# EUROPÄISCHE PATENTSCHRIFT

(11) **EP 1 781 640 B1**
(45) Veröffentlichungstag und Bekanntmachung des Hinweises auf die Patenterteilung: **07.01.2015**
(21) Anmeldenummer: 05777896.1
(22) Anmeldetag: 18.08.2005
(51) Int. Cl.: C07D 403/12, C07D 403/14, C07D 405/12, C07D 239/48, C07D 413/12, A61K 31/505, A61K 31/506, A61K 31/5513, A61K 31/553, A61P 29/00, A61P 35/00, A61P 37/00, C07D 401/12

(54) **2,4-DI(AMINOPHENYL)PYRIMIDINE ALS PLK INHIBITOREN**
2,4-DI(AMINOPHENYL)PYRIMIDINE DERIVATIVES AS PLK INHIBITORS
DÉRIVÉS DE 2,4-DI(AMINOPHÉNYL)PYRIMIDINE COMME INHIBITEURS DE PLK

(30) Priorität: 20.08.2004 EP 04019775
(43) Veröffentlichungstag der Anmeldung: 09.05.2007
(73) Patentinhaber: Boehringer Ingelheim International GmbH, 55216 Ingelheim (DE)
(72) Erfinder: REISER, Ulrich, A-1130 Wien (AT); ZAHN, Stephan Karl, A-1130 Wien (AT); HERFURTH, Lars, 55437 Appenheim (DE); STADTMUELLER, Heinz, A-1120 Wien (AT); ENGELHARDT, Harald, A-2483 Ebreichsdorf (AT); STEEGMAIER, Martin, 72762 Reutlingen (DE); BAUM, Anke, A-1050 Wien (AT); GUERTLER, Ulrich, A-1120 Wien (AT); SCHOOP, Andreas, A-1150 Wien (AT); QUANT, Jens, Juergen, A-2380 Perchtoldsdorf (AT); SOLCA, Flavio, A-1230 Wien (AT); HAUPTMANN, Rudolf, A-2483 Ebreichsdorf (AT)
(74) Vertreter: Simon, Elke Anna Maria
(86) Internationale Anmeldenummer: PCT/EP2005/054089
(87) Internationale Veröffentlichungsnummer: WO 2006/021544

(56) Entgegenhaltungen:
- WO-A1-00/12485
- WO-A1-00/39101
- WO-A1-02/04429
- WO-A1-2004/080980
- WO-A2-2004/074244

## Beschreibung

Die vorliegende Erfindung betrifft neue Pyrimidine der allgemeinen Formel (1), 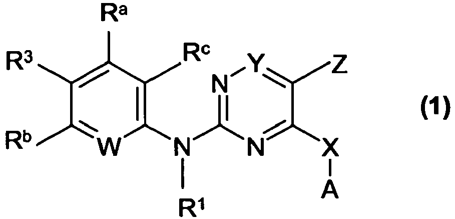 wobei die Reste A, W, X, Y, Z, R^{a}, R^{b}, R^{c}, R¹ und R³ die in den Ansprüchen und der Beschreibung genannten Bedeutungen haben, deren Isomere, Verfahren zur Herstellung dieser Pyrimidine sowie deren Verwendung als Arzneimittel.

### Hintergrund der Erfindung

Tumorzellen entziehen sich teilweise oder völlig der Regulation und Kontrolle durch den Organismus und zeichnen sich durch ein unkontrolliertes Wachstum aus. Dies beruht einerseits auf dem Verlust von Kontroll-Proteinen, wie z.B. Rb, p16, p21 und p53 als auch auf der Aktivierung von so genannten Beschleunigern des Zellzykluses, den cyclin-abhängigen Kinasen (CDK's).

Studien in Modellorganismen wie *Schizosaccharomyces pombe, Drosophila melanogaster* oder *Xenopus laevis* sowie Untersuchungen in menschlichen Zellen haben gezeigt, dass der Übergang von der G2 Phase zur Mitose durch die CDK1/Cyclin B Kinase reguliert wird (Nurse 1990, Nature 344: 503-508). Diese Kinase, die auch als "mitosis promoting factor" (MPF) bezeichnet wird, phosphoryliert und reguliert dadurch eine Vielzahl von Proteinen, wie z.B. nukleäre Lamine, Kinesin-ähnliche Motorproteine, Kondensine und Golgi Matrix Proteine, die eine wichtige Rolle beim Abbau der Kernhülle, bei der Zentrosomen Separation, dem Aufbau des mitotischen Spindelapparates, der Chromosomen Kondensation und Abbau des Golgi Apparates spielen (Nigg. E. 2001, Nat Rev Mol Cell Biol. 2(1):21-32). Eine murine Zell-Linie mit einer temperatursensitiven CDK-1 Kinasemutante zeigt nach Temperaturerhöhung einen raschen Abbau der CDK-1 Kinase und einen darauf folgenden Arrest in der G2/M Phase (Th'ng et al. 1990, Cell. 63(2):313-24). Die Behandlung von humanen Tumorzellen mit Inhibitoren gegen CDK1/Cyclin B, wie z.B. Butyrolacton, führt zu einem Arrest in der G2/M Phase und anschließender Apoptose (Nishio, et al. 1996, Anticancer Res. 16(6B):3387-95).

Darüber hinaus wird auch für die Proteinkinase Aurora B eine essentielle Funktion beim Eintritt in die Mitose beschrieben. Aurora B phosphoryliert Histon H3 an Ser10 und leitet damit die Chromosomenkondensation ein (Hsu et al. 2000, Cell 102:279-91). Ein spezifischer Zellzyklusarrest in der G2/M Phase kann aber auch z.B. durch Inhibition von spezifischen Phosphatasen wie z.B. Cdc25C (Russell and Nurse 1986, Cell 45:145-53) ausgelöst werden. Hefen mit defektem Cdc25 Gen arretieren in der G2 Phase, während eine Überexpression von Cdc25 zu einem verfrühten Eintritt in die Mitosephase führt (Russell und Nurse 1987, Cell 49:559-67). Desweiteren kann ein Arrest in der G2/M Phase auch durch Inhibition von bestimmten Motorproteinen, den so genannten Kinesinen wie z.B. Eg5 (Mayer et al. 1999, Science 286:971-4), oder durch Mikrotubuli stabilisierende oder destabilisierende Agentien (z.B. Colchicin, Taxol, Etoposid, Vinblastin, Vincristin) ausgelöst werden (Schiff und Horwitz 1980, Proc Natl Acad Sci U S A 77:1561-5).

Neben den Cyclin-abhängigen und den Aurora Kinasen spielen des weiteren die so genannten Polo-like Kinasen, eine kleine Familie von Serin/Threonin-Kinasen, eine wichtige Rolle bei der Regulation des eukaryontischen Zellzykluses. Bisher wurden die Polo-like Kinasen PLK-1, PLK-2, PLK-3 und PLK-4 in der Literatur beschrieben. Besonders für PLK-1 wurde eine zentrale Rolle in der Regulation der Mitosephase gezeigt. PLK-1 ist für die Reifung der Zentrosomen, für die Aktivierung der Phosphatase Cdc25C, sowie für die Aktivierung des Anaphase Promoting Complex verantwortlich (Glover et al. 1998, Genes Dev. 12:3777-87; Qian et al. 2001, Mol Biol Cell. 12:1791-9). Die Injektion von PLK-1 Antikörpern führt zu einem G2 Arrest in nicht transformierten Zellen, während Tumorzellen in der Mitosephase arretieren (Lane und Nigg 1996, J Cell Biol. 135:1701-13). Überexpression von PLK-1 konnte für verschiedene Tumorarten, wie nicht kleinzelliges Lungenkarzinom, Plattenepithelkarzinom, Brust- und kolorektales Karzinom (Wolf et al. 1997, Oncogene 14:543-549; Knecht etal. 1999, Cancer Res. 59:2794-2797; Wolf et al. 2000, Pathol. Res. Pract. 196:753-759; Takahashi et al. 2003, Cancer Sci. 94:148-52) gezeigt werden. Daher stellt diese Klasse von Proteinen ebenfalls einen interessanten Angriffspunkt zur therapeutischen Intervention proliferativer Krankheiten dar (Liu and Erikson 2003, Proc Natl Acad Sci USA 100:5789-5794).

Pyrimidine sind allgemein als Inhibitoren von Kinasen bekannt So werden z.B. Pyrimidine als aktive Komponente mit Antikrebswirkung in der Internationalen Patentanmeldung WO 00/53595, in der die Verwendung von 2,4,5-substituierten Pyrimidinen mit einem heterozyklischen Rest in 4-Position und einem Anilinorest in 2-Position, der seinerseits eine Seitenkette mit der Länge mindestens eines n-Propylrests aufweist, beschrieben.

Weiterhin wird in der Internationalen Patentanmeldung WO 00/39101 die Verwendung von 2, 4, 5- substituierten Pyrimidinen als Verbindungen mit Antikrebs Wirkung vorgeschlagen, welche in der 2- und 4-Position mit einem aromatischen oder heteroaromatischen Ring verknüpft sind, von denen mindestens einer eine Seitenkette mit der Länge mindestens eines n-Propylrests aufweist.

Die Internationale Patentanmeldung WO 97/19065 schlägt weiterhin die Verwendung von 2,4,5-substituierten Pyrimidinen mit einem 3,4-Dialkoxyanilinorest in Position 2 als Kinaseinhibitoren vor.

Die Internationale Patentanmeldung WO 02/04429 beschreibt 2,4,5-substituierte Pyrimidine mit einer Cyanogruppe in Position 5 und deren Zellzyklus inhibierenden Effekt.

In der Internationalen Patentanmeldung WO 03/063794 wird die Verwendung von 2,4-Pyrimidindiaminen als Inhibitoren der IgE und/oder IgG Rezeptor Signalkaskade beschrieben.

Antivirale 2,4,5-substituierte Pyrimidine, in denen die Reste R^{c} und R^{d} an dem Stickstoff der 4-Position einen heteroaromatischen Fünfring bilden, sind aus der Internationalen Patentanmeldung WO 99/41253 bekannt.

Für 2,4,5-substituierte Pyrimidine, die in Position 2 und 4 (Hetero-)Aryle tragen (WO00/27825), sowie für 2,4,5-substituierten Pyrimidinen, die in Position 2 oder 4 einen mit einer Nitrilgruppe funktionalisierten (Hetero-)Arylrest tragen (EP 0 945 443 A1), wird eine antivirale Wirkung beschrieben.

Die Resistenz vieler Tumorarten erfordert die Entwicklung neuer Arzneimittel zur Tumorbekämpfung. Der vorliegenden Erfindung liegt daher die Aufgabe zugrunde, neue Wirkstoffe aufzuzeigen, welche zur Vorbeugung und/oder Behandlung von Krankheiten, die durch exzessive oder anomale Zellproliferation charakterisiert sind, eingesetzt werden können.

### Detaillierte Beschreibung der Erfindung

Es wurde nun überraschenderweise gefunden, dass Verbindungen der allgemeinen Formel **(1)**, worin die Reste **A, W, X, Y, R^{a}, R^{b}, R^{c}, R¹, R²** und **R³** die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft Verbindungen der allgemeinen Formel **(1)** 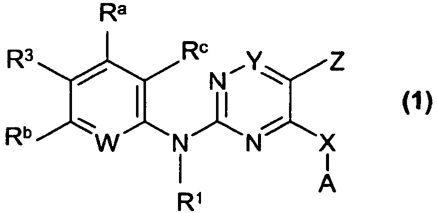 worin
- **W**: N oder C-R²,
- **X**: -NR^{1a}, O oder S,
- **Y**: CH oder N,
- **Z**: Halogen-C₁₋₃Alkyl-, -COH, -C(=O)-C₁₋₃Alkyl, -C(=O)-C₂₋₃Alkenyl, -C(=O)-C₂₋₃Alkinyl, -C(=O)C₁₋₃Alkyl-Halogen oder Pseudohalogen;
- **A**: ausgewählt ist aus den Formeln **(i), (ii)** oder **(iii)** 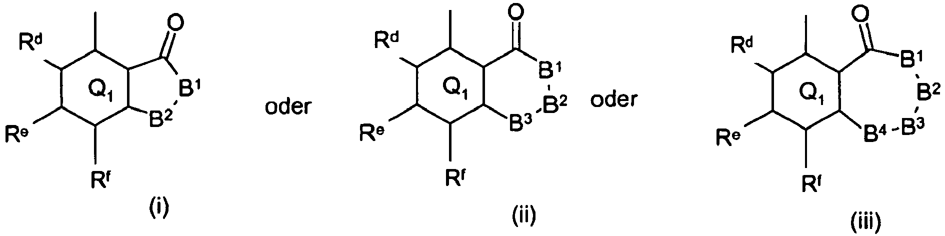
- **Q₁**: mono- oder bizyklische Arylverbindungen;
- **B¹, B², B³ und B⁴**: jeweils unabhängig voneinander C-R^{g}R^{h}, N-Rⁱ, O oder S, wobei benachbarte **B¹** - **B⁴** nicht jeweils -O- bedeuten;
- **R¹** und **R^{1a}**: jeweils unabhängig voneinander Wasserstoff oder Methyl,
- **R²**: ein Rest ausgewählt aus der Gruppe bestehend aus Wasserstoff, Halogen, -OR⁴, -C(=O)R⁴, -C(=O)NR⁴R⁵, -NR⁴R⁵, -NR⁴C(=O)R⁵, -NR⁴SO₂R⁵, -N=CR⁴R⁵, -C=NRⁱ, -SR⁴, -SOR⁴, -SO₂R⁴, -SO₂NR⁴R⁵ und Pseudohalogen, oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen;
- **R^{a}, R^{b}, R^{c}, R^{d}**, **R^{e}, R^{f}, R^{g}** und **R^{h}**: jeweils unabhängig voneinander ein Rest ausgewählt aus der Gruppe bestehend aus Wasserstoff, Halogen, =O, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -C=NRⁱ, -SR⁴, -SOR⁴, -SO₂R⁴, -SO₂NR⁴R⁵, -NR⁴SO₂NR⁵R⁶, -OSO₂NR⁵R⁶ und Pseudohalogen;
oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen; und gegebenenfalls können die am gleichen oder an benachbarten C-Atomen befindlichen **R^{g}** und **R^{h}** in jeder Kombination mit einer gemeinsamen gesättigten oder teilweise ungesättigten 3-5-gliedrigen Alkylbrücke, die gegebenenfalls ein bis zwei Heteroatome enthalten kann, verbunden sein;
- **R¹**: ein Rest ausgewählt aus der Gruppe bestehend aus Wasserstoff, =O, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen; oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, **-OSO₂NR⁵R⁶** und Pseudohalogen; und gegebenenfalls können die an benachbarten N-Atomen befindlichen **Rⁱ** miteinander oder **Rⁱ mit** an benachbarten C-Atomen befindlichen **R^{g}** oder **R^{h}** in jeder Kombination mit einer gemeinsamen gesättigten oder teilweise ungesättigten 3-5-gliedrigen Alkylbrücke, die gegebenenfalls ein bis zwei Heteroatome enthalten kann, verbunden sein;
- **R³**: ausgewählt aus den Formeln **(iv) - (x),**

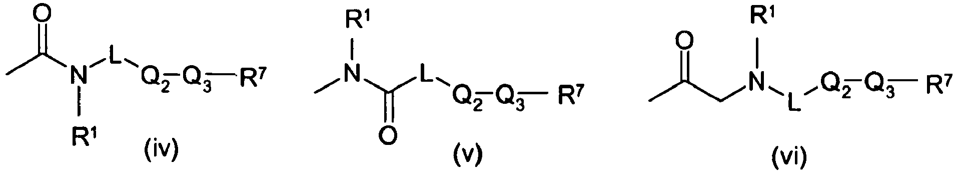

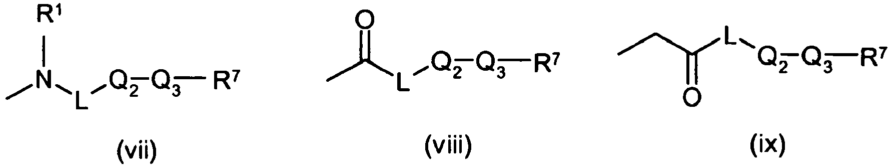

-L-Q₂-Q₃-R⁷ (x)
- **R⁴ R⁵** und **R⁶**: jeweils unabhängig voneinander Wasserstoff oder ein Rest ausgewählt aus der Gruppe bestehend aus gegebenenfalls einfach oder mehrfach substituiertem C₁₋₅Alkyl, C₂₋₅Alkenyl, C₂₋₅Alkinyl, C₃₋₁₀Cycloalkyl, Aryl, Heterocyclyl und Heteroaryl, wobei der/die Substituenten gleich oder verschieden sein können und ausgewählt werden aus der Gruppe bestehend aus C₃₋₁₀Cycloalkyl, Aryl, Heterocyclyl, Heteroaryl, Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
- **L**: eine Bindung oder ein Rest ausgewählt aus der Gruppe bestehend aus gegebenenfalls einfach oder mehrfach substituiertem C₁₋₁₆Alkyl, C₂₋₁₆Alkenyl, C₂₋₁₆Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
- **Q₂** und **Q₃**: jeweils unabhängig voneinander eine Bindung oder ein Rest ausgewählt aus der Gruppe bestehend aus gegebenenfalls einfach oder mehrfach substitutiertem C₁₋₁₆Alkyl, C₂₋₁₆Alkenyl, C₂₋₁₆Alkinyl, C₃₋₁₀Cycloalkyl, Aryl, Heterocyclyl und Heteroaryl wobei der/die Substituent(en) gleich oder verschieden sein können und ausgewählt werden aus der Gruppe bestehend aus Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹ -NR⁸SO₂NR⁹R¹⁰, -OSO₂NR⁸R⁹ und Pseudohalogen;
- **R⁷**: Wasserstoff oder ein Rest ausgewählt aus der Gruppe bestehend aus gegebenenfalls einfach oder mehrfach substituiertem C₁₋₁₆Alkyl, C₂₋₁₆Alkenyl, C₂₋₁₆Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen-, NO₂, -OR⁸, -C(=O)R⁸, -C(=O)OR⁸, -C(=O)NR⁸R⁹, -NR⁸R⁹, -NR⁸COR⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
- **R⁸**, **R⁹** und **R¹⁰**: jeweils unabhängig voneinander Wasserstoff oder ein Rest ausgewählt aus der Gruppe bestehend aus gegebenenfalls substituiertem C₁₋₈Alkyl, C₂₋₈Alkenyl, C₂₋₈Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen, Methyl, Ethyl, Amino, Methylamino, Dimethylamino, -OH und Pseudohalogen;
bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Ein Aspekt der Erfindung sind Verbindungen der allgemeinen Formel (1) worin
- **W**: für C-R² steht und die übrigen Reste wie vorstehend erwähnt definiert sind.

Ein weiterer Aspekt der Erfindung sind Verbindungen der allgemeinen Formel (1), worin
- **X**: -NR^{1a} oder Sauerstoff,
- **R¹** und **R^{1a}**: Wasserstoff;
- **R³**: Formel (iv) oder (x), 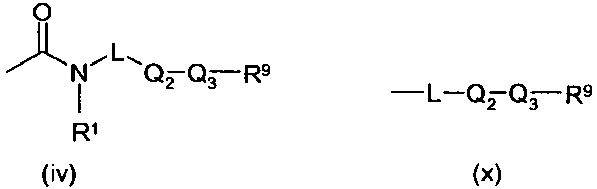
bedeuten und die übrigen Reste wie vorstehend erwähnt definiert sind.

Ein anderer Aspekt der Erfindung sind Verbindungen der allgemeinen Formel **(1)**, worin
- **Y**: CH und
- **Q₁**: monozyklische Arylverbindungen
bedeuten und die übrigen Reste wie vorstehend erwähnt definiert sind.

Ein Aspekt der Erfindung sind Verbindungen der allgemeinen Formel **(1),** worin
- **R^{c}**: ein Rest ausgewählt aus der Gruppe bestehend aus Wasserstoff, -F, -Cl, Methyl und Ethyl
bedeutet und die übrigen Reste wie vorstehend erwähnt definiert sind.

Ein zusätzlicher Aspekt dieser Erfindung sind Verbindungen der allgemeinen Formel **(1)**, worin
- **R^{a}** und **R^{b}**: jeweils unabhängig voneinander Wasserstoff oder Fluor; oder ein gegebenenfalls einfach oder mehrfach substituierter Rest ausgewählt aus der Gruppe bestehend aus C₁₋₂Alkyl, C₂Alkenyl, C₂Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁵, -SO₂R⁴, -SO₂NR⁴R⁵, -NR⁴, -SO₂NR⁴R⁵, -OSO₂NR⁴R⁵ und Pseudohalogen
bedeuten und die übrigen Reste wie vorstehend erwähnt definiert sind.

Ein weiterer Aspekt der Erfindung sind auch Verbindungen der allgemeinen Formel **(1)**, worin
**R^{a}** und **R^{b}** jeweils unabhängig voneinander Wasserstoff oder Fluor bedeuten und die übrigen Reste wie vorstehend erwähnt definiert sind.

Ein Aspekt der Erfindung sind Verbindungen der allgemeinen Formel **(1)**, oder deren pharmazeutisch wirksame Salze, als Arzneimittel.

Ein wesentlicher Aspekt der Erfindung sind Verbindungen der allgemeinen Formel **(1)**, oder deren pharmazeutisch wirksame Salze, zur Verwendung als Arzneimittel mit antiproliferativer Wirkung.

Ferner sind erfindungsgemäße Verbindungen der allgemeinen Formel **(1)**, oder deren pharmazeutisch wirksame Salze, zur Verwendung als Arzneimittel mit antiproliferativer Wirkung mit einem selektiven kinaseinhibierenden Wirkmechanismus umfasst.

Ein Aspekt der Erfindung ist die Verwendung von Verbindungen der allgemeinen Formel (1), oder deren pharmazeutisch wirksame Salze, zur Herstellung eines Arzneimittels mit antiproliferativer Wirkung mit einem PLK inhibierenden Wirkmechanismus.

Ein weiterer Aspekt der Erfindung sind pharmazeutische Zubereitungen, enthaltend als Wirkstoff eine oder mehrere Verbindungen der allgemeinen Formel **(1)**, oder deren physiologisch verträgliche Salze gegebenenfalls in Kombination mit üblichen Hilfs- und/oder Trägerstoffen.

Ein zusätzlicher Aspekt der Erfindung ist die Verwendung einer oder mehrerer Verbindungen der allgemeinen Formel **(1)** zur Herstellung eines Arzneimittels zur Behandlung und/oder Prävention von Krebs, Infektionen, Entzündungs- und Autoimmunerkrankungen.

Ein anderer Aspekt der Erfindung ist eine pharmazeutische Präperation enthaltend zumindest eine Verbindung der allgemeinen Formel **(1)**, 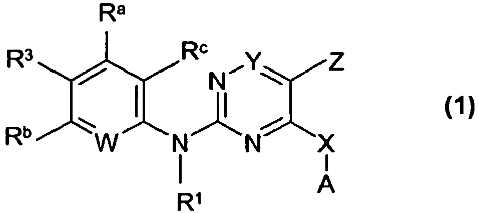 worin
- **W**: N oder C-R²,
- **X**: -NR^{1a}, O oder S,
- **Y**: CH oder N,
- **Z**: Halogen-C₁₋₃Alkyl-, -COH, -C(=O)-C₁₋₃Alkyl, -C(=O)-C₂₋₃Alkenyl, -C(=O)-C₂₋₃Alkinyl, -C(=O)C₁₋₃Alkyl-Halogen und Pseudohalogen;
- **A**: ausgewählt ist aus den Formeln (**i**), (**ii**) oder (**iii**) 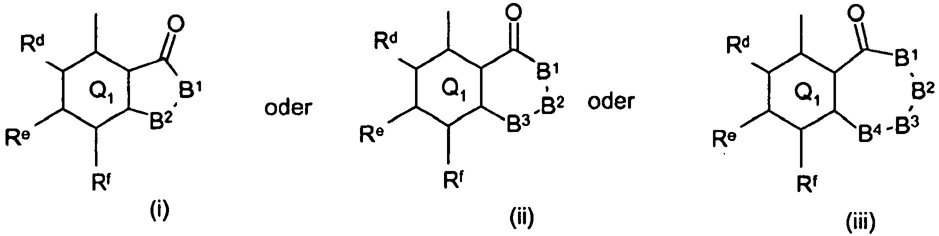
- **Q₁**: mono- oder bizyklische Arylverbindungen;
- **B¹, B², B³ und B⁴**: jeweils unabhängig voneinander C-R^{g}R^{h}, N-Rⁱ, O oder S, wobei benachbarte **B¹ - B⁴** nicht jeweils -O- bedeuten;
- **R¹ und R^{1a}**: jeweils unabhängig voneinander Wasserstoff oder Methyl,
- **R²**: ein Rest ausgewählt aus der Gruppe bestehend aus Wasserstoff, Halogen, -OR⁴, -C(=O)R⁴, -C(=O)NR⁴R⁵, -NR⁴R⁵, -NR⁴C(=O)R⁵, -NR⁴SO₂R⁵, -N=CR⁴R⁵, -C=NRⁱ, -SR⁴, -SOR⁴, -SO₂R⁴, -SO₂NR⁴R⁵ und Pseudohalogen, oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen;
- **R^{a}, R^{b}**, **R^{c}, R^{d}, R^{e}, R^{f}, R^{g} und R^{h}**: jeweils unabhängig voneinander ein Rest ausgewählt aus der Gruppe bestehend aus Wasserstoff, Halogen, =O, -NO₂, -OR⁴, -C(=O)R⁴, -C=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵ -N=CR⁴R⁵, -C=NRⁱ, -SR⁴, -SOR⁴, -SO₂R⁴, -SO₂NR⁴R⁵, -NR⁴SO₂NR⁵R⁶, -OSO₂NR⁵R⁶ und Pseudohalogen; oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=C)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR₄SO₂NR⁵R⁶, -OSO₂NR⁵R⁶ und Pseudohalogen; und gegebenenfalls können die am gleichen oder an benachbarten C-Atomen befindliche **R^{g}** und **R^{h}** in jeder Kombination mit einer gemeinsamen gesättigten oder teilweise ungesättigten 3-5-gliedrigen Alkylbrücke, die ein bis zwei Heteroatome enthalten kann, verbunden sein;
- **R¹**: ein Rest ausgewählt aus der Gruppe bestehend aus Wasserstoff, =O, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen; und gegebenenfalls können die an benachbarten N-Atomen befindlichen **R¹** miteinander oder **R¹** mit an benachbarten C-Atomen befindlichen **R^{g}** oder **R^{h}** in jeder Kombination mit einer gemeinsamen gesättigten oder teilweise ungesättigten 3-5-gliedrigen Alkylbrücke, die ein bis zwei Heteroatome enthalten kann, verbunden sein;
- **R³**: ausgewählt aus den Formeln **(iv) - (x)**, 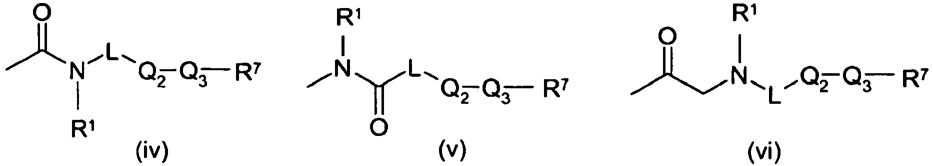 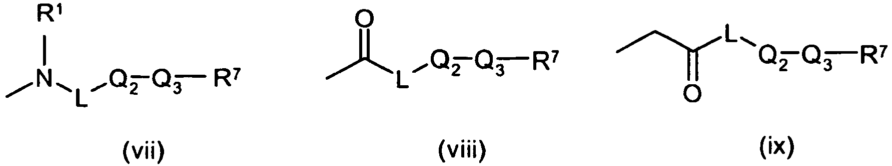 -L-Q₂-Q₃-R⁷ (x)
- **R⁴**, **R⁵ und R⁶**: jeweils unabhängig voneinander Wasserstoff oder ein Rest ausgewählt aus der Gruppe bestehend aus gegebenenfalls einfach oder mehrfach substituiertem C₁₋₅Alkyl, C₂₋₅-Alkenyl, C₂₋₅Alkinyl, C₃₋₁₀Cycloalkyl, Aryl, Heterocyclyl und Heteroaryl, wobei der/die Substituenten gleich oder verschieden sein können und ausgewählt werden aus der Gruppe bestehend aus C₃₋₁₀Cycloalkyl, Aryl, Heterocyclyl, Heteroaryl, Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
- **L**: eine Bindung oder ein Rest ausgewählt aus der Gruppe bestehend aus gegebenenfalls einfach oder mehrfach substituiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
- **Q₂ und Q₃**: jeweils unabhängig voneinander eine Bindung oder ein Rest ausgewählt aus der Gruppe bestehend aus gegebenenfalls einfach oder mehrfach substitutiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
- **R⁷**: Wasserstoff oder ein Rest ausgewählt aus der Gruppe bestehend aus gegebenenfalls einfach oder mehrfach substituiertem C₁₋₁₆Alkyl, C₂₋₁₆Alkenyl, C₂₋₁₆Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen-, NO₂, -OR⁸, -C(=O)R⁸, -C(=O)OR⁸, -C(=O)NR⁸R⁹, -NR⁸R⁹, -NR⁸COR⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
- **R⁸, R⁹ und R¹⁰**: jeweils unabhängig voneinander Wasserstoff oder ein Rest ausgewählt aus der Gruppe bestehend aus gegebenenfalls substituiertem C₁₋₈Alkyl, C₂₋₈Alkenyl, C₂₋₈Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen, -NH₂, -OH und Pseudohalogen;
bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, und
mindestens eine weitere zytostatische oder zytotoxische Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

### DEFINITIONEN

Wie hierin verwendet treffen folgenden Definitionen zu, falls nicht anders beschrieben.

Unter Alkyl-Substitutenten sind jeweils gesättigte, geradkettige oder verzweigte aliphatische Kohlenwasserstoffreste (Alkylrest) zu verstehen.

Alkenyl-Substituenten sind jeweils geradkettige oder verzweigte, ungesättigte Alkylreste, welche mindestens eine Doppelbindung aufweisen.

Unter Alkinyl-Substituenten sind jeweils geradkettige oder verzweigte, ungesättigte Alkylreste, die mindestens eine Dreifachbindung aufweisen, zu verstehen.

Halogenalkyl bezieht sich auf Alkylreste, in denen ein oder mehrere Wasserstoffatome durch Halogenatome ersetzt sind. Halogenalkyl umfasst sowohl gesättigte Alkylreste als auch ungesättigte Alkenyl- und Alkinylreste, wie beispielsweise -CF₃, -CH₂, -CH₂F, -CF₂CF₃,-CHFCF₃, -CH₂CF₃, -CF₂CH₃, -CHFCH₃, -CF₂CF₂CF₃, -CF₂CH₂CH₃, -CHFCH₂CH₃ und -CHFCH₂CF₃.

Halogen bezieht sich auf Fluor-, Chlor-, Brom- und/oder Jodatome.

Unter Pseudohalogen sind folgende Reste zu verstehen: -OCN, -SCN, -CF₃ und -CN. Cycloalkyl bedeutet ein mono- oder bizyklischer Ring, wobei das Ringsystem ein gesättigtes aber auch ein ungesättigtes, nichtaromatisches Ringsystem sein kann, welches gegebenenfalls auch Doppelbindungen enthalten kann. Beispielsweise genannt seien Cyclopropyl, Cyclopropenyl, Cyclobutyl, Cyclobutenyl, Cyclopentyl, Cyclopentenyl, Cyclohexyl, Cyclohexenyl, Norbornyl, Norbornenyl, Spiro[5.5]undecan, Spiro[5.4]decan und Spiro[4.4]nonan.

Aryl bezieht sich auf monozyklische oder bizyklische Ringe mit 6-12 Kohlenstoffatomen wie beispielsweise Phenyl und Naphthyl.
Unter Heteroaryl sind mono- oder bizyklische Ringe zu verstehen, welche anstelle eines oder mehrere Kohlenstoffatome ein oder mehrere, gleich oder verschiedene Heteroatome enthalten, wie z.B. Stickstoff-, Schwefel- oder Sauerstoffatome. Beispielsweise genannt seien Furyl, Thienyl, Pyrrolyl, Oxazolyl, Thiazolyl, Isoxazolyl, Isothiazolyl, Pyrazolyl, Imidazolyl, Triazolyl, Tetrazolyl, Oxadiazolyl, Thiadiazolyl, Pyridyl, Pyrimidyl, Pyridazinyl, Pyrazinyl und Triazinyl. Beispiele für bizyklische Heteroarylreste sind Indolyl, Isoindolyl, Benzofuranyl, Benzothienyl, Benzoxazolyl, Benzothiazolyl, Benzisoxazolyl, Benzisothiazolyl, Benzimidazolyl, Indazolyl, Isoquinolinyl, Quinolinyl, Quinoxalinyl, Cinnolinyl, Phthalazinyl, Quinazolinyl und Benzotriazinyl, Indolizinyl, Oxazolopyridinyl, Imidazopyridinyl, Naphthyridinyl, Indolinyl, Isochromanyl, Chromanyl, Tetrahydroisochinolinyl, Isoindolinyl, Isobenzotetrahydrofuranyl, Isobenzotetrahydrothienyl, Isobenzothienyl, Benzoxazolyl, Pyridopyridinyl, Benzotetrahydrofuranyl, Benzotetrahydrothienyl, Purinyl, Benzodioxolyl, Triazinyl, Phenoxazinyl, Phenothiazinyl, Pteridinyl, Benzothiazolyl, Imidazopyridinyl, Imidazothiazolyl, Dihydrobenzisoxazinyl, Benzisoxazinyl, Benzoxazinyl, Dihydrobenzisothiazinyl, Benzopyranyl, Benzothiopyranyl, Cumarinyl, Isocumarinyl, Chromonyl, Chromanonyl, Pyridinyl-*N*-oxid, Tetrahydroquinolinyl, Dihydroquinolinyl, Dihydroquinolinonyl, Dihydroisoquinolinonyl, Dihydrocumarinyl, Dihydroisocumarinyl, Isoindolinonyl, Benzodioxanyl, Benzoxazolinonyl, Pyrrolyl-*N*-oxid, Pyrimidinyl-*N*-oxid, Pyridazinyl-*N*-oxid, Pyrazinyl-*N*-oxid, Quinolinyl-*N*-oxid, Indolyl-*N*-oxid, Indolinyl-*N-*oxid, Isoquinolyl-*N*-oxid, Quinazolinyl-*N*-oxid, Quinoxalinyl-*N*-oxid, Phthalazinyl-*N*-oxid, Imidazolyl-*N*-oxid, Isoxazolyl-*N*-oxid, Oxazolyl-*N*-oxid, Thiazolyl-*N*-oxid, Indolizimyl-*N-*oxid, Indazolyl-*N*-oxid, Benzothiazolyl-*N*-oxid, Benzimidazolyl-*N*-oxid, Pyrrolyl-*N*-oxid, Oxadiazolyl-*N*-oxid, Thiadiazolyl-*N*-oxid, Triazolyl-*N*-oxid, Tetrazolyl-*N*-oxid, Benzothiopyranyl-*S*-oxid und Benzothiopyranyl-*S,S*-dioxid.

Heterocyclyl bezieht sich auf 5-12 Kohlenstoffatome umfassende, gesättigte oder ungesättigte, nicht aromatische mono-, bizyklische, überbrückte oder spirozyklische bizyklische Ringe, welche anstelle eines oder mehrere Kohlenstoffatome Heteroatome, wie Stickstoff, Sauerstoff oder Schwefel, tragen. Beispiele für solche Heterocyclylreste sind Tetrahydrofuranyl, Pyrrolidinyl, Pyrrolinyl, Imidazolidinyl, Imidazolinyl, Pyrazolidinyl, Pyrazolinyl, Piperidyl, Piperazinyl, Indolinyl, Isoindoliny, Morpholinyl, Thiomorpholinyl, Homomorpholinyl, Homopiperidyl, Homopiperazinyl, Thiomorpholinyl-*S*-oxide, Thiomorpholinyl-*S,S-*dioxide, Tetrahydropyranyl, Piperidinyl, Tetrahydrothienyl, Homopiperidinyl, Homothiomoipholinyl-*S,S*-dioxid, Oxazolidinonyl, Dihydropyrazolyl, Dihydropyrrolyl, Dihydropyrazinyl, Dihydropyridinyl, Dihydropyrimidinyl, Dihydrofuryl, Dihydropyranyl, Tetrahydrothienyl-*S*-oxid, Tetrahydrothienyl-*S,S*-dioxid, Homothiomorpholinyl-*S*-oxid, 2-Oxa-5-azabicyclo[2.2.1]heptan, 8-Oxa-3-azabicyclo[3.2.1]octan, 3,8-Diaza-bicyclo[3.2.1]octan, 2,5-Diaza-bicyclo[2.2.1]heptan, 3,8-Diaza-bicyclo[3.2.1]octan, 3,9-Diaza-bicyclo[4.2.1]nonan, 2,6-Diazabicyclo[3.2.2]nonan, 2,7-Diaza-spiro[3.5]nonan, 2,7-Diaza-spiro[4.4]nonan, 2,8-Diazaspiro[4.5]decan, 3,9-Diaza-spiro[5.5]undecan.

Die nachfolgenden Beispiele illustrieren die vorliegende Erfindung ohne sie jedoch in ihrem Umfang einzuschränken:

### Herstellung der erfindungsgemäßen Verbindungen:

Die Herstellung der erfindungsgemäßen Verbindungen kann nach den, im Folgenden beschriebenen Syntheseverfahren A bis C erfolgen, wobei die Substituenten der allgemeinen Formeln **(I** bis **XVI)** die zuvor genannten Bedeutungen haben.

### Verfahren A

### Stufe 1A

Die Herstellung der Zwischenverbindung **III** erfolgt durch Substitution einer Abgangsgruppe LG, beispielsweise Halogen, SCN oder Methoxy, vorzugsweise Chlor, an einem heteroaromatischem System **I** durch ein Nukleophil **II**.

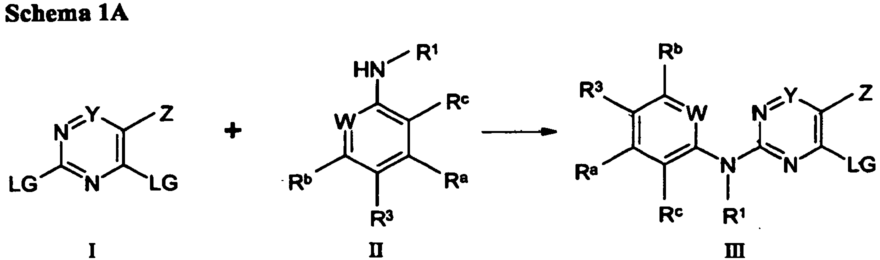

Es werden 1 Äquivalent der Verbindung **I** und 1 bis 1,5 Äquivalente der Verbindung **II** in einem Lösungsmittel, beispielsweise 1,4-Dioxan, Tetrahydrofuran, Ethanol, Isopropanal, *N,N-*Dimethylformamid oder *N,N-*Dimethylacetamid gerührt. Bei einer Temperatur von 15 bis 25°C werden 2 bis 2,5 Äquivalente einer Base, beispielsweise Kaliumcarbonat, Natriumcarbonat, Cäsiumcarbonat, *N*-Ethyl-*N,N-*diisopropylamin oder Triethylamin zugegeben. Das Reaktionsgemisch wird 6 bis 72 h bei einer Temperatur von 20 bis 100°C weitergerührt. Danach wird das Lösungsmittel abdestilliert und der Rückstand mit Wasser versetzt, welches mit einer Mineralsäure, beispielsweise Salzsäure oder Schwefelsäure auf einen pH-Wert zwischen 1-4 eingestellt wird. Dieses Gemisch wird 2 bis 3mal mit einem organischen Lösungsmittel, beispielsweise Diethylether, Ethylacetat oder Dichlormethan, extrahiert. Die vereinigten organischen Extrakte werden getrocknet und das Lösungsmittel abdestilliert. Der Rückstand wird chromatographisch gereinigt.

### Stufe 2A

Die Herstellung der Endverbindung **V** erfolgt durch Substitution einer Abgangsgruppe LG, beispielsweise Halogen, SCN oder Methoxy, vorzugsweise Chlor, an einem heteroaromatischem System **III** durch ein Nukleophil **IV**.

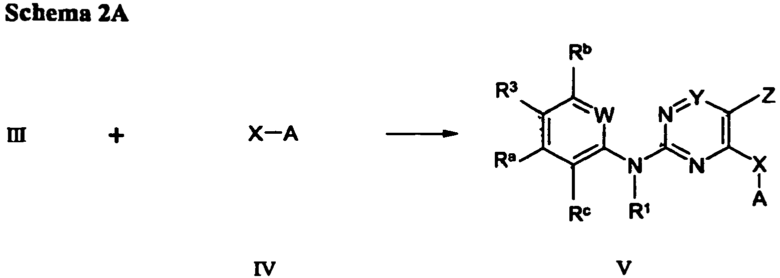

Es werden 1 Äquivalent der Verbindung **III** und 1 bis 3 Äquivalente der Verbindung **IV** in einem Lösungsmittel, beispielsweise 1,4-Dioxan, *N,N*-Dimethylfornamid, *N,N-*Dimethylacetamid oder *N*-Methyl-2-pyrrolidinon gerührt. Bei einer Temperatur von 15 bis 40 °C werden 1 bis 2 Äquivalente einer Mineralsäure, beispielsweise Schwefelsäure oder Salzsäure, zugegeben. Das Reaktionsgemisch wird 12 bis 72 h bei einer Temperatur von 20 bis 100 °C weitergerührt. Danach wird das Lösungsmittel abdestilliert und der Rückstand chromatographisch gereinigt.

### Verfahren B

### Stufe 1B

Die Herstellung der Zwischenverbindung **VII** erfolgt durch Substitution einer Abgangsgruppe LG, beispielsweise Halogen, SCN, Methoxy, vorzugsweise Chlor, an einem heteroaromatischem System **I** durch ein Nukleophil **VI**.

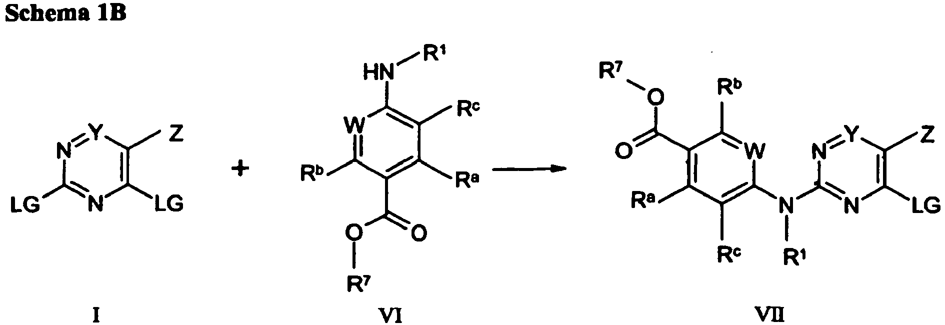

Es werden 1 Äquivalent der Verbindung **I** und 1 bis 1,5 Äquivalente der Verbindung **VI** in einem Lösungsmittel, beispielsweise 1,4-Dioxan, Tetrahydrofuran, Ethanol, Isopropanol, *N,N*-Dimethylfonnarnid oder *N,N*-Dimethylacetamid gerührt. Bei einer Temperatur von 15 bis 25°C werden 2 bis 2,5 Äquivalente einer Base, beispielsweise Kaliumcarbonat, Natriumcarbonat, Cäsiumcarbonat, Kaliumhydrogenphosphat, *N*-Ethyl-*N,N-*diisopropylamin oder Triethylamin zugegeben. Das Reaktionsgemisch wird 6 bis 72 h bei einer Temperatur von 20 bis 120°C weitergerührt. Das Reaktionsgemisch wird mit Wasser, welches mit einer anorganischen Base, beispielsweise Natriumhydrogencarbonat oder Kaliumcarbonat, auf einen pH-Wert von 8 bis 9 eingestellt wird, versetzt. Dieses Gemisch wird zwei bis dreimal mit einem organischen Lösungsmittel, beispielsweise Diethylether oder Ethylacetat extrahiert. Die vereinigten organischen Extrakte werden getrocknet und das Lösungsmittel abdestilliert. Der Rückstand wird durch Chromatographie oder mehrmalige Kristallisation gereinigt.

### Stufe 2B

Die Herstellung der Zwischenverbindung **VIII** erfolgt durch Substitution einer Abgangsgruppe LG, beispielsweise Halogen, SCN, Methoxy, vorzugsweise Chlor, an einem heteroaromatischem System **VII** durch ein Nukleophil **IV**.

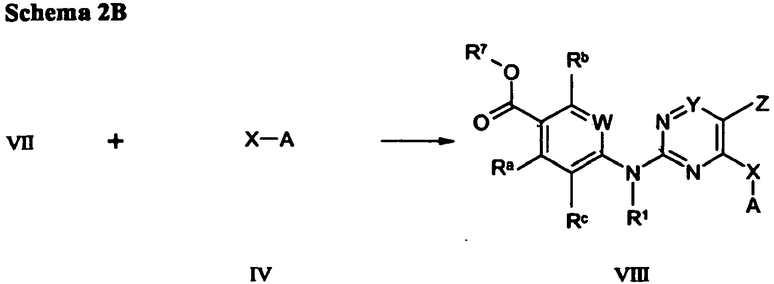

Es werden 1 Äquivalent der Verbindung **VII** und 1 bis 1,5 Äquivalente der Verbindung **IV** in einem Lösungsmittel, beispielsweise 1,4-Dioxan, *N,N*-Dimethylformamid, *N,N-*Dimethylacetamid oder *N*-Methyl-2-pyrrololidinon gerührt. Bei einer Temperatur von 15 bis 40°C werden 0,2 bis 1 Äquivalent einer Säure, beispielsweise Schwefelsäure oder Salzsäure, zugegeben. Das Reaktionsgemisch wird 12 bis 72 h bei einer Temperatur von 20 bis 100°C weitergerührt. Das Reaktionsgemisch wird in Wasser eingerührt und der entstehende Niederschlag abfiltriert und getrocknet. Der Niederschlag kann durch Chromatographie oder Kristallisation gereinigt oder als Rohprodukt in der nächsten Stufe eingesetzt werden.

### Stufe 3B

Verbindungen **VIII** deren Rest R⁷ Wasserstoff darstellt können direkt zur Herstellung der Endverbindungen **X** eingesetzt werden, wobei eine Verbindung **VIII** mit einer Verbindung **IX** umgesetzt wird. Verbindungen **VIII** mit einem Rest R⁷ ungleich Wasserstoff werden zuvor durch Hydrolyse oder ähnliche, dem Fachmann bekannte Verfahren in die Verbindungen überführt, bei denen der Rest R⁷ Wasserstoff repräsentiert.

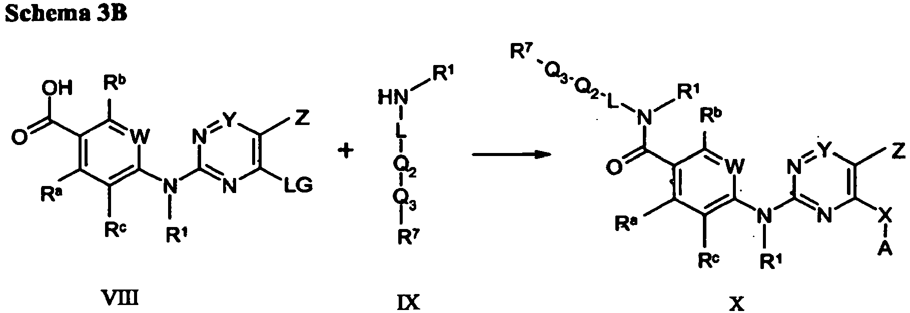

Es werden 1 Äquivalent der Verbindung **VIII,** 1 bis 1,5 Äquivalente der Verbindung **IX** und 1 bis 3 Äquivalente einer Base, beispielsweise Triethylamin oder Ethyldiisopropylamin in einem Lösungsmittel, beispielsweise 1,4-Dioxan, *N,N-*Dimethylformamid, *N,N-*Dimethylacetamid oder *N-*Methyl-2-pyrrolidinon gerührt. Bei einer Temperatur von 15 bis 25°C werden 1 bis 1,5 Äquivalente eines Kupplungsreagenzes, beispielsweise *N,N-*Dicyclohexylcarbodiimid, *N,N*-Diisopropylcarbodiimid, *O*-(Benzotriazol-1-yl)-*N,N,N',N'-*tetramethyluronium-tetrafluoroborat oder 1-(3-*N,N*-Dimethylaminopropyl)-3-ethylcarbodiimid zugegeben. Das Reaktionsgemisch wird 4 bis 24 h bei einer Temperatur von 15 bis 25°C weitergerührt. Danach wird das Lösungsmittel abdestilliert und der Rückstand chromatographisch gereinigt.

### Verfahren C

### Stufe 1C

Die Herstellung der Zwischenverbindung **XI** erfolgt durch Substitution einer Abgangsgruppe LG, beispielsweise Halogen, SCN, Methoxy, vorzugsweise Chlor, an einem heteroaromatischem System **I** durch ein Nukleophil **IV**.

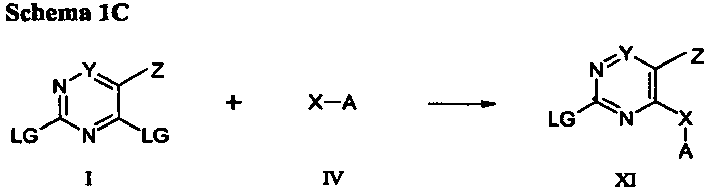

Es werden 1 Äquivalent der Verbindung **I** und 1 bis 3 Äquivalente einer Base, beispielsweise Triethylamin oder Ethyldiisopropylamin einem Lösungsmittel, beispielsweise 1,4-Dioxan, Tetrahydrofuran, *N,N-*Dimethylformamid oder *N,N-*Dimethylacetamid gerührt. Bei einer Temperatur von -60 bis 0°C werden 0,8 bis 1,5 Äquivalente einer Verbindung **IV** zugegeben. Das Reaktionsgemisch wird 6 bis 72 h bei einer Temperatur von 15 bis 75°C weitergerührt. Danach wird das Lösungsmittel abdestilliert und der Rückstand chromatographisch gereinigt.

### Stufe 2C

Die Herstellung der Endverbindung **V erfolgt** durch Substitution einer Abgangsgruppe LG, beispielsweise Halogen, SCN, Methoxy, vorzugsweise Chlor, an einem heteroaromatischem System **XI** durch ein Nukleophil **II.**

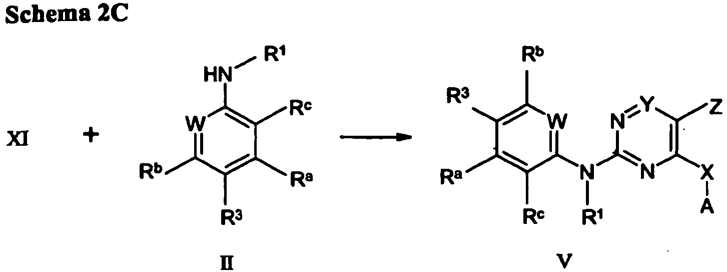

Es werden 1 Äquivalent der Verbindung **XI** und 1 bis 1,5 Äquivalente der Verbindung **II** in einem Lösungsmittel, beispielsweise 1,4-Dioxan, *N,N-*Dimethylformamid, *N,N-*Dimethylacetamid oder *N*-Methyl-2-pyrrolidinon gerührt. Bei einer Temperatur von 15 bis 40°C werden 1 bis 2 Äquivalente einer Säure, beispielsweise Schwefelsäure oder Salzsäure zugegeben. Das Reaktionsgemisch wird 6 bis 72 h bei einer Temperatur von 20 bis 100°C weitergerührt. Danach wird das Lösungsmittel abdestilliert und der Rückstand chromatographisch gereinigt.

### Chromatographie:

Für die Mitteldruck Chromatographie (MPLC) wird Kieselgel der Firma Millipore (Bezeichnung: Granula Silica Si-60A 35-70µm) oder C-18 RP-Kieselgel der Firma Macherey Nagel (Bezeichnung: Polygoprep 100-50 C18) eingesetzt.
Für die Hochdruck Chromatographie werden Säulen der Firma Waters (Bezeichnung: XTerra Prep. MS C18, 5 µM, 30*100 mm oder Symmetrie C18, 5 µm, 19*100) verwendet.

### Nuclear Magnet Resonanz (NMR) Spektroskopie:

Die Messung wird in deuteriertem Dimethylsulfoxid-d6 durchgeführt. Werden andere Lösungsmittel verwendet sind diese explizit in den Beispielen oder in den Methoden vermerkt. Die Messwerte werden auf einer Delta-Scala in der Einheit ppm angegeben. Als Standard wird Tetramethylsilan verwendet. Die Messung erfolgt auf einem Avance 400 (400MHz-NMR-Spektrometer) von der Firma Bruker Biospin GmbH.
Die NMR-Spektren sind rein beschreibend angegeben. Es werden grundsätzlich nur alle sichtbaren Molekül-Signale aufgeführt. Sind Molekül-Signale beispielsweise teilweise oder vollständig von Fremdsignalen wie beispielsweise Wasser-Signal, DMSO-Signal oder CDC13-Signal verdeckt werden solche nicht genannt.

### Massenspektroskopie / UV-Spektrometer:

Diese Daten werden mit Hilfe einer HPLC-MS Anlage (high performance liquid chromatography mit Massendetektor) der Firma Agilent erzeugt.
Die Anlage ist so aufgebaut, dass anschließend an die Chromatographie (Säule: Zorbax SB-C8, 3,5 µm, 2,1*50, Fa. Agilent) ein Diodenarry-Detektor (G1315B von Fa. Agilent) und ein Massendetektor (1100 LS-MSD SL; G1946D; Fa. Agilent) in Reihe geschalten sind. Die Anlage wird mit einem Fluß von 0,6 ml/min betrieben. Für einen Trennvorgang wird ein Gradient innerhalb von 3,5 min durchlaufen (Gradient Anfang: 95% Wasser und 5% Acetonitril; Gradient Ende: 5% Wasser und 95% Acetonitril; den beiden Lösungsmitteln wird jeweils 0,1%Ameisensäure beigemischt).

### Methode 1

### 2-(2-Methoxy-4-propylcarbamoyl-phenylamino)-4-chlor-5-trifluormethyl-pyrimidin

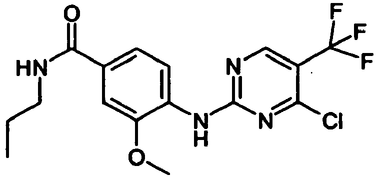

5 g (21,9 mmol) 2,4-Dichlor-5-trifluormethyl-pyrimidin werden in 50 ml 1,4-Dioxan gelöst und mit 5,5 g (21,9 mmol) 4-Amino-3-methoxybenzoesäure-propylamid Hydrochlorid (Zhuangyu Zhang, et al. 1989, J Pharml Sci. 78(10):829-32) versetzt. Diesem Reaktionsgemisch werden 7,5 ml (43,8 mmol) Ethyldiisopropylamin zugesetzt und 2 Tage bei Raumtemperatur gerührt. Anschließend wird das Reaktionsgemisch mit 250 ml Ethylacetat verdünnt und zunächst mit 300 ml wässriger 10%-iger KHSO₄-Lösung, dann mit 300 ml gesättigter, wässriger NaCl-Lösung gewaschen. Die organische Phase wird mit MgSO₄ getrocknet und das Lösungsmittel im Vakuum entfernt. Das Rohprodukt wird durch Säulenchromatographie gereinigt. Als Trägermaterial dient Kieselgel und als Eluent wird ein Gemisch bestehend aus Cyclohexan:Ethylacetat (75:25) verwendet.

| | |
|---|---|
| Ausbeute: | 2,30 g (5,9 mmol; 27 %) |
| ¹H-NMR: | 0.91 (t, 3H), 1.50 - 1.61 (m, 2H), 3.20 - 3.28 (m, 2H), 3.87 (s, 3H), 7.46-7.51 (m, 1H), 7.52 - 7.56 (m, 1H), 7.70 - 7.75 (m, 1H), 8.44 (t, 1H), 8.75 (s, 1H), 9.73 (s, 1H) |

### Methode 2

### 7-Amino-2,3-dihydro-isoindol-1-on

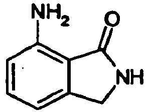

### a) 7-Nitro-2,3-dihydro-isoindol-1-on

1,5 g (5,473 mmol) 2-Brommethyl-6-nitro-benzosäuremethylester werden in 20 ml *N,N-*Dimethylformamid gelöst und mit 15 ml methanolischem Ammoniak (7 mmol/ml) versetzt. Nach 20 h bei 25°C wird mit 100 ml Essigester verdünnt und 3mal mit gesättigter Natriumhydrogencarbonat-Lösung extrahiert. Die organische Phase wird mit Magnesiumsulfat getrocknet und das Lösungsmittel im Vakuum entfernt.

| | |
|---|---|
| Ausbeute: | 960 mg (5,389 mmol, 99 %) |
| MS-ESI+: | m/z =179 [M+H]⁺ |

### b) 7-Amino-2,3-dihydro-isoindol-1-on

960 mg (5,389 mmol) 7-Nitro-2,3-dihydro-isoindol-1-on werden in 100 ml Tetrahydrofuran gelöst und mit 100 mg Palladium auf Kohle versetzt. Anschließend wird 20 h bei 25°C und 4 bar Wasserstoffdruck (H₂-Druck) gerührt. Der Katalysator wird abfiltriert und das Lösungsmittel im Vakuum entfernt.

| | |
|---|---|
| Ausbeute: | 734 mg (4,958 mmol, 92 %) |
| MS-ESI+: | m/z = 149 [M+H]⁺ |

Analog zu dieser Methode werden folgende 7-Amino-2,3-dihydro-isoindol-1-on Derivate hergestellt. Dabei wird anstelle von Ammoniak ein entsprechendes Amin eingesetzt:

| | **MS (ESI) (M+H)⁺** | | **MS (ESI) (M+H)⁺** |
|---|---|---|---|
| 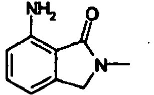 | 163 | 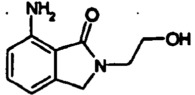 | 193 |
| 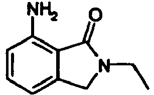 | 177 | 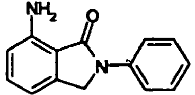 | 225 |
| 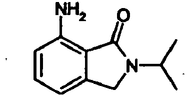 | 191 | 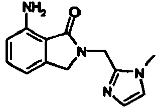 | 243 |
| 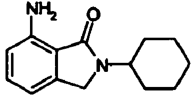 | 231 | 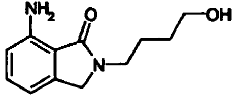 | 221 |
| 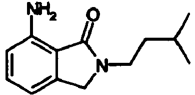 | 219 | 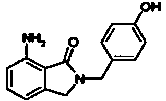 | 255 |
| 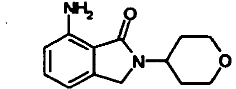 | 233 | 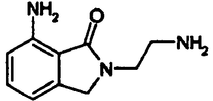 | 192 |
| 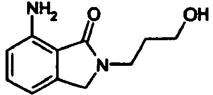 | 207 | 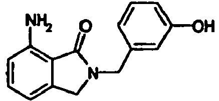 | 255 |
| 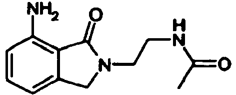 | 234 | 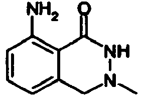 | 178 |
| 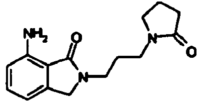 | 274 | 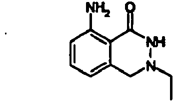 | 192 |
| 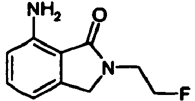 | 195 | 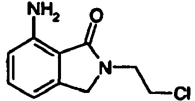 | 211/213 |
| 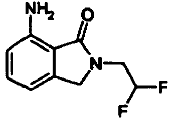 | 213 | 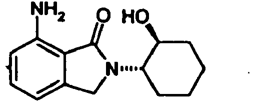 | 247 |
| 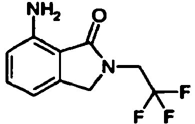 | 231 | 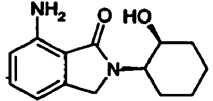 | 247 |
| 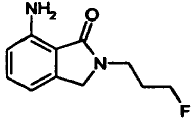 | 209 | 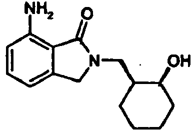 | 261 |
| 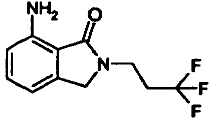 | 245 | 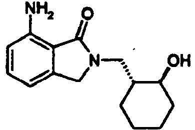 | 261 |
| 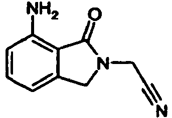 | 188 | 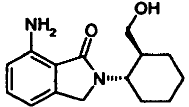 | 261 |
| 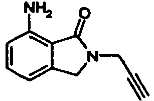 | 187 | 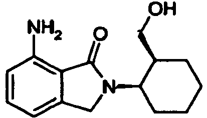 | 261 |
| 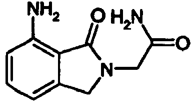 | 206 | 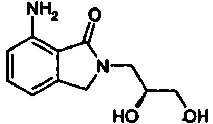 | 223 |
| 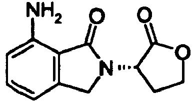 | 233 | 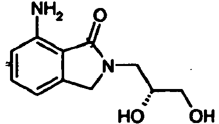 | 223 |
| 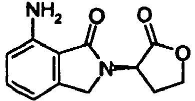 | 233 | 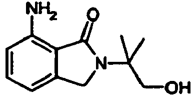 | 221 |
| 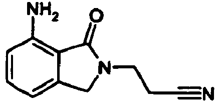 | 202 | 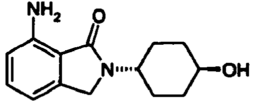 | 247 |
| 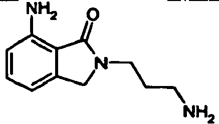 | 206 | 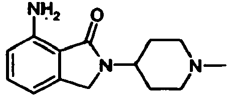 | 246 |
| 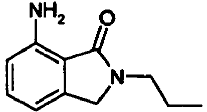 | 191 | 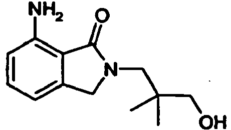 | 235 |
| 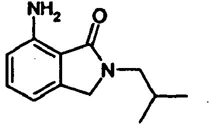 | 205 | 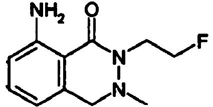 | 224 |
| 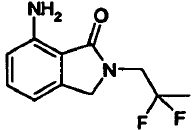 | 227 | 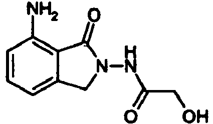 | 222 |
| 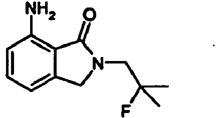 | 223 | | |

### Methode 3

### (4-Amino-3-oxo-1,3-dihydro-isobenzofuran-1-yl)-essigsäureethylester

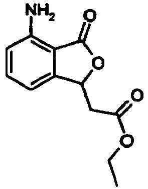

### a) (4-Amino-3-oxo-3H-isobenzofuran-1-yliden)-essigsäureethylester

500 mg (3,1 mmol) 4-Amino-isobenzofuran-1,3-dion und 1,13 g (3,1 mmol) (Ethoxy-carbonylmethylen)-triphenylphosphoran werden in 5 ml Tetrahydrofuran (THF) gelöst und 3 h unter Rückfluß erhitzt. Anschließend wird das Lösungsmittel im Vakuum entfernt. Das Rohprodukt wird durch Säulenchromatographie gereinigt. Als Trägermaterial dient Kieselgel und als Eluent wird ein Gemisch bestehend aus Cyclohexan:Ethylacetat (75:25) verwendet.

| | |
|---|---|
| Ausbeute: | 221 mg (0,95 mmol, 31 %) |
| MS-ESI+: | **m/z** = 234 **[M+H]⁺** |

### b) (4-Amino-3-oxo-1,3-dihydro-isobenzofuran-1-yl)-essigsäureethylester

120 mg (0,51 mmol) (4-Amino-3-oxo-3*H*-isobenzofuran-1-yliden)-essigsäurethylester werden in 50 ml Methanol gelöst und mit 50 mg Palladium auf Aktivkohle (10% Pd) versetzt. Das Reaktionsgemisch wird für 3 h bei 2 bar H₂-Druck und 25°C hydriert Danach wird der Katalysator abfiltriert und das Lösungsmittel im Vakuum entfernt

| | |
|---|---|
| Ausbeute: | 116 mg (0,49 mmol, 97 %) |
| MS (ESI): | m/z = 236 (M+H)⁺ |
| ¹H-NMR: | 1.17 (t, 3H), 2.68 - 2.78 (m, 1H), 3.08 - 3.16 (m, 1H), 4.10 (q, 2H), 5.67 - 5.74 (m, 1H), 6.28 (bs, 2H), 6.61- 6.70 (m, 2H), 7.30-7.38 (m, 1H) |

### Methode 4

### 5-Amino-3H-quinazolin-4-on

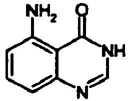

### a) 2,6-Diaminobenzamid

5 g (25,373 mmol) 2,6-Dinitro-benzonitril wird mit 20 ml einer wässrigen 80% Schwefelsäure versetzt und 2 h bei 80°C gerührt. Das Reaktionsgemisch wird mit 100 ml Tetrahydrofuran versetzt mit 10% wässriger Natronlauge neutralisiert. Die organische Phase wird abgetrennt, mit weiteren 100 ml Tetrahydrofuran und 200 mg Palladium auf Kohle versetzt und 20 h bei 8 bar H₂-Druck und 25°C gerührt. Die Feststoffe werden abfiltriert. Das Filtrat wird mit 300 ml Essigester versetzt und mit gesättigter Kaliumhydrogencarbonat-Lösung extrahiert. Die organische Phase wird abgetrennt, getrocknet und das Lösungsmitteln im Vakuum entfernt. Der Rückstand wird durch Chromatographie gereinigt. Als Trägermaterial dient Kieselgel und als Eluent wird Dichlormethan, dem 7% einer Mischung aus 90% Methanol und 10% gesättigter wässriger Ammoniak-Lösung zugefügt sind, verwendet.

| | |
|---|---|
| Ausbeute: | 900 mg (5,958 mmol; 23 %) |
| MS (ESI): | 152 (M+H)⁺ |

### b) 5-Amino-3H-quinazolin-4-on

900 mg (5,958 mmol) 2,6-Diaminobenzamid werden in 3,6 ml *N,N-*Dimethylacetamid gelöst und mit 6,3 ml (57,01 mmol) Trimethylorthoformiat und 792 µl (8,865 mmol) 98% Schwefelsäure versetzt. Nach 16 h bei 25°C wird das Reaktionsgemisch mit 20 ml Methanol aufgenommen und das Lösungsmittel im Vakuum entfernt. Der Rückstand wird erneut in 20 ml Methanol aufgenommen, mit konzentriertem Ammoniak neutralisiert. Das Lösungsmittel wird im Vakuum entfernt und der Rückstand durch Chromatographie gereinigt Als Trägermaterial dient Kieselgel und als Eluent wird Dichlormethan, dem 7% einer Mischung aus 90% Methanol und 10% gesättigter wässriger Ammoniak-Lösung zugefügt sind, verwendet.

| | |
|---|---|
| Ausbeute: | 782 mg (4,852 mmol; 81 %) |
| MS (ESI): | 162 (M+H)⁺ |

### Methode 5

### 9-Amino-2,3,4,5-tetrahydro-2-benzazepin-1-on

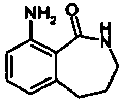

500 mg (1,825 mmol) 2-Brommethyl-6-nitro-methylbenzoat werden in 2 ml Phosphorigsäuretrimethylester 5 h lang auf 100°C erhitzt. 2-(Dimethylphosphonomethyl)-6-nitromethylbenzoat wird durch Eindampfen im Hochvakuum erhalten und direkt weiterverwendet. Dabei wird das Rohprodukt in 24 ml Tetrahydrofuran bei -70°C unter N₂ gelöst, 2,7 ml (2,7 mmol) einer 1 M Lithiumhexamethyldisilazid-Lösung in Tetrahydrofuran zugetropft und anschließend 430 mg (2,70 mmol) *tert*.-Butyl-*N*-(2-oxoethyl)-carbamat in 5 ml Tetrahydrofuran zugegeben. Die Reaktionsmischung wird langsam auf Raumtemperatur erwärmt, mit 5 ml einer 1 M HCl versetzt und mit Ethylacetat extrahiert. Die vereinigten organischen Phasen werden eingedampft und durch Chromatographie auf Kieselgel mit einem Cyclohexan-Ethylacetat-Gemisch im Verhältnis 95:5 bis 75:25 werden 338 mg (1,006 mmol, 55 %) des *E*/*Z*-Gemisches von 2-(3*-tert.-*Butoxycarbonylamino-prop-1-en-1-yl)-6-nitro-methylbenzoat erhalten. Dieses *E-*/*Z-*Gemisch wird 12 h lang mit 10 ml einer gesättigten methanolischen Kaliumhydroxid-Lösung behandelt. Nach Ansäuern mit wässriger 1 M HCl und Extraktion mit Ethylacetat erhält man 302 mg (0,938 mmol, 93%) des *E-*/*Z*-Gemisches von 2-(3-*tert*.-Butoxy-carbonylamino prop-1-en-1-yl)-6-nitro-methylbenzoesäure. Dazu werden 20 mg Raney-Nickel in 100 ml Methanol zugegeben und bei 5 bar H₂-Druck hydriert. Der Katalysator wird abfiltriert, das Filtrat eingedampft und mit einer 1:1-Mischung aus Trifluoressigsäure und Dichlormethan über Nacht bei Raumtemperatur gerührt. Nach dem Entfernen des Lösungsmittels erhält man 133 mg (0,686 mmol, 73%) 2-Amino-6-(3-amino-propyl)-benzoesäure. Die weitere Umsetzung erfolgt durch Lösen in 10 ml THF und 10 ml DCM mit Zugabe von 300 mg (1,570 mmol) *N*-(3-Dimethylaminopropyl)-*N*⁴-Ethylcarbodiimid Hydrochlorid sowie 134 µl (0,830 mmol) *N,N*-Diisopropyl-ethylamin und 48 h Rühren bei Raumtemperatur. Das Lösungsmittel wird im Vakuum entfernt und das Rohprodukt durch Chromatographie mit C18-RP-Kieselgel und einem Laufmittelgemisch aus Acetonitril und Wasser im Verhältnis 5:95 bis 95:5, dem 0,1 % Ameisensäure zugesetzt werden, gereinigt.

| | |
|---|---|
| Ausbeute: | 28 mg (0,160 mmol, 23 %) |
| MS (ESI): | m/z = 177 (M+H)⁺ |

### Methode 6

### 4-Amino-1-methyl-1,2-dihydro-indazol-3-on

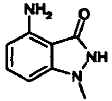

### a) 4-Nitro-1,2-dihydro-indazol-3-on

5 g (27,5 mmol) 2-Amino-6-nitro-benzoesäure werden mit 22,2 ml (225,3 mol) konzentrierter HCl und 45 ml (30,0 mol) 5 %igen wässrigen Natriumnitrit-Lösung versetzt und 1 h bei Raumtemperatur gerührt. Anschließend wird die Suspension mit 150 ml dest. H₂O verdünnt und in 350 ml destilliertes Wasser getropft, welches mit Schwefeldioxid gesättigt ist. Durch das Reaktionsgemisch wird weitere 30 min Schwefeldioxid geleitet. Danach wird das Reaktionsgemisch 30 min unter Rückfluß erhitzt und anschließend lässt man langsam auf 20°C abkühlen. Der dabei entstehende Niederschlag wird abfiltriert.

| | |
|---|---|
| Ausbeute: | 1,7 g (9,5 mmol, 35 %) |
| MS (ESI): | m/z = 180 (M+H)⁺ |

### b) 1-Methyl-4-nitro-1,2-dihydro-indazol-3-on

306 mg (1,7 mmol) 4-Nitro-1,2-dihydro-indazöl-3-on werden in 1 ml *N,N-*Dimethylacetamid gelöst, mit 150 µl (2,4 mmol) Methyljodid und 500 µl (2,32 mmol) *N-*Ethyldiisopropylamid versetzt und 2 h bei Raumtemperatur gerührt. Anschließend wird das Reaktionsgemisch mit 40 ml einer wässrigen 1 N Salzsäure versetzt und 2mal mit 50 ml Dichlormethan extrahiert. Danach wird die organische Phase mit MgSO₄ getrocknet, das Lösungsmittel im Vakuum entfernt und das Rohprodukt durch Chromatographie gereinigt. Als Trägermaterial dient C18-RP-Kieselgel und es wird ein Gradient durchlaufen, der am Startpunkt aus 95% Wasser und 5% Acetonitril und am Endpunkt aus 5% Wasser und 95% Acetonitril besteht.

| | |
|---|---|
| Ausbeute: | 144 mg (0,7 mmol, 44 %) |
| MS (ESI): | m/z = 194 (M+H)⁺ |
| ¹H-NMR: | 3.90 (s, 3H), 7.47-7.52 (m, 1H), 7.68-7.73 (m, 1H), 7.88-7.93 (m, 1H), 10.53 (s, 1H) |

### c) 4-Amino-1-methyl-1,2-dihydro-indazol-3-on

140 mg (0,7 mmol) 1-Methyl-4-nitro-1,2-dihydro-indazol-3-on werden in 6 ml Ethanol suspendiert und mit 600 mg (4,4 eq, 2,9 mmol) Natriumdithionit, gelöst in 2 ml destilliertem Wasser, versetzt und 15 min bei 25 °C gerührt. Anschließend wird das Reaktionsgemisch mit destilliertem Wasser versetzt und 2mal mit Ethylacetat extrahiert. Danach wird die organische Phase mit MgSO₄ getrocknet und das Lösungsmittel im Vakuum entfernt.

| | |
|---|---|
| Ausbeute: | 33 mg (0,2 mmol, 28 %) |
| MS(ESI): | m/z = 164 (M+H)⁺ |

Analog zu dieser Methode wird 4-Amino-1,2-dihydro-indazol-3-on sowie folgende Verbindungen hergestellt.

| | **MS (ESI) (M+H)⁺** | | **MS (ESI) (M+H)⁺** |
|---|---|---|---|
| 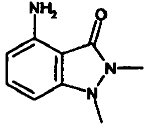 | 178 | 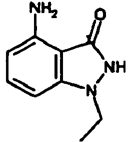 | 178 |
| 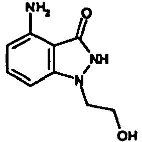 | 194 | | |

### Methode 7

### 8-Amino-4-methyl-3,4-dihydro-2H-isoquinolin-1-on

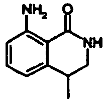

a) 2-(Cyanomethyl-2-methyl)-6-nitro-benzoesäuremethylester 400 mg (1,8 mmol) 2-Cyanomethyl-6-nitro-benzoesäuremethylester werden in 13 ml THF gelöst, mit 114 µl (1,8 mmol) Methyljodid versetzt und unter Stickstoffatmosphäre auf -20 °C abgekühlt Anschließend werden bei dieser Temperatur 250 mg (2,2 mmol) Kalium*tert*-Butylat zugegeben. Nach 1 h wird das Lösungsmittel im Vakuum entfernt und das Rohprodukt durch Chromatographie gereinigt. Als Trägermaterial dient C18-RP-Kieselgel und es wird ein Gradient durchlaufen, der am Startpunkt aus 95% Wasser und 5% Acetonitril und am Endpunkt aus 5% Wasser und 95% Acetonitril besteht.

| | |
|---|---|
| Ausbeute: | 289 mg (1,2 mmol, 68 %) |
| MS (ESI): | 233 (M-H)⁻ |

b) 8-Amino-4-methyl-3,4-dihydro-2*H*-isoquinolin-1-on 400 mg (1,8 mmol) 2-(Cyanomethyl-2-methyl)-6-nitro-benzoesäuremethylester werden in 13 ml Methanol gelöst und mit 50 mg Raney-Nickel versetzt. Das Reaktionsgemisch wird 16 h bei 4 bar H₂-Druck und 25 °C hydriert. Danach wird der Katalysator abfiltriert und das Lösungsmittel im Vakuum entfernt.

| | |
|---|---|
| Ausbeute: | 170 mg (0,8 mmol, 46 %) |
| MS (ESI): | 177 (M+H)⁺ |

Analog zu dieser Methode werden 8-Amino-3,4-dihydro-2*H*-isoquinolin-1-on und 8-Amino-4,4-dimethyl-3,4-dihydro-2*H*-isoquinolin-1-on sowie folgende Verbindungen hergestellt hergestellt.

| | **MS (ESI) (M+H)⁺** | | **MS (ESI) (M+H)⁺** |
|---|---|---|---|
| 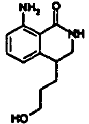 | 221 | 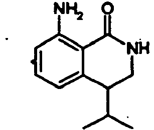 | 205 |
| 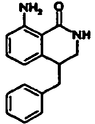 | 253 | | |

### Methode 8

### 7-Amino-indan-1-on

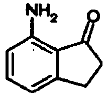

### a) Indan-4-ylamin

24 ml (349 mmol) 65 %ige Salpetersäure werden auf 0-5°C abgekühlt. Dazu werden langsam unter Eiskühlung 28 ml (518,5 mmol) konzentrierte Schwefelsäure getropft. Diese Lösung wird auf 5°C abgekühlt und langsam unter starkem Rühren und weiterer Eiskühlung auf 30 ml (232 mmol) 0-5°C abgekühltes Indan getropft. Das Reaktionsgemisch wird 30 min bei 0-5°C gerührt, und dann über 1 h unter Rühren auf 25°C erwärmt. Anschließend wird die Lösung in 150 ml Eis/Wasser getropft und 30 min nachgerührt. Die wässrige Phase wird 3mal mit je 200 ml Diethylether extrahiert. Die vereinigten organischen Phasen werden zweimal mit je 200 ml gesättigter Natriumhydrogencarbonat-Lösung und einmal mit 150 ml destilliertem Wasser gewaschen. Danach wird die organische Phase mit MgSO₄ getrocknet und das Lösungsmittel im Vakuum entfernt. Das Rohprodukt wird in 250 ml Methanol gelöst und mit 4,5 g Raney-Nickel versetzt. Das Reaktionsgemisch wird 16 h bei 3 bar H₂-Druck und 25° C hydriert. Danach wird der Katalysator abfiltriert und das Lösungsmittel im Vakuum entfernt. Das Rohprodukt wird durch Säulenchromatographie gereinigt. Als Trägermaterial dient Kieselgel und als Eluent wird ein Gemisch bestehend aus Cyclohexan:Ethylacetat (75:25) verwendet.

| | |
|---|---|
| Ausbeute: | 3,81 g (28,6 mmol, 12 %) |
| MS (ESI): | 134 (M+H)⁺ |
| ¹H-NMR: | 1.90-2.00 (m, 2H), 2.61 (t, 2H), 2.76 (t, 2H), 4.73 (s, 2H), 6.33-6.38 (m, 1H), 6.39-6.45 (m, 1H), 6.76-6.83 (m, 1H) |

### b) N-Indan-4-yl-acetamid

226 mg (1,7 mmol) Indan-4-ylamin werden mit 5 ml Essigsäureanhydrid versetzt. Die Suspension wird 16 h bei 70 °C gerührt. Die dabei entstehende Lösung wird in 40 ml destilliertes Wasser eingerührt, mit Natriumcarbonat auf pH 7 eingestellt und 3mal mit je 30 ml Ethylacetat extrahiert. Danach wird die organische Phase mit MgSO₄ getrocknet, das Lösungsmittel im Vakuum entfernt und das Rohprodukt durch Chromatographie gereinigt. Als Trägermaterial dient Kieselgel und als Eluent wird ein Gemisch bestehend aus Cyclohexan:Ethylacetat (70:30) verwendet.

| | |
|---|---|
| Ausbeute: | 152 mg (0,9 mmol, 51 %) |
| MS (ESI): | 176 (M+M)⁺ |
| ¹H-NMR: | 1.93-2.03 (m, 2H), 2.04 (s, 3H), 2.79 (t, 2H), 2.86 (t, 2H), 6.94-7.01 (m, 1H), 7.02-7.10 (m, 1H), 7.36-7.44 (m, 1H), 9.25 (s, 1H) |

### c) N-(3-Oxo-indan-4-yl)-acetamid

147 mg (0,84 mmol) *N*-Indan-4-yl-acetamid werden in 10 ml Aceton gelöst und mit 770 µl einer 15%igen wässrigen Magnesiumsulfat-Lösung versetzt. Die Lösung wird auf 0 °C abgekühlt und portionsweise mit 397 mg (2,490 mmol) Kaliumpermanganat versetzt. Nach 2 h wird mit 50 ml Wasser verdünnt, und 3mal mit je 20 ml Chloroform extrahiert. Die organische Phase wird mit Magnesiumsulfat getrocknet und das Lösungsmittel im Vakuum entfernt und das Rohprodukt durch Chromatographie gereinigt. Als Trägermaterial dient Kieselgel und als Eluent wird ein Gemisch bestehend aus Cyclohexan:Ethylacetat (85:15) verwendet.

| | |
|---|---|
| Ausbeute: | 95 mg (0,500 mmol, 60%) |
| MS (ESI): | 190 (M+H)⁺ |

### d) 7-Ainino-indan-1-on

500 mg (2,6 mmol) *N*-(3-Oxo-indan-4-yl)-acetamid werden in 5 ml Ethanol gelöst, mit 5 ml 18%iger Salzsäure versetzt und 3 h bei 70 °C gerührt. Anschließend wird das Reaktionsgemisch in 100 ml destilliertes Wasser eingerührt, mit Natriumcarbonat auf pH 7 eingestellt und 3mal mit je 30 ml Ethylacetat extrahiert. Danach wird die organische Phase mit Magnesiumsulfat getrocknet und das Lösungsmittel im Vakuum entfernt.

| | |
|---|---|
| Ausbeute: | 388 mg (2,6 mmol, 100 %) |

Analog zu dieser Methode wird 8-Amino-3,4-dihydro-2*H*-naphthalen-1-on hergestellt. Dabei geht man anstelle von Indan von 1,2,3,4-Tetrahydronaphthalin aus.

### Methode 9

### N-(7-Amino-1-oxo-1,3-dihydro-isoindol-2-yl)acetamid

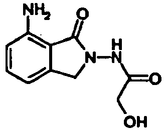

### a) 2-Benzyloxy-N-(7-nitro-1-oxo-1,3-dihydro-isoindol-2-yl)-acetamid

870 mg (4,5 mmol) 2-Amino-7-nitro-2,3-dihydro-isoindol-1-on (Herstellung analog Methode 2) werden in 82 ml Dichlormethan und 64 ml THF gelöst. Die Lösung wird mit 2,8 ml (3,3 eq, 20 mmol) Benzyloxyacetylchlorid, 4,8 ml (28,0 mmol) *N*-Ethyldiisopropylamin und 10 mg *N,N-*Dimethylaminopyridin versetzt und 3 h bei 25°C gerührt. Anschließend wird das Reaktionsgemisch mit 100 ml wässriger 0,1 N Salzsäure versetzt und 3mal mit je 50 ml Essigester extrahiert. Die organische Phase wird mit Magnesiumsulfat getrocknet, das Lösungsmittel im Vakuum entfernt und das Rohprodukt durch Chromatographie gereinigt. Als Trägermaterial dient Kieselgel und als Eluent wird ein Gemisch bestehend aus Dichlormethan:Methanol (95:5) verwendet

| | |
|---|---|
| Ausbeute: | 910 mg (2,7 mmol, 59 %) |

### b) N-(7-Amino-1-oxo-1,3-dihydro-isoindol-2-yl)-acetamid

790 mg (2,3 mmol) 2-Benzyloxy-*N*-(7-nitro-1-oxo-1,3-dihydro-isoindol-2-yl)-acetamid werden in 100 ml Methanol gelöst und mit 80 mg Palladiumhydroxid versetzt. Das Reaktionsgemisch wird 48 h bei 4 bar H₂-Druck und 25°C hydriert. Danach wird der Katalysator abfiltriert und das Lösungsmittel im Vakuum entfernt. Das Rohprodukt wird durch Chromatographie gereinigt. Als Trägermaterial dient Kieselgel und als Eluent wird ein Gemisch bestehend aus Dichlormethan:Methanol (90:10) verwendet.

| | |
|---|---|
| Ausbeute: | 210 mg (0,1 mmol, 41 %) |
| MS (ESI): | 222 (M+H)⁺ |

### Methode 10

### 6-Amino-2-ethyl-3,4-dihydro-2H-benzo[f]][1,4]oxazepin-5-on

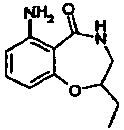

### a) 2-Amino-6-(1-aminomethyl-propoxy)-benzonitril

2,01 g (22 mmol) 1-Amino-2-butanol werden in 6,5 ml 1,4-Dioxan gelöst, mit 880 mg (7,8 mmol) Natriumhydrid versetzt und 30 min bei Raumtemperatur gerührt. Diesem Reaktionsgemisch werden 2 g(14,7 mmol) 2-Amino-6-fluorbenzonitril beigefügt und 24 h bei 50°C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 5% einer Mischung aus 90% Methanol und 10% gesättigter wässriger Ammoniak-Lösung zugefügt sind, verwendet

| | |
|---|---|
| Ausbeute: | 1,15 g (5,6 mmol, 38 %) |
| MS (ESI): | 206 (M+H)⁺ |

### b) 2-Amino-6-(1-aminomethyl-propoxy)-benzoesäure

1,15 g (5,6 mmol) 2-Amino-6-(1-aminomethyl-propoxy)-benzonitril werden in 10 ml 20%iger ethanolischer KOH gelöst und 24 h bei 80°C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 12% einer Mischung aus 90% Methanol und 10% gesättigter wässriger Ammoniak-Lösung zugefügt sind, verwendet.

| | |
|---|---|
| Ausbeute: | 262 mg (1,2 mmol, 21 %) |
| MS (ESI): | 225 (M+H)⁺ |

### c) 6-Amino-2-ethyl-3,4-dihydro-2H-benzo[f][1,4]oxazepin-5-on

262 mg (1,2 mmol) 2-Amino-6-(1-aminomethyl-propoxy)-benzoesäure werden in 26 ml THF gelöst, mit 680 mg (3,5 mmol) 1-(3-Dimethyl-aminopropyl)-3-ethylcarbodiimid Hydrochlorid und 0,6 ml (3,5 mmol) Diisopropyl-ethylamin versetzt und 3 h bei 50°C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 4% einer Mischung aus 90% Methanol und 10% gesättigter wässriger Ammoniak-Lösung zugefügt sind, verwendet.

| | |
|---|---|
| Ausbeute: | 50 mg (0,2 mmol, 21 %) |
| MS (ESI): | 207 (M+H)⁺ |

Analog zu dieser Methode werden folgende Verbindungen hergestellt. Dabei ersetzt man 1-Amino-2-butanol durch einen entsprechenden Aminoalkohol oder durch ein entsprechendes 1,2-Diaminoethylen.

| | **MS (ESI) (M+H)⁺** | | **MS (ESI) (M+H)⁺** |
|---|---|---|---|
| 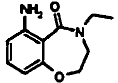 | 207 | 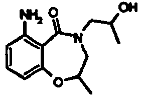 | 251 |
| 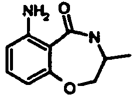 | 193 | 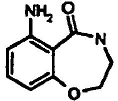 | 179 |
| 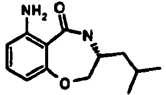 | 235 | 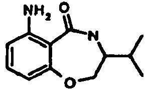 | 221 |
| 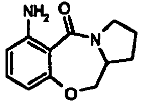 | 219 | 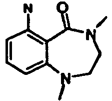 | 206 |
| 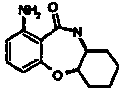 | 233 | 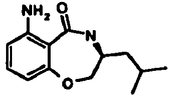 | 235 |
| 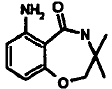 | 207 | 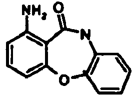 | 227 |
| 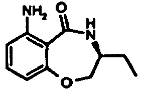 | 207 | 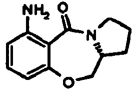 | 219 |
| 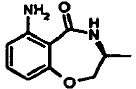 | 193 | 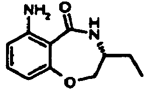 | 207 |
| 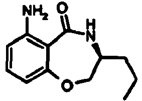 | 221 | 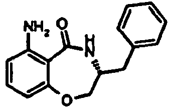 | 269 |
| 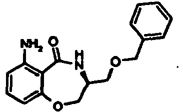 | 299 | 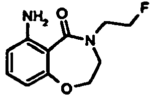 | 225 |
| 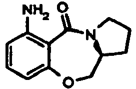 | 219 | 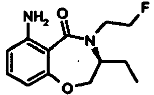 | 253 |
| 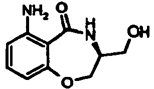 | 209 | 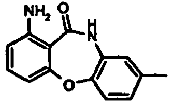 | 241 |
| 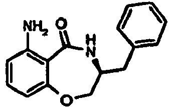 | 269 | 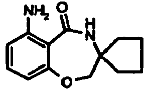 | 233 |

### Methode 11

### 6-Amino-3-benzyl-3,4-dihydro-1H-benzo[e][1,4]diazepin-2,5-dion

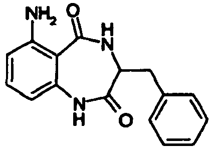

### a) 2-(2-Amino-6-nitro-benzoylamino)-3-phenyl-propionsäuremethylester

1,18 g (6,5 mmol) 2-Amino-6-nitrobenzoesäure, 1,0g (4,6 mmol) D,L-Phenylalanin-methylester Hydrochlorid, 4,05 ml (23,2 mmol) *N*-Ethyldiiopropylamin werden mit 2,5 ml Tetrahydrofuran versetzt. Diesem Reaktionsgemisch werden 1,71 g (5,1 mmol) ***O*-(Benzotriazol-1-yl)-*N*,*N*,*N',N*'-tetramethyluronium-tetrafluorborat** zugesetzt und 12 h bei 50°C erhitzt. Anschließend wird das Lösungsmittel im Vakuum entfernt und das Rohprodukt durch Chromatographie gereinigt. Als Trägermaterial dient Kieselgel und als Eluent wird ein Gemisch bestehend aus Cyclohexan:Ethylacetat (50:50) verwendet.

| | |
|---|---|
| Ausbeute: | 1,04 g (3,03 mmol, 65 %) |
| MS (ESI): | 344 (M+H)⁺ |

### b) 2-(2-Amino-6-nitro-benzoylamino)-3-phenyl-propionsäure

1,04 g (3,03 mmol) 2-(2-Amino-6-nitro-benzoylanzino)-3-phenyl-propionsäuremethylester werden in 3 ml 20% iger ethanolischer KOH gelöst und 1,5 h bei 50°C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 15% einer Mischung aus 90% Methanol und 10% gesättigter wässriger Ammoniak-Lösung zugefügt sind, verwendet.

| | |
|---|---|
| Ausbeute: | 636 mg (1,9 mmol, 64 %) |
| MS(ESI): | 329 (M+H)⁺ |
| ¹H-NMR: | 2.86 - 2.94 (m, 1H), 3.17 (s, 1H), 3.22 - 3.29 (m, 1H), 4.30 - 4.38 (m, 1H), 6.63 (s, 2H), 6.89 - 6.96 (m, 1H), 6.97 - 7.02 (m, 1H), 7.12 - 7.21 (m, 2H), 7.21 - 7.27 (m, 2H), 7.28 - 7.35 (m, 2H), 8.33 - 8.43 (m, 1H) |

### c) 2-(2,6-Diamino-benzoylamino)-3-phenyl-propionsäure

410 mg (1,25 mmol) 2-(2-Amino-6-nitro-benzoylamino)-3-phenyl-propionsäure werden in 50 ml Methanol gelöst und mit 40 mg Palladium auf Kohle (10% Pd) versetzt. Das Reaktionsgemisch wird 9 h bei 5 bar H₂-Druck und 25°C hydriert. Danach wird der Katalysator abfiltriert, das Lösungsmittel im Vakuum entfernt und das Rohprodukt durch Chromatographie gereinigt. Als Trägermaterial dient C18-RP-Kieselgel und es wird ein Gradient durchlaufen der am Startpunkt aus 95% Wasser und 5% Acetonitril und am Endpunkt aus 5% Wasser und 95% Acetonitril besteht.

| | |
|---|---|
| Ausbeute: | 88 mg (0,29 mmol, 24 %) |
| MS (ESI): | 300 (M+H)⁺ |

### d) 6-Amino-3-benzyl-3,4-dihydro-1H-benzo[e][1,4]diazepin-2,5-dion

88 mg (0,3 mmol) 2-(2,6-Diamino-benzoylamino)-3-phenyl-propionsäure werden in 2 ml THF gelöst, mit 143 mg (0,9 mol) l-(3-Dimethyl-aminopropyl)-3-ethylcarbodiimid Hydrochlorid und 103 µl (0,6 mmol) Diisopropyl-ethylamin versetzt und 17 h bei 50°C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 5% einer Mischung aus 90% Methanol und 10% gesättigter wässriger Ammoniak-Lösung zugefügt sind, verwendet.

| | |
|---|---|
| Ausbeute: | 22 mg (0,08 mmol, 27 %) |
| MS(ESI): | 282 (M+H)⁺ |

Analog zu Methode 11 werden folgende Verbindungen hergestellt.

| | **MS (ESI) (M+H)⁺** | | **MS (ESI) (M+H)⁺** |
|---|---|---|---|
| 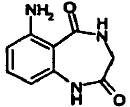 | 192 | 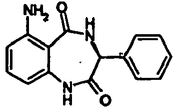 | 268 |
| 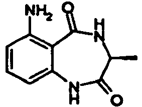 | 206 | 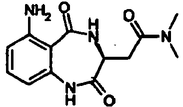 | 277 |
| 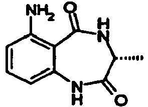 | 206 | 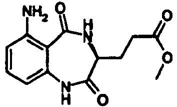 | 278 |
| 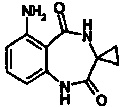 | 218 | 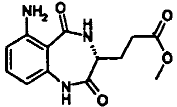 | 278 |
| 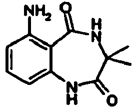 | 220 | 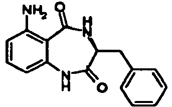 | 282 |
| 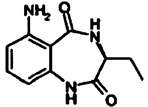 | 220 | 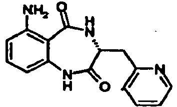 | 283 |
| 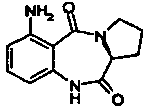 | 232 | 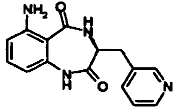 | 283 |
| 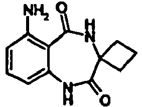 | 232 | 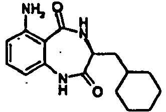 | 288 |
| 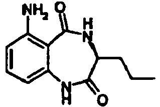 | 234 | 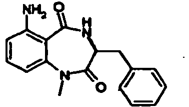 | 296 |
| 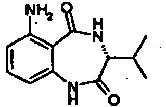 | 234 | 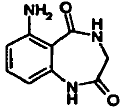 | 192 |
| 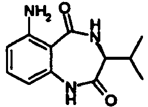 | 234 | 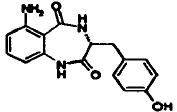 | 298 |
| 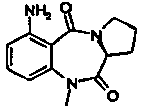 | 246 | 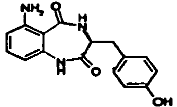 | 298 |
| 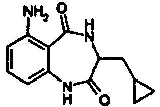 | 246 | 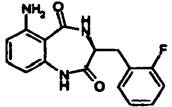 | 300 |
| 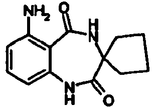 | 246 | 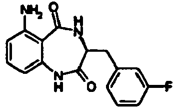 | 300 |
| 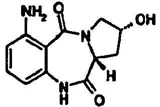 | 248 | 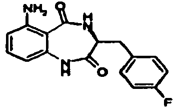 | 300 |
| 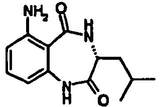 | 248 | 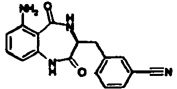 | 307 |
| 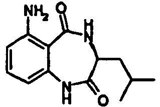 | 248 | 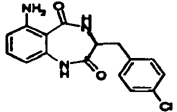 | 316/318 |
| 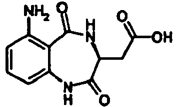 | 250 | 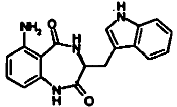 | 321 |
| 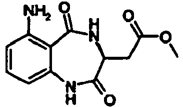 | 265 | 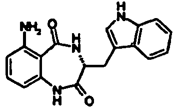 | 321 |
| 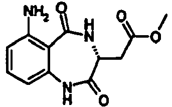 | 265 | 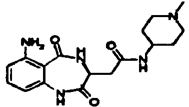 | 346 |

### Methode 12

### 2-(4-Carboxy-2-methox-phenylamino)-4-chlor-5-trifluormethyl-pyrimidin

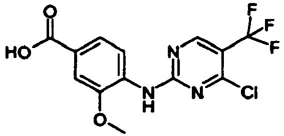

7,36 g (44 mmol) 4-Amino-3-methoxybenzoesäure werden in 80 ml einer wässrigen Phosphatpuffer-Lösung (pH 6,3) suspendiert und mit 9,5 g (44 mmol) 2,4-Dichlor-5-trifluor-methyl-pyrimidin, welches in 240 ml 1,4-Dioxan gelöst ist, versetzt. Nach 4 h bei 100°C wird das Reaktionsgemisch bei 0 °C zur Auskristallisation gebracht. Der Niederschlag wird abfiltriert, das Filtrat wird mit 150 ml Ethylacetat versetzt und 2mal mit je 200 ml einer gesättigten wässrigen Natriumhydrogencarbonat-Lösung gewaschen. Die organische Phase wird mit MgSO₄ getrocknet und das Lösungsmittel im Vakuum entfernt. Das Rohprodukt wird in 10 ml n-Hexan suspendiert und unter Rückfluss erhitzt. Der Niederschlag wird abfiltriert, in 48 ml einer gesättigten wässrigen Natriumhydrogencarbonat-Lösung suspendiert und 1 h bei 65°C erhitzt. Anschließend wird die Lösung bei 0° C zur Auskristallisation gebracht. Der Niederschlag wird abfiltriert, das Filtrat wird mit 1 N wässriger Salzsäure angesäuert und mit 100 ml Essigester versetzt. Die organische Phase wird abgetrennt, mit Magnesiumsulfat getrocknet und das Lösungsmitteln im Vakuum entfernt. Der Rückstand wird in Ethylacetat umkritallisiert

| | |
|---|---|
| Ausbeute: | 330 mg (0,95 mmol, 2 %) |
| MS (ESI): | 348 (M+H)⁺ |
| ¹H-NMR: | 1.55 (s, 1H), 4.01 (s, 3H), 7.61 - 7.64 (m, 1H), 7.79 - 7.85 (m, 1H), 8.34 (s, 1H), 8.59 - 8.63 (m, 1H), 8.66 (s, 1H) |

### Methode 13

### 4-(4-Amino-cyclohexyl)-morpholin

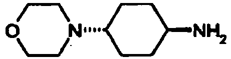

### a) Dibenzyl-(4-morpholino-4-yl-cyclohexyl)-amin

3,9 g (30 mmol) 4-Dibenzylamino-cyclohexanon werden in 100 ml Dichlormethan gelöst und mit 3,9 g (45 mmol) Morpholin und 9,5 g (45 mmol) Natriumtriacetoxyborhydrid 12 h bei Raumtemperatur gerührt. Anschließend wird mit Wasser und Kaliumcarbonat versetzt, die organische Phase abgetrennt, getrocknet und im Vakuum das Lösungsmittel abgezogen. Das Rohprodukt wird durch Säulenchromatographie gereinigt Als Trägermaterial dient Kieselgel und als Eluent wird Essigester, dem 10% einer Mischung aus 90% Methanol und 10% gesättigter wässriger Ammoniak-Lösung zugefügt sind, verwendet. Die geeigneten Fraktionen werden im Vakuum eingeengt.

| | |
|---|---|
| Ausbeute: | 6,6 g (18 mmol, 60%) cis-Isomer |
| | 2 g (5,4 mmol, 18%) trans-Isomer. |

### b) trans-4-Morpholino-4-yl-cyclohexylamin

7,2 g (16,4 mmol) trans-Dibenzyl-4-morpholino-cyclohexylamin wurden in 100 ml Methanol gelöst und an 1,4 g Palladium auf Kohle (10%Pd) bei 30-50°C hydriert. Das Lösungsmittel wird im Vakuum abgezogen und der Rückstand aus Ethanol und konzentrierter Salzsäure kristallisiert.

| | |
|---|---|
| Ausbeute: | 3,9 g (15,2 mmol, 93%) |
| Schmelzpunkt: | 312°C |

Analog zu Methode 13 werden folgende Verbindungen hergestellt:

| | **MS (ESI) (M+H)⁺** | | **MS (ESI) (M+H)^{*}** |
|---|---|---|---|
| 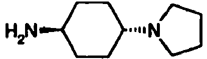 | 169 | 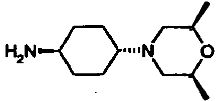 | 213 |
| 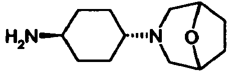 | 211 | 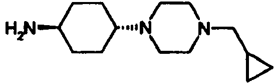 | 238 |

### Methode 14

### 2-(4-Carboxy-2-methoxy-phenylamino)-4-chlor-5-trifluormethyl-pyrimidin

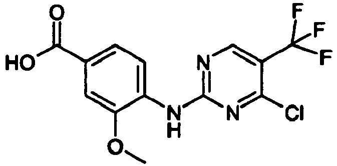

### a) 2-(4-Benzyloxycarbonyl -2-methoxy-phenylamino)-4-chlor-5-trifluormethyl-pyrimidin

2 g (9,217 mmol) 2,4-Dichlor-5-trifluormethylpyrimidin werden in 4 ml Dioxan gelöst und mit 6,01 g (18,430 mmol) Cäsiumcarbonat und 2,16 g (7,363 mmol) 4-Amino-3-methoxybenzoesäurebenzylester (WO 9825901) versetzt. Diese Suspension rührt man 30 h bei 100 °C. Die Suspension wird mit je 50 ml Dichlormethan und Methanol versetzt und von den unlöslichen Bestandteilen abfiltriert. Das Lösungsmittel wird im Vakuum entfernt und der Rückstand wird mittels Säulenchromatographie gereinigt. Als Trägermaterial dient Kieselgel und als Eluent wird ein Gemisch bestehen aus 85% Cyclohexan und 15% Ethylacetat verwendet.

| | |
|---|---|
| Ausbeute: | 1,03 g (2,360 mmol; 26 %) |
| UV max: | 320 nm |
| MS (ESI): | 438 / 440 (M+H)⁺ Cl-Verteilung |
| | 436 / 438 (M -H)⁻ Cl-Verteilung |

### b) 2-(4-Carboxy-2-methoxy-phenylamino)-4-chlor-5-trifluormethyl-pyrimidin

1 g (2,284 mmol) 2-(4-Benzyloxycarbonyl -2-methoxy-phenylamino)-4-chlor-5-trifluormethyl-pyrimidin werden in 50 ml THF gelöst und mit 100 mg Palladiumhydroxid versetzt. Die Reaktionsmischung wird 16 h bei Raumtemperatur und 4 bar Wasserstoffdruck gerührt. Anschließend wird vom Katalysator abfiltriert und das Lösungmittel im Vakuum entfernt.

| | |
|---|---|
| Ausbeute: | 0,76 g (2,192 mmol; 96 %) |
| UV max: | 288 nm |
| MS (ESI): | 346 / 348 (M -H)⁻ Cl-Veiteilung |

Analog zu diesem Verfahren werden folgende Verbindungen hergestellt:
2-((4-Carboxy-phenylamino)-4-chlor-5-trifluormethyl-pyrimidin

| | |
|---|---|
| MS (ESI): | 316 / 318 (M -H)⁻ Cl-Verteilung |

2-[4-(4-Benzyloxycarbonyl-piperazin-1-yl)-phenylaminol]-4-chlor-5-trifluormethyl-pyrimidin

| | |
|---|---|
| MS (ESI): | 492/494 (M +H)⁺ Cl-Verteilung |

2-[4-(4-Benzyloxycarbonyl-piperazin-1-yl)-2-methoxy-phenylamino]-4-chlor-5-trifluormethyl-pyrimidin

| | |
|---|---|
| MS (ESI): | 522/524 (M +H)⁺ Cl-Verteilung |

### Methode 15

### 3-Pyrrolidin-1-yl-cyclobutylamin

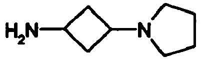

### a) (3-Benzyloxy-cyclobutyl)-carbaminsäure tert-butylester

9,28 g (45 mmol) 3-Benzyloxy-cyclobutancarbonsäure (Org. Lett. 6(11), 1853-1856, 2004) werden in 80 ml trockenem *tert*-Butanol suspendiert und mit 5,1 g (50 mmol) Triethylamin sowie mit 13,8 g (50 mmol) Phosphorsäurediphenylesterazid versetzt. Die Reaktionsmischung wird 20 h unter Rückflussbedingungen gerührt. Das Lösungsmittel wird im Vakuum entfernt und der Rückstand in Dichlormethan aufgenommen. Die organische Phase wird 3mal mit 2 N Natronlauge gewaschen, mit Natriumsulfat getrocknet und das Dichlormethan im Vakuum entfernt Das Rohprodukt wird in Acetonitril umkristallisiert (1g Rohprodukt:5 ml Acetonitril).

| | |
|---|---|
| Ausbeute: | 5,98 g (22 mmol; 48 %) |
| MS (ESI): | 178 (M +H -boc)⁺Boc-Abspaltung in Massendetektor |

### b) (3-Hydroxy-cyclobutyl)-carbaminsäure tert-butylester

2,77 g (10 mmol) (3-Benzyloxy-cyclobutyl)-carbaminsäure *tert*-butylester werden in 100 ml Methanol suspendiert und mit 200 mg Palladiumhydroxid versetzt. Die Reaktionsmischung wird 5 h bei 45°C und 45 bar H₂-Druck gerührt Anschließend wird vom Katalysator abfiltriert und das Lösungmittel im Vakuum entfernt. Der Rückstand wird in Chloroform aufgenommen und 3mal mit wässriger Natriumhydrogencarbonat Lösung gewaschen. Die organische Phase wird mit Magnesiumsulfat getrocknet und das Lösungsmittel im Vakuum entfernt.

| | |
|---|---|
| Ausbeute: | 1,53 g (8,2 mmol; 82 %) |
| MS(ESI): | 188 (M+H)⁺ |

### c) (3-Tosyl-cyclobutyl)-carbaminsäure tert-butylester

18,7 g (100 mmol) (3-Hydroxy-cyclobutyl)-carbaminsäure *tert*-butylester und 12,1 g (120 mmol) Triethylamin werden in 500 ml Chloroform vorgelegt. Unter Rühren tropft man zu dieser Lösung bei 0 °C 20,5 g (105 mmol) Tosylchlorid, welches in 150 ml Chloroform gelöst ist, zu. Anschließend lässt man auf Raumtemperatur kommen und rührt 2 h nach. Die organische Phase wird nacheinander mit Wasser, mit verdünnter Salzsäure, mit Natriumhydrogencarbonat-Lösung und mit Wasser gewaschen. Die organische Phase wird mit Magnesiumsulfat getrocknet und das Lösungsmittel im Vakuum entfernt.

| | |
|---|---|
| Ausbeute: | 28,30 g (83 mmol; 83 %) |
| MS (ESI): | 342 (M+H)⁺ |

### d) (3-Pyrrolidin-cyclobutyl)-carbaminsäure tert-butylester

34,1 g (100 mmol) (3-Tosyl-cyclobutyl)-carbaminsäure *tert*-butylester werden in 750 ml Pyrrolidin gelöst, und mit einer katalytischen Menge DMAP versetzt. Die Reaktionsmischung wird 20 h unter Rückflussbedingungen gerührt. Das Pyrrolidin wird im Vakuum entfernt, der Rückstand in 500 ml Essigester aufgenommen und 2mal mit gesättigter Natriumhydrogencarbonat-Lösung gewaschen. Die organische Phase wird mit Magnesiumsulfat getrocknet und das Lösungsmittel im Vakuum entfernt. Das Rohprodukt besteht - wie in allen analogen Umsetzungen - aus einem Gemisch von 2 isomeren Verbindungen welche säulenchromatographisch getrennt werden. Als stationäre Phase dient Kieselgel und als Eluent wird Dichlormethan, dem 9% einer Mischung aus 90% Methanol und 10% gesättigter wässriger Ammoniak-Lösung zugefügt sind, verwendet. Jene Substanzen die als erstes eluieren werden wie folgt bezeichnet:

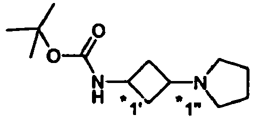

| | |
|---|---|
| Ausbeute Produkt A: | 1 g (4,17 mmol; 4 %) |

RF-Wert (Kieselgel; Dichlormethan:Methanol:konz. wässriger Ammoniak = 90:9:1)= 0,62 Jene Substanzen die als zweites eluieren werden wie folgt bezeichnet:

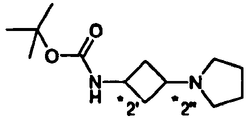

| | |
|---|---|
| Ausbeute Produkt C: | 2,00 g (8,33 mmol; 8 %) |

RF-Wert (Kieselgel; Dichlormethan:Methanol:konz. wässriger Ammoniak = 90:9:1)= 0,53

### e)(*1',*1")-3-Pyrrolidin-1-yl-cyclobutylamin

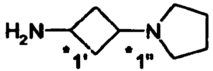

1 g (4,17 mmol) (3-Pyrrolidin-cyclobutyl)-carbaminsäure *tert*-butylester(Produkt A aus Vorstufe) werden in 20 ml einer 2 N wässrigen Salzsäurelösung 2 h bei 40 °C gerührt. Anschließend wird das Lösungsmittel im Vakuum entfernt und der Rückstand in Ethanol umkristallisiert.

| | |
|---|---|
| Ausbeute: | 0,43 g (2,786 mmol; 67 %) |
| MS (ESI): | 141 (M +H)⁺ |

Analog zu diesem Verfahren werden folgende Verbindungen hergestellt:

| | **MS (ESI) (M+H)⁺** | | **MS (ESI) (M+H)⁺** |
|---|---|---|---|
| 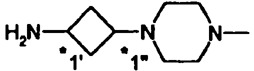 | 170 | 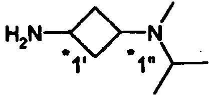 | 143 |
| 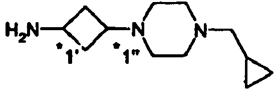 | 210 | 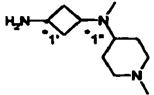 | 198 |
| 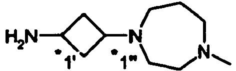 | 184 | 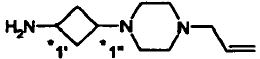 | 196 |
| 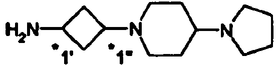 | 224 | 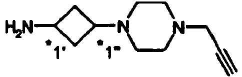 | 194 |
| 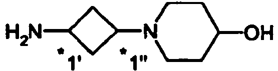 | 171 | 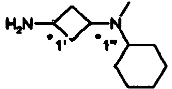 | 183 |
| 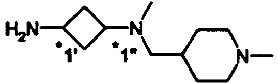 | 212 | | |

### (*2',*2")-3-Pyrrolidin-1-yl-cyclobutylamin

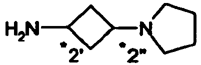

1 g (4,17 mmol) (3-Pyrrolidin-cyclobutyl)-carbaminsäure *tert*-butylester (Produkt C aus Vorstufe) werden in 20 ml einer 2 N wässrigen Salzsäurlösung 2 h bei 40 °C gerührt. Anschließend wird das Lösungsmittel im Vakuum entfernt und der Rückstand in Ethanol umkristallisiert.

| | |
|---|---|
| Ausbeute: | 0,43 g (3,09 mmol,; 74 %) |
| MS (ESI): | 141 (M+H)⁺ |

Analog zu diesem Verfahren werden folgende Verbindungen hergestellt:

| | **MS (ESI) (M+H)⁺** | | **MS (ESI) (M+H)⁺** |
|---|---|---|---|
| 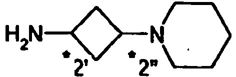 | 155 | 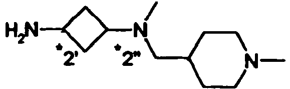 | 212 |
| 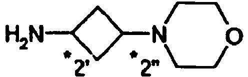 | 157 | 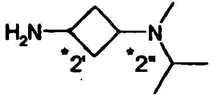 | 143 |
| 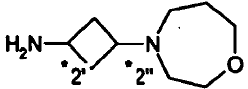 | 171 | 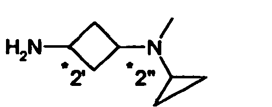 | 141 |
| 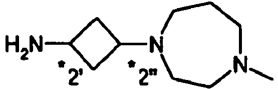 | 184 | 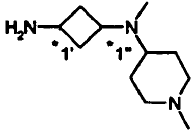 | 198 |
| 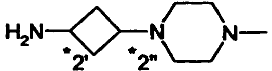 | 170 | 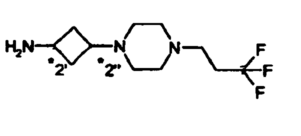 | 251 |
| 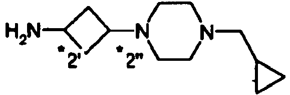 | 210 | 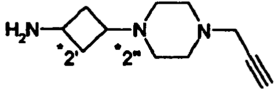 | 194 |
| 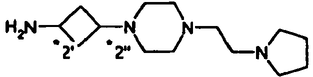 | 253 | 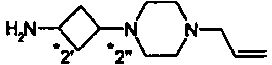 | 196 |
| 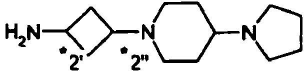 | 224 | 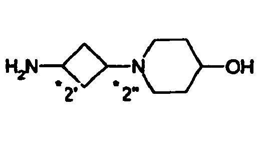 | 171 |
| 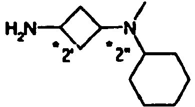 | 183 | | |

### Methode 16

### 2-(4-Carboxy-2-brom-phenylamino)-4-chlor-5-trifluormethyl-pyrimidin

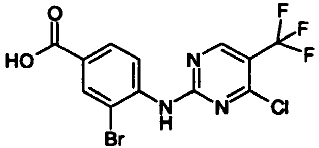

1 g (3,15 mmol) 2-(4-Carboxy-phenylamino)-4-chlor-5-trifluormethyl-pyrimidin werden in 5 ml DMF gelöst und portionsweise mit 3,36 g (18,89 mmol) *N*-Bromsuccinimid versetzt. Die Reaktionsmischung wird 16 h bei Raumtemperatur gerührt. Das Lösungsmittel wird im Vakuum entfernt und der Rückstand wird mittels Säulenchromatographie gereinigt. Als Trägermaterial dient C18-RP-Kieselgel und es wird ein Gradient durchlaufen der am Startpunkt aus 95% Wasser und 5% Acetonitril und am Endpunkt aus 2% Wasser und 98% Acetonitril besteht. Sowohl dem Wasser als auch dem Acetonitril sind jeweils 0,1% Ameisensäure beigemischt.

| | |
|---|---|
| Ausbeute: | 0,57 g (1,44 mmol; 46 %) |
| MS (ESI): | 396 / 398 (M -H)⁺Cl/Br-Verteilung |

### Methode 17

### 5-Amino-3-(2-fluor-ethyl)-3H-quinazolin-4-on

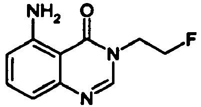

500 mg (3,102 mmol) 5-Amino-3*H*-quinazolin-4-on werden mit 2 ml (15,596 mmol) 1-Brom-2-fluorethan versetzt. Dazu gibt man 125 mg (3,125 mmol) Natriumhydrid und rührt 5 Tage bei Raumtemperatur. Die Reaktionsmischung wird mit 100 ml Essigester verdünnt und mit 100 ml gesättigter wässriger Natriumchloridlösung gewaschen. Die wässrige Phase wird mit 50 ml 1 N Natronlauge versetzt und 5mal mit Essigester extrahiert. Die vereinigten organischen Phasen werden getrocknet und das Lösungsmittel im Vakuum entfernt. Der Rückstand wird mittels Säulenchromatographie gereinigt. Als Trägermaterial dient C18-RP-Kieselgel und es wird ein Gradient durchlaufen der am Startpunkt aus 95% Wasser und 5% Acetonitril und am Endpunkt aus 5% Wasser und 95% Acetonitril besteht. Sowohl dem Wasser als auch dem Acetonitril sind jeweils 0,1% Ameisensäure beigemischt.

| | |
|---|---|
| Ausbeute: | 67 mg (0,323 mmol; 10 %) |
| MS (ESI): | 208 (M+H)⁺ |

### Methode 18

### 8-Amino-2-(2-fluor-ethyl)-3,4-dihydro-2H-isoquinolin-1-on

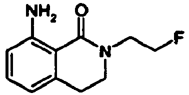

### a) 8-Dibenzylamino-3,4-dihydro-2H-isoquinolin-1-on

1,466 g (9,039 mmol) 8-Amino-3,4-dihydro-2*H*-isoquinolin-1-on werden in 15 ml DMF gelöst und mit 3,226 g (23,340 mmol) Kaliumcarbonat und mit 3,808 ml (31,420 mmol) Benzylbromid versetzt. Diese Reaktionsmischung wird 16 h bei 50°C gerührt. Die Reaktionsmischung wird mit Essigester verdünnt und mit Natriumhydrogencarbonat Lösung extrahiert. Die organischen Phasen werden getrocknet und das Lösungsmittel im Vakuum entfernt.

| | |
|---|---|
| Ausbeute: | 1,670 g (4,877 mmol; 54 %) |
| MS (ESI): | 343 (M+H)⁺ |

### b) 8-Dibenzylamino-2-(2-fluoro-ethyl)-3,4-dihydro-2H-isoquinolin-1-on

1,06 g (3,095 mmol) 8-Dibenzylamino-3,4-dihydro-2*H*-isoquinolin-1-on werden mit 1,5 ml (12 mmol) 1-Brom-2-fluor-ethan versetzt und bei Raumtemperatur portionsweise über einen Zeitraum von 30 h mit 780 mg (19,50 mmol) Natriumhydrid versetzt. Die Reaktionsmischung wird mit Essigester verdünnt und mit Natriumhydrogencarbonat Lösung extrahiert. Die organischen Phasen werden getrocknet und das Lösungsmittel im Vakuum entfernt. Das Rohprodukt wird durch Säulenchromatographie gereinigt. Als Trägermaterial dient Kieselgel und als Eluent wird Dichlormethan, dem 5% einer Mischung aus 90% Methanol und 10% gesättigter wässriger Ammoniak-Lösung zugefügt sind, verwendet.

| | |
|---|---|
| Ausbeute: | 0,83 g (2,136 mmol; 69 %) |
| MS (ESI): | 389 (M+H)⁺ |

### c) 8-Amino-2-(2-fluoro-ethyl)-3,4-dihydro-2H-isoquinolin-1-on

830 mg (2,136 mmol) 8-Dibenzylamino-2-(2-fluor-ethyl)-3,4-dihydro-2*H*-isoquinolin-1-on werden in 50 ml Methanol gelöst und mit 80 mg Palladiumhydroxid versetzt Die Reaktionsmischung wird 48 h bei Raumtemperatur und 4,5 bar H₂-Druck gerührt. Anschließend wird der Katalysator abfiltriert und das Lösungsmittel im Vakuum entfernt.

| | |
|---|---|
| Ausbeute: | 0,403 g (1,935 mmol; 91 %) |
| MS (ESI): | 209 (M+H)⁺ |

Analog zu diesem Verfahren werden folgende Verbindungen hergestellt:

| | **MS (ESI) (M+H)⁺** | | **MS (ESI) (M+H)⁺** |
|---|---|---|---|
| 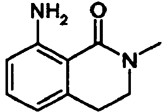 | 177 | 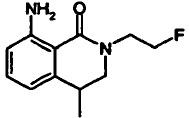 | 223 |
| 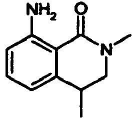 | 191 | | |

### Methode 19

### 7-Amino-5H-phenanthridin-6-on

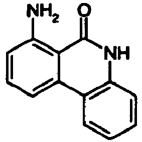

250 mg (1,16 mmol) 2-Chlor-6-nitro-benzoesäuremethylester, 458 mg (1,392 mol) Cäsiumcarbonat, 211 mg (1,218 mmol) 2-Nitrophenylboronsäure und 18 mg (0,035 mol) Bis(tri-*tert*-butylphosphin)palladium(0) werden unter Argon vorgelegt und mit 0,8 ml Dioxan versetzt. Diese Reaktionsmischung rührt man 48 h bei 80°C. Die Reaktionsmischung wird mit Essigester verdünnt und mit 1 N Salzsäure extrahiert. Die organische Phase wird getrocknet und das Lösungsmittel im Vakuum entfernt. Das Rohprodukt wird durch Säulenchromatographie gereinigt. Als Trägermaterial dient C18-RP-Kieselgel und es wird ein Gradient durchlaufen der am Startpunkt aus 95% Wasser und 5% Acetonitril und am Endpunkt aus 5% Wasser und 95% Acetonitril besteht Sowohl dem Wasser als auch dem Acetonitril sind jeweils 0,1% Ameisensäure beigemischt. Die geeigneten Fraktionen werden gefriergetrocknet. 71 mg des so gewonnenen Zwischenprodukts werden in 50 ml Methanol gelöst und mit 10 mg Palladium auf Kohle versetzt Die Reaktionsmischung wird 48 h bei Raumtemperatur und 4.5 bar H₂-Druck gerührt. Zu der Reaktionslösung gibt man 50 ml Dichlormethan, behandelt die Mischung 5 min im Ultraschallbad, und filtriert anschließend den Katalysator ab. Das Lösungsmittel wird im Vakuum entfernt.

| | |
|---|---|
| Ausbeute: | 46 mg (0,221 mmol; 94 %) |
| MS (ESI): | 211 (M+H)⁺ |

### Methode 20

### C-(5-Morpholin-4-ylmethyl-1H-[1,2,3]triazol-4-yl)-methylamin

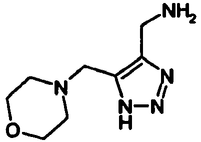

18,021 g (100 mmol) 1-Azido-4-morpholino-2-butin und 19,728 g (100 mmol) Dibenzylamin werden in 100 ml Dioxan gelöst und unter Rühren auf 80 °C erwärmt. Nachdem man 20 h bei dieser Temperatur gerührt hat wird das Lösungsmittel im Vakuum entfernt und der Rückstand wird mittels Säulenchromatographie gereinigt. Als Trägermaterial dient Kieselgel und als Eluent wird Dichlormethan, dem 5% einer Mischung aus 90% Methanol und 10% gesättigter wässriger Ammoniak-Lösung zugefügt sind, verwendet. Die geeigneten Fraktionen werden vereinigt und das Lösungsmittel im Vakuum entfernt. Der Rückstand wird in 480 ml Methanol gelöst und mit 30 ml konzentrierter wässriger Salzsäure und 1 g Palladium auf Kohle versetzt. Diese Reaktionsmischung wird 5 h bei 50°C und 50 bar H₂-Druck gerührt. Anschließend wird der Katalysator abfiltriert und das Lösungsmittel im Vakuum entfernt.

| | |
|---|---|
| Ausbeute: | 8,588 g (28,00 mmol; 28%) |
| MS (ESI): | 198 (M+H)⁺ |

### Methode 21

### 4-Morpholin-4-ylmethyl-cyclohexylamin

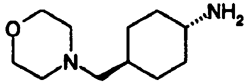

2,5 g (11 mmol) trans-(4-Formyl-cyclohexyl)-carbaminsäure *tert*-butylester werden in 25 ml Dimethylacetamid gelöst mit 1 ml (11 mmol) Morpholin und 0,7 ml Essigsäure versetzt. Zu dieser Mischung gibt man 2,4 g (11,3 mmol) Natriumtriacetoxyborhydrid, welches in 12,5 ml Dimethylacetamid gelöst ist. Die Reaktionsmischung wird 16 h bei Raumtemperatur gerührt. Anschließend gibt man die Reaktionsmischung auf 250 ml 10% Kaliumhydrogencarbonat Lösung und extrahiert diese Mischung 3mal mit je 100 mal Essigester. Die organischen Phasen werden vereinigt, getrocknet und anschließend wird das Lösungsmittel im Vakuum entfernt. Der Rückstand wird in 20 ml Dichlormethan und 20 ml Trifluoressigsäure aufgenommen und eine 1 h bei Raumtemperatur gerührt. Die Lösungsmittel werden im Vakuum entfernt.

| | |
|---|---|
| Ausbeute: | 4,22 g (9,9 mmol; 90 %) (zweifaches Trifluoressigsäure Salz) |
| MS (ESI): | 199 (M+H)⁺ |

Analog zu diesem Verfahren werden folgende Verbindungen hergestellt:

| | **MS (ESI) (M+H)⁺** | | |
|---|---|---|---|
| 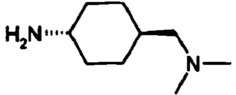 | 157 | 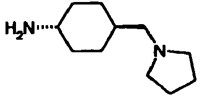 | 183 |
| 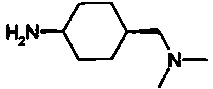 | 157 | 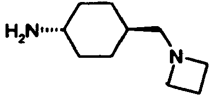 | 169 |

### Methode 22

### 7-Amino-2-(2-fluor-ethyl)-3-methyl-2,3-dihydro-isoindol-1-on

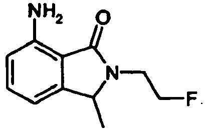

10 g (42,157 mmol) 2-Acetyl-6-nitro-benzoesäuremethylester (J. Org. Chem. (1952), 17, 164-76), 6,06 g (54,804 mmol) 2-Fluorethylamin und 9,32 ml (54,804 mmol) *N-*Ethyldiisopropylamin werden in 25 ml Toluol supendiert und 40 h unter Rückflussbedingungen gerührt. Die Reaktionsmischung wird mit 400 ml Methanol verdünnt und mit 2,5 g Palladium auf Kohle versetzt. Anschließend wird 48 h bei Raumtemperatur und 5 bar H₂-Druck gerührt. Der Katalysator wird abfiltriert und das Lösungsmittel im Vakuum entfernt. Der Rückstand wird in Dichlormethan aufgenommen und mit Wasser gewaschen. Die organische Phase wird mit Magnesiumsulfat getrocknet, das Lösungsmittel im Vakuum entfernt und das Rohprodukt durch Chromatographie gereinigt Als Trägermaterial dient Kieselgel und als Eluent wird ein Gemisch bestehend aus Cyclohexan:Ethylacetat (70:30) verwendet.

| | |
|---|---|
| Ausbeute: | 3,83 g (18,404 mmol, 43 %) |
| MS (ESI): | 209 (M+H)⁺ |
| UV max: | 318 nm |

Analog zu diesem Verfahren unter Verwendung des entsprechenden 6-Nitro-benzoesäuremethylester Derivats werden folgende Verbindungen hergestellt:

| | **MS (ESI) (M+H)⁺** | | **MS (ESI) (M+H)⁺** |
|---|---|---|---|
| 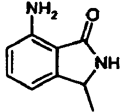 | 163 | 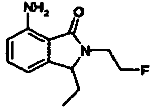 | 223 |
| 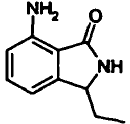 | 177 | 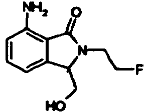 | 225 |
| 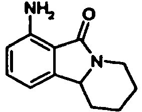 | 203 | 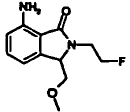 | 239 |
| 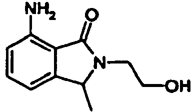 | 207 | 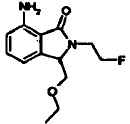 | 253 |
| 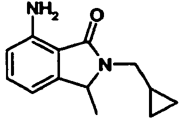 | 217 | 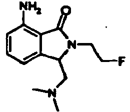 | 252 |
| 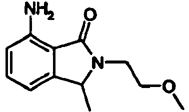 | 221 | 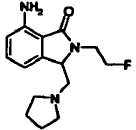 | 278 |
| 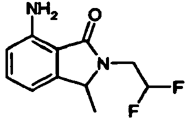 | 227 | 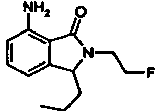 | 237 |
| 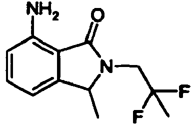 | 241 | 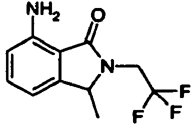 | 245 |

### Methode 23

### 2-Azetidin-1-yl-ethylamin

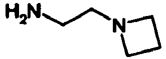

500 µl (7,49 mmol) Azetidin werden in 15 ml Acetonitril gelöst, mit 4,831 g (34,822 mmol) Kaliumcarbonat und 445 µl (7,038 mmol) Chloracetonitril versetzt. Diese Reaktionsmischung rührt man 20 h bei Raumtemperatur. Zu dieser Reaktionsmischung gibt man 20 ml Diethylether rührt die Suspension 10 min und filtriert von den festen Bestandteilen ab. Das Filtrat wird im Vakuum von den Lösungsmitteln befreit 463 mg (4,816 mmol) dieses Zwischenprodukts werden in 50 ml 7 N methanolischem Ammoniak gelöst und mit Raney-Nickel versetzt Die Reaktionsmischung wird 2 h bei 60°C und 20 bar H₂-Druck gerührt. Der Katalysator wird abfiltriert und das Lösungsmittel im Vakuum entfernt.

| | |
|---|---|
| Ausbeute: | 365 mg (3,664 mmol, 48 %) |
| MS (ESI): | 101 (M+H)⁺ |

Analog zu diesem Verfahren werden folgende Verbindungen hergestellt:

| | **MS (ESI) (M+H)⁺** | | **MS (ESI) (M+H)⁺** |
|---|---|---|---|
| 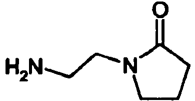 | 129 | 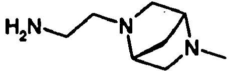 | 156 |
| 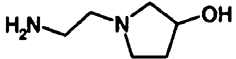 | 131 | 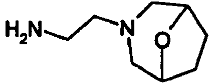 | 157 |
| 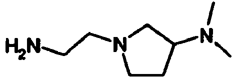 | 158 | 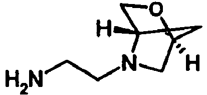 | 143 |
| 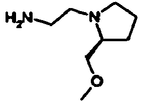 | 159 | 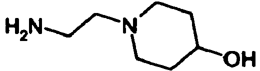 | 145 |
| 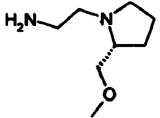 | 159 | 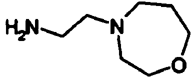 | 145 |
| 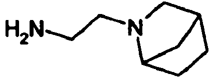 | 141 | 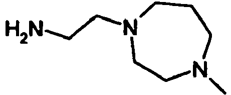 | 158 |
| 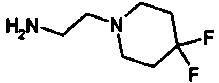 | 165 | 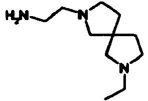 | 198 |
| 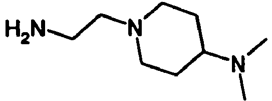 | 172 | 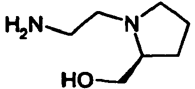 | 145 |

### Methode 24

### ((S)-3-Amino-pyrrolidin-1-yl-acetonitrile

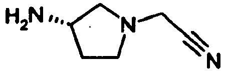

1 g (5,369 mmol) (S)-3-(Boc-amino)-pyrrolidin werden in 20 ml Acetonitril gelöst, mit 4,831 g (34,822 mmol) Kaliumcarbonat und 322 µl (5,101 mmol) Chloracetonitril versetzt. Diese Reaktionsmischung rührt man 20 h bei Raumtemperatur. Zu dieser Reaktionsmischung gibt man 20 ml Diethylether rührt die Suspension 10 min und filtriert von den festen Bestandteilen ab. Das Filtrat wird im Vakuum von den Lösungsmitteln befreit. Das Zwischenprodukt wird in 2 ml Dioxan gelöst und mit 13 ml 4 N dioxanischer Salzsäure versetzt und über Nacht bei RT gerührt. Anschließend wird das Lösungsmittel im Vakuum entfernt.

| | |
|---|---|
| Ausbeute: | 500 mg (3,995 mmol, 74 %) |
| MS (ESI): | 126 (M+H)⁺ |

### Methode 25

### (R)-2-Pyrrolidin-1-yl-propylamine

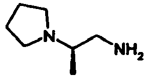

### a) (R)-2-Pyrrolidin-1-yl-propionamide

2 g (16,055 mmol) R-Alaninamid Hydrochlorid, 6,67 g (16,083 mmol) Kaliumcarbonat und 8 mg (0,048 mmol) Kaliumjodid werden in 50 ml Acetonitril suspendiert und anschließend mit 1,921 ml (16,083 mmol) 1,4-Dibrombutan versetzt. Diese Reaktionsmischung wird 14 h unter Rückflussbedingungen gerührt. Zu der Reaktionsmischung gibt man 100 ml 1 N Salzsäure und 100 ml Dichlormethan. Die organische Phase wird abgetrennt und verworfen. Die wässrige Phase wird mit Natronlauge basisch gestellt und 3mal mit Dichlormethan extrahiert. Die organischen Phasen werden vereinigt, getrocknet und im Vakuum vom Lösungsmittel befreit.

| | |
|---|---|
| Ausbeute: | 1,305 g (9,177 mmol, 57 %) |
| MS (ESI): | 143 (M+H)⁺ |

### b) (R)-2-Pyrrolidin-1-yl-propylamine

Unter Stickstoffatmosphäre werden 31,65 ml 1 M Lithiumaluminiumhydrid Lösung (THF) vorgelegt und mit 1 g (7,032 mmol) (R)-2-Pyrrolidin-1-yl-propionamid, welches in 2 ml THF gelöst ist, bei 0 °C versetzt. Die Reaktionsmischung wird 48 h bei 50 °C gerührt. Die Reaktionsmischung wird unter Eiskühlung mit 100 ml Methanol und anschließend mit der gleichen Menge Dichlormethan versetzt. Zu dieser Mischung gibt man ca. 25 g Kieselgel und entfernt das Lösungsmittel im Vakuum. Dieses Kieselgel wird auf eine Nutsche aufgebracht, welche vorher mit ca. 75 g Kieselgel beschickt wurde. Die Nutsche wird portionsweise mit insgesamt 500 ml einer Mischung aus Dichlormethan, Methanol und wässrigen konz. Ammoniak (90:9:1) gewaschen. Ein Großteil des Lösungsmittels wird bei einem Vakuum von 200 mbar und einer Sumpftemperatur von ca. 50 °C entfernt Das Produkt wird bei 69-71 °C und 10 mbar destilliert.

| | |
|---|---|
| Ausbeute: | 160 mg (1,248 mmol, 18 %) |
| MS (ESI): | 129 (M+H)⁺ |

Analog zu diesem Verfahren werden folgende Verbindungen hergestellt:

| | **MS (ESI) (M+H)⁺** | | **MS (ESI) (M**+**H)⁺** |
|---|---|---|---|
| 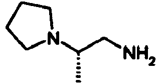 | 129 | 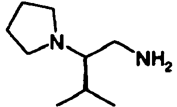 | 157 |
| 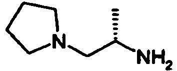 | 129 | 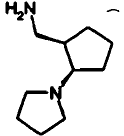 | 169 |
| 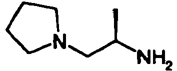 | 129 | 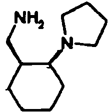 | 183 |
| 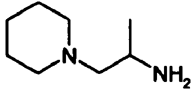 | 143 | 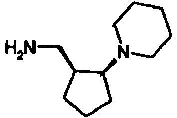 | 183 |
| 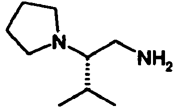 | 157 | 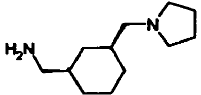 | 197 |

### Methode 26

### 2-Chlor-4-(2-(2-fluor-ethyl)-1-methyl-3-oxo-2,3-dihydro-1H-isoindol-4-ylamino)-5-trifluormethyl-pyrimidin

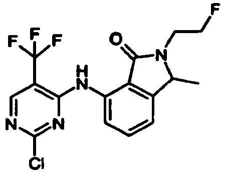

1,1 g (5,07 mmol) 2,4-Dichlor-5-trifluormethylpyrimidin werden in 1 ml Dioxan gelöst und mit 0,9 g (4,322 mmol) 7-Amino-2-(2-fluor-ethyl)-3-methyl-2,3-dihydro-isoindol-1-on (Methode 22) und 0,9 ml (5,257 mmol) Diisopropyethylamin versetzt. Diese Mischung rührt man 1 h bei 80 °C. Anschließend entfernt man das Lösungsmittel im Vakuum. Das Rohprodukt wird durch Säulenchromatographie gereinigt. Als Trägermaterial dient C18-RP-Kieselgel und es wird ein Gradient durchlaufen der am Startpunkt aus 95% Wasser und 5% Acetonitril und am Endpunkt aus 20% Wasser und 80% Acetonitril besteht. Sowohl dem Wasser als auch dem Acetonitril sind jeweils 0,1% Ameisensäure beigemischt. Die geeigneten Fraktionen werden mit Dichlormethan versetzt, die organische Phase abgetrennt, getrocknet und vom Lösungsmittel im Vakuum befreit.

| | |
|---|---|
| Ausbeute: | 485 mg (1,250 mmol, 25 %) |
| MS (ESI): | 389/391 (M+H)⁺; Cl-Verteilung |

Analog zu diesem Verfahren werden folgende Verbindungen hergestellt. Die eingesetzten Anilin-Derivate sind in den Ergänzungen zu Methode 2, in Methode 10 und in den Ergänzungen zu Methode 10 beschrieben. Die Herstellung der 2,4-Dichlorpyrimidin-Derivaten ist literaturbekannt oder kann analog literaturbekannter Verfahren durchgeführt werden.

| | **MS (ESI) (M+H)⁺** | | **MS (ESI) (M+H)⁺** |
|---|---|---|---|
| 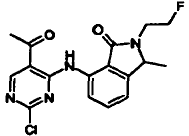 | 363/365 | 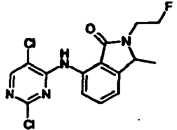 | 355/357 |
| 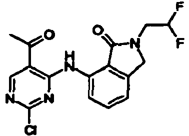 | 367/369 | 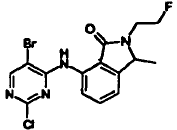 | 399/401 |
| 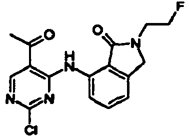 | 349/351 | 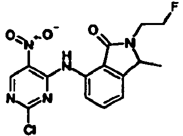 | 366/368 |
| 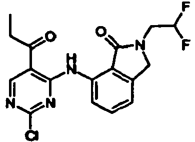 | 381/383 | 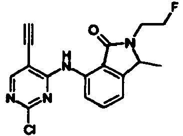 | 345/347 |
| 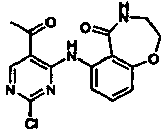 | 333/335 | 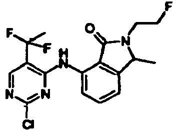 | 385/387 |
| 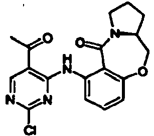 | 373/375 | 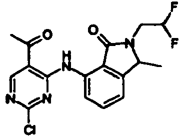 | 381/383 |
| 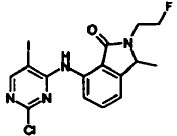 | 447/449 | | |

### Methode 27

### 2-[2-(4-Amino-3-methoxy-phenyl)-1H-imidazol-4-yl]-ethanol

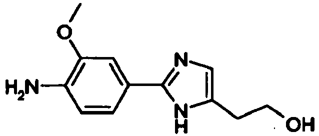

### a) 3-Methoxy-4-nitro-benzonitril

25 g (150,504 mmol) 3-Fluor-4-nitrobenzonitril und 25 g (462,757 mmol) Natriummethoxid werden bei 0°C in 125 ml THF gelöst. Diese Reaktionsmischung wird 30 min gerührt. Die Reaktionsmischung wird mit Essigester und 1 N Salzsäure extrahiert. Die organische Phase wird mit Magnesiumsulfat getrocknet und das Lösungsmittel im Vakuum entfernt.

| | |
|---|---|
| Ausbeute: | 25,092g (140,852 mmol, 94%) |
| UV max: | 334 nm |

### b) 3-Methoxy-4-nitro-benzamidin

99 ml (99mmol) Lithium-bis-trimethylsilylamid-Lösung (1 mol/l in THF) werden mit 640 ml THF verdünnt, auf 10°C gekühlt und mit 8,3 g (46,591 mmol) 3-Methoxy-4-nitro-benzonitril versetzt. Das Reaktionsgemisch wird 10 min bei 20°C gerührt. Der Ansatz wird auf 0°C gekühlt und mit 80 ml 3 N Salzsäure versetzt Das Reaktionsgemisch wird im Vakuum eingeengt und mit Wasser und Essigester extrahiert. Die wässrige Phase wird mit 3 N Natronlauge auf pH 14 gestellt. Das Produkt wird anschließend abgesaugt.

| | |
|---|---|
| Ausbeute: | 14,30 g (Rohprodukt: 60% Reinheit) |
| MS (ESI): | 196 (M+H)⁺ |
| UV max: | 334 nm |

### c) [2-(3-Methoxy-4-nitro-phenyl)-1H-imidazol-4-yl]-essigsäure

7 g (60% Reinheit, 21,519 mol) 3-Methoxy-4-nitro-benzamidin werden in Methanol gelöst und mit 11 ml (44 mmol) 4 N dioxanischer Salzsäure versetzt, die Lösungsmittel werden im Vakuum entfernt. Der Rückstand und 6,13 g (44,384 mmol) Kaliumcarbonat werden in 350 ml Acetonitril suspendiert und mit 3,24 ml (22,764 mmol) 4-Chloracetessigsäureethylester und mit 880 mg (5,301 mmol) Kaliumjodid versetzt. Das Reaktionsgemisch wird 16 h bei 45 °C gerührt. Die Reaktionsmischung wird mit Wasser verdünnt und mit 1 N Natronlauge versetzt, es wird mit Essigester extrahiert. Die wässrige Phase wird mit 1 N HCL auf pH 1 gestellt und mit Natriumchlorid gesättigt. Das Produkt wird anschließend abgesaugt.

| | |
|---|---|
| Ausbeute: | 1,45 g (5,230 mmol, 24%) |
| MS (ESI): | 278 (M+H)⁺ |
| UV max: | 294 nm |

### d) 2-[2-(3-Methoxy-4-nitro-phenyl)-1H-imidazol-4-yl]-ethanol

1,45 g (5,23 mmol) [2-(3-Methoxy-4-nitro-phenyl)-1*H*-imidazol-4-yl]-essigsäure werden in 36 ml THF gelöst und auf 0 °C gekühlt und mit 10 ml (18 mol) Boran-THF-Komplex (1,8 mol/l) versetzt. Nach 1 h wird die Mischung auf 20 °C erwärmt und 16 h gerührt. Es wird Wasser zugegeben bis die Gasentwicklung beendet ist. Anschließend wird mit gesättigter wässriger Natriumhydrogencarbonatlösung und Essigester 2mal extrahiert. Die organischen Phasen werden vereinigt, getrocknet und im Vakuum vom Lösungsmittel befreit.

| | |
|---|---|
| Ausbeute: | 0,65 g (2,465 mmol, 47%) |
| MS (ESI): | 264 (M+H)⁺ |
| UV max: | 298 nm |

### e) 2-[2-(4-Amino-3-methoxy-phenyl)-1H-imidazol-4-yl]-ethanol

0,144 g (0,547 mmol) 2-[2-(3-Methoxy-4-nitro-phenyl)-1*H*-imidazol-4-yl]-ethanol werden in 100 ml Methanol gelöst und mit 0,08 g (5%) Palladium auf Kohle versetzt. Die Reaktionsmischung wird 16 h bei 20 °C und 4 bar H₂-Druck hydriert. Das Palladium auf Kohle wird abgesaugt und das Methanol im Vakuum entfernt.

| | |
|---|---|
| Ausbeute: | 87 mg (0,373 mmol, 68%) |
| MS (APCI): | 234 (M+H)⁺ |
| UV max: | 314 nm |

Analog zu diesem Verfahren werden folgende Verbindungen hergestellt:

| | **MS (ESI) (M+H)⁺** | | **MS (ESI) (M+H)⁺** |
|---|---|---|---|
| 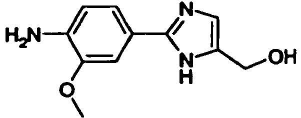 | 220 | 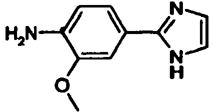 | 190 |
| 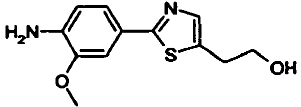 | 251 | | |

2-[2-(4-Amino-3-methoxy-phenyl)-thiazol-5-yl]-ethanol wird analog zu den oben beschriebenen Verfahren hergestellt Für die Zyklisierung verwendet man anstelle des 3-Methoxy-4-nitro-benzamidin das 4-Amino-3-methoxy-thiobenzamid(analog J. Am. Soc. 82, 2656, 1960) 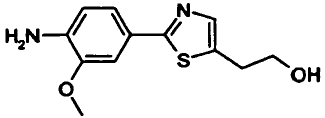 MS (ESI): 251 (M+H)⁺

### Methode 28

### 2-Methoxy-N⁴-(3-pyrrolidin-1-yl-propyl)-benzol-1,4-diamin

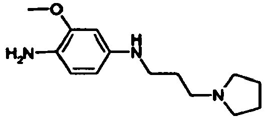

### a) (3-Methoxy-4-nitro-phenyl)-(3-pyrrolidin-1-yl-propyl)-amin

1 g (5,884 mmol) 4-Fluor-2-methoxy-1-nitro-benzol, 975 mg (7,369 mmol) 1-(3-Aminopropyl)pyrrolidin und 1,5 ml (8,765 mmol) Diisopropyethylamin werden in 5 ml Dioxan gelöst und 24 h bei 95 °C gerührt. Die Lösungsmittel werden im Vakuum entfernt und das Rohprodukt wird durch Säulenchromatographie gereinigt. Als Trägermaterial dient Kieselgel und als Eluent wird Dichlormethan, dem 15% einer Mischung aus 90% Methanol und 10% gesättigter wässriger Ammoniak-Lösung zugefügt sind, verwendet.

| | |
|---|---|
| Ausbeute: | 1,07 g (3,827 mmol; 65 %) |
| MS (ESI): | 280 (M+H)⁺ |

### b) 2-Methoxy-N⁴-(3-pyrrolidin-1-yl-propyl)-benzol-1,4-diamin

200 mg (0,716 mmol) (3-Methoxy-4-nitro-phenyl)-(3-pyrrolidin-1-yl-propyl)-amin werden in 10 ml Methanol gelöst und mit 537 µl (2,148 mmol) dioxanischer Salzsäure und 20 mg Palladium auf Kohle versetzt. Die Reaktionsmischung wird 1 h bei Raumtemperatur und 5 bar H₂-Druck gerührt. Der Katalysator wird abfiltriert und das Lösungsmitteln im Vakuum entfernt.

| | |
|---|---|
| Ausbeute: | 213 mg (0,661 mmol, 92 %) |
| MS (ESI): | 250 (M+H)⁺ |

Analog zu diesem Verfahren werden folgende Verbindungen hergestellt:

| | **MS (ESI) (M+H)⁺** | | **MS (ESI) (M+H)⁺** |
|---|---|---|---|
| 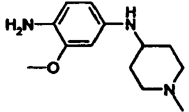 | 236 | 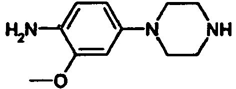 | 208 |
| 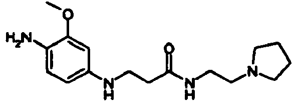 | 307 | 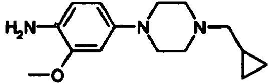 | 262 |
| 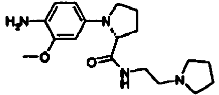 | 333 | 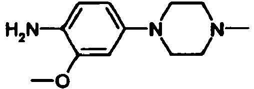 | 222 |
| 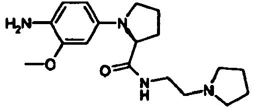 | 333 | 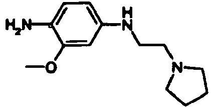 | 236 |
| 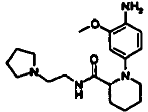 | 347 | 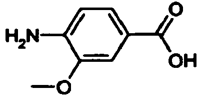 | 168 |
| 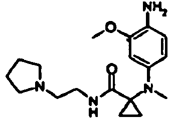 | 333 | 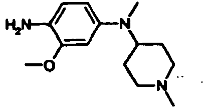 | 250 |
| 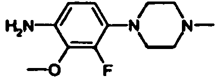 | 240 | 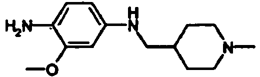 | 250 |
| 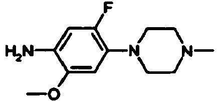 | 240 | 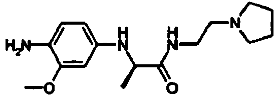 | 307 |
| 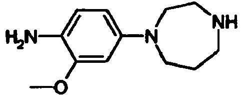 | 222 | 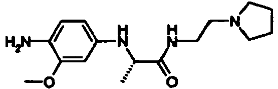 | 307 |

### Methode 29

### 2-(4-Carboxy-2-brom-phenylamino)-4-chlor-5-trifluormethyl-pyrimidin

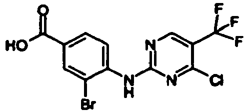

1 g (3,148 mmol) 2-(4-Carboxy-2-methoxy-phenylamino)-4-chlor-5-trifluormethyl-pyrimidin (Methode 12 oder 14) werden in 5 ml DMF gelöst und portionsweise mit 3,36 g (18,889 mmol) *N*-Bromsuccinimid versetzt. Diese Reaktionsmischung lässt man 16 h bei Raumtemperatur rühren. Anschließend wird das Lösungsmittel im Vakuum entfernt und der Rückstand wird mittels Säulenchromatographie gereinigt. Als Trägermaterial dient C18-RP-Kieselgel und es wird ein Gradient durchlaufen der am Startpunkt aus 95% Wasser und 5% Acetonitril und am Endpunkt aus 2% Wasser und 98% Acetonitril besteht. Sowohl dem Wasser als auch dem Acetonitril sind jeweils 0,1% Ameisensäure beigemischt.

| | |
|---|---|
| Ausbeute: | 571 mg (1,440 mmol, 46 %) |
| MS (ESI): | 396 / 398 (M+H)⁺ |

### Methode 30

### 2-(4-Acryloylamino-2-methoxy-phenylamino)-4-(2-(2-fluor-ethyl)-1-methyl-3-oxo-2,3-dihydro-1H-isoindol-4-ylamino)-5-trifluormethyl-pyrimidin

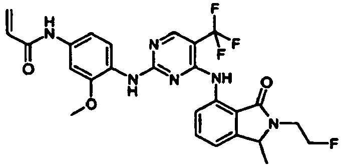

### a) 4-Amino-2-methoxy-phenylamino)-4-(2-(2-fluor-ethyl)-1-methyl-3-oxo-2,3-dihydro-1H-isoindol-4-ylamino)-5-trifluormethyl-pyrimidin

1 g (1,925 mmol) 2-(4-Carboxy-2-methoxy-phenylamino)-4-[2-(2-fluor-ethyl)-1-methyl-3-oxo-2,3-dihydro-1*H*-isoindol-4-ylamino]-5-trifluormethyl-pyrimidine (Herstellung analog Beispiel 53) werden in 2 ml Toluol gelöst und nacheinander mit 0,43 ml (2,503 mmol) Diisopropylethylamin, mit 1,8 ml *tert*-Butanol und mit 0,49 ml (2,310 mmol) Diphenylphosphorylazid versetzt und für 18 h auf 80°C erhitzt. Die Reaktionsmischung wird abgekühlt mit 100 ml Essigester verdünnt und 2mal mit 0,5 N Natronlauge gewaschen. Die organische Phase wird mit Magnesiumsulfat getrocknet und das Lösungsmittel im Vakuum entfernt. er Rückstand wird in Dichlormethan aufgenommen und mit 4 M dioxanischer Salzsäure versetzt. Man lässt 72 h bei Raumtemperatur rühren. Es wird mit Essigester verdünnt und 4mal mit 1 N Salzsäure extrahiert. Die wässrigen Phasen werden vereinigt und 1mal mit Essigester extrahiert. Die wässrige Phase wird mit Natronlauge basisch gestellt und 3mal mit Essigester extrahiert. Die organische Phasen werden vereinigt, getrocknet und das Lösungsmittel im Vakuum entfernt.

| | |
|---|---|
| Ausbeute: | 606 mg (1,236 mmol, 64 %) |
| MS (ESI): | 491 (M+H)⁺ |

### b) 2-(4-Acryloylamino-2-methoxy-phenylamino)-4-(2-(2-fluor-ethyl)-1-methyl-3-oxo-2,3-dihydro-1H-isoindol-4-ylamino)-5-trifluormethyl-pyrimidin

311 mg (0,634 mmol) 2-(4-Amino-2-methoxy-phenylamino)-4-(2-(2-fluor-ethyl)-1-methyl-3-oxo-2,3-dihydro-1*H*-isoindol-4-ylamino)-5-trifluormethyl-pyrimidin werden in 10 ml THF gelöst und mit 115 µl (0,824 mmol) Triethylamin und 62 µl (0,761 mmol) Acrylchlorid versetzt. Diese Mischung rührt man 1 h bei Raumtemperatur. Anschließend wird mit Essigester verdünnt und 3mal mit Wasser extrahiert. Die organische Phase wird mit Magnesiumsulfat getrocknet und das Lösungsmittel im Vakuum entfernt.

| | |
|---|---|
| Ausbeute: | 340 mg (0,625 mmol, 98 %) |
| MS (ESI): | 545 (M+H)⁺ |

Analog zu diesem Verfahren werden folgende Verbindungen hergestellt:

| | **MS(ESI) (M+H)⁺** | | **MS (ESI) (M+H)⁺** |
|---|---|---|---|
| 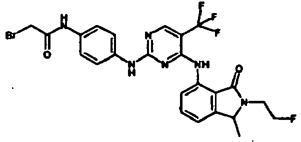 | 581 | 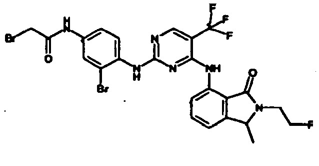 | 659 |
| 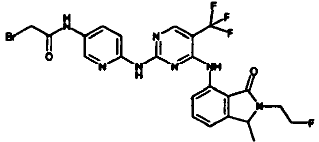 | 582 | 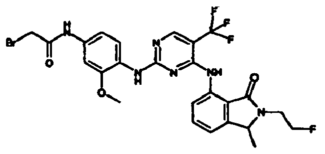 | 611 |

### Methode 31

### Trennung des racemischen 7-Amino-2-(2-fluor-ethyl)-3-methyl-2,3-dihydro-isoindol-1-on (Methode 22) in die beiden Enantiomeren

Die Trennung erfolgt über eine preparative Chromatographie mit folgenden Bedingungen: Säule: 280 x 110 mm CHIRALPAK^{®} AD 20 µm
Eluent: 95% Acetonitril/5% Isopropanol (v/v)
Flußrate: 570 ml/min
Temperatur: Raumtemperatur

Das Enantiomer, welches als erstes eluiert, wird als Enantiomer 1 bezeichnet und trägt in den chemischen Formel das Symbol *1:

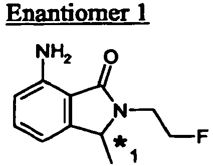

Das Enantiomer, welches als zweites eluiert, wird als Enantiomer 2 bezeichnet und trägt in den chemischen Formel das Symbol *2:

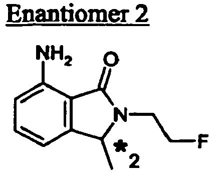

### Methode 32

### 7-Amino-3-ethyl-indan-1-one

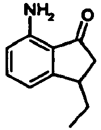

262 mg (1,364 mmol) Kupferjodid werden vorgelegt und im Argonstrom ausgeheizt. Anschließend suspendiert man das Kupferjodid in Ether und kühlt auf -78 °C ab. Bei dieser Temperatur gibt man 0,8 ml einer 3 M Ethylmagnesiumbromid Lösung (in Ether) zu und rührt 10 min und lässt anschließend auf 0 °C auftauen. Nachdem man 15 min bei dieser Temperatur gerührt hat kühlt man wieder auf -78 °C ab und tropft 200 mg (0,802 mmol) *N*-(3-Oxo-3*H*-inden-4-yl)-benzamid, gelöst in 9 ml THF, zu und läst bei 0 °C für 1 h rühren. Die Reaktionsmischung wird mit Dichlormethan verdünnt und 3mal mit konzentrierter wässriger Ammoniaklösung gewaschen. Die organische Phase wird mit Magnesiumsulfat getrocknet und das Lösungsmittel im Vakuum entfernt. Der Rückstand wird mittels Säulenchromatographie gereinigt. Als Trägermaterial dient C18-RP-Kieselgel und es wird ein Gradient durchlaufen der am Startpunkt aus 98% Wasser und 2% Acetonitril und am Endpunkt aus 2% Wasser und 98% Acetonitril besteht. Sowohl dem Wasser als auch dem Acetonitril sind jeweils 0,1% Ameisensäure beigemischt. Die geeigneten Fraktionen werden gefriergetrocknet Dieses Zwischenprodukt wird in 2 ml Dioxan gelöst und mit 5 ml konzentrierter Salzsäure versetzt. Die Reaktionsmischung wird 24 h unter Rückflussbedingungen gerührt. Anschließend wird mit Wasser verdünnt und 3mal mit Dichlormethan extrahiert. Die vereinigten organischen Phasen werden nochmals mit Wasser gewaschen, getrocknet und das Lösungsmittel entfernt. Der Rückstand wird mittels Säulenchromatographie gereinigt. Als Trägermaterial dient Kieselgel und als Eluent wird Dichlormethan, dem 5% einer Mischung aus 90% Methanol und 10% gesättigter wässriger Ammoniak-Lösung zugefügt sind, verwendet.

| | |
|---|---|
| Ausbeute: | 70 mg (0,399 mmol; 29 %) |
| MS (ESI): | 176 (M+H)⁺ |

Analog zu diesem Verfahren werden folgende Verbindungen hergestellt:

| | **MS (ESI) (M+H)⁺** | | **MS (ESI) (M+H)⁺** |
|---|---|---|---|
| 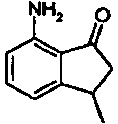 | 162 | 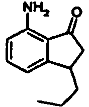 | 190 |

### Methode 33

### 7-Amino-3,3-dimethyl-3H-isobenzofuran-1-on

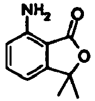

250 mg (0,609 mmol) 2-Dibenzylamino-benzoesäuremethylester werden unter Argon mit 0,609 ml einer 1 M Lithiumchlorid Lösung (THF) versetzt Diese Lösung kühlt man auf - Raumtemperatur ab und dosiert langsam 0,914 ml (1,827 mmol) einer 2 M Isopropylmagnesiumchlorid Lösung zu. Nachdem man 16 h bei dieser Temperatur gerührt hat gibt man tropfenweise 45 µl (0,609 mmol) Aceton zu und rührt bei Raumtemperatur für 4 h. Die Reaktionslösung wird mit Natriumhydrogencarbonat Lösung versetzt und 3mal mit Dichlormethan extrahiert. Die vereinigten organischen Phasen werden getrocknet und das Lösungsmittel im Vakuum entfernt. Der Rückstand wird mittels Säulenchromatographie gereinigt Als Trägermaterial dient C18-RP-Kieselgel und es wird ein Gradient durchlaufen der am Startpunkt aus 95% Wasser und 5% Acetonitril und am Endpunkt aus 5% Wasser und 95% Acetonitril besteht. Sowohl dem Wasser als auch dem Acetonitril sind jeweils 0,1% Ameisensäure beigemischt. Die geeigneten Fraktionen werden gefriergetrocknet. Dieses Zwischenprodukt wird in 50 ml Methanol gelöst mit 10 mg Palladium auf Kohle versetzt und 20 h bei 5 bar Wasserstoffdruck und Raumtemperatur hydriert. Anschließend wird der Katalysator abfiltriert und das Lösungsmittel im Vakuum entfernt. Der Rückstand wird mittels Säulenchromatographiegereinigt. Als Trägermaterial dient C18-RP-Kieselgel und es wird ein Gradient durchlaufen der am Startpunkt aus 95% Wasser und 5% Acetonitril und am Endpunkt aus 5% Wasser und 95% Acetonitril besteht. Sowohl dem Wasser als auch dem Acetonitril sind jeweils 0,1% Ameisensäure beigemischt. Die geeigneten Fraktionen werden gefriergetrocknet.

| | |
|---|---|
| Ausbeute: | 34 mg (0,192 mmol; 32 %) |
| MS (ESI): | 178 (M+H)⁺ |

Analog zu diesem Verfahren werden folgende Verbindungen hergestellt:

| | **MS (ESI) (M+H)⁺** | | **MS (ESI) (M+H)⁺** |
|---|---|---|---|
| 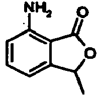 | 164 | 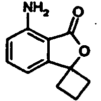 | 190 |
| 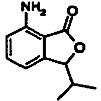 | 192 | 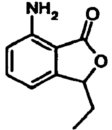 | 178 |
| 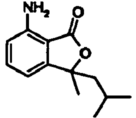 | 220 | | |

### Methode 34

### 7-Amino-2-(2-fluor-ethyl)-3,3-dimethyl-2,3-dihydro-isoindol-1-on

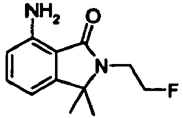

### a) 2-(Cyano-dimethyl-methyl)-6-nitro-benzoesäuremethylester

3 g (13,625 mmol) 2-Cyanomethyl-6-nitro-benzoesäuremethylester (WO 9518097) werden in 20 ml THF gelöst mit 4,33 ml (68,788 mmol) Iodmethan versetzt und auf 0 °C abgekühlt. Bei dieser Temperatur tropft man langsam 40,87 ml einer 1 M Kalium-*tert-*butylat-Lösung zu. Man läst auf Raumtemperatur aufwärmen und rührt 16 h bei dieser Temperatur. Die Reaktionsmischung wird mit Essigester verdünnt und 3mal mit 1 M Salzsäure extrahiert. Die vereinigten organischen Phasen werden getrocknet und das Lösungsmittel im Vakuum entfernt.

| | |
|---|---|
| Ausbeute: | 3,11 g (12,535 mmol; 92 %) |

### b) 3,3-Dimethyl-7-nitro-2,3-dihydro-isoindol-1-on

### Reaktionsmischung 1

1 g (4,028 mmol) 2-(Cyano-dimethyl-methyl)-6-nitro-benzoesäuremethylester werden in 20%iger ethanolischer Kaliumhydroxid Lösung suspendiert und 24 h bei Raumtemperatur gerührt.

### Reaktionsmischung 2

Man löst 1,9 g (47,577 mmol) Natriumhydroxid in 40 ml Wasser kühlt auf 0 °C ab und versetzt mit 0,5 ml (28,899 mmol) Brom. Zu dieser Lösung tropft man langsam die Reaktionsmischung 1. Nach 8 h gibt man nochmals die gleiche Menge Reaktionsmischung 1 zu. Man lässt weitere 48 h bei RT rühren. Anschließend gibt man Natriumsulfit-Lösung zu, lässt 20 min rühren und stellt anschließend mit Kaliumhydrogensulfat-Lösung sauer. Es wird 3mal mit Essigester extrahiert. Die vereinigten organischen Phasen werden getrocknet und das Lösungsmittel im Vakuum entfernt. Der Rückstand wird mittels Säulenchromatographie gereinigt. Als Trägermaterial dient Kieselgel und als Eluent wird ein Gemisch bestehend aus Cyclohexan:Ethylacetat(3:1) verwendet.

| | |
|---|---|
| Ausbeute: | 67 mg (0,325 mmol, 8 %) |
| MS (ESI): | 207 (M+H)⁺ |

### c) 3,3-Dimethyl-7-amino-2,3-dihydro-isoindol-1-on

67 mg (0,325 mmol) 3,3-Dimethyl-7-nitro-2,3-dihydro-isoindol-1-on werden in 50 ml Methanol gelöst und mit 10 mg Palladium auf Kohle versetzt. Man hydriert 16 h bei 4 bar H₂-Druck und Raumtemperatur. Anschließend wird der Katalysator abfiltriert und das Lösungsmittel im Vakuum entfernt.

| | |
|---|---|
| Ausbeute: | 50 mg (0,284 mmol, 93 %) |
| MS (ESI): | 177 (M+H)⁺ |

### d) 7-Dibenzylamino-3,3-dimethyl-2,3-dihydro-isoindol-1-on

50 mg (0,284 mmol) 3,3-Dimethyl-7-amino-2,3-dihydro-isoindol-1-on werden in 0,5 ml DMF gelöst und mit 141 mg (1,021 mmol) Kaliumcarbonat und 10 mg (0,028 mmol) Tetrabutylammoniumjodid versetzt. Man erwärmt diese Mischung auf 50 °C und tropft 155 µl (1,277 mmol) Benzylbromid zu. Nachdem man 16 h bei dieser Temperatur gerührt hat verdünnt man mit Essigester und extrahiert 3mal mit 1 M Salzsäure. Die vereinigten organischen Phasen werden getrocknet und das Lösungsmittel im Vakuum entfernt.

| | |
|---|---|
| Ausbeute: | 67 mg (0,188 mmol; 66 %) |
| MS (ESI): | 357 (M+H)⁺ |

### e) 7-Dibenzylamino-2-(2-fluor-ethyl)-3,3-dimethyl-2,3-dihydro-isoindol-1-on

67 mg (0,188 mmol) 7-Dibenzylamino-3,3-dimethyl-2,3-dihydro-isoindol-1-on werden in 1 ml (7,877 mmol) 1-Brom-2-fluorethan gelöst und mit 52 mg (0,376 mmol) Natriumhydrid versetzt. Nachdem man 4 h bei Raumtemperatur gerührt hat verdünnt man mit Essigester und extrahiert 3mal mit 1 M Salzsäure. Die vereinigten organischen Phasen werden getrocknet und das Lösungsmittel im Vakuum entfernt.

| | |
|---|---|
| Ausbeute: | 75 mg (0,188 mmol; 100 %) |
| MS (ESI): | 403 (M+H)⁺ |

### f) 7-Amino-2-(2-fluor-ethyl)-3,3-dimethyl-2,3-dihydro-isoindol-1-on

75 mg (0,188 mmol) 7-Dibenzylamino-2-(2-fluor-ethyl)-3,3-dimethyl-2,3-dihydro-isoindol-1-on werden in 50 ml Methanol gelöst und mit 10 mg Palladium auf Kohle versetzt. Man hydriert 16 h bei 5 bar H₂-Druck und Raumtemperatur. Anschließend wird der Katalysator abfiltriert und das Lösungsmittel im Vakuum entfernt.

| | |
|---|---|
| Ausbeute: | 36 mg (0,162 mmol, 87 %) |
| MS (ESI): | 223 (M+H)⁺ |

### Beispiele 1

### 2-(2-Methoxy-4-N-propylcarbamoyl-phenylamino)-4-(3-oxo-2,3-dihydro-1H-isoindol-4-ylamino)-5-trifluormethyl-pyrimidin

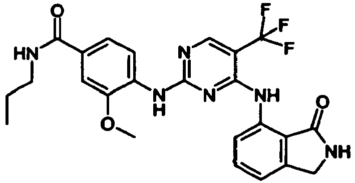

100 mg (0,257 mmol) 2-(2-Methoxy-4-propylcarbamoyl-phenylamino)-4-chlor-5-trifluormethyl-pyrimidin (Methode 1) werden in 1 ml *N,N*-Dimethylacetamid gelöst und mit 83 mg (0,565 mmol) 7-Amino-2,3-dihydro-isoindol-1-on versetzt (Methode 2). Diesem Reaktionsgemisch werden 48 µl einer 4 molaren Lösung von HCl(0,193 mmol) in 1,4-Dioxan zudosiert. Nach zwei Tagen bei 50 °C wird das Lösungsmittel im Vakuum entfernt. Das Rohprodukt wird durch Säulenchromatographie gereinigt. Als Trägermaterial dient Kieselgel und als Eluent wird Dichlormethan, dem 5% einer Mischung aus 90% Methanol und 10% gesättigter wässriger Ammoniak-Lösung zugefügt sind, verwendet. Das angereicherte Rohprodukt wird nochmals mittels Säulenchromatographie gereinigt. Als Trägermaterial dient C18-RP-Kieselgel und es wird ein Gradient durchlaufen der am Startpunkt aus 80% Wasser und 20% Acetonitril und am Endpunkt aus 60% Wasser und 40% Acetonitril besteht.

| | |
|---|---|
| Ausbeute: | 42 mg (0,084 mmol; 33 %) |
| UV max: | 318 nm |
| MS (ESI): | 501 (M+H)⁺ |
| ¹H-NMR: | 0.92 (t, 3H), 1.51-1.63 (m, 2H), 3.21 - 3.29 (m, 2H), 3.86 (s, 3H), 4.37 (s, 2H), 7.14 - 7.21 (m, 1H), 7.33 (t, 1H), 7.47 - 7.54 (m, 1H), 7.55 - 7.60 (m, 1H), 7.73 - 7.82 (m, 1H), 8.35 - 8.50 (m, 3H), 8.75 (s, 1H), 9.09 (s, 1H), 10.66 (s, 1H) |

### Beispiele 2-17

Die folgenden Verbindungen sind über ein analoges Verfahren, wie in Beispiel 1 beschrieben, hergestellt Dabei wird 2-(2-Methoxy-4-propylcarbamoyl-phenylamino)-4-chlor-5-trifluormethylpyrimidin und ein entsprechendes 7-Amino-2,3-dihydro-isoindol-1-on Derivat (Methode 2) verwendet. Als Lösungsmittel wird *N-*Methyl-2-pyrrolidinon oder *N,N*-Dimethylacetamid verwendet.

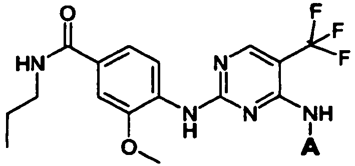

| **#** | **A** | **UV max [nm]** | **MS (ESI) (M+H)⁺** |
|---|---|---|---|
| 2 | 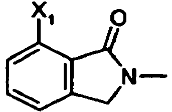 | 322 | 515 |
| 3 | 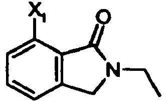 | 314 | 529 |
| 4 | 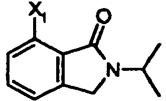 | 285 | 543 |
| 5 | 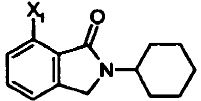 | 286/310 | 583 |
| 6 | 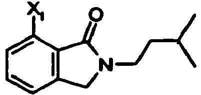 | 322 | 571 |
| 7 | 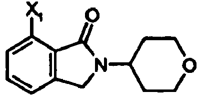 | 285/321 | 585 |
| 8 | 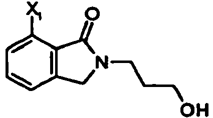 | 285/318 | 559 |
| 9 | 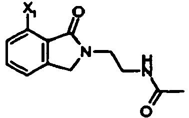 | 285/318 | 586 |
| 10 | 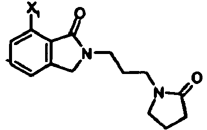 | 281/316 | 626 |
| 11 | 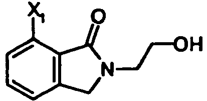 | 284/316 | 545 |
| 12 | 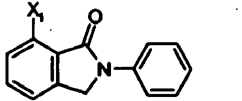 | 325 | 577 |
| 13 | 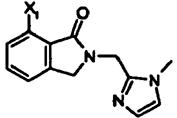 | 282/318 | 595 |
| 14 | 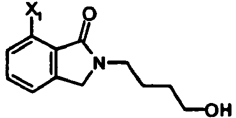 | 284/322 | 573 |
| 15 | 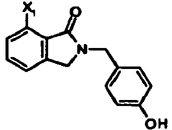 | 286,306 | 607 |
| 16 | 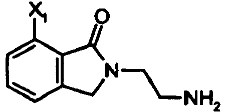 | 325 | |
| 17 | 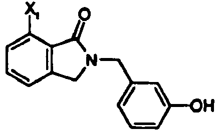 | 318/282 | 607 |

### Beispiel 18

### 2-(2-Methoxy-4-N-propylcarbamoyl-phenylamino)-4-(3-oxo-1,3-dihydro-isobenzofuran-4-ylamino)-5-trifluormethyl-pyrimidin

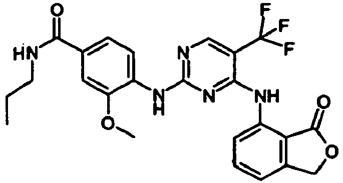

100 mg (0,257 mmol) 2-(2-Methoxy-4-propylcarbamoyl-phenylamino)-4-chlor-5-trifluormethyl-pyrimidin (Methode 1) werden in 1 ml *N,N-*Dimethylacetamid gelöst und mit 46 mg (0,308 mmol) 7-Amino-3*H*-isobenzofuran-1-on (Safdar Hayat et al., Tetrahedron Lett 2001, 42(9):1647-1649) versetzt. Diesem Reaktionsgemisch werden 48 µl einer 4 molaren Lösung von HCl (0,193 mmol) in 1,4-Dioxan zudosiert. Nach 4 Tagen bei 50 °C wird das Lösungsmittel im Vakuum entfernt. Das Rohprodukt wird durch Säulenchromatographie gereinigt. Als Trägermaterial dient Kieselgel und als Eluent wird Dichlormethan, dem 4% einer Mischung aus 90% Methanol und 10% gesättigter wässriger Ammoniak-Lösung zugefügt sind, verwendet.

| | |
|---|---|
| Ausbeute: | 26 mg (0,051 mmol; 20 %) |
| UV max: | 322 nm |
| MS (ESI): | 502 (M+H)⁺ |
| ¹H-NMR: | 0.92 (t, 3H), 1.51 - 1.63 (m, 2H), 3.22 - 3.28 (m, 2H), 3.86 (s, 3H), 5.42 (s, 2H), 7.24 - 7.30 (m, 1H), 7.44 - 7.55 (m, 2H), 7.55 - 7.60 (m, 1H), 7.67 - 7.78 (m, 1H), 8.38 - 8.48 (m, 2H), 8.50 (s, 1H), 9.21 (s, 1H), 9.64 (s, 1H) |

### Beispiele 19-37

Die folgenden Verbindungen sind über analoge Verfahren, wie in Beispiel 1 und Beispiel 18 beschrieben, hergestellt. Dabei wird 2-(2-Methoxy-4-propylcarbamoyl-phenylamino)-4-chlor-5-trifluormethylpyrimidin (Methode 1) verwendet. Das entsprechende Anilin-Derivat ist kommerziell erhältlich, literaturbekannt oder wird nach in Methode 2 und 4 bis 9 beschriebenen Verfahren hergestellt. Als Lösungsmittel wird *N*-Methyl-2-pyrrolidinon oder *N,N-*Dimethylacetamid verwendet.

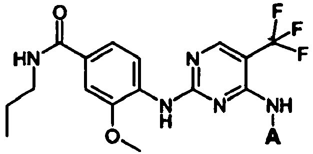

| **#** | **A** | **UV max [nm]** | **MS (ESI) (M+H)⁺** |
|---|---|---|---|
| 19 | 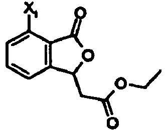 | 235 | 586 |
| 20 | 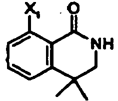 | 323/226 | 543 |
| 21 | 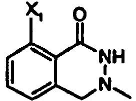 | 325 | 530 |
| 22 | 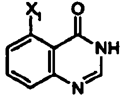 | 262 | 514 |
| 23 | 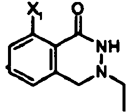 | 320 | 544 |
| 24 | 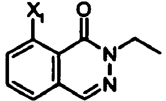 | 318 | 542 |
| 25 | 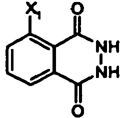 | 312 | 530 |
| 26 | 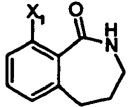 | 315 | 529 |
| 27 | 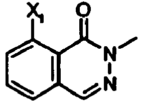 | 314 | 528 |
| 28 | 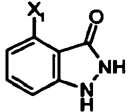 | 317 | 502 |
| 29 | 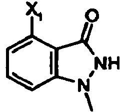 | 316 | 516 |
| 30 | 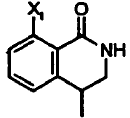 | 322 | 529 |
| 31 | 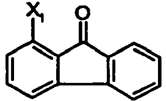 | 255 | 548 |
| 32 | 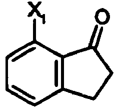 | 320 | 500 |
| 33 | 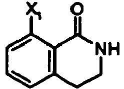 | 325 | 515 |
| 34 | 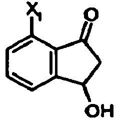 | 250/286/ 318 | 516 |
| 35 | 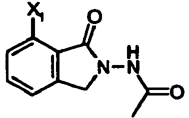 | 320 | 558 |
| 36 | 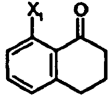 | 316 | 514 |
| 37 | 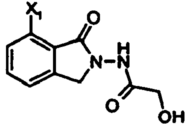 | 321 | |

### Bespiel 38

### 2-(2-Methoxy-4-N-propylcarbamoyl-phenylamino)-4-(4-methyl-5-oxo-2,3,4,5-tetrahydro-benzo[f][1,4]oxazepin-6-ylamino)-5-trifluormethyl-pyrimidin

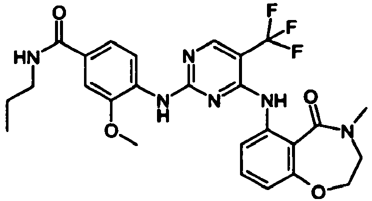

50 mg (0,129 mmol) 2-(2-Methoxy-4-propylcarbamoyl-phenylamino)-4-chlor-5-trifluor-methyl-pyrimidin (Methode 1) werden in 200 µl 1,4-Dioxan gelöst und mit 25 mg (0,13 mmol) 6-Amino-4-methyl-3,4-dihydro-2*H*-benzo[f][1,4]oxazepin-5-on (Methode 10) versetzt. Diesem Reaktionsgemisch werden 36 µl einer 4 molaren Lösung von HCl (0,144 mmol) in 1,4-Dioxan zudosiert. Nach 4 Tagen bei 50 °C wird das Lösungsmittel im Vakuum entfernt. Das Rohprodukt wird durch Säulenchromatographie gereinigt. Als Trägermaterial dient Kieselgel und als Eluent wird ein Gemisch bestehend aus Dichlormethan und Essigester verwendet (1:1).

| | |
|---|---|
| Ausbeute: | 23 mg (0,042 mmol; 33 %) |
| UV max: | 318 nm |
| MS (ESI): | 545 (M+H)⁺ |
| ¹H-NMR: | 0.91 (t, 3H), 1.49 - 1.61 (m, 2H), 3.09 (s, 3H), 3.20 - 3.28 (m, 2H), 3.49 (t, 2H), 3.88 (s, 3H), 4.31 (t, 2H), 6.83 - 6.88 (m, 1H), 7.34 - 7.45 (m, 2H), 7.50 - 7.54 (m, 1H), 7.88 - 8.00 (m, 2H), 8.37 - 8.44 (m, 2H), 8.62 (s, 1H), 9.97 (s, 1H) |

### Beispiele 39-52

Die folgenden Verbindungen sind über analoge Verfahren, wie in Beispiel 1 und 18 beschrieben, hergestellt. Dabei wird 2-(2-Methoxy-4-propylcarbamoyl-phenylamino)-4-chlor-5-trifluormethylpyrimidin (Methode 1) verwendet. Das entsprechende Anilin-Derivat ist kommerziell erhältlich, literaturbekannt oder wird nach in Methode 10 und 11 beschriebenen Verfahren hergestellt. Als Lösungsmittel wird *N-*Methyl-2-pyrrolidinon oder *N,N-*Dimethylacetamid verwendet.

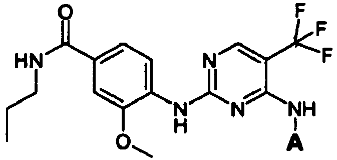

| **#** | **A** | **UV max [nm]** | **MS (ESI) (M+H)⁺** |
|---|---|---|---|
| 39 | 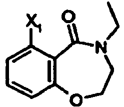 | 229/279/ 315 | 559 |
| 40 | 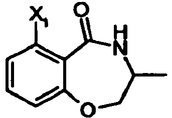 | 282/314 | 545 |
| 41 | 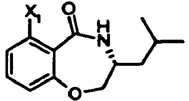 | 282/318 | 587 |
| 42 | 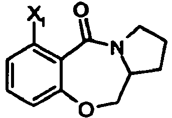 | 282/314 | 571 |
| 43 | 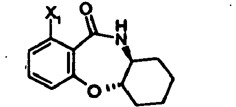 | 282/318 | 585 |
| 44 | 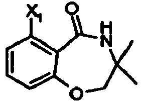 | 318 | 559 |
| 45 | 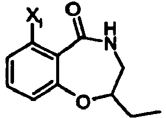 | 234/320 | 559 |
| 46 | 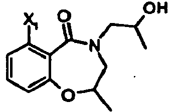 | 282/218 | 603 |
| 47 | 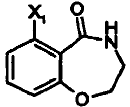 | 278/318 | 531 |
| 48 | 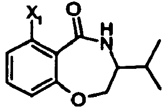 | 286/314 | 573 |
| 49 | 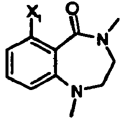 | 274/314 | 558 |
| 50 | 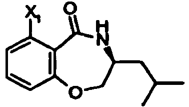 | 318 | 587 |
| 51 | 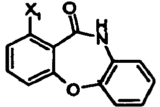 | 223/282/318 | 579 |
| 52 | 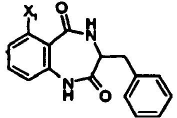 | 318 | 634 |

### Beispiel 53

### 2-[2-Methoxy-4-(4-morpholin-4-yl)-(1,4-trans-cyclohexyl)carbamoyl)-phenylaminol-4-(2-carbamoyl-3-fluor-phenylamino)-5-trifluormethyl-pyrimidin

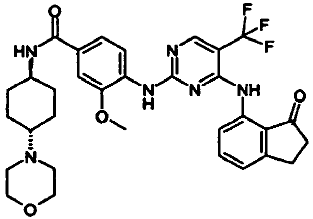

102 mg (0,29 mmol) 2-(4-Carboxyamino-2-methoxy-phenylamino)-4-chlor-5-trifluormethyl-pyrimidin (Methode 12) werden in 250 µl *N-*Methyl-2-pyrrolidinon gelöst und mit 47 mg (0,319 mmol) 7-Amino-indan-1-on (Methode 8) versetzt. Diesem Reaktionsgemisch werden 15 µl einer 4 M Lösung von HCl (0,058 mmol) in 1,4-Dioxan zudosiert. Nach 16 h bei 90°C wird das Reaktionsgemisch in 150 ml einer wässrigen 1 N Salzsäure eingerührt. Der Niederschlag wird abfiltriert und im Vakuum getrocknet. 100 mg (0,174 mmol) dieses Niederschlages, 150 µl (0,875 mmol) *N*-Ethyldiisopropyl-amin, 68 mg (0,210 mmol) *O*-(Benzotriazol-1-yl)-*N,N,N',N'*-tetramethyluronium-tetrafluorborat und 30 mg (0,163 mmol) trans-4-Morpholin-4-yl-clohexylamin (Methode 13) werden in 5 ml *N,N-*Dimethylformamid gelöst. Nach 15 h bei Raumtemperatur wird das Lösungsmittel im Vakuum entfernt. Das Rohprodukt wird durch Säulenchromatographie gereinigt. Als Trägermaterial dient Kieselgel und als Eluent wird Dichlormethan, dem 7% einer Mischung aus 90% Methanol und 10% gesättigter wässriger Ammoniak-Lösung zugefügt sind, verwendet.

| | |
|---|---|
| Ausbeute: | 55 mg (0,100 mmol; 57 %) |
| UV max: | 318 nm |
| MS (ESI): | 555 (M+H)⁺ |
| ¹H-NMR: | 1.55 - 1.69 (m, 2H), 1.74 - 1.84 (m, 2H), 1.91 - 2.02 (m, 2H), 2.18 (s, 3H), 2.69 - 2.75 (m, 2H), 2.75 - 2.84 (m, 2H), 3.03 - 3.10 (m, 2H), 3.70 - 3.83 (m, 1H), 3.86 (s, 3H), 7.15 - 7.21 (m, 1H), 7.36 - 7.46 (m, 1H), 7.48 - 7.54 (m, 1H), 7.54 - 7.58 (m, 1H), 7.71 - 7.79 (m, 1H), 8.18 - 8.25 (m, 1H), 8.30 - 8.45 (m, 1H), 8.48 (s, 1H), 9.16 (s, 1H), 10.59(s, 1H) |

### Beispiele 54-77

Die folgenden Verbindungen sind über ein analoges Verfahren, wie in Beispiel 53 beschrieben, hergestellt. Das entsprechende Anilin ist in Methode 2, 7, 8, oder 9 beschrieben oder literaturbekannt. Das für die Darstellung des Amids eingesetzte Amin ist kommerziell erhältlich oder ist in Methode 13 beschrieben.

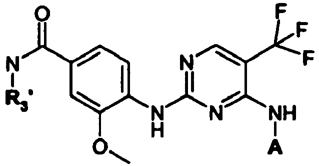

| **#** | **A** | **R₃'** | **UV max [nm]** | **MS (ESI) (M+H)⁺** |
|---|---|---|---|---|
| 54 | 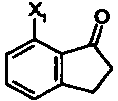 | 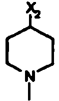 | 318 | 555 |
| 55 | 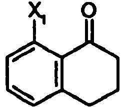 | 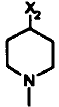 | 318 | 569 |
| 56 | 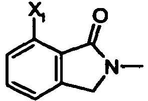 | 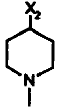 | 322 | 570 |
| 57 | 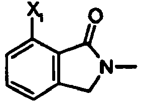 | 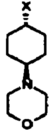 | 320 | 640 |
| 58 | 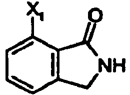 | 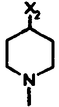 | 284, 322 | 556 |
| 59 | 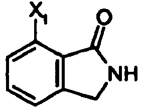 | 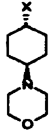 | 282, 318 | 626 |
| 60 | 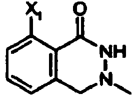 | 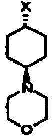 | 325 | 655 |
| 61 | 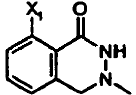 | 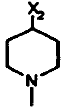 | 325 | 585 |
| 62 | 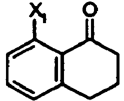 | 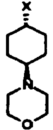 | 254, 286, 318 | 639 |
| 63 | 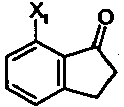 | 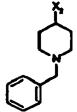 | 321 | 631 |
| 64 | 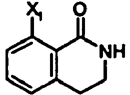 | 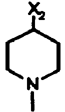 | 322 | 570 |
| 65 | 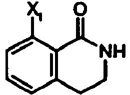 | 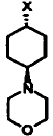 | 322 | 640 |
| 66 | 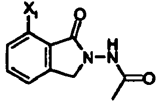 | 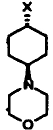 | 322 | 683 |
| 67 | 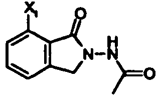 | 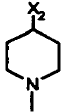 | 322 | 613 |
| 68 | 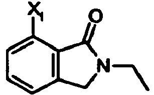 | 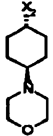 | 286, 322 | 654 |
| 69 | 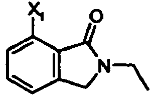 | 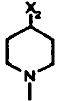 | 286, 322 | 584 |
| 70 | 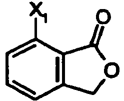 | 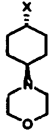 | 282, 322 | 627 |
| 71 | 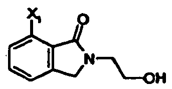 | 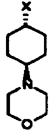 | 322 | 670 |
| 72 | 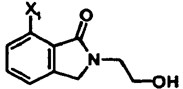 | 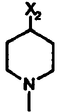 | 286, 322 | 600 |
| 73 | 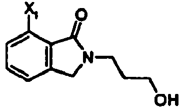 | 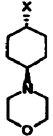 | 322 | 684 |
| 74 | 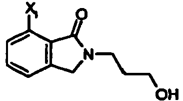 | 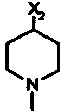 | 286, 322 | 614 |
| 75 | 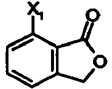 | 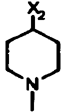 | 322 | 557 |
| 76 | 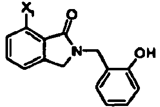 | 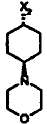 | 330 | 732 |
| 77 | 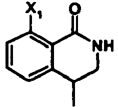 | 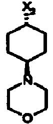 | 325 | 654 |

### Beispiel 78-140

Die folgenden Verbindungen werden über ein analoges Verfahren, wie in Beispiel 53 beschrieben, hergestellt. 2-(4-Carboxy-2-methoxy-phenylamino)-4-chlor-5-trifluormethyl-pyrimidin kann nach Methode 12 beziehungsweise 14 hergestellt werden. Das entsprechende Anilin ist in den Ergänzungen zu Methode 10 beschrieben. Das für die Darstellung des Amids eingesetzte Amin ist kommerziell erhältlich beziehungsweise ist in Methode 13, in den Ergänzungen zu Methode 13, 15 beziehungsweise 25 beschrieben.

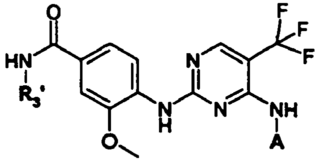

| **#** | **A** | **R₃'** | **UV max [nm]** | **MS (ESI) (M+H)⁺** |
|---|---|---|---|---|
| 78 | 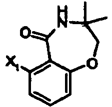 | 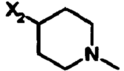 | 318 | 308 |
| 79 | 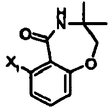 | 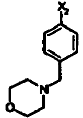 | 326 | 346 |
| 80 | 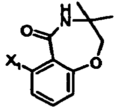 | 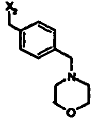 | 318 | 706 |
| 81 | 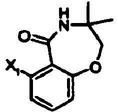 | 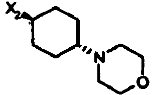 | 318 | 584 |
| 82 | 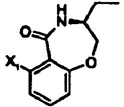 | 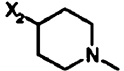 | 318 | 614 |
| 83 | 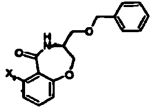 | 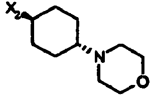 | 318 | 776 |
| 84 | 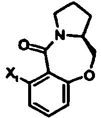 | 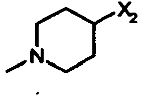 | 318 | 626 |
| 85 | 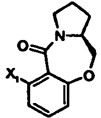 | 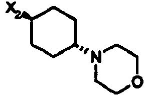 | 318 | 348 |
| 86 | 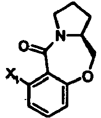 | 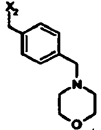 | 318 | 718 |
| 87 | 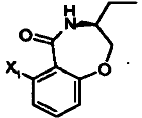 | 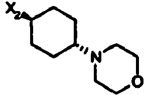 | 318 | 684 |
| 88 | 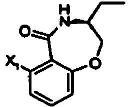 | 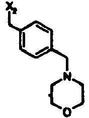 | 318 | 353 |
| 89 | 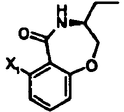 | 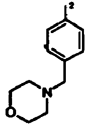 | 322 | 346 |
| 90 | 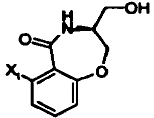 | 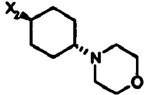 | 318 | 686 |
| 91 | 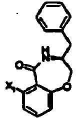 | 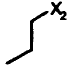 | 310 | 621 |
| 92 | 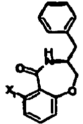 | 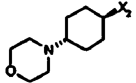 | 318 | 746 |
| 93 | 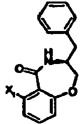 | 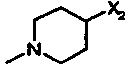 | 318 | 676 |
| 94 | 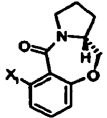 | 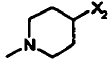 | 318 | 316 |
| 95 | 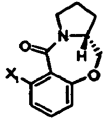 | 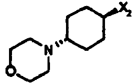 | 318 | 696 |
| 96 | 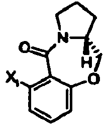 | 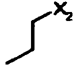 | 282; 310 | 571 |
| 97 | 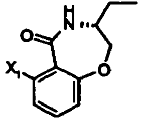 | 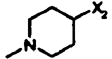 | 318 | 614 |
| 98 | 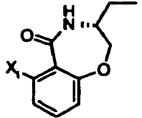 | 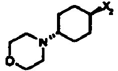 | 318 | 684 |
| 99 | 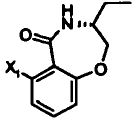 | 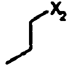 | 315 | 559 |
| 100 | 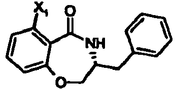 | 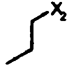 | 314 | 621 |
| 101 | 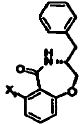 | 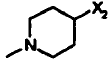 | 314 | 676 |
| 102 | 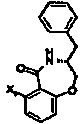 | 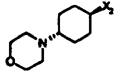 | 318 | 747 |
| 103 | 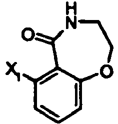 | 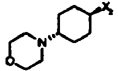 | 318 | 656 |
| 104 | 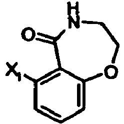 | 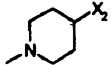 | 318 | 586 |
| 105 | 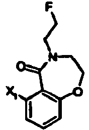 | 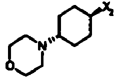 | 318 | (M |
| 106 | 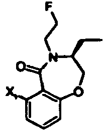 | 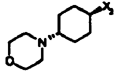 | 318 | 730 |
| 107 | 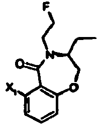 | 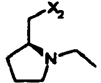 | 322 | 674 |
| 108 | 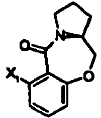 | 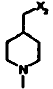 | 318 | 640 |
| 109 | 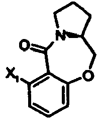 | 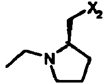 | 322 | 640 |
| 110 | 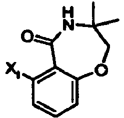 | 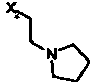 | 282, 318 | 614 |
| 111 | 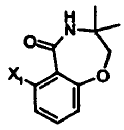 | 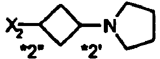 | 226, 282, 318 | 640 |
| 112 | 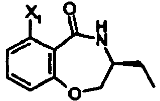 | 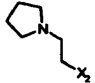 | 318 | 614 |
| 113 | 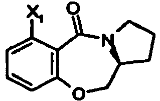 | 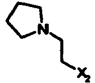 | | 626 |
| 114 | 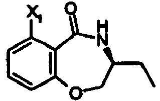 | 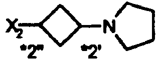 | 318 | 640 |
| 115 | 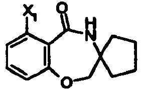 | 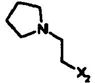 | 318 | 640 |
| 116 | 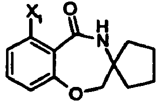 | 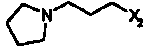 | 318 | 654 |
| 117 | 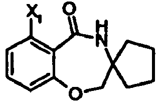 | 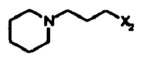 | 318 | 668 |
| 118 | 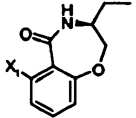 | 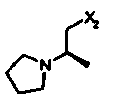 | 318 | 628 |
| 119 | 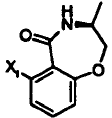 | 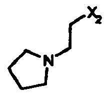 | 318 | 600 |
| 120 | 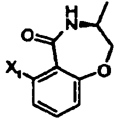 | 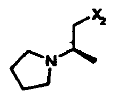 | 318-322 | 614 |
| 121 | 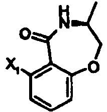 | 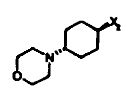 | 318 | 670 |
| 122 | 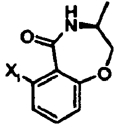 | 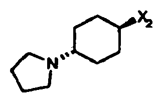 | 318 | 654 |
| 123 | 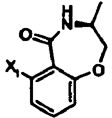 | 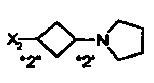 | 318 | 626 |
| 124 | 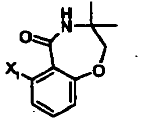 | 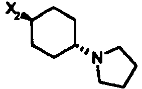 | 282, 318 | 668 |
| 125 | 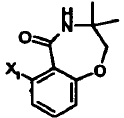 | 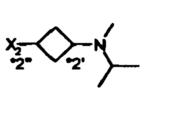 | 282, 318 | 642 |
| 126 | 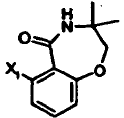 | 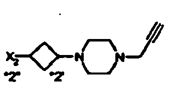 | 282, 318 | 693 |
| 127 | 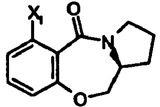 | 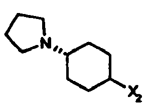 | 318 | 680 |
| 128 | 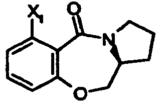 | 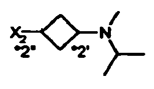 | 318 | 654 |
| 129 | 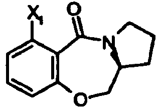 | 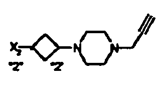 | 318 | 705 |
| 130 | 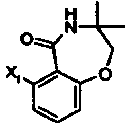 | 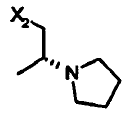 | 226, 282, 318 | 628 |
| 131 | 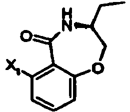 | 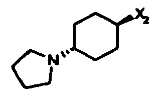 | 318 | 668 |
| 132 | 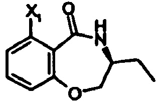 | 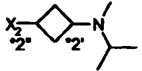 | 318-322 | 642 |
| 133 | 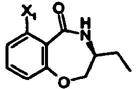 | 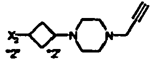 | 318 | 693 |
| 134 | 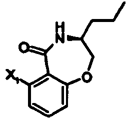 | 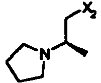 | 318-322 | 642 |
| 135 | 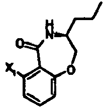 | 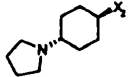 | 318 | 682 |
| 136 | 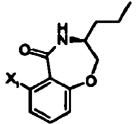 | 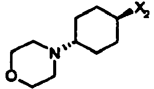 | 318 | 698 |
| 137 | 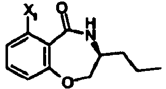 | 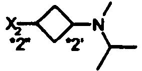 | 318-322 | 656 |
| 138 | 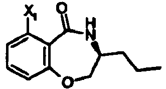 | 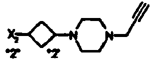 | 318-322 | 707 |
| 139 | 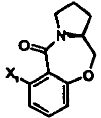 | 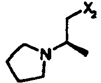 | 318-322 | 640 |
| 140 | 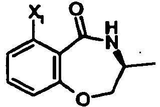 | 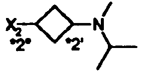 | 318-322 | 628 |

### Beispiele 141-166

Die folgenden Verbindungen sind über ein analoges Verfahren, wie in Beispiel 53 beschrieben, hergestellt. Die Herstellung von 2-(4-Carboxy-phenylamino)-4-chlor-5-trifluormethyl-pyrimidin ist in Methode 14 beschrieben. Das entsprechende Anilin ist in Methode 10 beschrieben. Das für die Darstellung des Amids eingesetzte Amin ist kommerziell erhältlich beziehungsweise ist in Methode 13, 15 beziehungsweise 25 beschrieben.

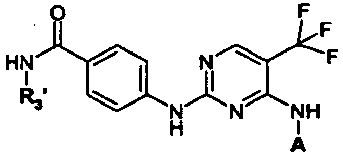

| **#** | **A** | **R₃'** | **UV max [nm]** | **MS(ESI) (M+H)⁺** |
|---|---|---|---|---|
| 141 | 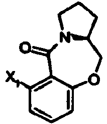 | 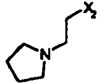 | 302 | 596 |
| 142 | 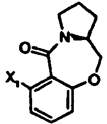 | 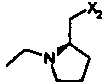 | 302 | 610 |
| 143 | 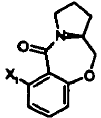 | 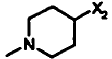 | 302 | 596 |
| 144 | 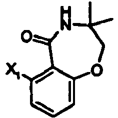 | 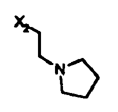 | 302 | 584 |
| 145 | 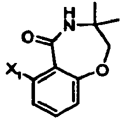 | 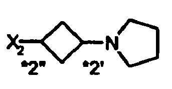 | 302 | 610 |
| 146 | 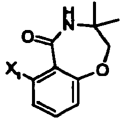 | 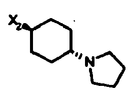 | 302 | 638 |
| 147 | 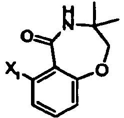 | 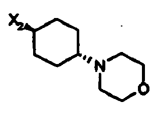 | 298 | 654 |
| 148 | 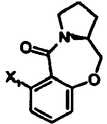 | 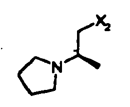 | 302 | 610 |
| 149 | 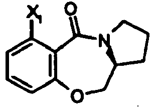 | 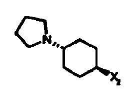 | 302 | 650 |
| 150 | 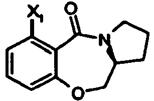 | 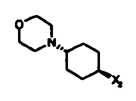 | 298-302 | 666 |
| 151 | 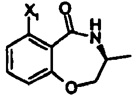 | 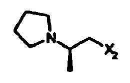 | 302 | 584 |
| 152 | 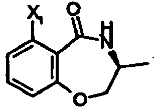 | 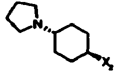 | 302 | 624 |
| 153 | 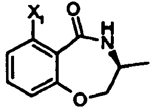 | 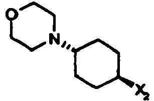 | 298-302 | 640 |
| 154 | 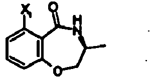 | 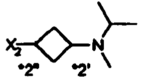 | 302 | 598 |
| 155 | 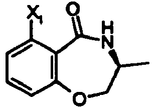 | 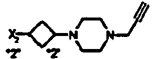 | 298-302 | 649 |
| 156 | 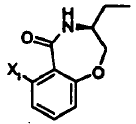 | 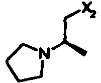 | 302 | 598 |
| 157 | 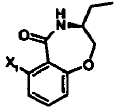 | 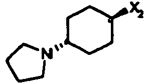 | 302 | 638 |
| 158 | 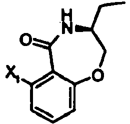 | 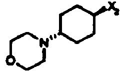 | 298-302 | 654 |
| 159 | 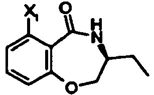 | 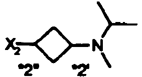 | 302 | 612 |
| 160 | 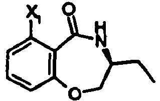 | 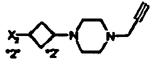 | 302 | 663 |
| 161 | 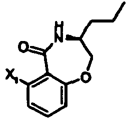 | 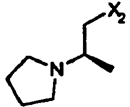 | 302 | 612 |
| 162 | 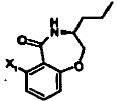 | 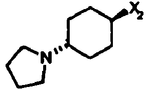 | 302 | 652 |
| 163 | 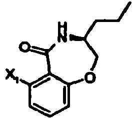 | 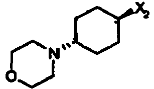 | 298-302 | 668 |
| 164 | 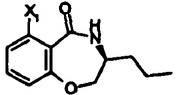 | 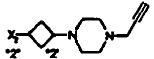 | 302 | 677 |
| 165 | 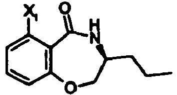 | 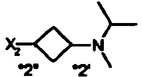 | 302 | 626 |
| 166 | 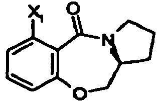 | 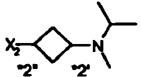 | 302 | 624 |

### Beispiel 167

### 2-(2-Methoxy-4-piperazin-1-yl-phenylamino)-4-(3,3-dimethyl-5-oxo-2,3,4,5-tetrahydro-benzo[f][1,4]oxazepin-6-ylamino)-5-trifluormethyl-pyrimidin

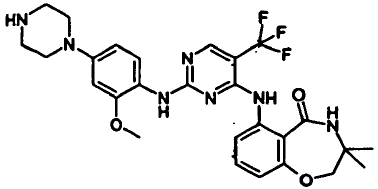

500 mg (0,958 mmol) 2-[4-(4-Benzyloxycarbonyl-piperazin-1-yl)-phenylamino]-4-chlor-5-trifluormethyl-pyrimidin (Methode 14) werden in 0,5 ml NMP gelöst, mit 198 mg(0,960 mmol) 6-Amino-3,3-dimethyl-3,4-dihydro-2*H*-benzo[*f*][1,4]oxazepin-5-on (Methode 10) und mit 25 µl (0,1 mmol) dioxanischer Salzsäure versetzt. Diese Reaktionsmischung wird 1,5 h bei 100 °C gerührt. Das Lösungsmittel wird im Vakuum entfernt und der Rückstand wird mittels Säulenchromatographie gereinigt. Als Trägermaterial dient C18-RP-Kieselgel und es wird ein Gradient durchlaufen der am Startpunkt aus 95% Wasser und 5% Acetonitril und am Endpunkt aus 5% Wasser und 95% Acetonitril besteht. Sowohl dem Wasser als auch dem Acetonitril sind jeweils 0,1% Ameisensäure beigemischt.

| | |
|---|---|
| Ausbeute: | 0,59 g (0,86 mmol; 90 %) |

0,59 g (0,86 mmol) des oben genannten Zwischenprodukts werden in 50 ml Dimethylformamid gelöst und mit soviel destilliertem Wasser versetzt, dass gerade nichts ausfüllt. Zu dieser Lösung gibt man 60 mg Palladium auf Kohle und hydriert bei 7 bar H₂-Druck und 20°C 6 h lang. Der Katalysator wird abfiltriert und das Lösungsmittel im Vakuum entfernt. Der Rückstand wird mittels Säulenchromatographie gereinigt. Als Trägermaterial dient C18-RP-Kieselgel und es wird ein Gradient durchlaufen der am Startpunkt aus 60% Wasser und 40% Acetonitril und am Endpunkt aus 15% Wasser und 85% Acetonitril besteht. Im Wasser ist 10 mmol/l Ammoniumhydrogencarbonat und 20 mmol/l Ammoniak gelöst. Die geeigneten Fraktionen werden gefriergetrocknet. Der Rückstand wird in Acetonitril gelöst und mit 2 ml einer 1 M Salzsäurelösung versetzt. Anschließend wird das Lösungsmittel im Vakuum entfernt. Die Substanz liegt als Dihydrochlorid vor.

| | |
|---|---|
| Ausbeute: | 0,46 g (0,73 mmol; 85 %) |
| UV max: | 284 nm |
| MS (ESI): | 558 (M+H)⁺ |

| | |
|---|---|
| ¹H-NMR: | 1.19 (s, 6H), 3.19 - 3.28 (m, 4H), 3.41 - 3.49 (m, 4H), 3.80(s, 3H), 4.07 (s, 1H), 6.54 - 6.60 (m, 1H), 6.72 - 6.76 (m, 1H), 6.83 - 6.89 (m, 1H), 7.21 - 7.42 (m, 2H), 7.85 - 8.20 (m, 1H), 8.33 - 8.60 (m, 1H), 8.74 (s, 1H), 9.30 - 9.71 (m, 3H), 12.84 (s, 1H) |

### Beispiel 168

### 2-(2-Methoxy-4-piperazin-1-yl-phenylamino)-4-((S)-4-oxo-2,3,10,10a-tetrahydro-1H,4H-9-oxa-3a-aza-benzo[f]azulen-5-ylamino-5-trifluormethyl-pyrimidin

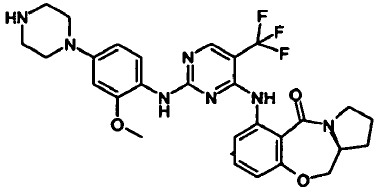

Diese Verbindung wird analog zu Beispiel 167 hergestellt. Das eingesetzte Anilin ist in Methode 10 beschrieben.

| | |
|---|---|
| Ausbeute: | 0,23 g (0,41 mmol; 91 %) |
| UV max: | 282 nm |
| MS (ESI): | 570 (M+H)⁺ |
| ¹H-NMR: | 1,53-1,71 (m, 1H), 1,79-2,06 (m, 3H), 3,15-3,32 (m, 4H), 3,32-3,55 (m, 5H), 3,58-3,72 (m, 1H), 3,72-3,94 (m, 4H), 4,00-4,23 (m, 2H), 6,48-6,61 (m, 1H), 6,68-6,77 (m, 1H), 6,83-7,00 (m, 1H), 7,19-7,50(m, 2H), 7,78-8,10 (m, 1H), 8,23-8,60 (m, 1 H), 9,18-9,64 (m, 3H), 10,54-10,86 (m, 1H) |

### Beispiel 169

### 2-[4-(4-Ethyl-piperazin-1-yl)-2-methoxy-phenylamino]-4-((S)-4-oxo-2,3,10,10a-tetrahydro-1H,4H-9-oxa-3a-aza-benzo[f]azulen-5-ylamino-5-trifluormethyl-pyrimidin

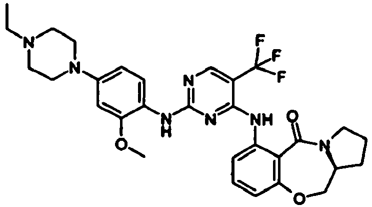

60 mg (0,11 mmol) 2-(2-Methoxy-4-piperazin-1-yl-phenylamino-4-((S)-4-oxo-2,3,10,10a-tetrahydro-1*H*,4*H*-9-oxa-3a-aza-benzo[*f*]azulen-5-ylamino-5-trifluormethyl-pyrimidin (Beispiel 168) werden in 300 µl Dimethylformamid gelöst und mit 12 µl(0,21 mmol) Acetaldehyd und 47 mg (0,21 mmol) Natriumtriacetoxyborhydrid versetzt. Diese Reaktionsmischung rührt man bei 20°C 20 h. Das Lösungsmittel wird im Vakuum entfernt und der Rückstand wird mittels Säulenchromatographie gereinigt. Als Trägermaterial dient C18-RP-Kieselgel und es wird ein Gradient durchlaufen der am Startpunkt aus 95% Wasser und 5% Acetonitril und am Endpunkt aus 50% Wasser und 50% Acetonitril besteht. Sowohl dem Wasser als auch dem Acetonitril sind jeweils 0,1% Ameisensäure beigemischt. Die geeigneten Fraktionen werden mit 500 µl einer 1 N Salzsäure versetzt und gefriergetrocknet. Das Produkt liegt als Dihydrochlorid vor.

| | |
|---|---|
| Ausbeute: | 49 mg (0,074 mmol; 71 %) |
| UV max: | 282 nm |
| MS (ESI): | 598 (M+H)⁺ |
| ¹H-NMR: | 1,23-1,37 (m, 3H), 1,57-1,72 (m, 1H), 1,80-2,06 (m, 3H), 3,02-3,27 (m, 6H), 3,34-3,48 (m, 1H), 3,48-3,71 (m, 3H), 3,71-3,94 (m, 7H), 6,48-6,61 (m, 1H), 6,68-6,79 (m, 1H), 6,84-6,97 (m, 1H), 7,18-7,43 (m, 2H), 7,78-8,08 (m, 1H), 8,26-8,53 (m, 1H), 9,14-9,44 (m, 1H), 10,49-10,74 (m, 1H), 10,80-11,08 (m, 1H) |

### Beispiel 170

### 2-[4-(4-Methyl-piperazin-1-yl)-2-methoxy-phenylamino]-4-((S)-4-oxo-2,3,10,10a-tetrahydro-1H,4H-9-oxa-3a-aza-benzo[f]azulen-5-ylamino-5-trifluormethyl-pyrimidin

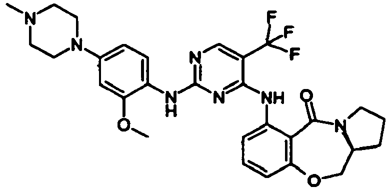

Zur Herstellung dieser Verbindung wird anstelle von Acetaldehyd Formaldehyd eingesetzt. Ansonsten wird analog zu Beispiel 169 verfahren.

| | |
|---|---|
| Ausbeute: | 16 mg (0,024 mmol; 28 %) |
| UV max: | 278 nm |
| MS (ESI): | 584 (M+H)⁺ |
| ¹H-NMR: | 1,58-1,71 (m, 1H), 1,81-2,06 (m, 3H), 2,78-2,88 (m, 3H), 3,00-3,23 (m, 4H), 4,03-4,21 (m, 2H), 6,48-6,59 (m, 1H), 6,69-6,78 (m, 1H), 6,80-6,91 (m, 1H), 7,17-7,44 (m, 2H), 7,92-8,15 (m, 1H), 8,34 (s, 1H), 8,86-9,04 (m, 1H), 10,38-10,64 (m, 2H) |

### Beispiel 171-180

Analog zu Beispiel 169 und 170 werden die folgenden Beispiele hergestellt. Das entsprechende Anilin ist in den Ergänzungen zu Methode 10 beschrieben.

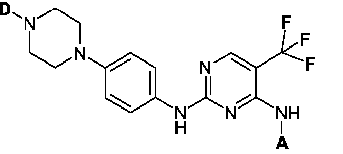

| **#** | **A** | **D** | **UV max [nm]** | **MS (ESI) (M+H)⁺** |
|---|---|---|---|---|
| 171 | 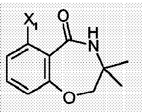 | 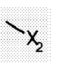 | 226, 282 | 572 |
| 172 | 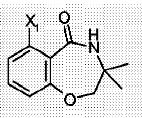 | 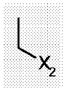 | 250, 282 | 586 |
| 173 | 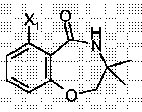 | 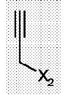 | 250, 282 | 596 |
| 174 | 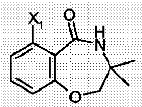 | 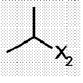 | 250, 282 | 600 |
| 175 | 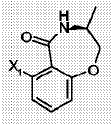 | 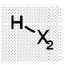 | 282 | 544 |
| 176 | 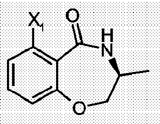 | 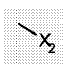 | 282 | 558 |
| 177 | 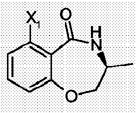 | 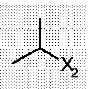 | 218; 282 | 586 |
| 178 | 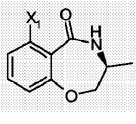 | 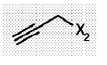 | 282 | 582 |
| 179 | 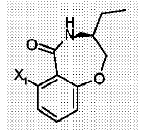 | 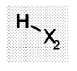 | 226 | 558 |
| 180 | 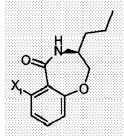 | 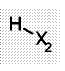 | 226 | 572 |

### Beispiele 181-332

Die folgenden Verbindungen sind über ein analoges Verfahren, wie in Beispiel 53 beschrieben, hergestellt. 2-(4-Carboxy-2-methoxy-phenylamino)-4-chlor-5-trifluormethyl-pyrimidin ist nach Methode 12 beziehungsweise 14 zugänglich. Das entsprechende Anilin ist in Methode 11 beschrieben. Das für die Darstellung des Amids eingesetzte Amin ist kommerziell erhältlich beziehungsweise in Methode 13,15 und 25 beschrieben.

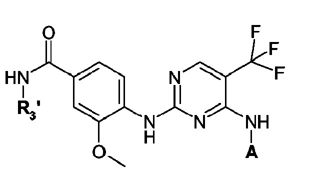

| **#** | **A** | **R₃'** | **UV max [nm]** | **MS (ESI) (M+H)⁺** |
|---|---|---|---|---|
| 181 | 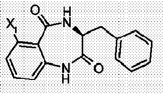 | 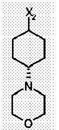 | 318, 282, 234 | 380 |
| 182 | 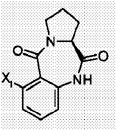 | 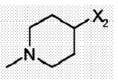 | 238 | 639 |
| 183 | 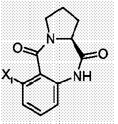 | 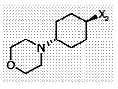 | 234; 318 | 709 |
| 184 | 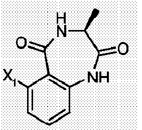 | 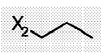 | 318, 282, 248 | 558 |
| 185 | 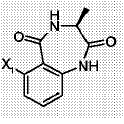 | 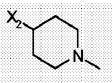 | 318, 280 | 613 |
| 186 | 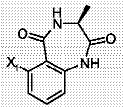 | 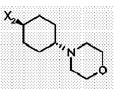 | 316, 282, 234 | 342 |
| 187 | 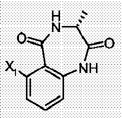 | 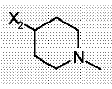 | 318, 284, 238 | 307 |
| 188 | 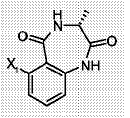 | 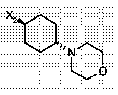 | 318, 282, 242 | 342 |
| 189 | 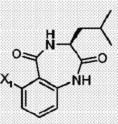 | 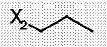 | 314, 282, 242 | 600 |
| 190 | 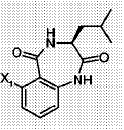 | 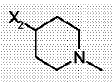 | 318, 282, 234 | 328 |
| 191 | 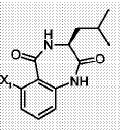 | 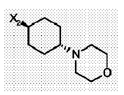 | 318 | 363 |
| 192 | 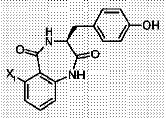 | 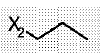 | 318, 230 | 650 |
| 193 | 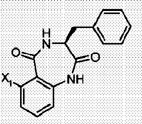 | 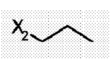 | 314 | 634 |
| 194 | 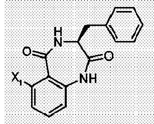 | 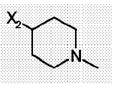 | 318 | 634 |
| 195 | 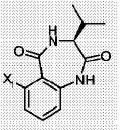 | 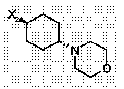 | 318 | 671 |
| 196 | 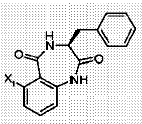 | 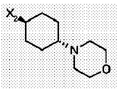 | 318, 230 | 380 |
| 197 | 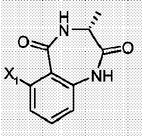 | 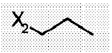 | 314, 282, 250 | 558 |
| 198 | 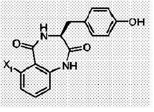 | 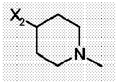 | 319 | 705 |
| 199 | 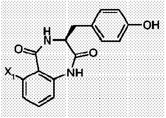 | 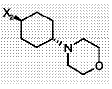 | 318, 226 | 775 |
| 200 | 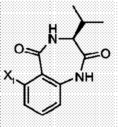 | 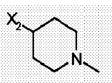 | 318 | 634 |
| 201 | 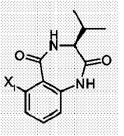 | 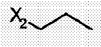 | 314 | 634 |
| 202 | 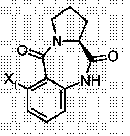 | 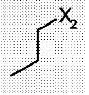 | 230; 318 | 584 |
| 203 | 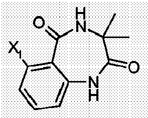 | 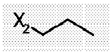 | 317 | 572 |
| 204 | 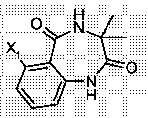 | 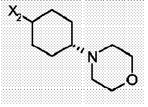 | 318, 230 | 697 |
| 205 | 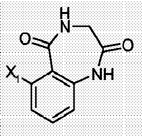 | 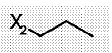 | 318, 234 | 544 |
| 206 | 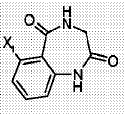 | 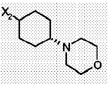 | 318 | 669 |
| 207 | 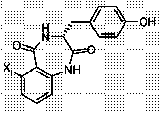 | 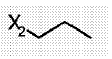 | 318, 230 | 650 |
| 208 | 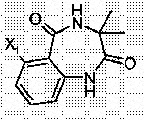 | 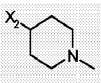 | 317 | 627 |
| 209 | 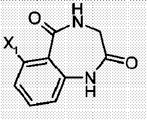 | 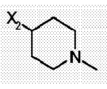 | 318, 230 | 599 |
| 210 | 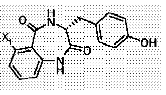 | 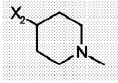 | 318, 230 | 705 |
| 211 | 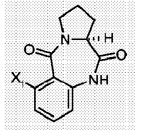 | 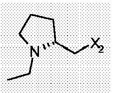 | 230; 322 | 653 |
| 212 | 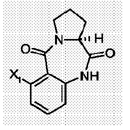 | 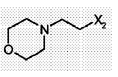 | 230; 322 | 655 |
| 213 | 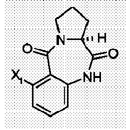 | 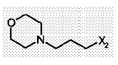 | 230; 318 | 669 |
| 214 | 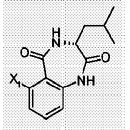 | 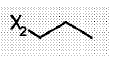 | 230, 282, 314 | 634 |
| 215 | 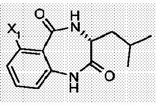 | 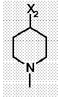 | 318 | 655 |
| 216 | 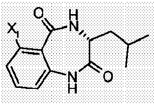 | 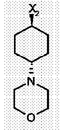 | 318, 234 | 725 |
| 217 | 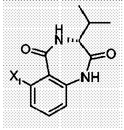 | 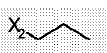 | 314, 235 | 586 |
| 218 | 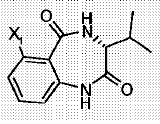 | 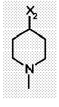 | 318, 230 | 641 |
| 219 | 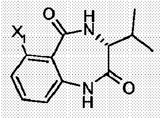 | 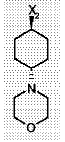 | 318, 226 | 711 |
| 220 | 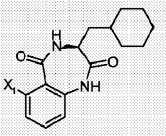 | 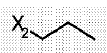 | 318, 230 | 640 |
| 221 | 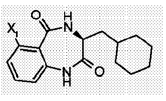 | 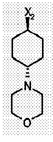 | 318 | 765 |
| 222 | 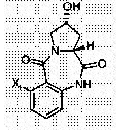 | 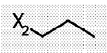 | 318 | 600 |
| 223 | 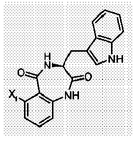 | 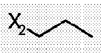 | 315 | 673 |
| 224 | 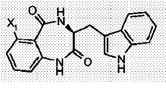 | 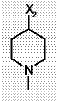 | 319, 226 | 728 |
| 225 | 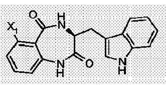 | 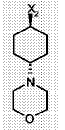 | 318, 226 | 798 |
| 226 | 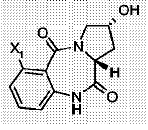 | 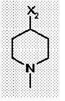 | 318, 234 | 655 |
| 227 | 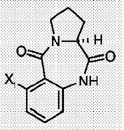 | 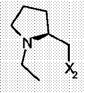 | 230; 322 | 653 |
| 228 | 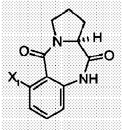 | 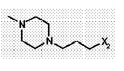 | 230; 318 | 682 |
| 229 | 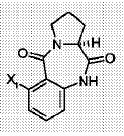 | 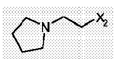 | 234; 318 | 639 |
| 230 | 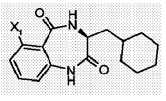 | 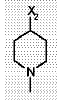 | 318, 226 | 695 |
| 231 | 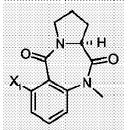 | 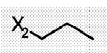 | 234, 282, 318 | 598 |
| 232 | 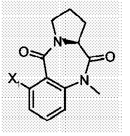 | 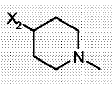 | 230, 282, 318 | 653 |
| 233 | 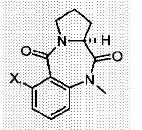 | 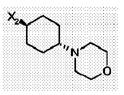 | 234, 282, 318 | 723 |
| 234 | 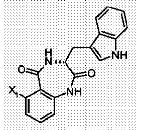 | 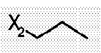 | 318, 222 | 673 |
| 235 | 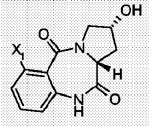 | 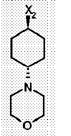 | 318 | 725 |
| 236 | 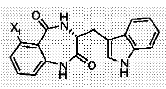 | 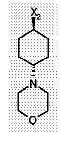 | 318, 282, 226 | 798 |
| 237 | 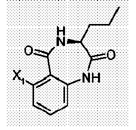 | 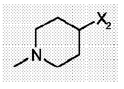 | 230; 318 | 641 |
| 238 | 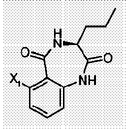 | 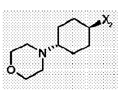 | 230; 318 | 711 |
| 239 | 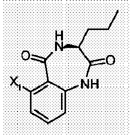 | 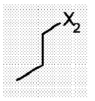 | 234; 318 | 586 |
| 240 | 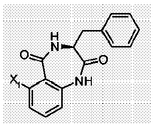 | 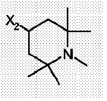 | 318, 226 | 745 |
| 241 | 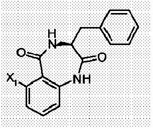 | 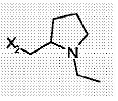 | 322 | 703 |
| 242 | 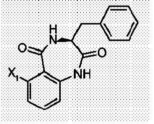 | 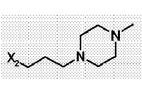 | 320, 226 | 732 |
| 243 | 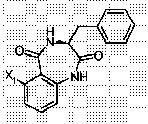 | 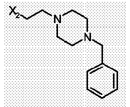 | 321, 221 | 694 |
| 244 | 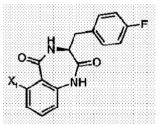 | 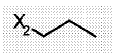 | 230, 282, 318 | 652 |
| 245 | 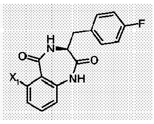 | 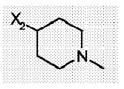 | 234, 282, 318 | 707 |
| 246 | 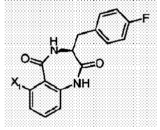 | 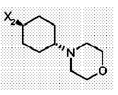 | 230, 282, 318 | 777 |
| 247 | 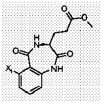 | 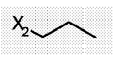 | 230, 282, 318 | 630 |
| 248 | 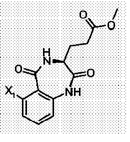 | 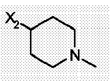 | 234, 282, 318 | 685 |
| 249 | 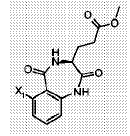 | 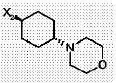 | 234, 282, 318 | 755 |
| 250 | 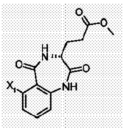 | 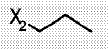 | 230, 282, 318 | 630 |
| 251 | 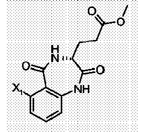 | 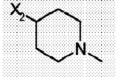 | 230, 282, 318 | 685 |
| 252 | 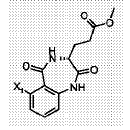 | 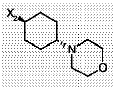 | 230, 282, 318 | 755 |
| 253 | 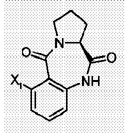 | 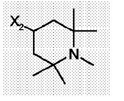 | 230; 318 | 695 |
| 254 | 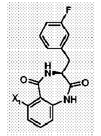 | 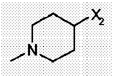 | 230; 318 | 70 |
| 255 | 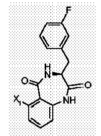 | 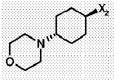 | 230; 318 | 389 |
| 256 | 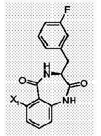 | 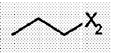 | 230; 318 | 652 |
| 257 | 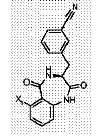 | 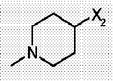 | 230 | 357 |
| 258 | 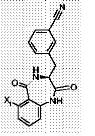 | 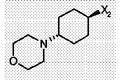 | 230 | 784 |
| 259 | 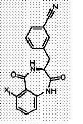 | 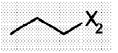 | 230 | 659 |
| 260 | 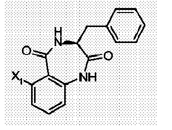 | 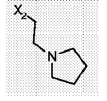 | 319, 230 | 689 |
| 261 | 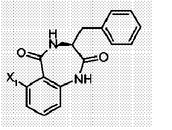 | 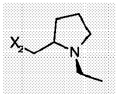 | 322 | 703 |
| 262 | 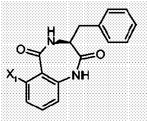 | 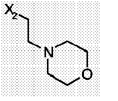 | 322 | 705 |
| 263 | 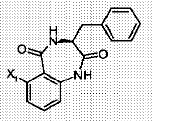 | 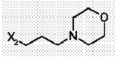 | 320 | 719 |
| 264 | 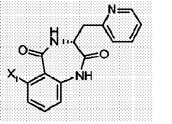 | 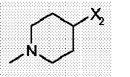 | 226 | 690 |
| 265 | 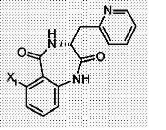 | 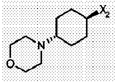 | 226; 318 | 760 |
| 266 | 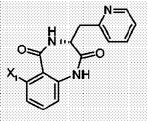 | 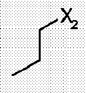 | 230 | 635 |
| 267 | 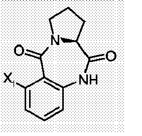 | 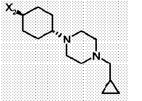 | 230; 318 | 381 |
| 268 | 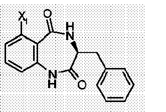 | 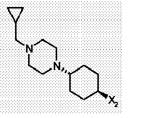 | 318 | 812 |
| 269 | 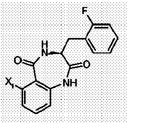 | 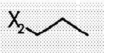 | 318 | 652 |
| 270 | 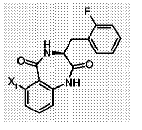 | 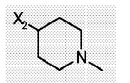 | 318 | 707 |
| 271 | 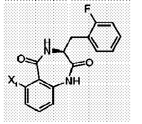 | 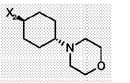 | 318, 226 | 777 |
| 272 | 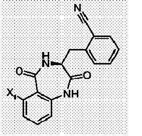 | 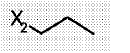 | 318 | 659 |
| 273 | 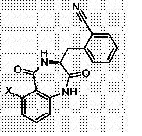 | 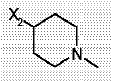 | 318 | 714 |
| 274 | 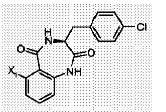 | 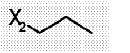 | 315, 239 | 669 |
| 275 | 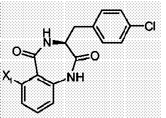 | 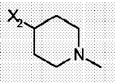 | 319, 222 | 723 |
| 276 | 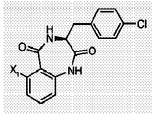 | 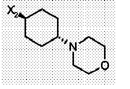 | 318, 226 | 793 |
| 277 | 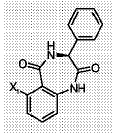 | 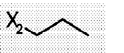 | 316 | 620 |
| 278 | 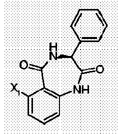 | 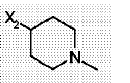 | 318 | 675 |
| 279 | 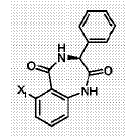 | 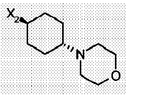 | 318, 226 | 745 |
| 280 | 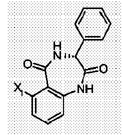 | 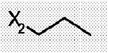 | 317, 226 | 620 |
| 281 | 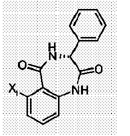 | 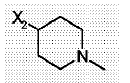 | 318 | 675 |
| 282 | 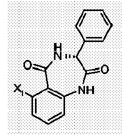 | 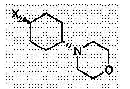 | 318, 230 | 745 |
| 283 | 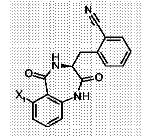 | 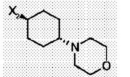 | 318 | 784 |
| 284 | 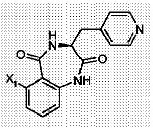 | 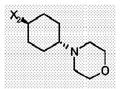 | 318 | 758 |
| 285 | 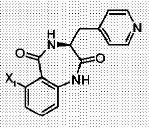 | 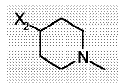 | 318 | 688 |
| 286 | 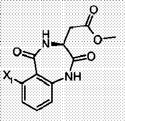 | 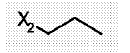 | 238, 282, 314 | 616 |
| 287 | 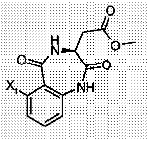 | 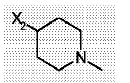 | 230, 282, 318 | 671 |
| 288 | 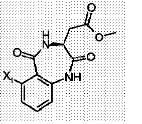 | 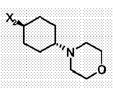 | 230, 282, 318 | 741 |
| 289 | 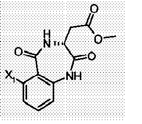 | 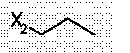 | 234, 282, 318 | 616 |
| 290 | 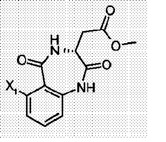 | 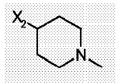 | 226, 282, 318 | 671 |
| 291 | 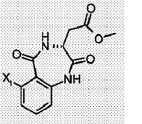 | 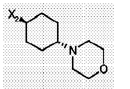 | 234, 282, 318 | 741 |
| 292 | 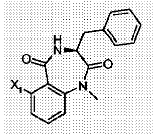 | 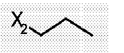 | 234, 282, 318 | 648 |
| 293 | 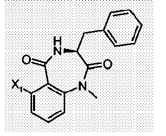 | 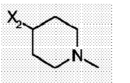 | 230, 282, 318 | 703 |
| 294 | 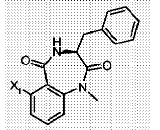 | 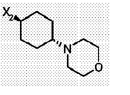 | 226, 282, 318 | 773 |
| 295 | 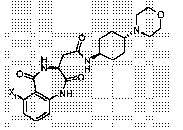 | 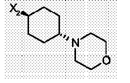 | 226, 282, 318 | 893 |
| 296 | 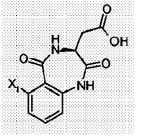 | 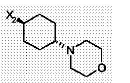 | 226, 282, 318 | 727 |
| 297 | 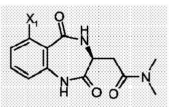 | 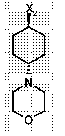 | 226, 282, 318 | 754 |
| 298 | 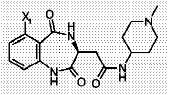 | 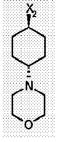 | 230, 282, 318 | 823 |
| 299 | 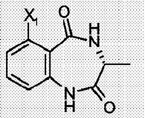 | 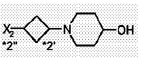 | 282, 318 | 669 |
| 300 | 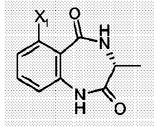 | 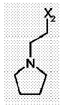 | 282, 318 | 613 |
| 301 | 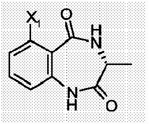 | 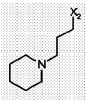 | 282, 318 | 641 |
| 302 | 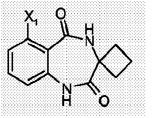 | 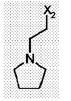 | 286, 318 | 639 |
| 303 | 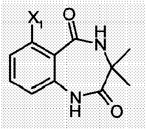 | 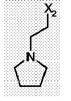 | 286, 318 | 627 |
| 304 | 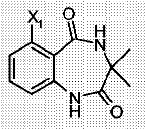 | 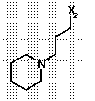 | 286, 318 | 655 |
| 305 | 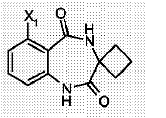 | 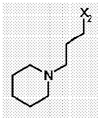 | 286, 318 | 667 |
| 306 | 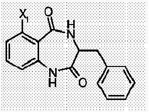 | 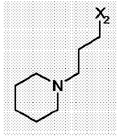 | 286, 318 | 717 |
| 307 | 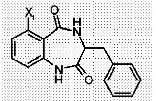 | 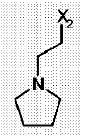 | 286, 318 | 689 |
| 308 | 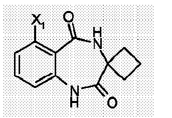 | 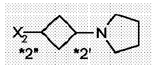 | 286, 318 | 665 |
| 309 | 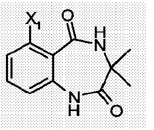 | 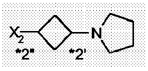 | 230, 286, 318 | 653 |
| 310 | 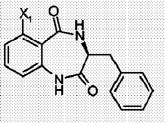 | 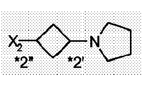 | 230, 282, 318 | 715 |
| 311 | 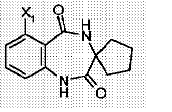 | 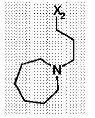 | 286, 322 | 695 |
| 312 | 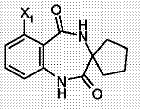 | 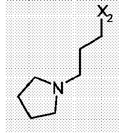 | 234, 286, 318 | 667 |
| 313 | 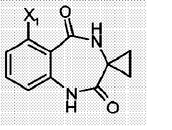 | 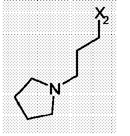 | 230, 282, 318 | 639 |
| 314 | 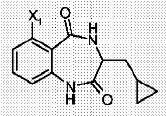 | 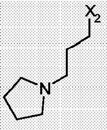 | 230, 282, 318 | 667 |
| 315 | 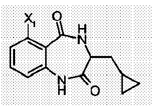 | 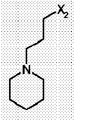 | 230, 282, 318 | 681 |
| 316 | 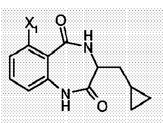 | 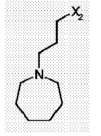 | 230, 282, 318 | 695 |
| 317 | 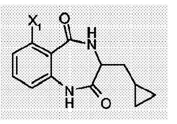 | 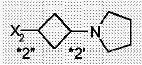 | | 679 |
| 318 | 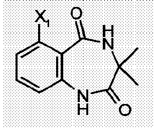 | 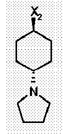 | 226, 284, 318 | 681 |
| 319 | 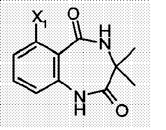 | 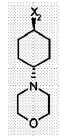 | 230, 284, 318 | 697 |
| 320 | 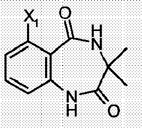 | 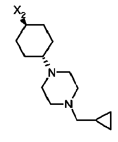 | 226, 284, 314 | 750 |
| 321 | 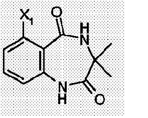 | 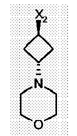 | 230, 286, 318 | 669 |
| 322 | 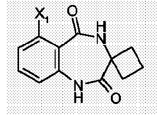 | 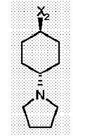 | 230, 282, 318 | 693 |
| 323 | 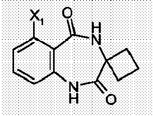 | 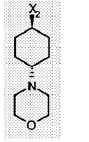 | 230, 282, 314 | 709 |
| 324 | 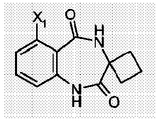 | 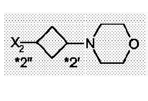 | 230, 286, 314 | 681 |
| 325 | 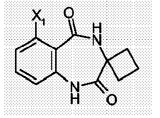 | 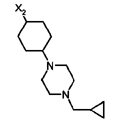 | 226, 286, 314 | 762 |
| 326 | 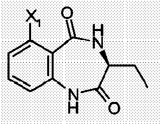 | 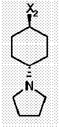 | 230, 282, 318 | 681 |
| 327 | 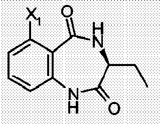 | 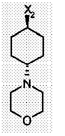 | 230, 282, 314 | 697 |
| 328 | 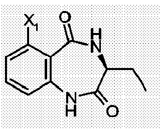 | 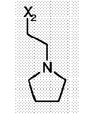 | 234, 282, 318 | 627 |
| 329 | 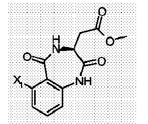 | 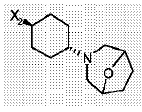 | 226, 282, 318 | 767 |
| 330 | 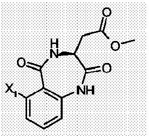 | 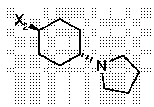 | 226, 282, 318 | 725 |
| 331 | 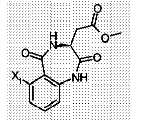 | 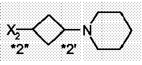 | 230, 286, 318 | 711 |
| 332 | 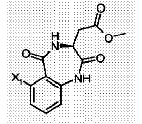 | 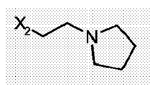 | 226, 282, 318 | 671 |
| 333 | 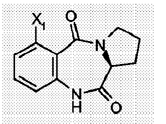 | 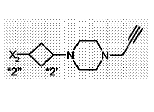 | 234, 282, 314 | 718 |
| 334 | 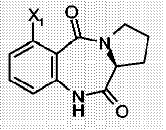 | 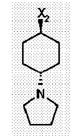 | 234, 282, 318 | 693 |
| 335 | 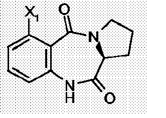 | 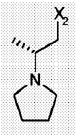 | 234, 286, 318 | 653 |
| 336 | 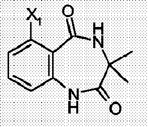 | 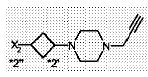 | 284, 318 | 706 |
| 337 | 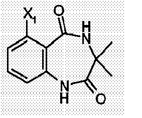 | 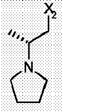 | 230, 282, 318 | 641 |
| 338 | 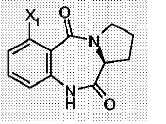 | 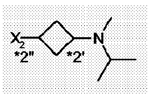 | 230, 282, 314 | 667 |
| 339 | 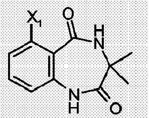 | 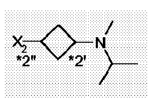 | 283, 318 | 655 |
| 340 | 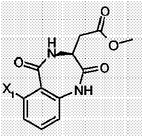 | 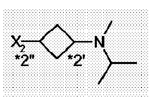 | 230, 286, 318 | 699 |
| 341 | 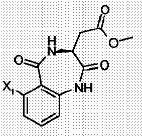 | 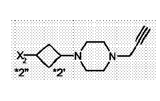 | 230, 282, 318 | 750 |
| 342 | 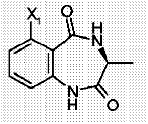 | 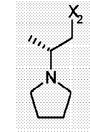 | 230, 282, 318 | 627 |
| 343 | 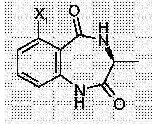 | 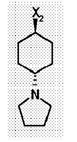 | 250, 282 ,318 | 667 |
| 344 | 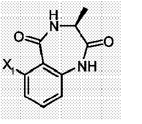 | 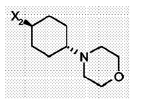 | 230, 282, 318 | 683 |
| 345 | 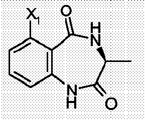 | 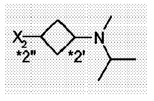 | 238, 282, 314 | 641 |
| 346 | 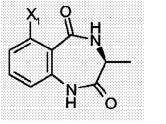 | 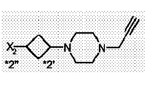 | 230, 314 | 692 |
| 347 | 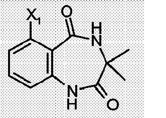 | 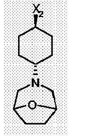 | 282, 318 | 723 |
| 348 | 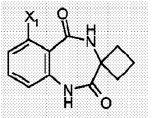 | 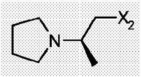 | 234, 286, 314 | 653 |
| 349 | 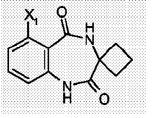 | 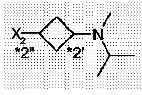 | 286, 318 | 667 |
| 350 | 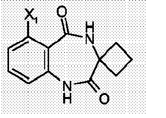 | 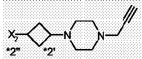 | 234, 286, 314 | 718 |
| 351 | 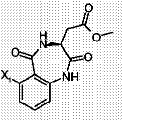 | 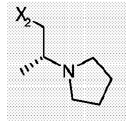 | 230, 286, 318 | 685 |

### Beispiele 352-372

Die folgenden Verbindungen sind über ein analoges Verfahren, wie in Beispiel 53 beschrieben, hergestellt. 2-(4-Carboxy-phenylamino)-4-chlor-5-trifluormethyl-pyrimidin kann nach Methode 14 hergestellt werden. Das entsprechende Anilin ist in Methode 11 beschrieben. Das für die Darstellung des Amids eingesetzte Amin ist kommerziell erhältlich beziehungsweise ist in Methode 13, 15 beziehungsweise 25 beschrieben.

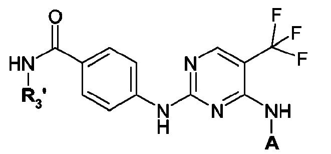

| **#** | **A** | **R₃'** | **UV max [nm]** | **MS (ESI) (M+H)⁺** |
|---|---|---|---|---|
| 352 | 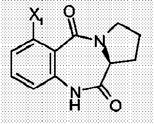 | 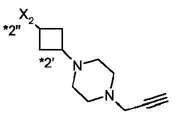 | 222, 302 | 688 |
| 353 | 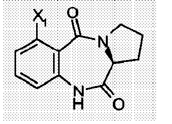 | 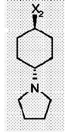 | 246, 298 | 663 |
| 354 | 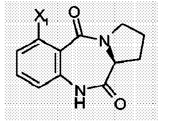 | 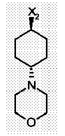 | 234, 298 | 679 |
| 355 | 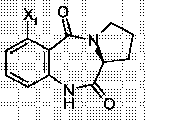 | 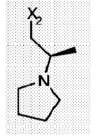 | 234, 302 | 623 |
| 356 | 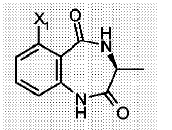 | 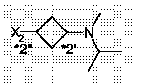 | 298 | 611 |
| 357 | 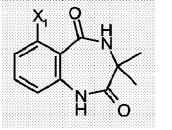 | 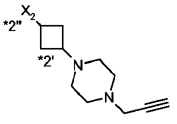 | 246, 302 | 676 |
| 358 | 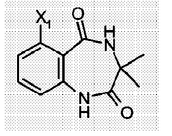 | 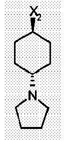 | | 651 |
| 359 | 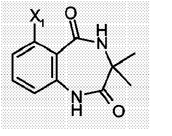 | 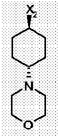 | | 667 |
| 360 | 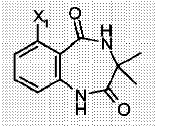 | 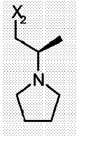 | 246, 302 | 611 |
| 361 | 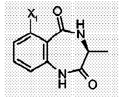 | 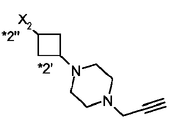 | 298 | 662 |
| 362 | 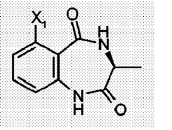 | 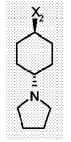 | | 637 |
| 363 | 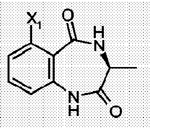 | 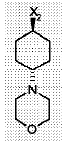 | 234, 298 | 653 |
| 364 | 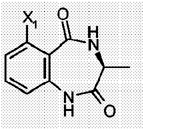 | 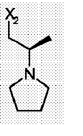 | 226, 302 | 597 |
| 365 | 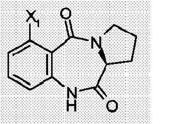 | 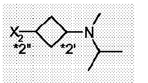 | 302 | 637 |
| 366 | 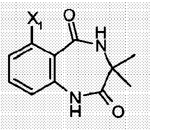 | 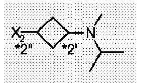 | 246, 302 | 625 |
| 367 | 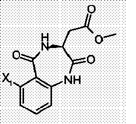 | 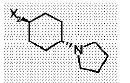 | 302 | 695 |
| 368 | 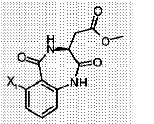 | 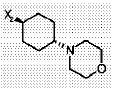 | 302 | 711 |
| 369 | 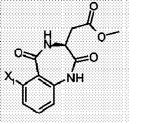 | 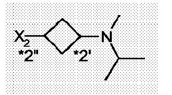 | 302 | 669 |
| 370 | 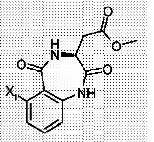 | 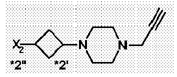 | 302 | 720 |
| 371 | 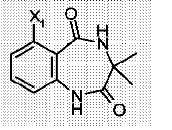 | 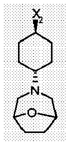 | 300 | 693 |
| 372 | 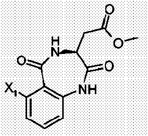 | 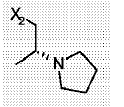 | 242, 302 | 655 |

### Beispiele 373-386

Analog zu Beispiel 169 und 170 werden die folgenden Beispiele hergestellt. Das entsprechende Anilin ist in Methode 11 beschrieben.

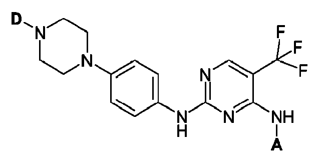

| **#** | **A** | **D** | **UV max [nm]** | **MS (ESI) (M+H)⁺** |
|---|---|---|---|---|
| 373 | 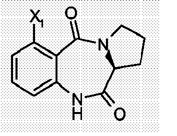 | 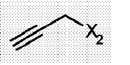 | 246 | 621 |
| 374 | 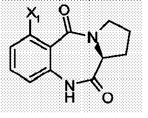 | 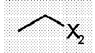 | 246 | 611 |
| 375 | 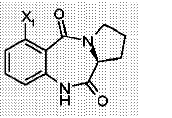 | 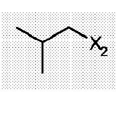 | 234 | 639 |
| 376 | 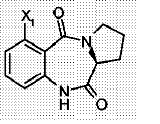 | 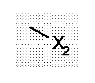 | 238 | 597 |
| 377 | 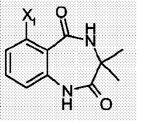 | 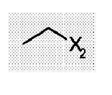 | 250 | 599 |
| 378 | 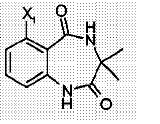 | 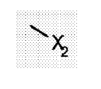 | 250 | 585 |
| 379 | 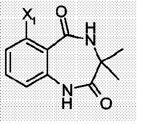 | 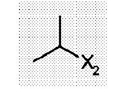 | 250 | 613 |
| 380 | 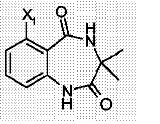 | 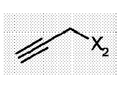 | 250 | 609 |
| 381 | 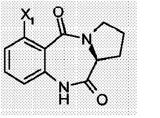 | 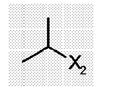 | 246 | 625 |
| 382 | 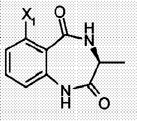 | 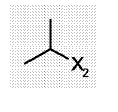 | 250 | 599 |
| 383 | 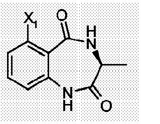 | 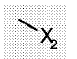 | 230 | 571 |
| 384 | 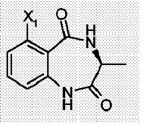 | 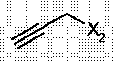 | 246 | 595 |
| 385 | 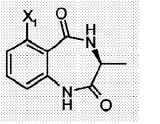 | 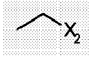 | 250 | 585 |
| 386 | 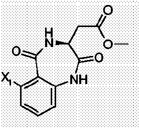 | 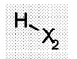 | 246, 286 | 615 |

### Beispiele 387-388

Die folgenden Verbindungen sind über ein analoges Verfahren, wie in Beispiel 53 beschrieben, hergestellt. 2-(4-Carboxy-2-methoxy-phenylamino)-4-chlor-5-trifluormethyl-pyrimidin kann nach Methode 12 beziehungsweise 14 hergestellt werden. Das entsprechende Anilin ist in Methode 4 beziehungsweise Methode 17 beschrieben. Das für die Darstellung des Amids eingesetzte Amin ist kommerziell erhältlich.

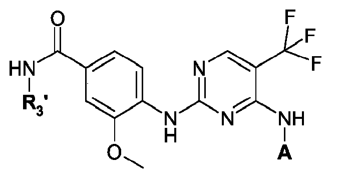

| **#** | **A** | **R₃'** | **UV max [nm]** | **MS (ESI) (M+H)⁺** |
|---|---|---|---|---|
| 387 | 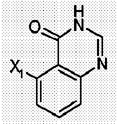 | 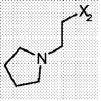 | 262, 318 | 569 |
| 388 | 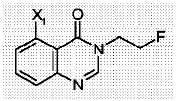 | 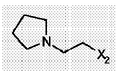 | 278, 318 | 615 |

### Beispiele 389-404

Die folgenden Verbindungen sind über ein analoges Verfahren, wie in Beispiel 53 beschrieben, hergestellt. 2-(4-Carboxy-2-methoxy-phenylamino)4-chlor-5-trifluormethyl-pyrimidin kann nach Methode 12 beziehungsweise 14 hergestellt werden. Das entsprechende Anilin ist in Methode 7, in Methode 18 beziehungsweise 19 beschrieben. Das für die Darstellung des Amids eingesetzte Amin ist kommerziell erhältlich beziehungsweise ist in Methode 13 beschrieben.

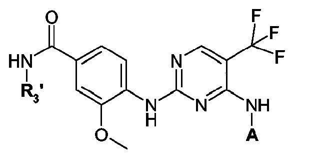

| **#** | **A** | **R₃'** | **UV max [nm]** | **MS (ESI) (M+H)⁺** |
|---|---|---|---|---|
| 389 | 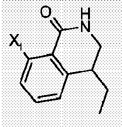 | 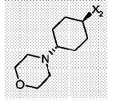 | 284, 322 | 668 |
| 390 | 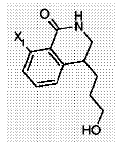 | 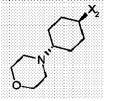 | 230, 285, 325 | 698 |
| 391 | 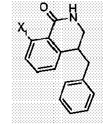 | 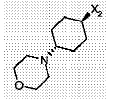 | 280, 325 | 730 |
| 392 | 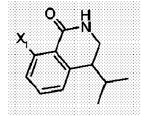 | 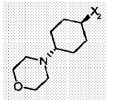 | 230, 285, 325 | 682 |
| 393 | 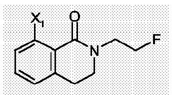 | 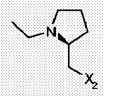 | 285, 325 | 630 |
| 394 | 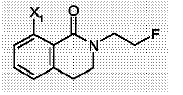 | 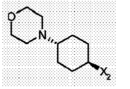 | 284, 322 | 686 |
| 395 | 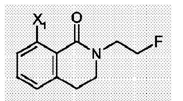 | 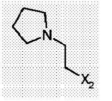 | 285, 325 | 616 |
| 396 | 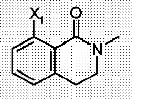 | 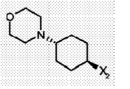 | 285, 322 | 654 |
| 397 | 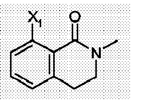 | 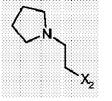 | 285, 325 | 584 |
| 398 | 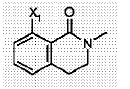 | 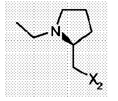 | 285, 325 | 598 |
| 399 | 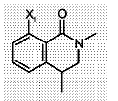 | 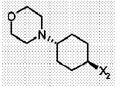 | 285, 325 | 668 |
| 400 | 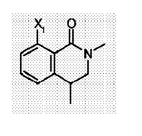 | 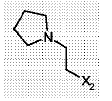 | 285, 325 | 598 |
| 401 | 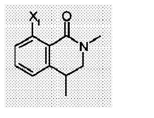 | 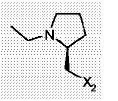 | 285, 325 | 612 |
| 402 | 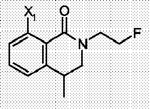 | 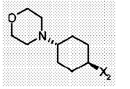 | 285, 322 | 700 |
| 403 | 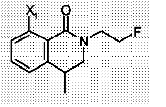 | 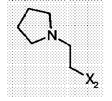 | 285, 322 | 630 |
| 404 | 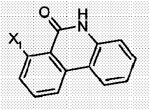 | 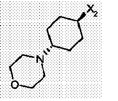 | 262 | 688 |

### Beispiel 405

### 2-[4-([1,4']Bipiperidinyl-4-ylcarbamoyl)-2-methoxy-phenylamino]-4-(2-(2-fluor-ethyl)-1-methyl-3-oxo-2,3-dihydro-1H-isoindol-4-ylamino)-5-trifluormethyl-pyrimidin

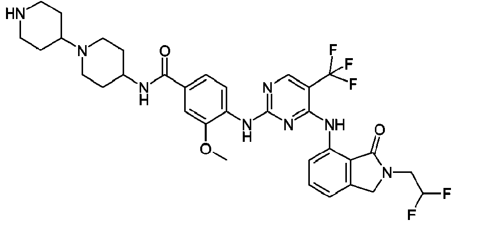

1150 mg (3,308 mmol) 2-(4-Carboxy-2-methoxy-phenylamino)-4-chlor-5-trifluormethyl-pyrimidin (Methode 12 beziehungsweise 14) werden in 2,5 ml *N*-Methyl-2-pyrrolidinon gelöst und mit 883 mg (4,161 mmol) 7-Amino-2-(2,2-difluor-ethyl)-2,3-dihydro-isoindol-1-on (Methode 2) versetzt. Diesem Reaktionsgemisch werden 115 µl einer 4 M Lösung von HCl (0,460 mol) in 1,4-Dioxan zudosiert. Nach 16 h bei 90 °C wird das Reaktionsgemisch in 150 ml einer wässrigen 1 N Salzsäure eingerührt. Der Niederschlag wird abfiltriert und im Vakuum getrocknet.

| | |
|---|---|
| Ausbeute: | 1626 mg (3,110 mmol; 94 %) |
| MS (ESI): | 524 (M+H)⁺ |

100 mg (0,191 mmol) dieses Niederschlages, 240 µl (1,402 mmol) *N*-Ethyldiisopropyl-amin, 89 mg (0,279 mmol) O-(Benzotriazol-1-yl)-*N,N,N'*,*N*'-tetramethyluronium-tetrafluorborat und 76 mg (0,267 mmol) 4-Amino-[1,4']bipiperidinyl-1'-carbonsäure *tert-*butylester werden in 3 ml *N,N*-Dimethylformamid gelöst. Nach 15 h bei 20 °C wird das Lösungsmittel im Vakuum entfernt. Der Rückstand wird in 20 ml Dichlormethan und 5 ml Methanol aufgenommen und über Aluminiumoxid filtriert. Das Aluminiumoxid wird mehrmals mit einem Gemisch aus Dichlormethan und Methanol (4:1) nach gewaschen. Das Lösungsmittel der vereinigten Fraktionen wird im Vakuum entfernt. Der Rückstand wird in 5 ml Dichlormethan gelöst und mit 5 ml Trifluoressigsäure versetzt. Diese Mischung last man 3 h bei 20 °C rühren und entfernt anschließend das Lösungsmittel im Vakuum. Das Rohprodukt wird durch Säulenchromatographie gereinigt. Als Trägermaterial dient C18-RP-Kieselgel und es wird ein Gradient durchlaufen der am Startpunkt aus 90% Wasser und 10% Acetonitril und am Endpunkt aus 5% Wasser und 95% Acetonitril besteht. Sowohl dem Wasser als auch dem Acetonitril sind jeweils 0,1% Ameisensäure beigemischt. Die geeigneten Fraktionen werden mit 500 µl einer 1 N Salzsäure versetzt und gefriergetrocknet. Das Produkt liegt als Trihydrochlorid vor.

| | |
|---|---|
| Ausbeute: | 42 mg (0,053 mmol; 28 %) |
| UV max: | 322 nm |
| MS (ESI): | 689 (M+H)⁺ |
| ¹H-NMR: | 1.92 - 2.19 (m, 6H), 2.28 - 2.37 (m, 2H), 2.86 - 3.00 (m, 2H), 3.07 - 3.19 (m, 3H), 3.84 - 4.18 (m, 7H), 4.59 (s, 2H), 6.15 - 6.47 (m, 1H), 7.23 - 7.28 (m, 1H), 7.35 - 7.43 (m, 1H), 7.54 - 7.64 (m, 2H), 7.75 - 7.82 (m, 1H), 8.40 - 8.64 (m, 3H), 8.90 - 9.01 (m, 1H), 9.10 - 9.25 (m, 2H), 10.40 - 10.47 (m, 1H), 10.91 - 11.27 (m, 1H) |

### Beispiele 406-407

Die folgenden Verbindungen sind über ein analoges Verfahren, wie in Beispiel 405 beschrieben, hergestellt.

| **#** | | **UV max [nm]** | **MS (ESI) (M+H)⁺** |
|---|---|---|---|
| 406 | 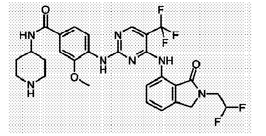 | 318 | 606 |
| 407 | 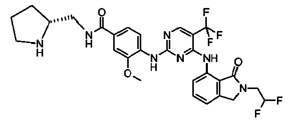 | 322,286 | 606 |

### Beispiel 408

### 2-[2-methoxy-4-(1'-methyl-[1,4']bipiperidinyl-4-ylcarbamoyl)-phenylamino]-4-(2-(2-fluorethyl)-1-methyl-3-oxo-2,3-dihydro-1H-isoindol-4-ylamino)-5-trifluormethyl-pyrimidin

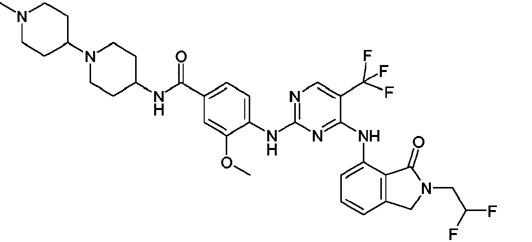

70 mg (0,087 mmol) 2-[4-([1,4']Bipiperidinyl-4-ylcarbamoyl)-2-methoxy-phenylamino]-4-(2-(2-fluor-ethyl)-1-methyl-3-oxo-2,3-dihydro-1*H*-isoindol-4-ylamino)-5-trifluormethyl-pyrimidin (Beispiel 405) werden in 3 ml Methanol gelöst, mit 8,5 µl (0,508 mmol) Essigsäure und mit 8 µl (0,107 mmol) einer 37% wässrigen Formaldehyd-Lösung versetzt. Anschließend gibt man bei 20 °C 7,0 mg (0,112 mmol) Natriumcyanoborhydrid zu. Diese Mischung rührt man 16 h bei 20 °C. Das Lösungsmittel wird im Vakuum entfernt und das Rohprodukt wird durch Säulenchromatographie gereinigt. Als Trägermaterial dient C18-RP-Kieselgel und es wird ein Gradient durchlaufen der am Startpunkt aus 95% Wasser und 5% Acetonitril und am Endpunkt aus 5% Wasser und 95% Acetonitril besteht. Sowohl dem Wasser als auch dem Acetonitril sind jeweils 0,1% Ameisensäure beigemischt Die geeigneten Fraktionen werden mit 500 µl einer 1 N Salzsäure versetzt und gefriergetrocknet. Das Produkt liegt als Trihydrochlorid vor.

| | |
|---|---|
| Ausbeute: | 18 mg (0,022 mmol; 25 %) |
| UV max: | 322 nm |
| MS (ESI): | 703 (M+H)⁺ |

### Beispiele 409-491

Die folgenden Verbindungen sind über ein analoges Verfahren, wie in Beispiel 53 beschrieben, hergestellt. 2-(4-Carboxy-2-methoxy-phenylamino)-4-chlor-5-trifluormethyl-pyrimidin kann nach Methode 12 beziehungsweise 14 hergestellt werden. Das entsprechende Anilin ist in Methode 2 beschrieben. Das für die Darstellung des Amids eingesetzte Amin ist kommerziell erhältlich beziehungsweise ist in Methode 13,20 beziehungsweise 21 beschrieben.

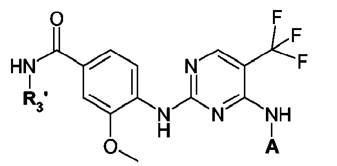

| **#** | **A** | **R₃'** | **UV max [nm]** | **MS (ESI) (M+H)⁺** |
|---|---|---|---|---|
| 409 | 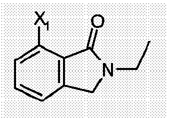 | 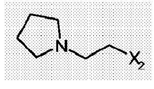 | 285, 320 | 584 |
| 410 | 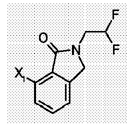 | 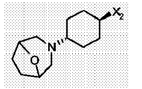 | 322 | 716 |
| 411 | 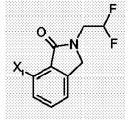 | 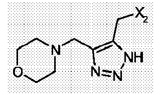 | 326 | 703 |
| 412 | 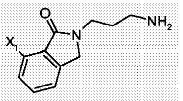 | 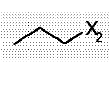 | | 558 |
| 413 | 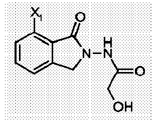 | 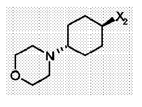 | 282, 318 | 699 |
| 414 | 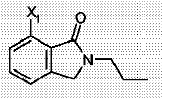 | 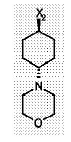 | 322, 286 | 668 |
| 415 | 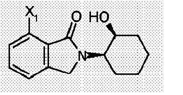 | 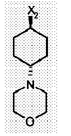 | 322,3 | 724 |
| 416 | 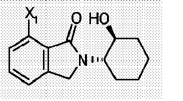 | 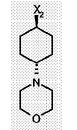 | 322,3 | 362 |
| 417 | 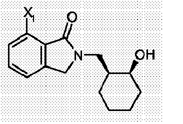 | 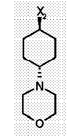 | 322, 286 | 738 |
| 418 | 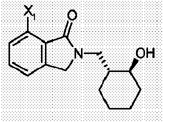 | 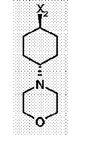 | 322, 286 | 738 |
| 419 | 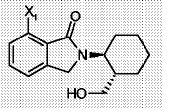 | 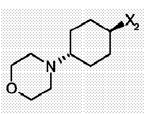 | 282, 314 | 738 |
| 420 | 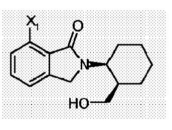 | 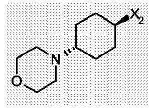 | 286, 314 | 738 |
| 421 | 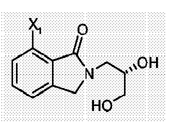 | 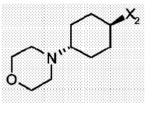 | 286, 318 | 700 |
| 422 | 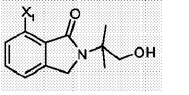 | 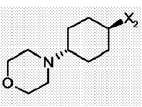 | 286, 322 | 698 |
| 423 | 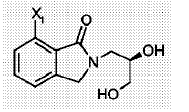 | 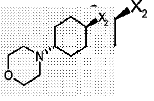 | 286, 318 | 700 |
| 424 | 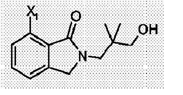 | 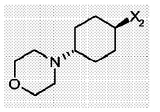 | 286, 322 | 712 |
| 425 | 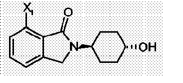 | 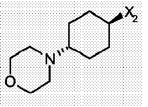 | 286, 322 | 724 |
| 426 | 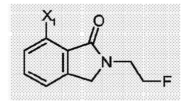 | 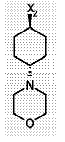 | 322, 286 | 672 |
| 427 | 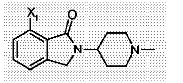 | 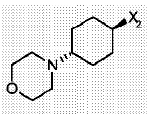 | 282, 322 | 723 |
| 428 | 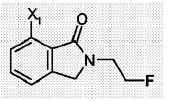 | 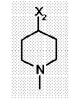 | 322, 285 | 602 |
| 429 | 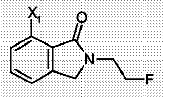 | 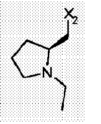 | 326,3 | 616 |
| 430 | 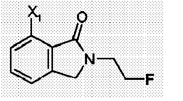 | 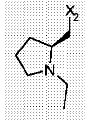 | 322, 286 | 616 |
| 431 | 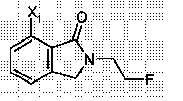 | 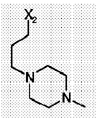 | 318, 286 | 645 |
| 432 | 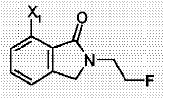 | 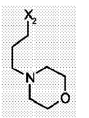 | 321, 284 | 632 |
| 433 | 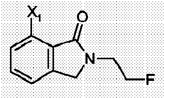 | 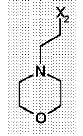 | 322, 286 | 618 |
| 434 | 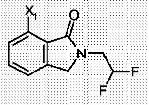 | 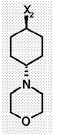 | 318, 282 | 690 |
| 435 | 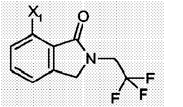 | 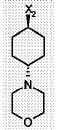 | 322, 282 | 708 |
| 436 | 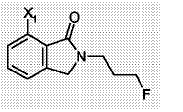 | 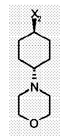 | 322, 286 | 686 |
| 437 | 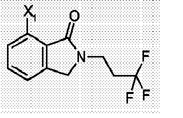 | 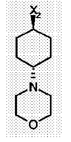 | 322, 284 | 722 |
| 438 | 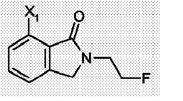 | 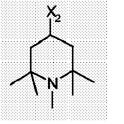 | 322, 282 | 658 |
| 439 | 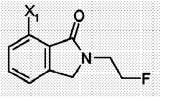 | 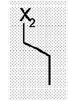 | 322, 285 | 547 |
| 440 | 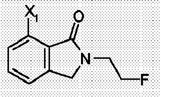 | 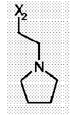 | 322, 286 | 602 |
| 441 | 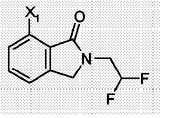 | 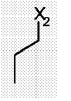 | 286,3 | 565 |
| 442 | 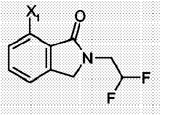 | 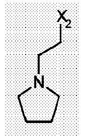 | 322, 286 | 620 |
| 443 | 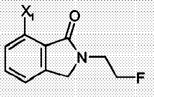 | 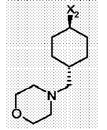 | 322, 284 | 686 |
| 444 | 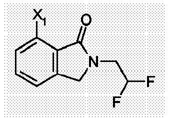 | 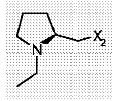 | 326,3 | 634 |
| 445 | 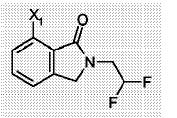 | 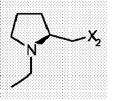 | 326, 286 | 634 |
| 446 | 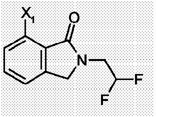 | 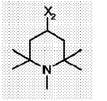 | 322, 284 | 676 |
| 447 | 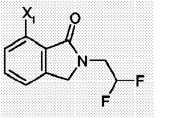 | 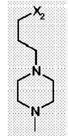 | 322,3 | 663 |
| 448 | 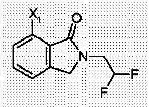 | 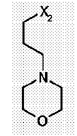 | 325,3 | 650 |
| 449 | 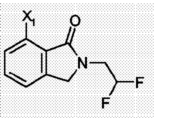 | 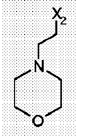 | 325,3 | 635 |
| 450 | 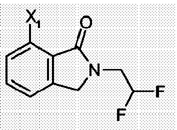 | 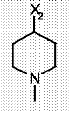 | 322, 282 | 620 |
| 451 | 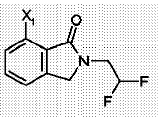 | 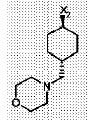 | 322, 282 | 704 |
| 452 | 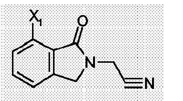 | 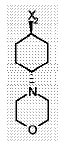 | 322, 282 | 665 |
| 453 | 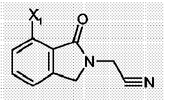 | 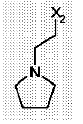 | 326, 282 | 595 |
| 454 | 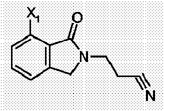 | 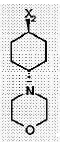 | 322, 284 | 677 |
| 455 | 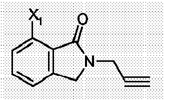 | 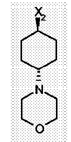 | 322,3 | 664 |
| 456 | 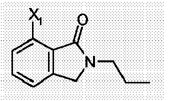 | 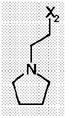 | 326, 286 | 594 |
| 457 | 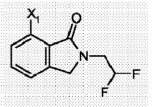 | 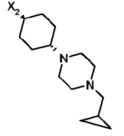 | 322, 282 | 743 |
| 458 | 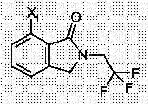 | 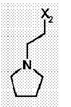 | 326, 286 | 638 |
| 459 | 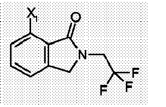 | 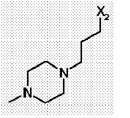 | 326, 283 | 681 |
| 460 | 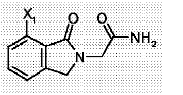 | 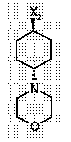 | 318, 284 | 681 |
| 461 | 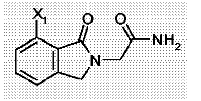 | 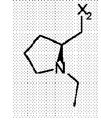 | 318, 286 | 627 |
| 462 | 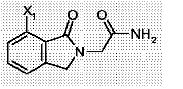 | 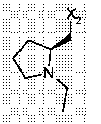 | 322, 286 | 627 |
| 463 | 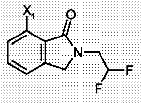 | 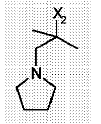 | 326, 286 | 648 |
| 464 | 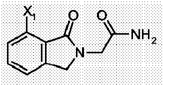 | 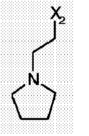 | 322, 286 | 611 |
| 465 | 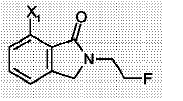 | 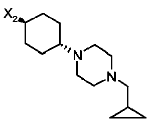 | 322, 286 | 723 |
| 466 | 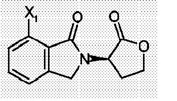 | 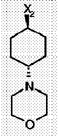 | 322, 282 | 710 |
| 467 | 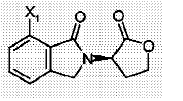 | 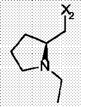 | 326, 286 | 654 |
| 468 | 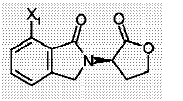 | 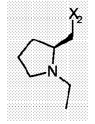 | 326, 286 | 654 |
| 469 | 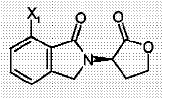 | 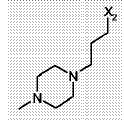 | 322, 284 | 683 |
| 470 | 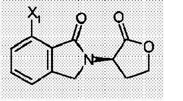 | 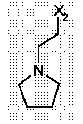 | 326, 286 | 640 |
| 471 | 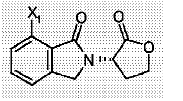 | 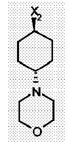 | 318, 283 | 710 |
| 472 | 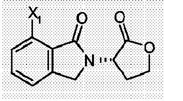 | 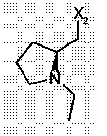 | 326, 286 | 654 |
| 473 | 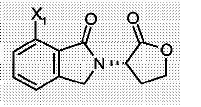 | 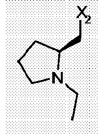 | 326, 286 | 654 |
| 474 | 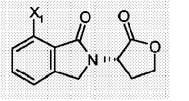 | 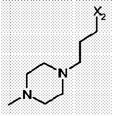 | 321, 285 | 683 |
| 475 | 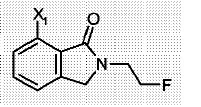 | 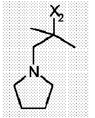 | 326, 286 | 630 |
| 476 | 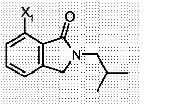 | 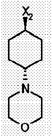 | 322, 286 | 682 |
| 477 | 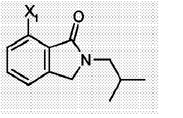 | 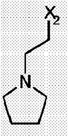 | 318, 286 | 612 |
| 478 | 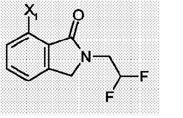 | 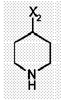 | 318,3 | 606 |
| 479 | 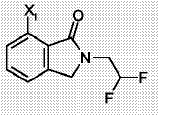 | 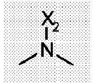 | 322, 286 | 566 |
| 480 | 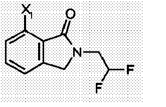 | 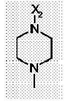 | 322, 286 | 621 |
| 481 | 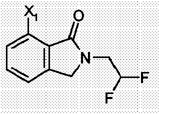 | 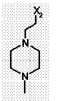 | 318, 286 | 649 |
| 482 | 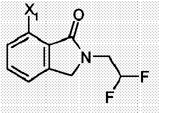 | 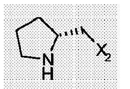 | 322, 286 | 606 |
| 483 | 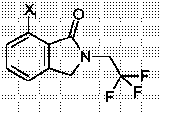 | 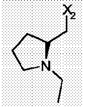 | 326, 286 | 652 |
| 484 | 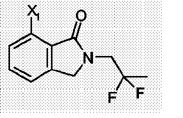 | 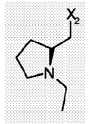 | 326, 286 | 648 |
| 485 | 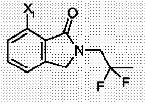 | 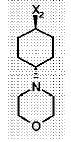 | 322, 284 | 704 |
| 486 | 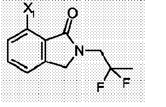 | 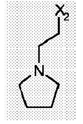 | 326, 286 | 634 |
| 487 | 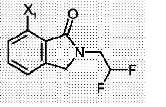 | 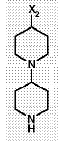 | 322, 285 | 689 |
| 488 | 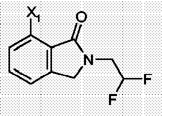 | 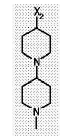 | 322, 285 | 703 |
| 489 | 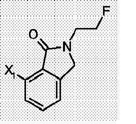 | 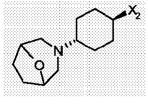 | 322 | 698 |
| 490 | 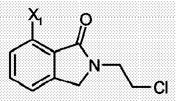 | 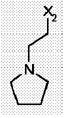 | 322, 286 | 619 |
| 491 | 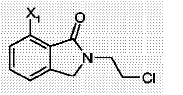 | 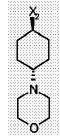 | 322, 286 | 689 |

### Beispiele 492-621

Die folgenden Verbindungen sind über ein analoges Verfahren, wie in Beispiel 53 beschrieben, hergestellt. 2-(4-Carboxy-2-methoxy-phenylamino)-4-chlor-5-trifluormethyl-pyrimidin kann nach Methode 12 beziehungsweise 14 hergestellt werden. Das entsprechende Anilin ist in Methode 22 beschrieben. Das für die Darstellung des Amids eingesetzte Amin ist kommerziell erhältlich, in Methode 13, 15, 20, 21, 23, 24 und 25 beziehungsweise in J. Med. Chem. 2003, 46(5), 702-715 beschrieben.

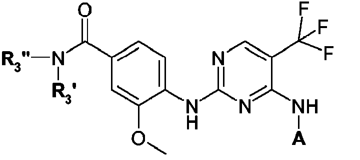

| **#** | **A** | **R₃'** | **R₃"** | **UV max [nm]** | **MS (ESI) (M+H)⁺** |
|---|---|---|---|---|---|
| 492 | 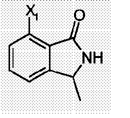 | 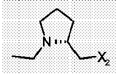 | H | 286, 322 | 584 |
| 493 | 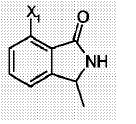 | 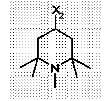 | H | 286, 322 | 826 |
| 494 | 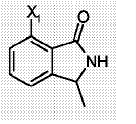 | 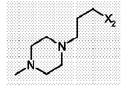 | H | 284, 322 | 613 |
| 495 | 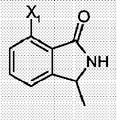 | 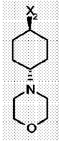 | H | 282, 322 | 640 |
| 496 | 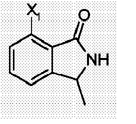 | 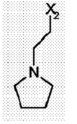 | H | 286, 320 | 570 |
| 497 | 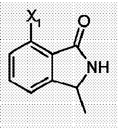 | 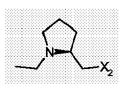 | H | 286, 322 | 584 |
| 498 | 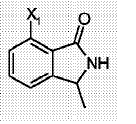 | 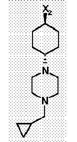 | H | 282, 322 | 693 |
| 499 | 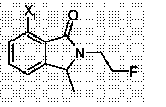 | 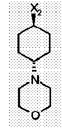 | H | 286, 322 | 686 |
| 500 | 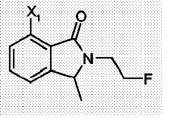 | 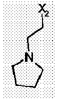 | H | 286, 326 | 616 |
| 501 | 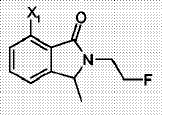 | 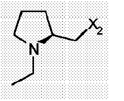 | H | 286, 326 | 630 |
| 502 | 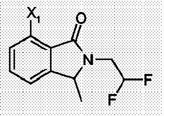 | 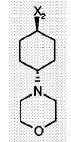 | H | 282, 325 | 704 |
| 503 | 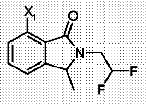 | 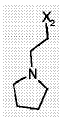 | H | 286, 326 | 634 |
| 504 | 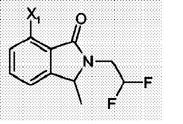 | 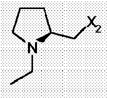 | H | 286, 326 | 648 |
| 505 | 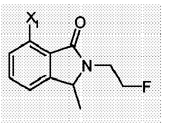 | 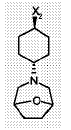 | H | 286, 322 | 712 |
| 506 | 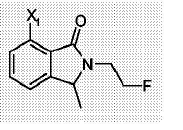 | 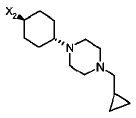 | H | 322, 286 | 739 |
| 507 | 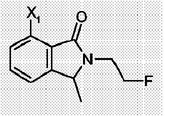 | 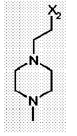 | H | 322, 286 | 645 |
| 508 | 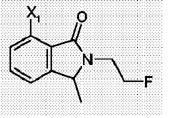 | 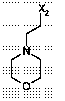 | H | 326, 286 | 632 |
| 509 | 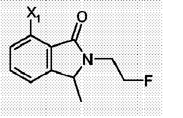 | 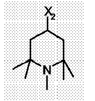 | H | 322, 286 | 672 |
| 510 | 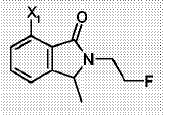 | 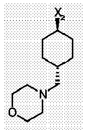 | H | 322, 284 | 700 |
| 511 | 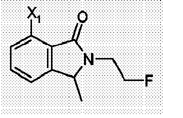 | 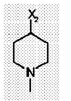 | H | 314, 286 | 616 |
| 512 | 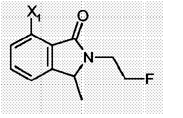 | 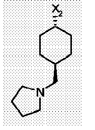 | H | 286, 322 | 684 |
| 513 | 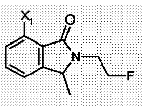 | 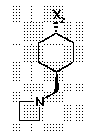 | H | 286, 322 | 670 |
| 514 | 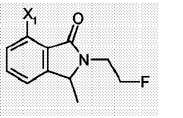 | 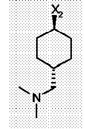 | H | 282, 322 | 658 |
| 515 | 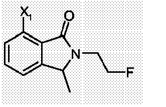 | 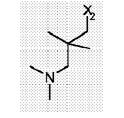 | H | 322, 286 | 632 |
| 516 | 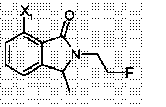 | 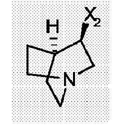 | H | 326, 286 | 628 |
| 517 | 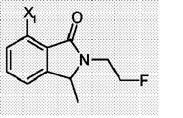 | 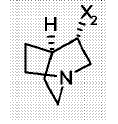 | H | 325, 286 | 628 |
| 518 | 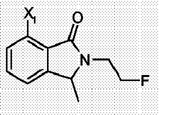 | 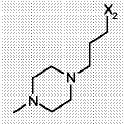 | H | 326, 286 | 659 |
| 519 | 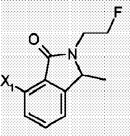 | 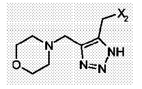 | H | 326 | 699 |
| 520 | 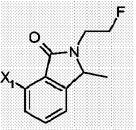 | 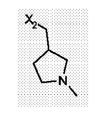 | H | 284, 326 | 616 |
| 521 | 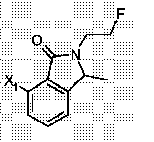 | 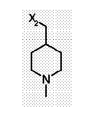 | H | 234, 282, 314 | 630 |
| 522 | 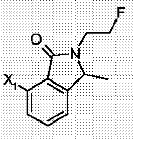 | 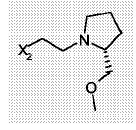 | H | 326 | 660 |
| 523 | 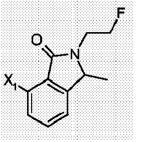 | 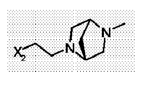 | H | 326 | 657 |
| 524 | 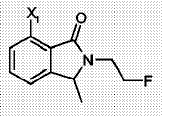 | 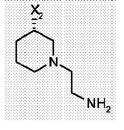 | H | | 645 |
| 525 | 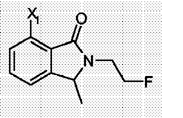 | 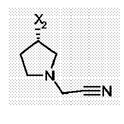 | H | 326 | 627 |
| 526 | 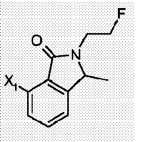 | 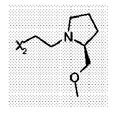 | H | 326 | 660 |
| 527 | 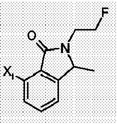 | 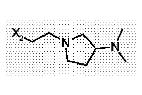 | H | 326 | 659 |
| 528 | 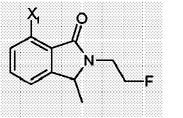 | 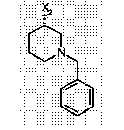 | H | 326 | 692 |
| 529 | 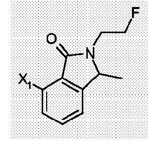 | 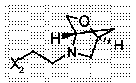 | H | 326 | 644 |
| 530 | 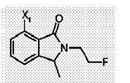 | 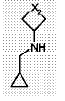 | H | 326 | 628 |
| 531 | 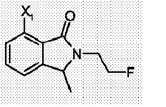 | 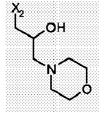 | H | 322 | 662 |
| 532 | 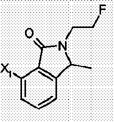 | 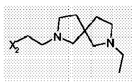 | H | 326 | 699 |
| 533 | 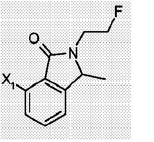 | 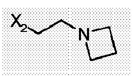 | H | 326 | 602 |
| 534 | 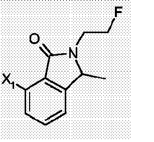 | 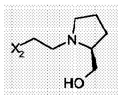 | H | | 646 |
| 535 | 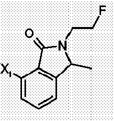 | 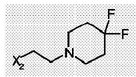 | H | 326 | 666 |
| 536 | 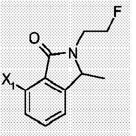 | 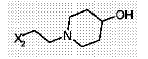 | H | 326 | 646 |
| 537 | 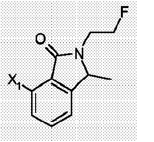 | 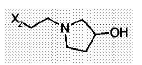 | H | 326 | - |
| 538 | 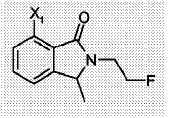 | 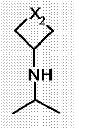 | H | 322 | 616 |
| 539 | 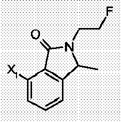 | 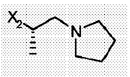 | H | 318 | 630 |
| 540 | 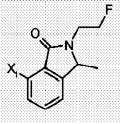 | 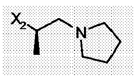 | H | 318 | 630 |
| 541 | 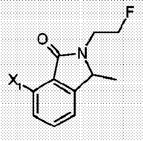 | 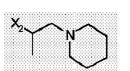 | H | 274 | 644 |
| 542 | 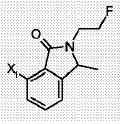 | 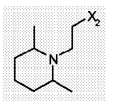 | H | 326 | 658 |
| 543 | 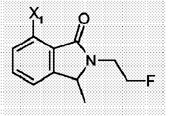 | 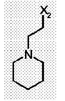 | H | 286, 324 | 630 |
| 544 | 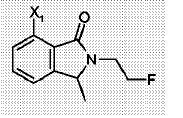 | 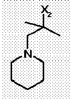 | H | 286, 326 | 658 |
| 545 | 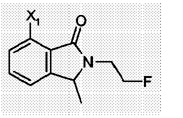 | 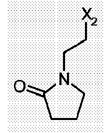 | H | 286, 322 | 630 |
| 546 | 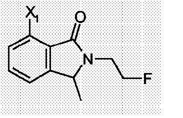 | 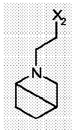 | H | 286, 326 | 642 |
| 547 | 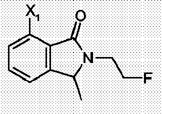 | 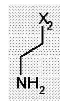 | H | 286, 322 | 562 |
| 548 | 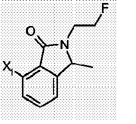 | 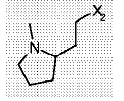 | H | 322-326 | 630 |
| 549 | 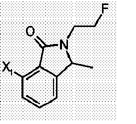 | 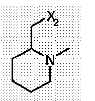 | H | 326 | 630 |
| 550 | 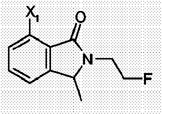 | 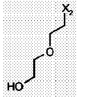 | H | 286, 322 | 607 |
| 551 | 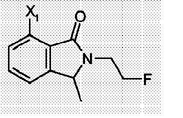 | 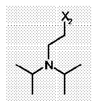 | H | | 646 |
| 552 | 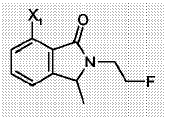 | 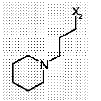 | H | | 644 |
| 553 | 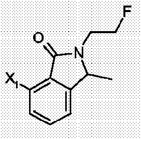 | 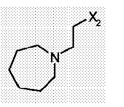 | H | 326 | 644 |
| 554 | 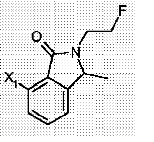 | 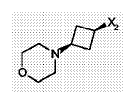 | H | 322-326 | 658 |
| 555 | 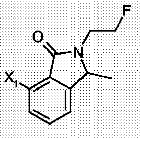 | 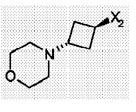 | H | 322-326 | 658 |
| 556 | 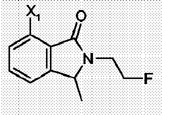 | 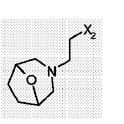 | H | 286, 326 | 658 |
| 557 | 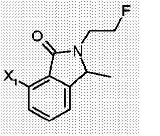 | 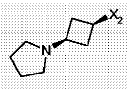 | H | 322-326 | 642 |
| 558 | 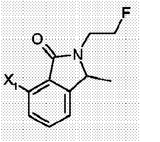 | 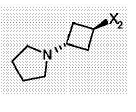 | H | 322-326 | 642 |
| 559 | 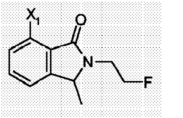 | 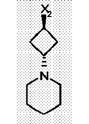 | H | 286, 322 | 656 |
| 560 | 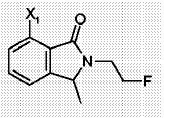 | 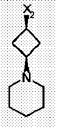 | H | 286, 322 | 656 |
| 561 | 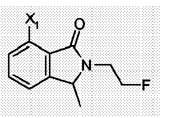 | 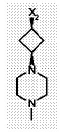 | H | 286, 322 | 671 |
| 562 | 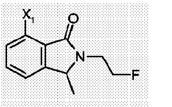 | 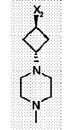 | H | 286, 322 | 671 |
| 563 | 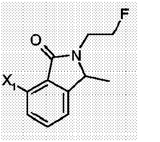 | 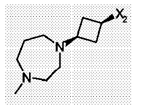 | H | 318 | 685 |
| 564 | 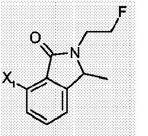 | 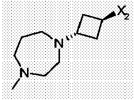 | H | 322-326 | 685 |
| 565 | 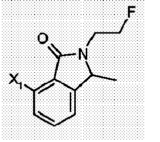 | 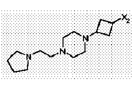 | H | 322-326 | 754 |
| 566 | 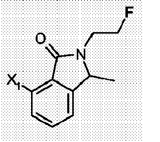 | 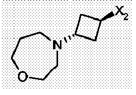 | H | 322-326 | 672 |
| 567 | 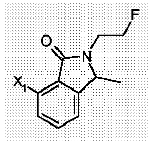 | 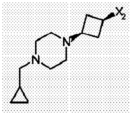 | H | 322 | 711 |
| 568 | 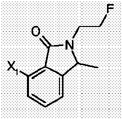 | 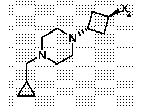 | H | 322-326 | 711 |
| 569 | 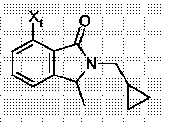 | 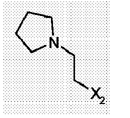 | H | 326 | 624 |
| 570 | 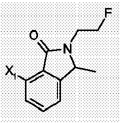 | 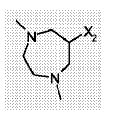 | H | 326 | 645 |
| 571 | 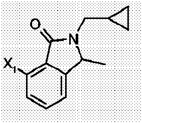 | 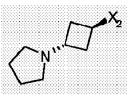 | H | 322-326 | 650 |
| 572 | 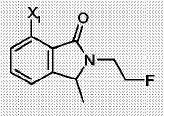 | 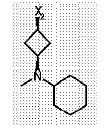 | H | 286, 326 | 684 |
| 573 | 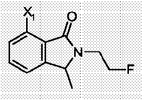 | 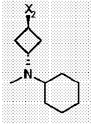 | H | 286, 326 | 684 |
| 574 | 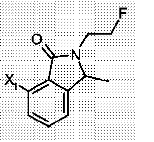 | 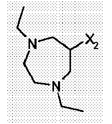 | H | 326 | 673 |
| 575 | 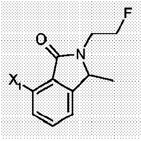 | 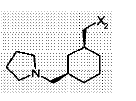 | H | 322 | 698 |
| 576 | 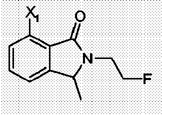 | 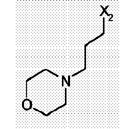 | H | 326, 286 | 646 |
| 577 | 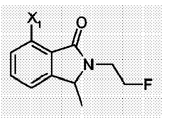 | 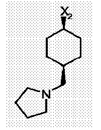 | H | 286, 322 | 684 |
| 578 | 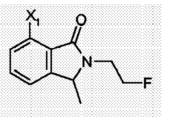 | 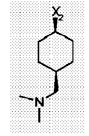 | H | 282, 322 | 658 |
| 579 | 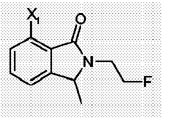 | 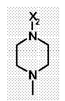 | H | 322, 286 | 617 |
| 580 | 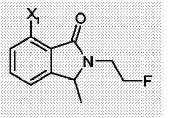 | 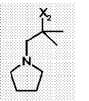 | H | 326, 286 | 644 |
| 581 | 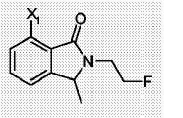 | 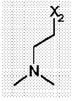 | H | 326, 286 | 590 |
| 582 | 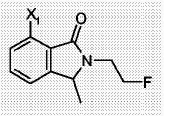 | 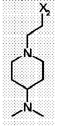 | H | 286, 326 | 673 |
| 583 | 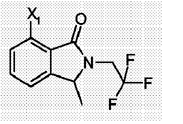 | 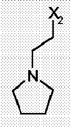 | H | 326, 285 | 652 |
| 584 | 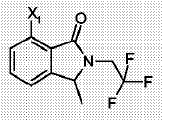 | 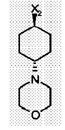 | H | 326, 282 | 722 |
| 585 | 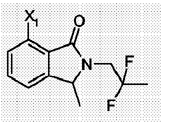 | 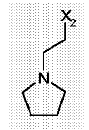 | H | 326, 286 | 648 |
| 586 | 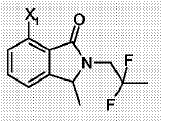 | 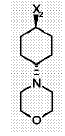 | H | 326, 285 | 718 |
| 587 | 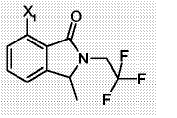 | 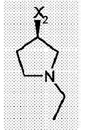 | H | 326, 286 | 652 |
| 588 | 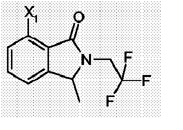 | 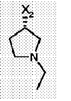 | H | 326, 284 | 652 |
| 589 | 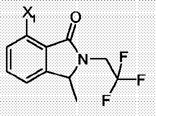 | 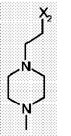 | H | 325, 283 | 681 |
| 590 | 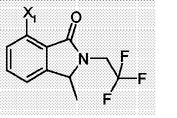 | 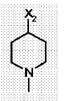 | H | 325,3 | 652 |
| 591 | 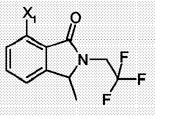 | 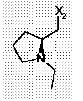 | H | 326,3 | 666 |
| 592 | 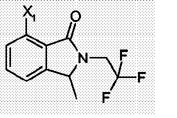 | 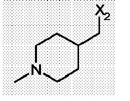 | H | 325, 283 | 666 |
| 593 | 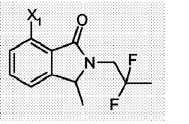 | 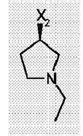 | H | 325,3 | 648 |
| 594 | 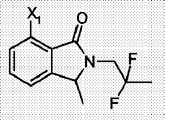 | 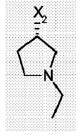 | H | 325, 284 | 648 |
| 595 | 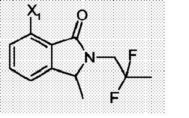 | 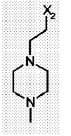 | H | 325, 284 | 677 |
| 596 | 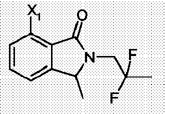 | 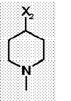 | H | 325, 284 | 648 |
| 597 | 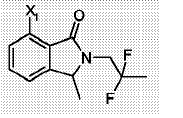 | 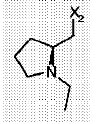 | H | 326, 285 | 662 |
| 598 | 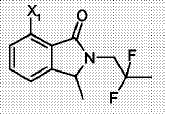 | 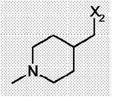 | H | 325, 284 | 662 |
| 599 | 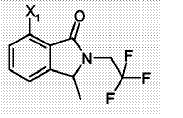 | 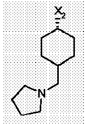 | H | 326, 282 | 720 |
| 600 | 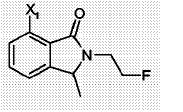 | 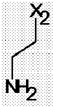 | 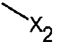 | 314, 283 | 576 |
| 601 | 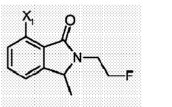 | 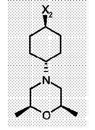 | H | 322, 286 | 714 |
| 602 | 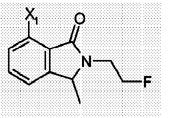 | 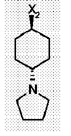 | H | 286, 322 | 670 |
| 603 | 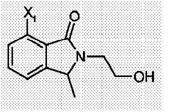 | 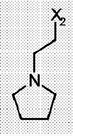 | H | 324, 285 | 614 |
| 604 | 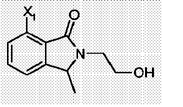 | 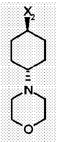 | H | 324, 284 | 684 |
| 605 | 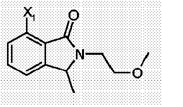 | 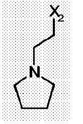 | H | 324, 285 | 628 |
| 606 | 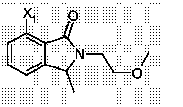 | 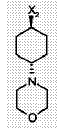 | H | 324, 284 | 698 |
| 607 | 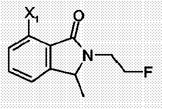 | 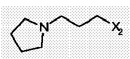 | H | 285, 322 | 630 |
| 608 | 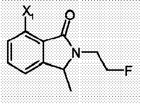 | 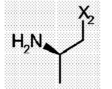 | H | 325, 284 | 576 |
| 609 | 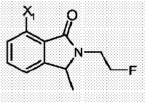 | 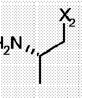 | H | 325, 284 | 576 |
| 610 | 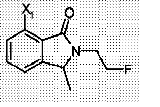 | 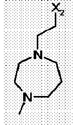 | H | 326, 286 | 659 |
| 611 | 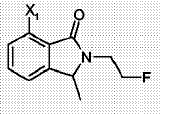 | 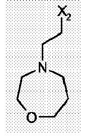 | H | 326, 286 | 646 |
| 612 | 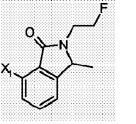 | 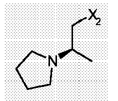 | H | 325, 285 | 630 |
| 613 | 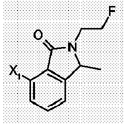 | 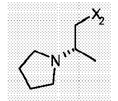 | H | 325, 284 | 630 |
| 614 | 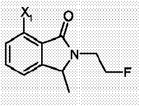 | 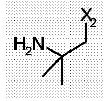 | H | 325, 285 | 590 |
| 615 | 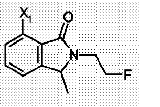 | 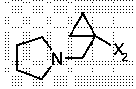 | H | 285, 325 | 642 |
| 616 | 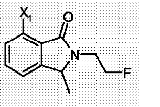 | 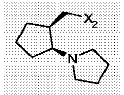 | H | 325, 285 | 670 |
| 617 | 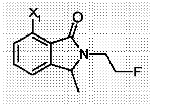 | 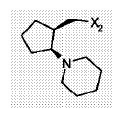 | H | 326, 286 | 684 |
| 618 | 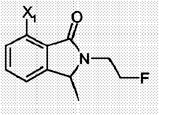 | 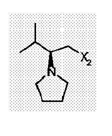 | H | 326, 286 | 658 |
| 619 | 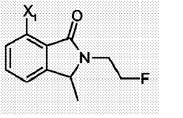 | 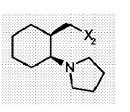 | H | 285, 324 | 684 |
| 620 | 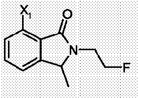 | 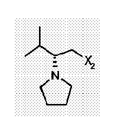 | H | 326, 286 | 658 |
| 621 | 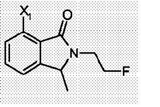 | 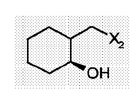 | H | 280, 320 | 631 |

### Beispiel 622

### 2-(2-Methoxy-4-[2-(2-pyrrolidin-1-yl-ethylcarbamoyl)-ethylamino]-phenylamino)-4-(2-(2-fluor-ethyl)-1-methyl-3-oxo-2,3-dihydro-1H-isoindol-4-ylamino)-5-trifluormethyl-pyrimidin

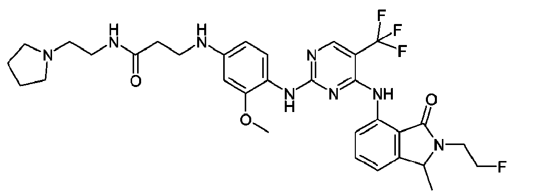

73 mg (0,193 mmol) 3-(4-Amino-3-methoxy-phenylamino)-*N*-(2-pyrrolidin-1-yl-ethyl)-propionsäureamid Hydrochlorid (Methode 28) werden in 3 ml 2-Butanol gelöst und mit 50 mg (0,129 mmol) 2-Chlor-4-(2-(2-fluorethyl)-1-methyl-3-oxo-2,3-dihydro-1*H*-isoindol-4-ylamino)-5-trifluormethyl-pyrimidin (Methode 26) versetzt. Diese Reaktionsmischung rührt man 16 h bei 100°C. Das Lösungsmittel wird im Vakuum entfernt und der Rückstand wird mittels Säulenchromatographie gereinigt. Als Trägermaterial dient C18-RP-Kieselgel und es wird ein Gradient durchlaufen der am Startpunkt aus 90% Wasser und 10% Acetonitril und am Endpunkt aus 55% Wasser und 45% Acetonitril besteht. Sowohl dem Wasser als auch dem Acetonitril sind jeweils 0,1% Ameisensäure beigemischt. Die geeigneten Fraktionen werden mit 500 µl einer 1 M wässrigen Salzsäure versetzt und gefriergetrocknet. Das Produkt liegt als Dihydrochlorid vor.

| | |
|---|---|
| Ausbeute: | 33 mg (0,045 mmol; 35 %) |
| UVmax: | 314 nm |
| MS (ESI): | 659 (M+H)⁺ |
| ¹H-NMR: | 1.35 - 1.48 (m, 3H), 1.64 - 1.78 (m, 4H), 2.37 - 2.46 (m, 2H), 3.48 - 3.75 (m, 4H), 3.97 - 4.14 (m, 1H), 4.50 - 4.78 (m, 3H), 5.55 - 5.71 (m, 1H), 6.14 - 6.42 (m, 2H), 6.96 - 7.32 (m, 3H), 7.86 - 7.98 (m, 1H), 8.32 (s, 1H), 8.84 (s, 1H), 10.41 (s, 1H) |

### Beispiel 623

### 2-(2-Fluor-ethyl)-7-(2-{4-[4-(2-hydroxy-ethyl)-1H-imidazol-2-yl]-2-methoxy-phenylamin}-4-(2-(2-fluor-ethyl)-1-methyl-3-oxo-2,3-dihydro-1H-isoindol-4-ylamino)-5-trifluormethyl-pyrimidin

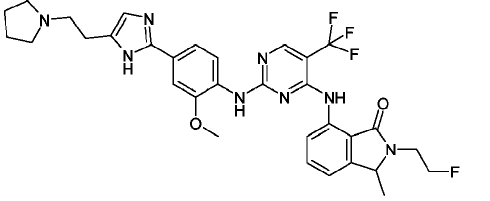

0,07 g (0,3 mmol) 2-[2-(4-Amino-3-methoxy-phenyl)-1*H*-imidazol-4-yl]-ethanol (Methode 27) werden in 2 ml Dioxan suspendiert und im Ultraschalbad bei 50 °C in Lösung gebracht. Es wird mit 0,8 ml (3,20 mmol) 4 N dioxanischer Salzsäure versetzt. Im Vakuum wird das Dioxan abgezogen, mit 0,096 g (0,247 mmol) 7-(2-Chlor-5-trifluormethyl-pyrimidin-4-ylamin)-2-(2-fluor-ethyl)-3-methyl-2,3-dihydro-isoindol-1-on versetzt und in Butanol suspendiert. Es wird 16 h bei 100 °C rühren gelassen. Das Rohprodukt wird durch Säulenchromatographie gereinigt. Als Trägermaterial dient C18-RP-Kieselgel. Es wird ein Gradient durchlaufen der am Startpunkt aus 75% Wasser und 25 % Acetonitril und am Endpunkt aus 30% Wasser und 70% Acetonitril besteht. Dem Wasser ist 0,1% Ammoniak beigemischt 23 mg dieses Zwischenprodukts und 0,018g (0,094 mmol) p-Toluensulfonylchloride werden in 0,9 ml Tetrahydrofuran und 0,02 ml (0,139 mmol) Triethylamin suspendiert und mit 0,007g (0,057 mmol) 4-Dimethylaminopyridine versetzt. Diese Reaktionsmischung rührt man 16 h bei 20 °C. Anschließend wird mit 0,36 ml (5,064 mmol) Pyrrolidin versetzt und man rührt man 16 h bei 60 °C. Das Rohprodukt wird durch Säulenchromatographie gereinigt. Als Trägermaterial dient C18-RP-Kieselgel. Es wird ein Gradient durchlaufen der am Startpunkt aus 90% Wasser und 10% Acetonitril und am Endpunkt aus 60% Wasser und 40% Acetonitril besteht. Dem Wasser ist 0,1 % Ameisensäure beigemischt.

| | |
|---|---|
| Ausbeute: | 7 mg (0,011 mmol, 28%) |
| MS (ESI): | 639 (M+H)⁺ |
| UV max: | 330 nm |
| NMR: | 1.42 - 1.46 (m, 3H), 1.78 - 2.08 (m, 6H), 2.29 (s, 1H), 3.95 - 4.16 (m, 4H), 4.52 - 4.78 (m, 3H), 7.09 - 7.13 (m, 1H), 7.24 - 7.28 (m, 1H), 7.46 - 7.50 (m, 1H), 7.52 - 7.58 (m, 2H), 7.64 - 7.67 (m, 1H), 7.82 - 7.88 (m, 1H), 8.02 - 8.13 (m, 2H), 8.50 - 8.60 (m, 2H), 9.20 - 9.23 (m, 1H), 10.52 - 10,82 (m, 2H). |

### Beispiele 624-638

Die folgenden Verbindungen sind über ein analoges Verfahren, wie in Beispiel 622 beziehungsweise 623 beschrieben, hergestellt Das entsprechende Anilin ist in Methode 27 und 28 beschrieben.

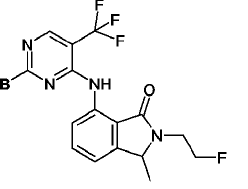

| **#** | **B** | **UV max [nm]** | **MS (ESI) (M+H)⁺** |
|---|---|---|---|
| 624 | 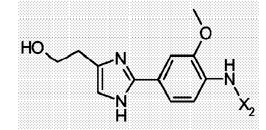 | 290,326 | 586 |
| 625 | 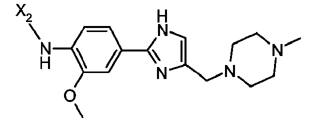 | 290,330 | 654 |
| 626 | 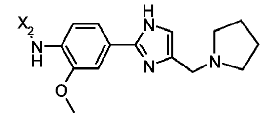 | 290,326 | 625 |
| 627 | 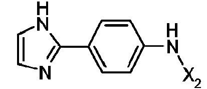 | 326 | 512 |
| 628 | 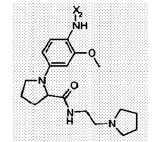 | 314 | 685 |
| 629 | 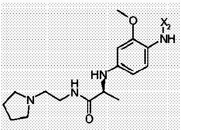 | 290,314 | 659 |
| 630 | 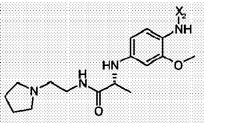 | | 659 |
| 631 | 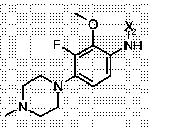 | 278 | 592 |
| 632 | 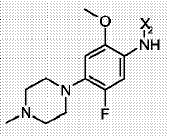 | 314 | 592 |
| 633 | 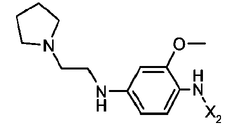 | 314 | 588 |
| 634 | 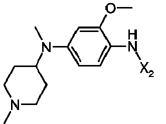 | 314 | 602 |
| 635 | 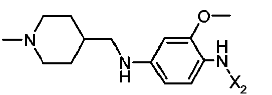 | 314 | 602 |
| 636 | 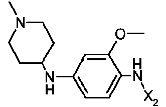 | 314 | 588 |
| 637 | 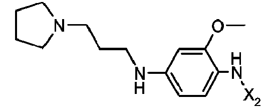 | 314 | 602 |
| 638 | 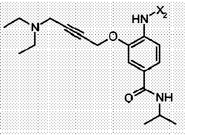 | | 670 |

### Beispiel 639

### 2-(4-(4-isonropyl-[1,4]diazepin-1-yl)-2-methoxy-phenylamino)-4-(2-(2-fluor-ethyl)-1-methyl-3-oxo-2,3-dihydro-1H-isoindol-4-ylamino)-5-trifluormethyl-pyrimidin

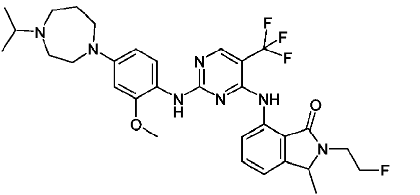

50 mg (0,087 mmol) 2-(4-(4-[1,4]diazepan-1-yl)-2-methoxy-phenylamino)-4-(2-(2-fluorethyl)-1-methyl-3-oxo-2,3-dihydro-1*H*-isoindol-4-ylamino)-5-trifluormethyl-pyrimidin (Verfahren aus Beispiel 622, Anilin aus Methode 28) werden in 0,5 ml Dimethylacetamid gelöst und mit 13 µl (0,174 mmol) Aceton versetzt. Zu dieser Reaktionsmischung gibt man 37 mg (0,175 mmol) Natriumtriacetoxyborhydrid. Nach 16 h bei 20 °C wird das Lösungsmittel im Vakuum entfernt. Der Rückstand wird durch Säulenchromatographie gereinigt. Als Trägermaterial dient C18-RP-Kieselgel und es wird ein Gradient innerhalb von 15 min durchlaufen der am Startpunkt aus 95% Wasser und 5% Acetonitril und am Endpunkt aus 5% Wasser und 95% Acetonitril besteht. Sowohl dem Wasser als auch dem Acetonitril sind jeweils 0,1 % Ameisensäure beigemischt. Die geeigneten Fraktionen werden mit 500 µl einer 1 M wässrigen Salzsäure versetzt und gefriergetrocknet. Das Produkt liegt als Dihydrochlorid vor.

| | |
|---|---|
| Ausbeute: | 51 mg (0,074 mmol; 85 %) |
| UV max: | 314 nm |
| MS (ESI): | 616 (M+H)⁺ |
| ¹H-NMR: | 1,23-1,35 (m, 6H), 1,35-1,51 (m, 3H), 2,16-2,29 (m, 1H), 2,95-3,05 (m, 1H), 3,12-3,23 (m, 1H), 3,42-3,66 (m, 6H), 3,78 (s, 3H), 3,83-4,00 (m, 2H), 4,00-4,16 (m, 1H), 4,50-4,79 (m, 3H), 6,32-6,63 (m, 2H), 7,08-8,59 (m, 4H), 9,24-9,76 (m, 1H), 10,67 (s, 2H) |

### Beispiele 640-648

Die folgenden Verbindungen sind über ein analoges Verfahren, wie in Beispiel 639 beschrieben, hergestellt

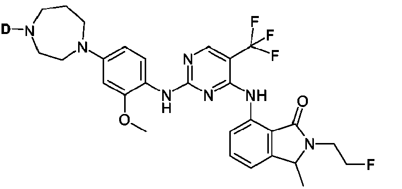

| **#** | **D** | **UV max [nm]** | **MS (ESI) (M+H)⁺** |
|---|---|---|---|
| 640 | 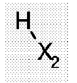 | 314 | 574 |
| 641 | 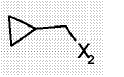 | 310-314 | 628 |
| 642 | 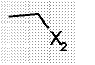 | 310-314 | 602 |
| 643 | 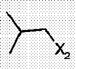 | 310-314 | 630 |
| 644 | 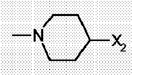 | 314 | 671 |
| 645 | 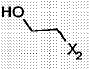 | 310-314 | 618 |
| 646 | 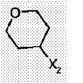 | 314 | 658 |
| 647 | 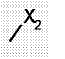 | 314 | 588 |

### Beispiele 648-659

Die folgenden Verbindungen sind über ein analoges Verfahren, wie in Beispiel 639 beschrieben, hergestellt. Für die reduktive Aminierung wird 2-(2-methoxy-4-piperazin-1-yl-phenylamino)-4-(2-(2-fluor-ethyl)-1-methyl-3-oxo-2,3-dihydro-1*H*-isoindol-4-ylamino)-5-trifluormethyl-pyrimidin eingesetzt. Das Anilin für die Herstellung diese Verbindung ist in Methode 28 beschrieben.

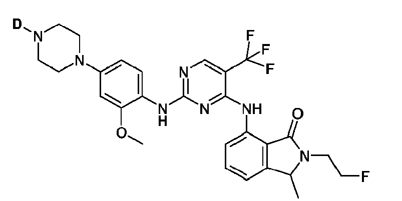

| **#** | **D** | **UV max [nm]** | **MS (ESI) (M+H)⁺** |
|---|---|---|---|
| 648 | 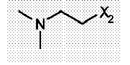 | 286, 314 | 631 |
| 649 | 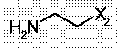 | 286, 314 | 603 |
| 650 | 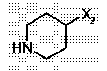 | 282, 314 | 643 |
| 651 | 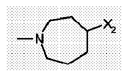 | 282, 314 | 671 |
| 652 | 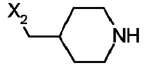 | 286, 314 | 657 |
| 653 | 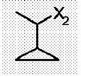 | 282, 314 | 628 |
| 654 | 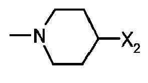 | 286, 314 | 657 |
| 655 | 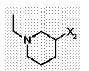 | 286, 314 | 671 |
| 656 | 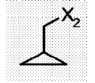 | 282, 314 | 614 |
| 657 | 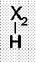 | 282, 314 | 560 |
| 658 | 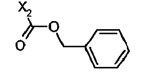 | 234, 283, 314 | 694 |
| 659 | 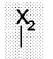 | 286, 314 | 574 |

### Beispiele 660-666

Die folgenden Verbindungen sind über ein analoges Verfahren, wie in Beispiel 53 beschrieben, hergestellt. 2-(4-Carboxy-2-brom-phenylamino)-4-chlor-5-trifluormethyl-pyrimidin kann nach Methode 29 hergestellt werden. Das entsprechende Anilin ist in Methode 22 beschrieben. Das für die Darstellung des Amids eingesetzte Amin ist kommerziell erhältlich beziehungsweise in Methode 13 beschrieben.

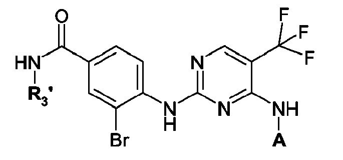

| **#** | **A** | **R₃'** | **UV max [nm]** | **MS (ESI) (M+H)⁺** |
|---|---|---|---|---|
| 660 | 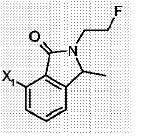 | 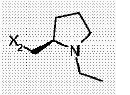 | 314 | 678/680 |
| 661 | 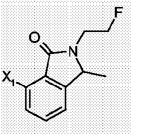 | 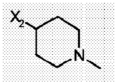 | 314 | 626/628 |
| 662 | 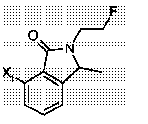 | 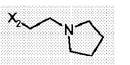 | 314 | 626/628 |
| 663 | 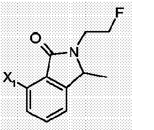 | 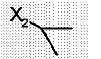 | 286 | 609/611 |
| 664 | 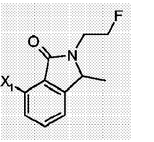 | 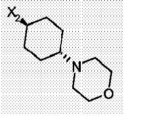 | 314 | 734/736 |
| 665 | 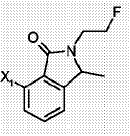 | 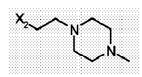 | 314 | 693/695 |
| 666 | 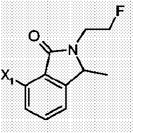 | 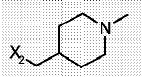 | 286 | 678/680 |

### Beispiele 667-681

Die folgenden Verbindungen sind über ein analoges Verfahren, wie in Beispiel 53 beschrieben, hergestellt. 2-(4-Carboxy-phenylamino)-4-chlor-5-trifluormethyl-pyrimidin kann nach Methode 14 hergestellt werden. Das entsprechende Anilin ist in Methode 22 beschrieben. Das für die Darstellung des Amids eingesetzte Amin ist kommerziell erhältlich oder in Methode 13 beschrieben. Außerdem kann der Rest R₃' analog zu Beispiel 639 über eine reduktive Aminierung aufgebaut werden. Dabei wird ein Amin eingesetzt, welches in der Seitenkette eine weitere geschützte Aminofunktion besitzt. Als Schutzgruppe kommt eine *tert*-Butoxycarbonyl-, Benzyloxycarbonyl- oder Benzyl-Gruppe in Frage. Die Abspaltung dieser Schutzgruppe erfolgt nach einer dem Fachmann geläufigen Prozedur und eine reduktive Aminierung (analog Beispiel 639) oder eine Alkylierung (analog Methode 34 beziehungsweise WO2004052857) stellen die letzten Schritte in dieser Sequenz dar.

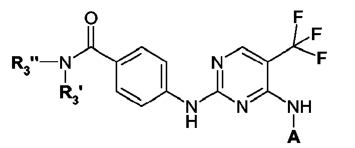

| **#** | **A** | **R₃'** | **R₃"** | **UV max [nm]** | **MS (ESI) (M+H)⁺** |
|---|---|---|---|---|---|
| 667 | 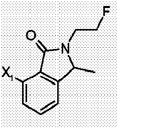 | 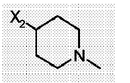 | H | 314 | 586 |
| 668 | 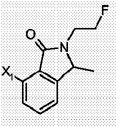 | 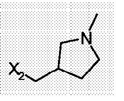 | H | 314 | 586 |
| 669 | 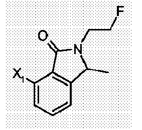 | 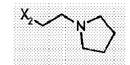 | H | 314 | 586 |
| 670 | 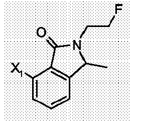 | 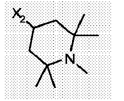 | H | 314 | 642 |
| 671 | 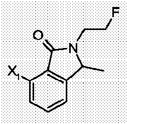 | 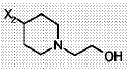 | H | 314 | 616 |
| 672 | 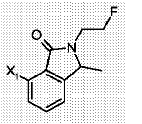 | 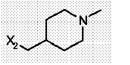 | H | 290 | 600 |
| 673 | 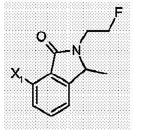 | 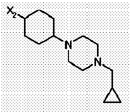 | H | 290 | 709 |
| 674 | 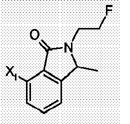 | 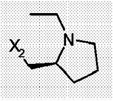 | H | 314 | 600 |
| 675 | 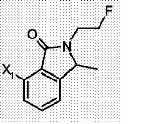 | 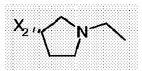 | H | 314 | 586 |
| 676 | 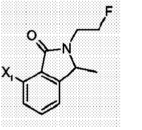 | 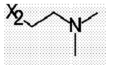 | 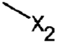 | 286 | 574 |
| 677 | 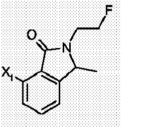 | 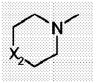 | H | 286 | 572 |
| 678 | 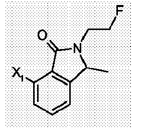 | 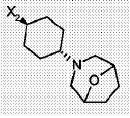 | H | 290 | 682 |
| 679 | 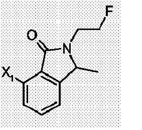 | 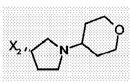 | H | 314 | 642 |
| 680 | 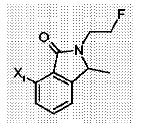 | 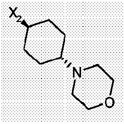 | H | 290 | 656 |
| 681 | 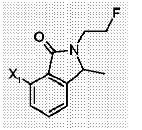 | 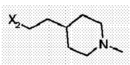 | H | 314 | 615 |

### Beispiel 682

### 2-(2-methoxy-4-[3-(4-methyl-piperazin-1-yl)-propionylamino]-phenylamino)-4-(2-(2-fluor-ethyl)-1-methyl-3-oxo-2,3-dihydro-1H-isoindol-4-ylamino)-5-trifluormethyl-pyrimidin

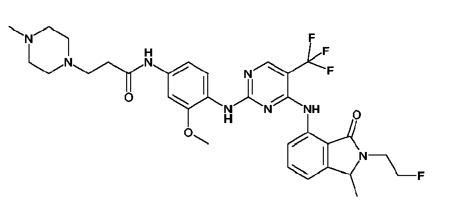

63 mg (0,116 mmol) 2-(4-Acryloylamino-2-methoxy-phenylamino)-4-(2-(2-fluor-ethyl)-1-methyl-3-oxo-2,3 -dihydro-1*H*-isoindol-4-ylamino)-5-trifluormethyl-pyrimidin (Methode 30) werden in 1 ml Methanol gelöst und mit 70 mg (0,699 mmol) N-Methyl-piperazin versetzt. Nachdem man 48 h bei 20 °C gerührt hat wird das Lösungsmittel im Vakuum entfernt. Der Rückstand wird durch Säulenchromatographie gereinigt. Als Trägermaterial dient C18-RP-Kieselgel und es wird ein Gradient innerhalb von 20 min durchlaufen der am Startpunkt aus 95% Wasser und 5% Acetonitril und am Endpunkt aus 2% Wasser und 98% Acetonitril besteht. Sowohl dem Wasser als auch dem Acetonitril sind jeweils 0,1% Ameisensäure beigemischt. Die geeigneten Fraktionen werden mit 500 µl einer 1 M wässrigen Salzsäure versetzt und gefriergetrocknet. Das Produkt liegt als Dihydrochlorid vor.

| | |
|---|---|
| Ausbeute: | 58 mg (0,081 mmol; 70 %) |
| UV max: | 282 nm |
| MS (ESI): | 645 (M+H)⁺ |
| ¹H-NMR: | 1.42 (d, 3H), 2.18 (s, 3H), 2.29 - 2.43 (m, 4H), 2.65 - 2.70 (m, 2H), 3.50 - 3.62 (m, 1H), 3.72 (s, 3H), 4.00 - 4.12 (m, 1H), 4.52 - 4.76 (m, 3H), 7.12 - 7.17 (m, 1H), 7.12 - 7.42 (m 4H), 7.51 (s, 1H), 8.17 (s, 1H), 8.38 (s, 1H), 9.08 (s, 1H), 10.18 (s, 1H), 10.46 (s, 1H) |

### Beispiele 683-692

Die folgenden Verbindungen sind über ein analoges Verfahren, wie in Beispiel 682 beschrieben, hergestellt.

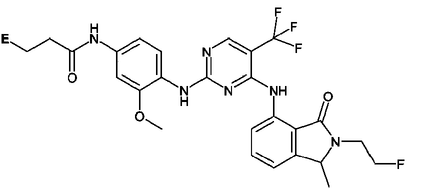

| **#** | **E** | **UV max [nm]** | **MS (ESI) (M+H)⁺** |
|---|---|---|---|
| 683 | 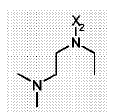 | 282 | 661 |
| 684 | 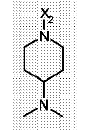 | 282 | 673 |
| 685 | 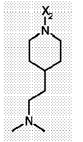 | 282 | 701 |
| 686 | 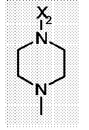 | 282 | 645 |
| 687 | 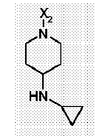 | 282 | 685 |
| 688 | 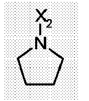 | 282 | 616 |
| 689 | 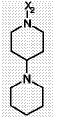 | 282 | 713 |
| 690 | 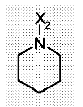 | 282 | 630 |
| 691 | 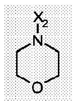 | 282 | 632 |
| 692 | 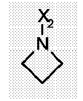 | 282 | 602 |

### Beispiele 693-704

Die folgenden Verbindungen sind über ein analoges Verfahren, wie in Beispiel 682 beschrieben, hergestellt. 2-(4-(2-Bromo-acetylamino)-2-methoxy-phenylamino)-4-(2-(2-fluor-ethyl)-1-methyl-3-oxo-2,3-dihydro-1*H*-isoindol-4-ylamino)-5-trifluormethyl-pyrimidin beziehungsweise 2-(4-(2-Bromo-acetylamino)-2-brom-phenylamino)-4-(2-(2-fluor-ethyl)-1-methyl-3-oxo-2,3-dihydro-1*H*-isoindol-4-ylamino)-5-trifluormethyl-pyrimidin oder 2-[5-(2-Bromo-acetylamino)-pyridin-2-ylamino]-4-(2-(2-fluor-ethyl)-1-methyl-3-oxo-2,3-dihydro-1*H*-isoindol-4-ylamino)-5-trifluormethyl-pyrimidin, welche in Methode 30 beschrieben sind, werden als Edukt für die nukleophile Substitution eingesetzt.

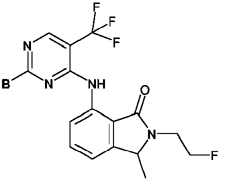

| **#** | **B** | **UV max [nm]:** | **MS (ESI) (M+H)⁺:** |
|---|---|---|---|
| 693 | 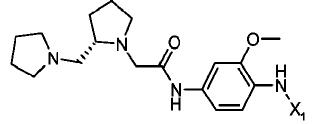 | 282 | 685 |
| 694 | 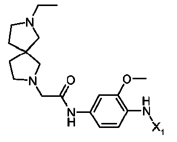 | 282 | 685 |
| 695 | 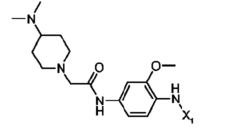 | 314 | 659 |
| 696 | 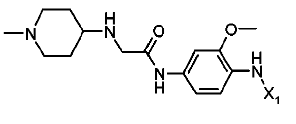 | 282 | 645 |
| 697 | 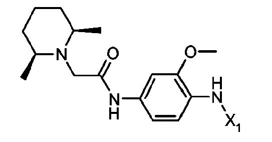 | 282 | 644 |
| 698 | 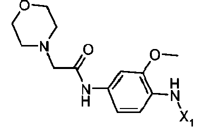 | 282 | 618 |
| 699 | 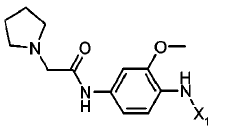 | 282 | 602 |
| 700 | 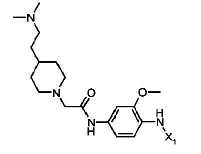 | 282 | 687 |
| 701 | 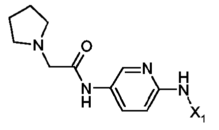 | 322 | 573 |
| 702 | 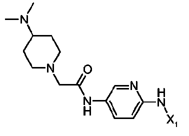 | 322 | 630 |
| 703 | 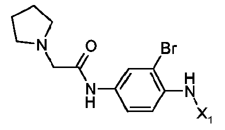 | 222 | 650 |
| 704 | 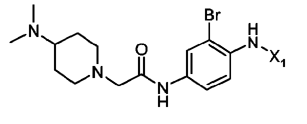 | 278 | 707 |

### Beispiel 705

### 2-(2-methoxy-4-[3-(3-pyrrolidin-1-yl-ethyl)-ureido]-phenylamino)-4-(2-(2-fluor-ethyl)-1-methyl-3-oxo-2,3-dihydro-1H-isoindol-4-ylamino)-5-trifluormethyl-pyrimidin

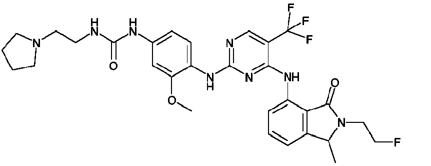

70 mg (0,135 mmol) 2-(4-Carboxy-2-methoxy-phenylamino)-4-(2-(2-fluor-ethyl)-1-methyl-3-oxo-2,3-dihydro-1*H*-isoindol-4-ylamino)-5-trifluormethyl-pyrimidin (analog Beispiel 53) werden in 2 ml Toluol gelöst und mit 190 µl (1,348 mmol) Triethylamin und 60 µl (0,270 mmol) Diphenylphosphorylazid versetzt. Diese Reaktionsmischung wird 48 h bei 20 °C gerührt. Anschließend temperiert man die Suspension 2 h auf 95 °C, wobei eine braune klare Lösung entsteht. Daraufhin gibt man 31 mg (0,270 mmol) 1-(2-Aminoethyl)-pyrrolidin zu und lässt nochmals 1 h bei 95 °C rühren. Das Lösungsmittel wird im Vakuum entfernt. Der Rückstand wird durch Säulenchromatographie gereinigt. Als Trägermaterial dient C18-RP-Kieselgel und es wird ein Gradient innerhalb von 15 min durchlaufen der am Startpunkt aus 95% Wasser und 5% Acetonitril und am Endpunkt aus 2% Wasser und 98% Acetonitril besteht. Sowohl dem Wasser als auch dem Acetonitril sind jeweils 0,1% Ameisensäure beigemischt. Die geeigneten Fraktionen werden mit 5 M Natronlauge basisch gestellt und 4mal mit je 50 ml Dichlormethan extrahiert. Die vereinigten organischen Phasen werden getrocknet und das Lösungsmitteln im Vakuum entfernt.

| | |
|---|---|
| Ausbeute: | 42 mg (0,067 mmol; 50 %) |
| UV max: | 282 nm |
| MS (ESI): | 631 (M+H)⁺ |
| ¹H-NMR: | 1.42 - 1.48 (m, 3H), 1.69 -1.79 (m, 4H), 3.22 - 3.28 (m, 2H), 3.49 - 3.62 (m, 1M, 3.70 (s, 3H), 3.99 - 4.12 (m, 1H), 4.53 - 4.76 (m, 3H), 6.17 (s, 1H), 6.84 - 6.91 (m, 1H), 7.15 - 7.33 (m, 3H), 7.40 (s, 1H), 8.36 (s, 1H), 8.76 (s, 1H), 9.01 (s, 1H), 10.44 (s, 1H) |

### Beispiel 706

### 2-(2-methoxy-4-ureido-phenylamino)-4-(2-(2-fluor-ethyl)-1-methyl-3-oxo-2,3-dihydro-1H-isoindol-4-ylamino)-5-trifluormethyl-pyrimidin

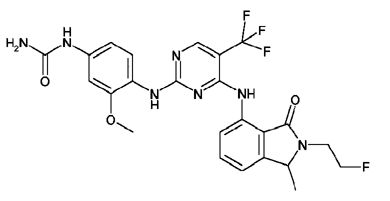

Diese Verbindung wird analog zu Beispiel 705 hergestellt.

| | |
|---|---|
| UV max: | 282 / 314 nm |
| MS (ESI): | 534 (M+H)⁺ |
| ¹H-NMR: | 1.42 (d, 3H), 3.48 - 3.64 (m, 1H), 3.69 (s, 3H), 3.98 - 4.13 (m, 1H), 4.50 - 4.77 (m, 3H), 5.89 (s, 2 H), 6.94 (d, 1H), 7.16 - 7.30 (m, 2H), 7.36 (s, 1H), 8.33 - 8.41 (m, 2H), 8.38 (s, 1H), 8.73 (s, 1H), 9.00 (s, 1H), 10.44 (s, 1H) |

### Beispiel 707

### 2-(2-methoxy-4-[(1-methyl-piperidin-4-carbonyl)-amino]-phenylamino)-4-(2-(2-fluorethyl)-1-methyl-3-oxo-2,3-dihydro-1H-isoindol-4-ylamino)-5-trifluormethyl-pyrimidin

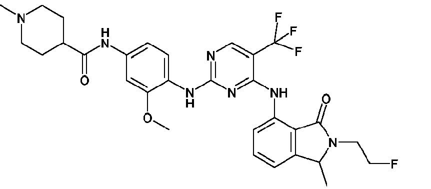

Ausgehend von 2-(4-Amino-2-methoxy-phenylamino)-4-(2-(2-fluor-ethyl)-1-methyl-3-oxo-2,3-dihydro-1*H*-isoindol-4-ylamino)-5-trifluormethyl-pyrimidin (Methode 30) wird über eine dem Fachmann geläufige Amidknüpfungsmethode oben genanntes Produkt hergestellt (siehe auch Beispiel 53 beziehungsweise 1032). Die Substanz liegt als freie Base vor.

| | |
|---|---|
| UV max: | 282 nm |
| MS (ESI): | 616 (M+H)⁺ |
| ¹H-NMR (400 MHz, CDCl₃): | 1.51 (d, 3H), 2.25 - 2.32 (m, 1H), 2.36 (s, 3H), 3.00 - 3.07 (m, 2H), 3.53 - 3.65 (m, 1H), 3.92 (s, 3H), 4.13 - 4.27 (m, 1H), 4.56 - 4.77 (m, 3H), 6.84 (d, 1H), 7.07 (d, 1H), 7.44 (s, 1H), 7.47 - 7.54 (m, 1H), 7.57 (s, 1H), 7.62 (s, 1H), 8.16 - 8.24 (m, 1H), 8.39 (s, 1H), 8.60 - 8.68 (m, 1H), 10.42 (s, 1H) |

### Beispiele 708-795

Über ein analoges Verfahren wie in Beispiel 53 beschrieben wird an 2-(4-Carboxy-2-methoxy-phenylamino)-4-[2-(2-fluor-ethyl)-1-methyl-3-oxo-2,3-dihydro-1*H*-isoindol-4-ylamino]-5-trifluormethyl-pyrimidin ein primäres Amin gekuppelt, welches in der Seitenkette eine weitere geschützte Aminofunktion besitzt. Als Schutzgruppe kommt eine *tert*-Butoxycarbonyl-, Benzyloxycarbonyl- oder Benzyl-Gruppe in Frage. Die Abspaltung dieser Schutzgruppe nach einer dem Fachmann geläufigen Prozedur und eine reduktive Aminierung (analog Beispiel 639) oder Alkylierung (analog Methode 34 beziehungsweise WO2004052857) stellen die letzten Schritte in dieser Sequenz dar.

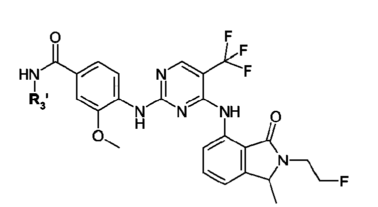

| **#** | **R₃'** | **UV max [nm]** | **MS (ESI) (M+H)⁺** |
|---|---|---|---|
| 708 | 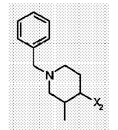 | 285, 322 | 706 |
| 709 | 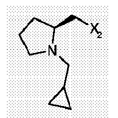 | 285, 322 | 656 |
| 710 | 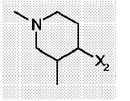 | 285, 322 | 630 |
| 711 | 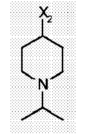 | 322, 286 | 644 |
| 712 | 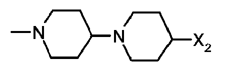 | 325, 286 | 699 |
| 713 | 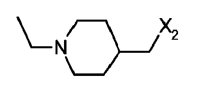 | 282, 318 | 644 |
| 714 | 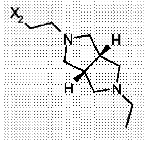 | 326 | 685 |
| 715 | 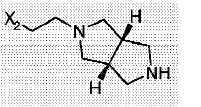 | 326 | 658 |
| 716 | 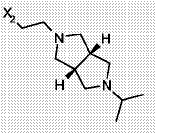 | 326 | 699 |
| 717 | 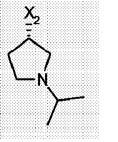 | 326 | 630 |
| 718 | 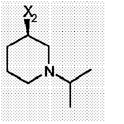 | 326 | 644 |
| 719 | 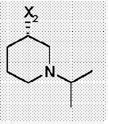 | 322 | 644 |
| 720 | 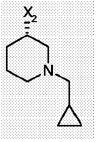 | 326 | 656 |
| 721 | 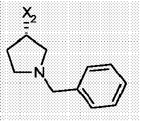 | 326 | 678 |
| 722 | 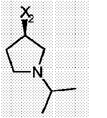 | 314 | 630 |
| 723 | 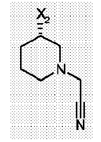 | 322 | 641 |
| 724 | 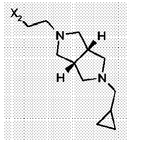 | 326 | 712 |
| 725 | 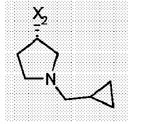 | 326 | 642 |
| 726 | 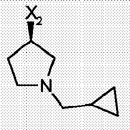 | 322 | 642 |
| 727 | 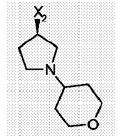 | 318 | 672 |
| 728 | 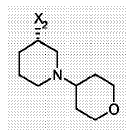 | 301 | 686 |
| 729 | 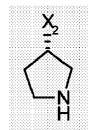 | 326 | 588 |
| 730 | 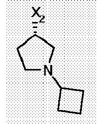 | 326 | 642 |
| 731 | 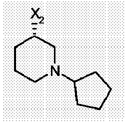 | 326 | 670 |
| 732 | 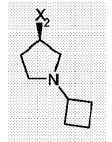 | 326 | 642 |
| 733 | 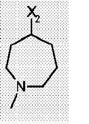 | 326 | 630 |
| 734 | 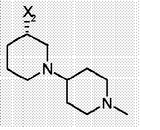 | 326 | 699 |
| 735 | 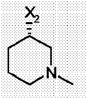 | 310 | 616 |
| 736 | 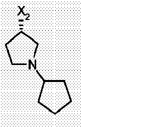 | 326 | 656 |
| 737 | 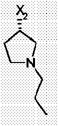 | 322 | 630 |
| 738 | 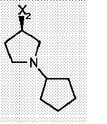 | 326 | 656 |
| 739 | 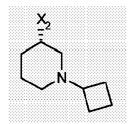 | 326 | 656 |
| 740 | 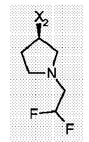 | 266 | 652 |
| 741 | 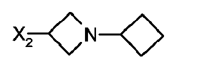 | 326 | 629 |
| 742 | 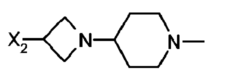 | 326 | 671 |
| 743 | 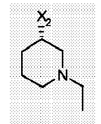 | 326 | 630 |
| 744 | 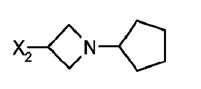 | 326 | 642 |
| 745 | 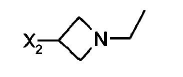 | 326 | 602 |
| 746 | 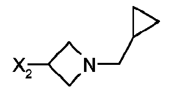 | 326 | 628 |
| 747 | 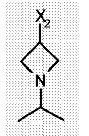 | 326 | 616 |
| 748 | 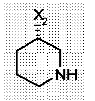 | 326 | 602 |
| 749 | 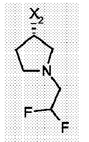 | 322 | 652 |
| 750 | 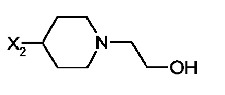 | 326 | 646 |
| 751 | 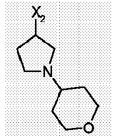 | 326 | 672 |
| 752 | 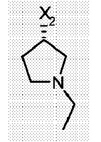 | 326 | 616 |
| 753 | 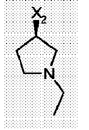 | 326 | 616 |
| 754 | 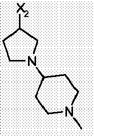 | 326 | 685 |
| 755 | 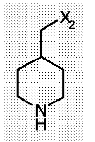 | 322 | 616 |
| 756 | 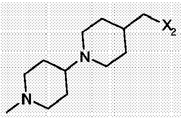 | 318 | 713 |
| 757 | 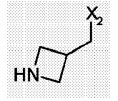 | 286, 322 | 588 |
| 758 | 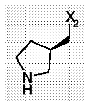 | 226, 286, 322 | 602 |
| 759 | 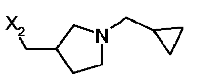 | 322-326 | 656 |
| 760 | 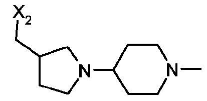 | 322-326 | 699 |
| 761 | 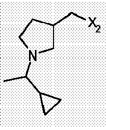 | 322-326 | 670 |
| 762 | 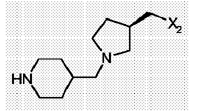 | 322-326 | 699 |
| 763 | 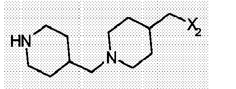 | 322 | 713 |
| 764 | 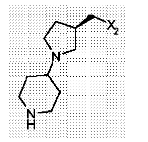 | 326 | 685 |
| 765 | 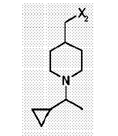 | 322 | 684 |
| 766 | 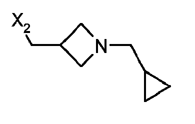 | 326 | 642 |
| 767 | 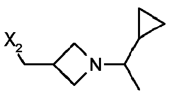 | 322-326 | 656 |
| 768 | 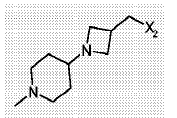 | 322-326 | 685 |
| 769 | 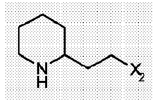 | 322-326 | 630 |
| 770 | 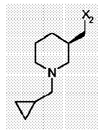 | 286,322 | 670 |
| 771 | 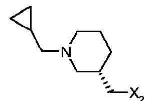 | 286, 322 | 670 |
| 772 | 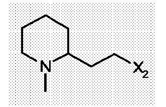 | 322-326 | 644 |
| 773 | 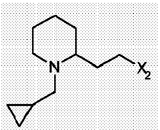 | 322 | 684 |
| 774 | 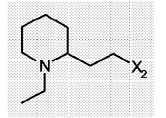 | 322-326 | 658 |
| 775 | 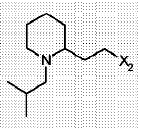 | 322 | 686 |
| 776 | 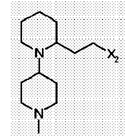 | 322-326 | 727 |
| 777 | 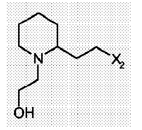 | 322-326 | 674 |
| 778 | 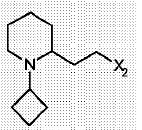 | 322-326 | 684 |
| 796 | 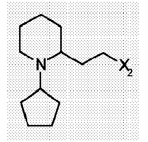 | 322-326 | 698 |
| 780 | 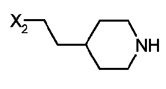 | 286, 322 | 630 |
| 781 | 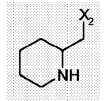 | 282, 314 | 616 |
| 782 | 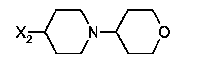 | 322, 286 | 686 |
| 783 | 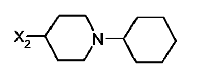 | 326 | 684 |
| 784 | 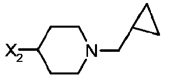 | 324, 286 | 656 |
| 785 | 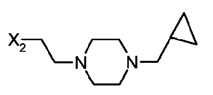 | 326, 286 | 685 |
| 786 | 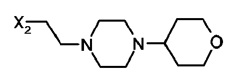 | 322, 286 | 715 |
| 787 | 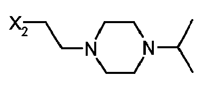 | 322, 286 | 673 |
| 788 | 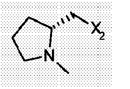 | 285, 322 | 616 |
| 789 | 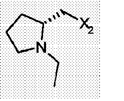 | 285, 322 | 630 |
| 790 | 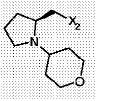 | 285, 322 | 686 |
| 791 | 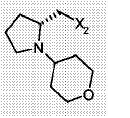 | 285, 322 | 686 |
| 792 | 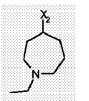 | 326 | 644 |
| 793 | 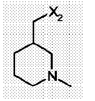 | 322 | 630 |
| 794 | 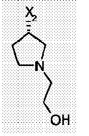 | 326 | 631 |
| 795 | 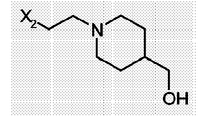 | 326 | 660 |

### Beispiel 796

### 2-[2-methoxy-4-(2-methyl-2-pyrrolidin-1-yl-propylcarbamoyl)-phenylamino]-4-(2-(2-fluor-ethyl)-1-methyl-3-oxo-2,3-dihydro-1H-isoindol-4-ylamino)-5-trifluormethyl-pyrimidin

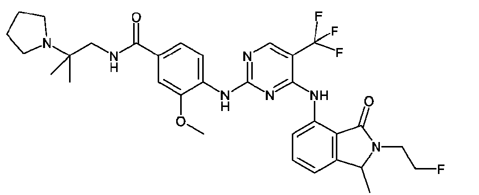

200 mg (0,385 mmol) 2-(4-Carboxy-2-methoxy-phenylamino)-4-[2-(2-fluor-ethyl)-1-methyl-3-oxo-2,3-dihydro-1*H*-isoindol-4-ylamino]-5-trifluormethyl-pyrimidin (analog Beispiel 53) werden in 1 ml Dimethylformamid gelöst auf 0 °C abgekühlt und mit 520 µl (3,038 mmol) Diisopropylethylamin und 160 mg (0,498 mmol) *O*-(Benzotriazol-1-yl)-*N,N,N,N'*-tetramethyluronium-tetrafluorborat versetzt. Diese Lösung tropft man nach 10 min langsam zu 56 µl (0,539 mmol) 1,2-Diamino-2-methylpropan, welches in 300 µl Dimethylformamid gelöst ist. Die Reaktionsmischung rührt man 24 h bei 20°C und entfernt anschließend das Lösungsmittel im Vakuum. Der Rückstand wird durch Säulenchromatographie gereinigt. Als Trägermaterial dient C18-RP-Kieselgel und es wird ein Gradient innerhalb von 15 min durchlaufen der am Startpunkt aus 90% Wasser und 10% Acetonitril und am Endpunkt aus 50% Wasser und 50% Acetonitril besteht. Sowohl dem Wasser als auch dem Acetonitril sind jeweils 0,1% Ameisensäure beigemischt. Die geeigneten Fraktionen werden gefriergetrocknet. Dieses Zwischenprodukt wird mit 70 mg (0,515 mmol) Kaliumcarbonat und mit 84 mg (0,506 mmol) Kaliumjodid versetzt und in 2 ml Acetonitril suspendiert. Zu dieser Mischung gibt man 20 µl (0,170 mmol) 1,4-Dibrombutan und rührt unter Rückflussbedingungen 16 h. Anschließend werden die Lösungsmittel im Vakuum entfernt und der Rückstand wird durch Säulenchromatographie gereinigt Als Trägermaterial dient C18-RP-Kieselgel und es wird ein Gradient innerhalb von 15 min durchlaufen der am Startpunkt aus 90% Wasser und 10% Acetonitril und am Endpunkt aus 50% Wasser und 50% Acetonitril besteht. Sowohl dem Wasser als auch dem Acetonitril sind jeweils 0,1% Ameisensäure beigemischt. Die geeigneten Fraktionen werden mit 0,5 ml 1 N Salzsäure versetzt und gefriergetrocknet. Das Produkt liegt als Dihydrochlorid vor.

| | |
|---|---|
| Ausbeute: | 20 mg (0,032 mmol, 8%) |
| UV max: | 325 nm |
| MS (ESI): | 644 (M+H)⁺ |
| ¹H-NMR (400 MHz): | 1.30 - 1.47 (m, 9H), 1.85 - 2.01 (m, 4H), 3.20 - 3.31 (m, 2H), 3.91 (s, 3H), 3.99 - 4.15 (m, 1H), 4.51 - 4.78 (m, 3H), 7.23 - 7.29 (m, 1H), 7.39 - 7.47 (m, 1H), 7.63 - 7.69 (m, 1H), 7.73 - 7.77 (m, 1H), 7.79 - 7.87 (m, 1H), 8.40 - 8.59 (m, 2H), 8.75 - 8.82 (m, 1H), 9.16 - 9.21 (m, 1H), 10.50 - 10.63 (m, 2H) |

### Beispiele 797-806

Über ein analoges Verfahren wie in Beispiel 796 beschrieben werden folgende Verbindungen hergestellt:

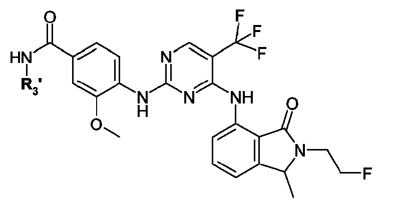

| **#** | **R₃'** | **UV max [nm]** | **MS (ESI) (M+H)⁺** |
|---|---|---|---|
| 797 | 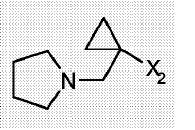 | 285, 325 | 642 |
| 798 | 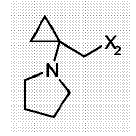 | 284, 325 | 642 |
| 799 | 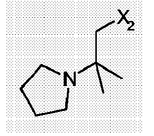 | 325, 285 | 644 |
| 800 | 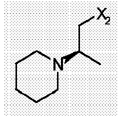 | 325, 285 | 644 |
| 801 | 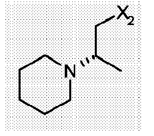 | 325, 285 | 644 |
| 802 | 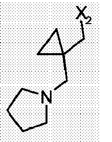 | 325, 285 | 656 |
| 803 | 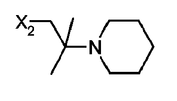 | 325, 285 | 658 |
| 804 | 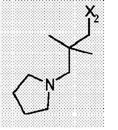 | 325, 284 | 658 |
| 805 | 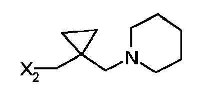 | 326, 286 | 670 |
| 806 | 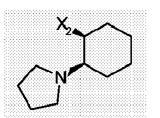 | 324, 285 | 670 |

### Beispiele 807-821

Die folgenden Verbindungen sind über ein analoges Verfahren, wie in Beispiel 53 beschrieben, hergestellt. Das entsprechende Anilin ist in Methode 31 beschrieben. Das für die Darstellung des Amids eingesetzte Amin ist kommerziell erhältlich beziehungsweise ist in Methode 13, 21 beziehungsweise in Methode 25 beschrieben.

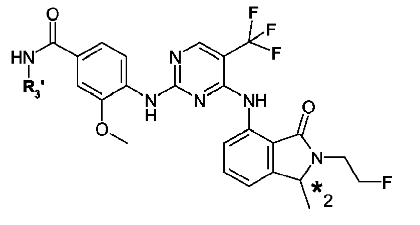

| **#** | **R₃'** | **UV max [nm]** | **MS (ESI) (M+H)⁺** |
|---|---|---|---|
| 807 | 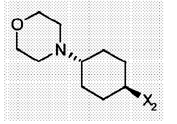 | 286, 322 | 686 |
| 808 | 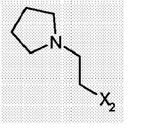 | 286, 322 | 616 |
| 809 | 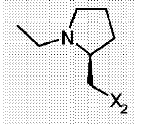 | 286, 322 | 630 |
| 810 | 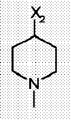 | 286, 322 | 616 |
| 811 | 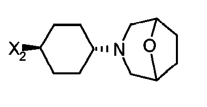 | 286, 322 | 712 |
| 812 | 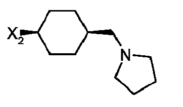 | 322, 286 | 684 |
| 813 | 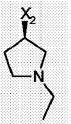 | | 689 |
| 814 | 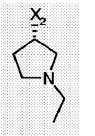 | 278 | 689 |
| 815 | 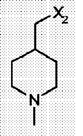 | 322 | 630 |
| 816 | 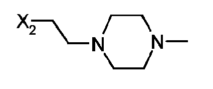 | 286, 326 | 645 |
| 817 | 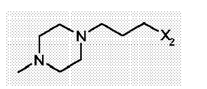 | 285, 322 | 659 |
| 818 | 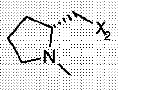 | 285, 322 | 616 |
| 819 | 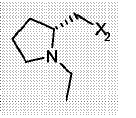 | 285, 322 | 630 |
| 820 | 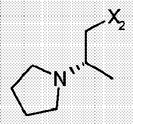 | | 630 |
| 821 | 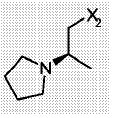 | 322, 286 | 630 |

### Beispiele 822-885

Die folgenden Verbindungen sind über ein analoges Verfahren, wie in Beispiel 53 beschrieben, hergestellt. Das entsprechende Anilin ist in Methode 31 beschrieben. Das für die Darstellung des Amids eingesetzte Amin ist kommerziell erhältlich, in Methode 13, 15, 20,21,23,24 und 25 beziehungsweise in J. Med. Chem. 2003, 46(5), 702-715 beschrieben.

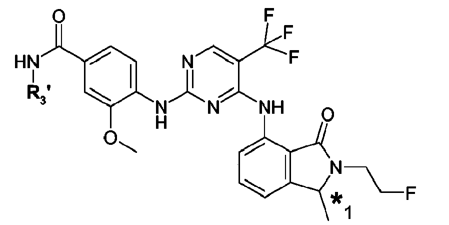

| **#** | **R₃'** | **UV max [nm]** | **MS (ESI) (M+H)⁺** |
|---|---|---|---|
| 822 | 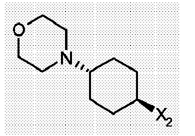 | 286, 322 | 686 |
| 823 | 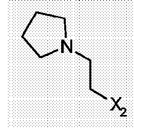 | 325, 284 | 616 |
| 824 | 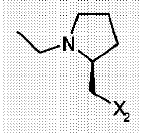 | 286, 326 | 630 |
| 825 | 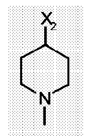 | 286, 322 | 616 |
| 826 | 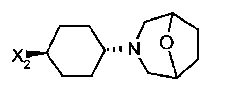 | 286, 318 | 712 |
| 827 | 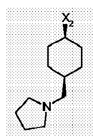 | 286, 322 | 684 |
| 828 | 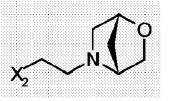 | 326 | 645 |
| 829 | 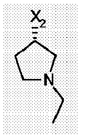 | 316 | 689 |
| 830 | 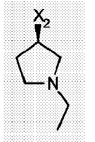 | 322 | 689 |
| 831 | 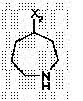 | | 616 |
| 832 | 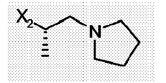 | 318 | 630 |
| 833 | 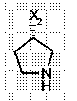 | 326 | 588 |
| 834 | 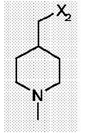 | 322 | 630 |
| 835 | 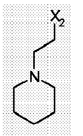 | 286, 322 | 630 |
| 836 | 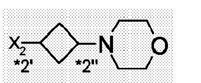 | | 658 |
| 837 | 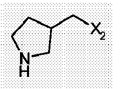 | 322-326 | 602 |
| 838 | 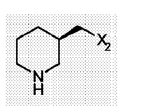 | 322-326 | 616 |
| 839 | 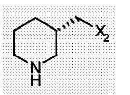 | 322 | 616 |
| 840 | 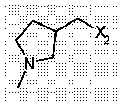 | 322-326 | 616 |
| 841 | 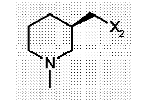 | 322-326 | 630 |
| 842 | 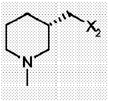 | 322-326 | 630 |
| 843 | 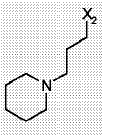 | 286, 322 | 644 |
| 844 | 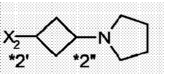 | 286, 322 | 642 |
| 845 | 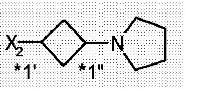 | 286, 322 | 642 |
| 846 | 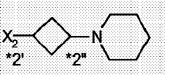 | 286, 322 | 656 |
| 847 | 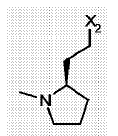 | 282, 318 | 630 |
| 848 | 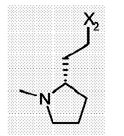 | 282, 322 | 630 |
| 849 | 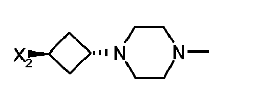 | 286, 318 | 671 |
| 850 | 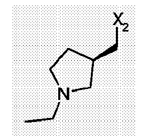 | 286, 322 | 630 |
| 851 | 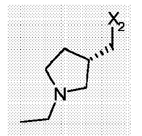 | 286, 322 | 630 |
| 852 | 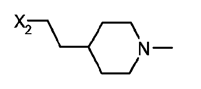 | 286, 322 | 644 |
| 853 | 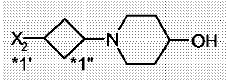 | 322-326 | 672 |
| 854 | 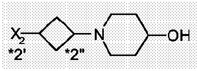 | 322 | 672 |
| 855 | 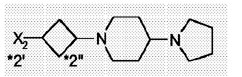 | 286, 322 | 725 |
| 856 | 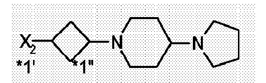 | 286, 322 | 725 |
| 857 | 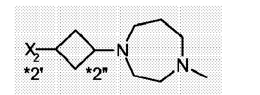 | 322-326 | 685 |
| 858 | 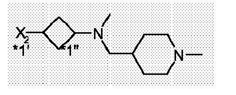 | 286, 322 | 713 |
| 859 | 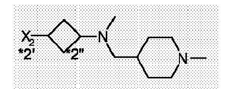 | 286, 322 | 713 |
| 860 | 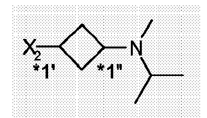 | 286, 322 | 644 |
| 861 | 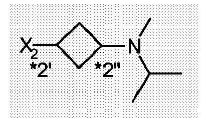 | 286, 322 | 644 |
| 862 | 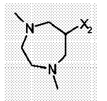 | 318-322 | 645 |
| 863 | 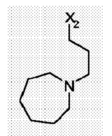 | 286, 322 | 658 |
| 864 | 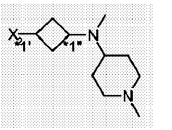 | 286, 322 | 699 |
| 865 | 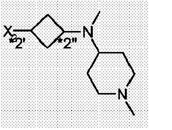 | 286, 322 | 699 |
| 866 | 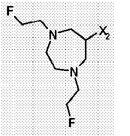 | 326 | 709 |
| 867 | 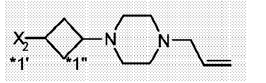 | 322 | 697 |
| 868 | 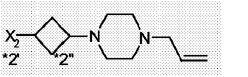 | 322 | 697 |
| 869 | 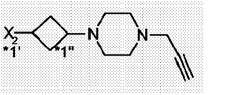 | 318 | 695 |
| 870 | 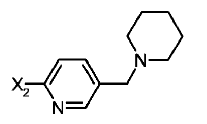 | 290,3 | 693 |
| 871 | 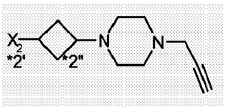 | 322 | 695 |
| 872 | 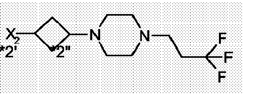 | 286, 322 | 753 |
| 873 | 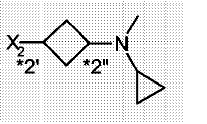 | 286, 326 | 642 |
| 874 | 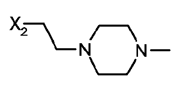 | 286, 322 | 645 |
| 875 | 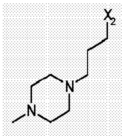 | 322, 286 | 659 |
| 876 | 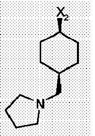 | 282, 322 | 684 |
| 877 | 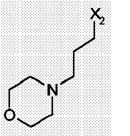 | 324, 284 | 646 |
| 878 | 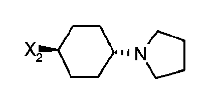 | 286, 322 | 670 |
| 879 | 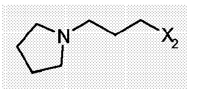 | 325, 284 | 630 |
| 880 | 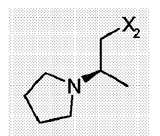 | 322, 286 | 630 |
| 881 | 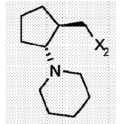 | 322, 286 | 684 |
| 882 | 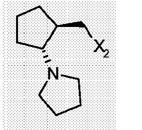 | 325, 286 | 670 |
| 883 | 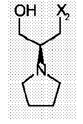 | 322, 286 | 646 |
| 884 | 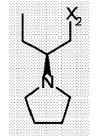 | 326, 286 | 644 |
| 885 | 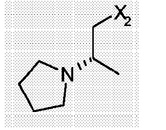 | 325, 285 | 630 |

### Beispiele 886-891

Die folgenden Verbindungen sind über ein analoges Verfahren, wie in Beispiel 622 beziehungsweise 623 beschrieben, hergestellt. Das entsprechende Anilin ist in Methode 27 beziehungsweise 28 beschrieben.

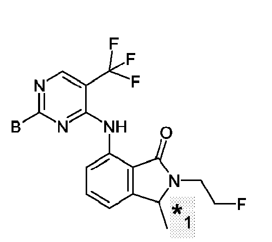

| # | **B** | **UV max [nm]** | **MS (ESI) (M+H)⁺** |
|---|---|---|---|
| 886 | 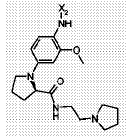 | 314 | 685 |
| 887 | 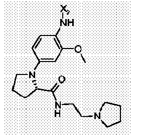 | 314 | 685 |
| 888 | 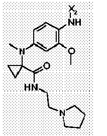 | 286, 310 | 685 |
| 889 | 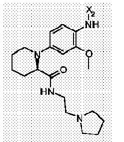 | 282, 314 | 699 |
| 890 | 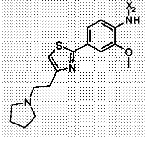 | 338 | 656 |
| 891 | 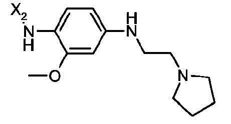 | 314 | 588 |

### Beispiele 892-894

Die folgenden Verbindungen sind über ein analoges Verfahren, wie in Beispiel 53 beschrieben, hergestellt. 2-(4-Carboxy-2-brom-phenylamino)-4-chlor-5-trifluormethyl-pyrimidin ist in Methode 29 beschrieben. Das entsprechende Anilin ist in Methode 31 beschrieben. Das für die Darstellung des Amids eingesetzte Amin ist kommerziell erhältlich.

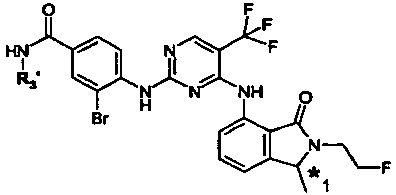

| # | **R₃'** | **UV max [nm]** | **MS (ESI) (M+H)⁺** |
|---|---|---|---|
| 892 | 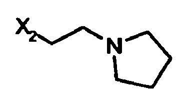 | 314 | 665 |
| 893 | 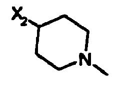 | 270 | 665 |
| 894 | 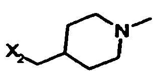 | 270 | 680 |

### Beispiel 895

### 2-(2-methoxy-4-[(1-methyl-piperidin-4-carbonyl)-amino]-phenylamino)-4-(2-(2-fluorethyl)-1-methyl-3-oxo-2,3-dihydro-1H-isoindol-4-ylamino)-5-trifluormethyl-pyrimidin Enantiomer 1

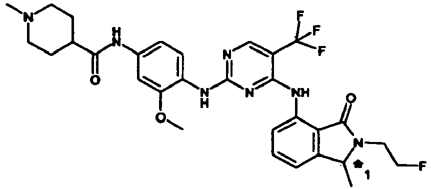

Ausgehend von 2-(4-Amino-2-methoxy phenylamino)-4-(2-(2-fluor-ethyl)-1-methyl-3-oxo-2,3-dihydro-1*H*-isoindol-4-ylamino)-5-trifluormethyl-pyrimidin Enantiomer 1 (analog Methode 30) wird über eine dem Fachmann geläufige Amidknüpfungsmethode oben genanntes Produkt hergestellt (siehe auch Beispiel 1032). Dieses liegt als Dihydrochlorid vor.

| | |
|---|---|
| UV max: | 310 nm |
| MS (ESI): | 616 (M+H)⁺ |
| ¹H-NMR (500MHz): | 1.42 (d, 3H), 1.69 - 1.77 (m, 2H), 1.77 - 1.84 (m, 2H), 1.94 - 2.03 (m, 2H), 2.23 (s, 3H), 2.29 - 2.38 (m, 1H), 2.86 - 2.93 (m, 2H), 3.72 (s, 3H), 4.00 - 4.12 (m, 1H), 4.52 - 4.75 (m, 3H), 7.16 (d, 3H), 7.18 - 7.24 (m, 1H), 7.32 - 7.41 (m, 1H), 7.57 (s, 1H), 8.18 (s, 1H), 8.38 (s, 1H), 9.07 (s, 1H), 9.95 (s, 1H), 10.46 (s, 1H) |

### Beispiel 896

### 2-(2-methoxy-4-(2-pyrrolidin-1-yl-acetylamino)-phenylamino)-4-(2-(2-fluor-ethyl)-1-methyl-3-oxo-2,3-dihydro-1H-isoindol-4-ylamino)-5-trifluormethyl-pyrrimidin Enantiomer 1

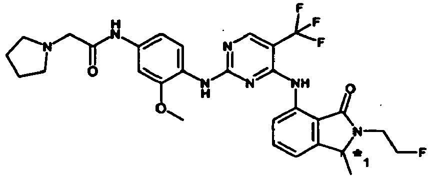

Ausgehend von 2-(4-Amino-2-methoxy-phenylamino)-4-(2-(2-fluor-ethyl)-1-methyl-3-oxo-2,3-dihydro-1*H*-isoindol-4-ylamino)-5-trifluormethyl-pyrimidin Enantiomer 1 (analog Methode 30) wird über eine dem Fachmann geläufige Amidknüpfungsmethode oben genanntes Produkt hergestellt (siehe auch Beispiel 1032). Dieses liegt als Dihydrochlorid vor.

| | |
|---|---|
| UV max: | 282 nm |
| MS (ESI): | 602 (M+H)⁺ |
| ¹H-NMR (500MHz): | 1.43 (d, 3H), 1.87 - 2.00 (m, 2H), 2.00 - 2.10 (m, 2H), 3.12 - 3.22 (m, 2H), 3.74 (s, 3H), 4.00 - 4.13 (m, 1H), 4.28 - 4.32 (m, 2H), 4.53 - 4.76 (m, 3H), 7.19 - 7.49 (m, 4H), 7.51 (s, 1H), 8.41 (s, 1H), 9.26 (s, 1H), 10.20 - 10.31 (m, 1H), 10.54 (s, 1H), 10.86 (s, 1H) |

### Beispiele 897-952

Über ein analoges Verfahren wie in Beispiel 53 beschrieben wird an 2-(4-Carboxy-2-methoxy-phenylamino)-4-[2-(2-fluoro-ethyl)-1-methyl-3-oxo-2,3-dihydro-1*H*-isoindol-4-ylamino]-5-trifluoromethyl-pyrimidin Enantiomer 1 ein primäres Amin gekuppelt, welches in der Seitenkette eine weitere geschützte Aminofunktion besitzt. Als Schutzgruppe kommt eine *tert*-Butoxycarbonyl-, Benzyloxycarbonyl- oder Benzyl-Gruppe in Frage. Die Abspaltung dieser Schutzgruppe nach einer dem Fachmann geläufigen Prozedur und eine reduktive Aminierung (analog Beispiel 639) oder Alkylierung (analog Methode 34 beziehungsweise WO2004052857) stellen die letzten Schritte in dieser Sequenz dar.

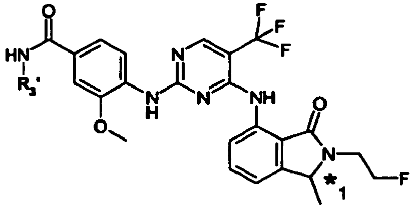

| # | **R₃'** | **UV max [nm]** | **MS (ESI) (M+H)⁺** |
|---|---|---|---|
| 897 | 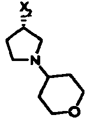 | | 672 |
| 898 | 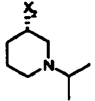 | 322 | 644 |
| 899 | 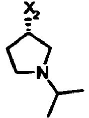 | 326 | 630 |
| 900 | 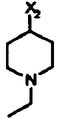 | 326 | 630 |
| 901 | 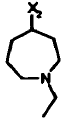 | 322 | 644 |
| 902 | 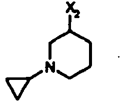 | 322 | 642 |
| 903 | 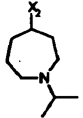 | 322 | 658 |
| 904 | 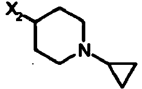 | 326 | 615 |
| 905 | 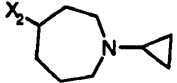 | 322 | 656 |
| 906 | 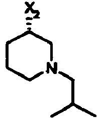 | 326 | 658 |
| 907 | 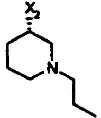 | 326 | 644 |
| 908 | 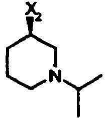 | 322 | 644 |
| 909 | 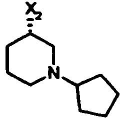 | 322 | 670 |
| 910 | 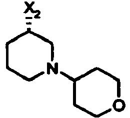 | 306 | 686 |
| 911 | 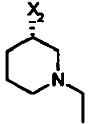 | 326 | 630 |
| 912 | 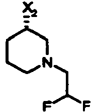 | | 666 |
| 913 | 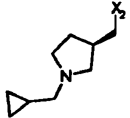 | 286, 322 | 656 |
| 914 | 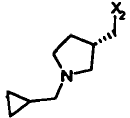 | 286, 322 | 656 |
| 915 | 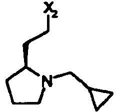 | 286, 318 | 670 |
| 916 | 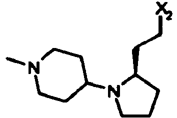 | 286, 322 | 713 |
| 917 | 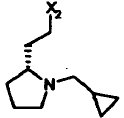 | 286, 322 | 670 |
| 918 | 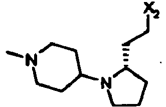 | 286,3 | 713 |
| 919 | 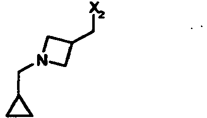 | 286, 322 | 642 |
| 920 | 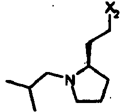 | 286, 322 | 672 |
| 921 | 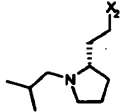 | 286, 322 | 672 |
| 922 | 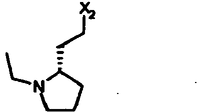 | 286, 322 | 644 |
| 923 | 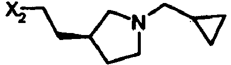 | 286, 322 | 670 |
| 924 | 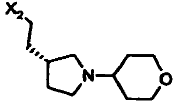 | 286, 322 | 700 |
| 925 | 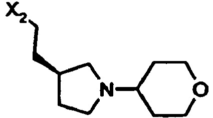 | 286, 322 | 700 |
| 926 | 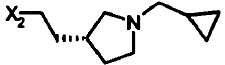 | 286, 322 | 670 |
| 927 | 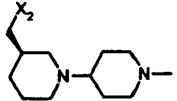 | 326 | 713 |
| 928 | 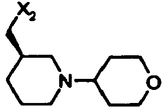 | 322-326 | 700 |
| 929 | 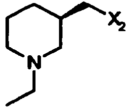 | 322-326 | 644 |
| 930 | 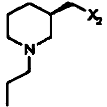 | 322 | 658 |
| 931 | 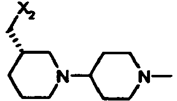 | 322-326 | 713 |
| 932 | 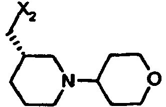 | 322 | 700 |
| 933 | 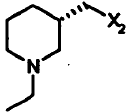 | 322-326 | 644 |
| 934 | 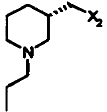 | 322 | 658 |
| 935 | 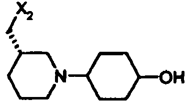 | 322-326 | 714 |
| 936 | 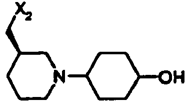 | 322 | 714 |
| 937 | 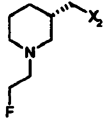 | 322 | 662 |
| 938 | 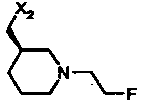 | 322-326 | 662 |
| 939 | 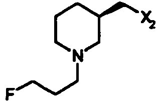 | | 676 |
| 940 | 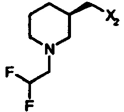 | 322-326 | 680 |
| 941 | 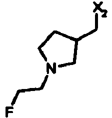 | 286, 322 | 648 |
| 942 | 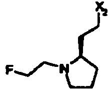 | 230, 286, 318 | 662 |
| 943 | 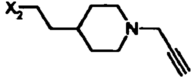 | 284, 324 | 668 |
| 944 | 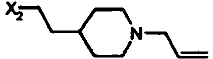 | 282, 322 | 670 |
| 945 | 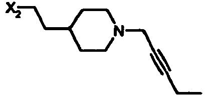 | 282, 322 | 696 |
| 946 | 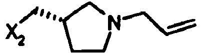 | 228, 284, 322 | 642 |
| 947 | 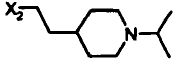 | 226, 286, 322 | 672 |
| 948 | 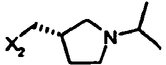 | 286, 322 | 644 |
| 949 | 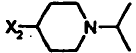 | 324, 284 | 644 |
| 950 | 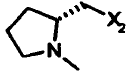 | 285, 322 | 616 |
| 951 | 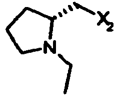 | 285, 325 | 630 |
| 952 | 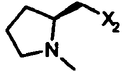 | 285, 325 | 616 |

### Beispiele 953-958

Über ein analoges Verfahren wie in Beispiel 796 beschrieben werden folgende Verbindungen hergestellt:

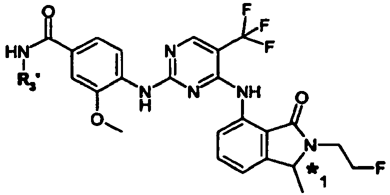

| **#** | **R₃'** | **UV max [nm]** | **MS (ESI) (M+H)⁺** |
|---|---|---|---|
| 953 | 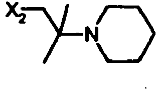 | 326, 286 | 658 |
| 954 | 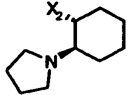 | 325, 285 | 670 |
| 955 | 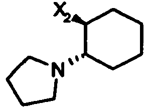 | 325, 285 | 670 |
| 956 | 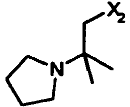 | 325, 284 | 644 |
| 957 | 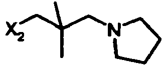 | 325, 284 | 658 |
| 958 | 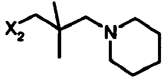 | 325, 285 | 672 |

### Beispiel 959

### 2-(2-methoxy-4-(2-pyrrolidin-1-yl-ethylcarbamoyl)-phenylamino)-4-(2-(2-fluor-ethyl)-1-ethyl-3-oxo-2,3-dihydro-1H-isoindol-4-ylamino)-5-trifluormethyl-pyrimidin

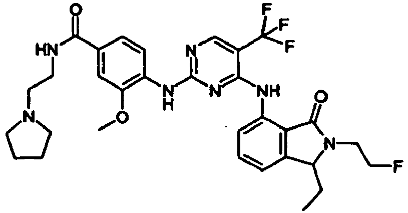

Die racemische Synthese der oben genannten Verbindung erfolgt über ein analoges Verfahren, wie in Beispiel 53 beschrieben. Das entsprechende Anilin ist in Methode 22 beschrieben. Über eine präparative Chromatographie werden die beiden Enantiomere isoliert:

| | |
|---|---|
| Säule: | 250 x 4,6 mm CHIRALPAKADH^{®} |
| Eluent: | 25 Ethanol / 75 Methanol (v/v) (beiden Lösungsmittel ist 0,03% Triethylamin zugemischt) |
| Flußrate: | 0,5 ml/min |
| Temperatur: | 20°C |

Das Enantiomer, welches als erstes eluiert, wird als Enantiomer 1 bezeichnet und trägt in den chemischen Formel das Symbol *1.

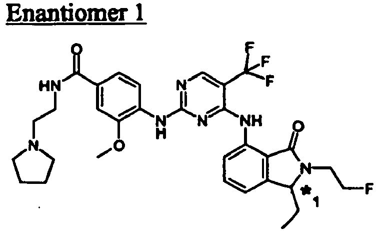

| | |
|---|---|
| Retentionszeit: | 9,96 min |

Das Enantiomer, welches als zweites eluiert, wird als Enantiomer 2 bezeichnet und trägt in den chemischen Formel das Symbol *2.

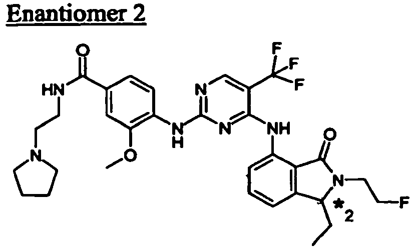

| | |
|---|---|
| Retentionszeit: | 12,60 min |

### Beispiele 960-976

Die folgenden Verbindungen sind über ein analoges Verfahren, wie in Beispiel 53 beschrieben, hergestellt. Das entsprechende Anilin ist in Methode 22 beschrieben. Das für die Darstellung des Amids eingesetzte Amin ist kommerziell erhältlich beziehungsweise ist in Methode 13 beschrieben.

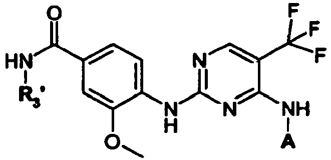

| **#** | **A** | **R₃'** | **UV max [nm]** | **MS (ESI) (M+H)⁺** |
|---|---|---|---|---|
| 960 | 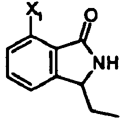 | 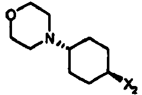 | 280, 320 | 654 |
| 961 | 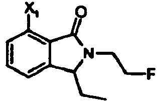 | 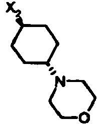 | 282, 318 | |
| 962 | 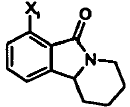 | 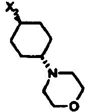 | 286, 322 | 680 |
| 963 | 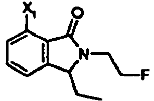 | 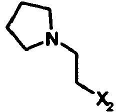 | 286, 326 | 630 |
| 964 | 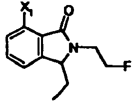 | 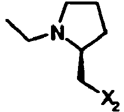 | 286, 326 | 644 |
| 965 | 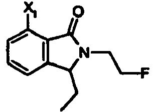 | 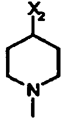 | 286, 326 | 630 |
| 966 | 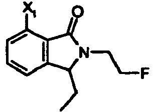 | 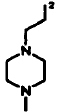 | 286, 326 | 659 |
| 967 | 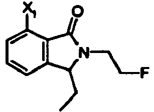 | 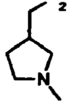 | 286, 326 | 630 |
| 968 | 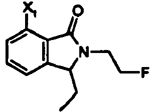 | 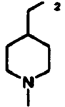 | 286, 322 | 644 |
| 969 | 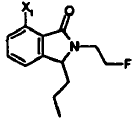 | 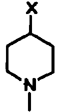 | 286, 326 | 644 |
| 970 | 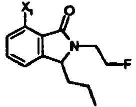 | 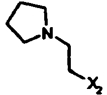 | 286, 326 | 644 |
| 971 | 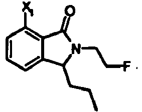 | 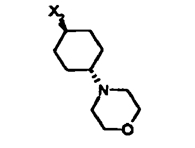 | 286, 326 | 714 |
| 972 | 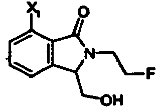 | 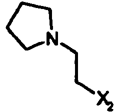 | 286, 322 | 632 |
| 973 | 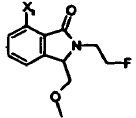 | 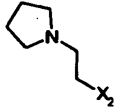 | 286, 326 | 646 |
| 974 | 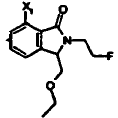 | 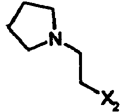 | 286, 326 | 660 |
| 975 | 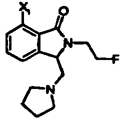 | 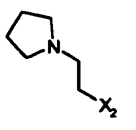 | 282, 326 | 685 |
| 976 | 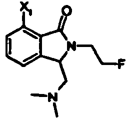 | 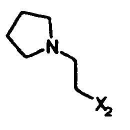 | 282, 326 | 659 |

### Beispiele 977-980

Die folgenden Verbindungen sind über ein analoges Verfahren, wie in Beispiel 53 beschrieben, hergestellt. Das entsprechende Anilin ist in Methode 6 beschrieben. Das für die Darstellung des Amids eingesetzte Amin ist in Methode 13 beschrieben.

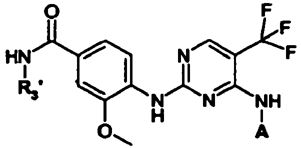

| **#** | **A** | **R₃'** | **UV max [nm]** | **MS (ESI) (M+H)⁺** |
|---|---|---|---|---|
| 977 | 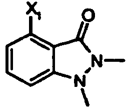 | 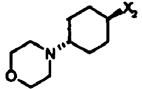 | 234, 282, 318 | 655 |
| 978 | 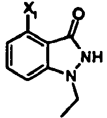 | 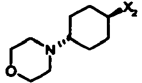 | 226, 282, 318 | 655 |
| 979 | 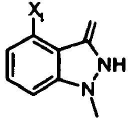 | 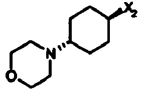 | 222, 282, 318 | 641 |
| 980 | 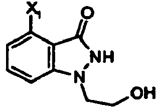 | 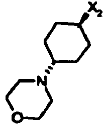 | 230, 282, 314 | 671 |

### Beispiele 981-999

Die folgenden Verbindungen sind über ein analoges Verfahren, wie in Beispiel 53 beschrieben, hergestellt. Das entsprechende Anilin ist in Methode 32 beschrieben. Das für die Darstellung des Amids eingesetzte Amin ist kommerziell erhältlich beziehungsweise in Methode 13 beschrieben.

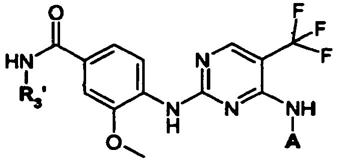

| **#** | **A** | **R₃'** | **UV max [nm]** | **MS (ESI) (M+H)⁺** |
|---|---|---|---|---|
| 981 | 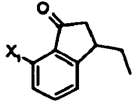 | 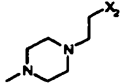 | 318 | 612 |
| 982 | 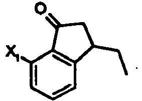 | 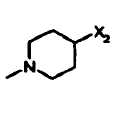 | 318 | 583 |
| 983 | 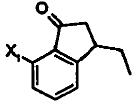 | 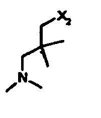 | 322 | 599 |
| 984 | 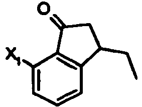 | 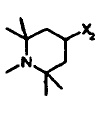 | | 639 |
| 985 | 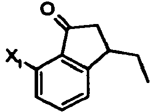 | 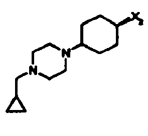 | 286 | 706 |
| 986 | 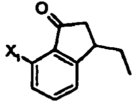 | 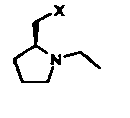 | 322 | 597 |
| 987 | 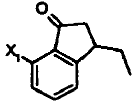 | 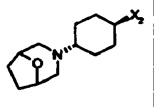 | 318 | 679 |
| 988 | 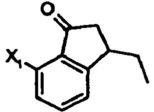 | 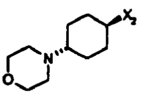 | 286 | 653 |
| 989 | 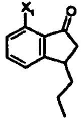 | 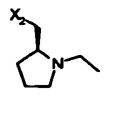 | 322 | 611 |
| 990 | 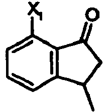 | 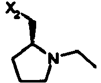 | 322 | 583 |
| 991 | 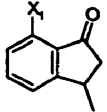 | 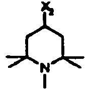 | 318 | 625 |
| 992 | 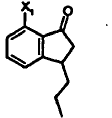 | 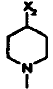 | 318 | 597 |
| 993 | 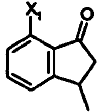 | 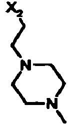 | 318 | 598 |
| 994 | 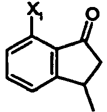 | 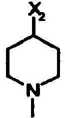 | 318 | 569 |
| 995 | 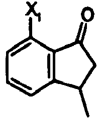 | 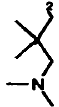 | 322 | 585 |
| 996 | 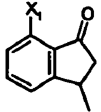 | 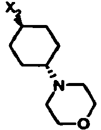 | 286 | 639 |
| 997 | 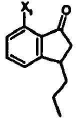 | 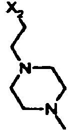 | 318 | 626 |
| 998 | 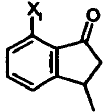 | 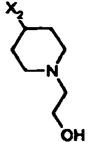 | 318 | 599 |
| 999 | 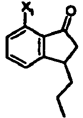 | 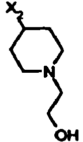 | 318 | 318 |

### Beispiele 1000-1024

Die folgenden Verbindungen sind über ein analoges Verfahren, wie in Beispiel 53 beschrieben, hergestellt. Das entsprechende Anilin ist in Methode 33 beschrieben. Das für die Darstellung des Amids eingesetzte Amin ist kommerziell erhältlich beziehungsweise in Methode 13 oder 21 beschrieben.

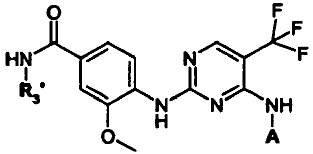

| **#** | **A** | **R₃'** | **UV max [nm]** | **MS (ESI) (M+H)⁺** |
|---|---|---|---|---|
| 1000 | 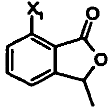 | 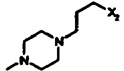 | 282, 322 | 614 |
| 1001 | 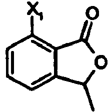 | 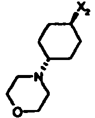 | 282, 322 | 841 |
| 1002 | 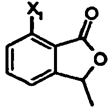 | 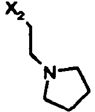 | 282, 326 | 571 |
| 1003 | 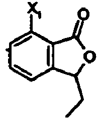 | 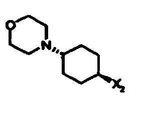 | 280, 322 | 655 |
| 1004 | 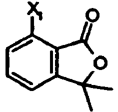 | 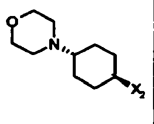 | 280,325 | 655 |
| 1005 | 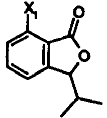 | 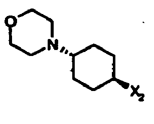 | 280, 322 | 669 |
| 1006 | 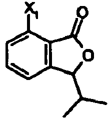 | 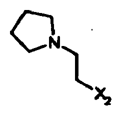 | 280, 325 | 599 |
| 1007 | 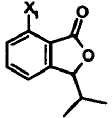 | 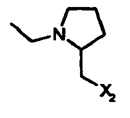 | 282, 327 | 613 |
| 1008 | 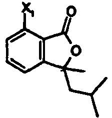 | 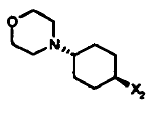 | 280, 322 | 697 |
| 1009 | 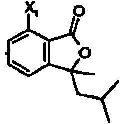 | 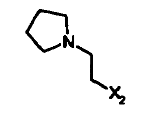 | 282, 325 | 627 |
| 1010 | 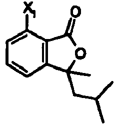 | 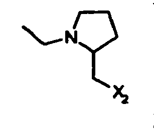 | 283, 328 | 641 |
| 1011 | 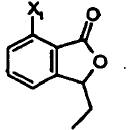 | 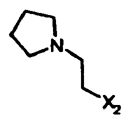 | 280, 325 | 585 |
| 1012 | 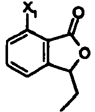 | 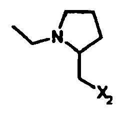 | 280, 325 | 599 |
| 1013 | 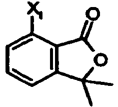 | 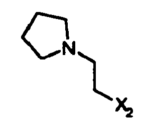 | 326, 283 | 585 |
| 1014 | 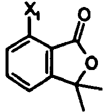 | 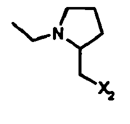 | 282, 327 | 599 |
| 1015 | 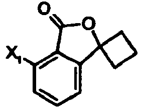 | 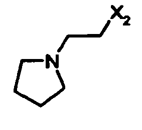 | 322-326 | 597 |
| 1016 | 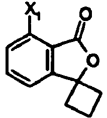 | 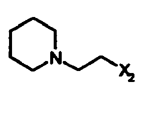 | 326 | 611 |
| 1017 | 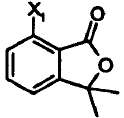 | 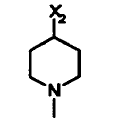 | 280, 325 | 585 |
| 1018 | 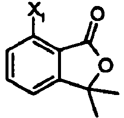 | 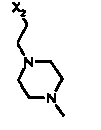 | 280, 325 | 614 |
| 1019 | 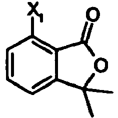 | 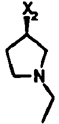 | 280, 325 | 585 |
| 1020 | 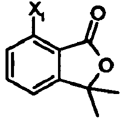 | 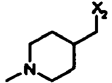 | 280, 322 | 599 |
| 1021 | 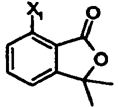 | 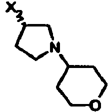 | 280, 325 | 641 |
| 1022 | 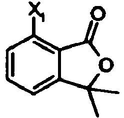 | 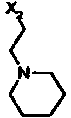 | 280, 325 | 599 |
| 1023 | 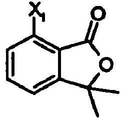 | 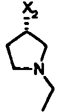 | 280, 325 | 585 |
| 1024 | 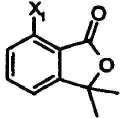 | 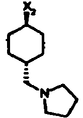 | 280, 322 | 653 |

### Beispiele 1025-1032

Die folgenden Verbindungen sind über ein analoges Verfahren, wie in Beispiel 53 beschrieben, hergestellt. Das entsprechende Anilin ist in Methode 10 beschrieben. Das für die Darstellung des Amids eingesetzte Amin ist kommerziell erhältlich beziehungsweise in Methode 13 beschrieben.

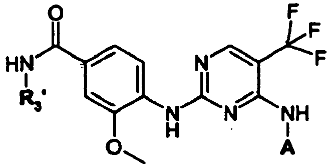

| **#** | **A** | **R₃'** | **UV max [nm]** | **MS (ESI) (M+H)⁺** |
|---|---|---|---|---|
| 1025 | 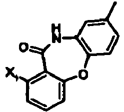 | 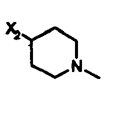 | 318 | 648 |
| 1026 | 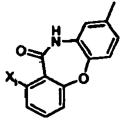 | 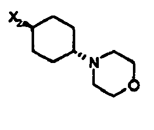 | 318 | 359 |
| 1027 | 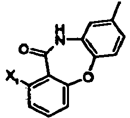 | 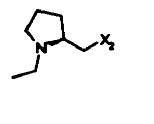 | 322 | 662 |
| 1028 | 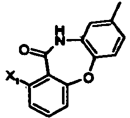 | 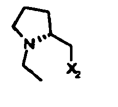 | 322 | 662 |
| 1029 | 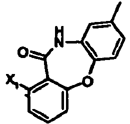 | 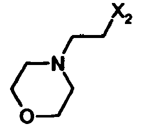 | 322 | 664 |
| 1030 | 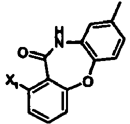 | 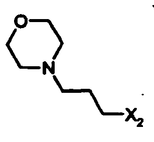 | 226, 318 | 678 |
| 1031 | 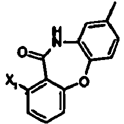 | 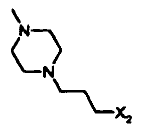 | 226, 318 | 691 |
| 1032 | 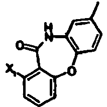 | 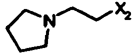 | 322 | 648 |

### Beispiele 1033-1035

Die folgenden Verbindungen sind über ein analoges Verfahren, wie in Beispiel 53 beschrieben, hergestellt. Das entsprechende Anilin ist in Methode 2 beschrieben. Das für die Darstellung des Amids eingesetzte Amin ist in Methode 13 beschrieben.

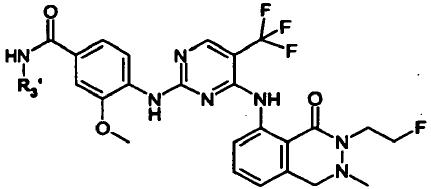

| **#** | **R₃'** | **MS (ESI) (M+H)⁺** | **Salzform** |
|---|---|---|---|
| 1033 | 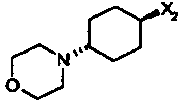 | 701 | Base |
| 1034 | 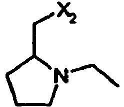 | 645 | Formiat |
| 1035 | 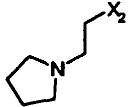 | 631 | Formiat |

### Beispiel 1036

### 2-(2-methoxy-4-(2-pyrrolidin-1-yl-ethylcarbamoyl)-phenylamino)-4-(2-(2-fluor-ethyl)-1,1-dimethyl-3-oxo-2,3-dihydro-1H-isoindol-4-ylamino)-5-trifluormethyl-pyrimidin

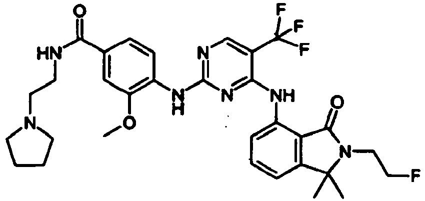

Die oben genannte Verbindung wird über ein analoges Verfahren, wie in Beispiel 53 beschrieben, hergestellt. Das entsprechende Anilin ist in Methode 34 beschrieben. Das für die Darstellung des Amids eingesetzte Amin ist kommerziell erhältlich. Die Substanz liegt als Dihydrochlorid vor.

| | |
|---|---|
| UV max: | 326, 286 nm |
| MS (ESI): | 630 (M+H)⁺ |
| ¹H-NMR (400MHz): | 1.44 - 1.50 (m, 6H), 1.84 - 1.95 (m, 2H), 1.98 - 2.07 (m, 2H), 3.02 - 3.12 (m, 2H), 3.62 - 3.70 (m, 4H), 3.71 - 3.76 (m, 1H), 3.77 - 3.81 (m, 1H), 3.89 (s, 3H), 4.57 - 4.61 (m, 1H), 4.69 - 4.73 (m, 1H), 7.27 - 7.31 (m, 1H), 7.39 - 7.45 (m, 1H), 7.55 - 7.59 (m, 1H), 7.63 - 7.66 (m, 1H), 7.84 - 7.88 (m, 1H), 8.44 - 8.55 (m, 2H), 8.77 - 8.82 (m, 1H), 9.11 - 9.15 (m, 1H), 9.91 - 10.03 (m, 1H), 10.51 - 10.55 (m, 1H) |

### Beispiele 1037

### 2-(2-methoxy-4-[2-(4-methyl-piperazin-1-yl)-ethylcarbamoyl]-phenylamino)-4-2-(2-fluor-ethyl)-3-oxo-2,3-dihydro-1H-isoindol-4-ylamino)-5-acetyl-pyrrimidin

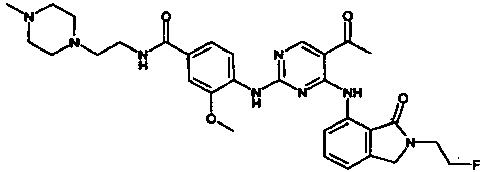

50 mg (0,104 mmol) 2-(4-carboxy-2-methoxy-phenylamino)-4-(2-(2-fluor-ethyl)-3-oxo-2,3-dihydro-1*H*-isoindol-4-ylamino)-5-acetyl-pyrimidin (wird über ein analoges Verfahren wie in Beispiel 622 beziehungsweise 623 hergestellt) werden in 0,5 ml Dimethylformamid gelöst und mit 72 µl (0,520 mmol) und 34 mg (0,104 mmol) O-(Benzotriazol-1-yl)-*N,N,N',N*'-tetramethyluronium-tetrafluorborat versetzt. Nachdem man 20 min bei 20 °C gerührt hat gibt man 23 mg (0,156 mmol) 2-(4-Methylpiperazin-1-yl)-ethylamin zu. Die Reaktion ist nach 2 h bei 20 °C abgeschlossen. Anschließend entfernt man das Lösungsmittel im Vakuum und der Rückstand wird mittels Säulenchromatographie gereinigt Als Trägermaterial dient C18-RP-Kieselgel und es wird ein Gradient innerhalb von 20 min durchlaufen der am Startpunkt aus 95% Wasser und 5% Acetonitril und am Endpunkt aus 5% Wasser und 95% Acetonitril besteht. Sowohl dem Wasser als auch dem Acetonitril sind jeweils 0,1% Ameisensäure beigemischt. Die geeigneten Fraktionen werden mit 500 µl einer 1 M wässrigen Salzsäure versetzt und gefriergetrocknet. Das Produkt liegt als Trihydrochlorid vor.

| | |
|---|---|
| UV max: | 326 nm |
| MS (ESI): | 605 (M+H)⁺ |
| ¹H-NMR (500MHz): | 2.53-2.58 (m, 3H), 2.80-2.92 (m, 3H), 3.62-3.88 (m, 9H), 3.88-4.01 (m, 4H), 4.54 (s, 2H), 4.58-4.66(m, 1H), 4.69-4.77 (m, 1H), 7.14-7.32 (m, 1H), 7.32-7.50 (m, 1H), 7.50-7.59 (m, 1H), 7.63-7.75 (m, 1H), 7.78-8.01 (m, 1H), 8.29-8.60 (m, 1H), 8.73-8.99 (m, 2H), 9.03-9.18 (m, 1H), 12.31-12.41 (m, 1H) |

### Beispiele 1038-1060

Die folgenden Verbindungen sind über ein analoges Verfahren, wie in Beispiel 1037 beschrieben, hergestellt. Das eingesetzte Anilin ist in Methode 28 beschrieben. Das für die Darstellung des Amids eingesetzte Amin ist kommerziell erhältlich beziehungsweise in Methode 13 beschrieben.

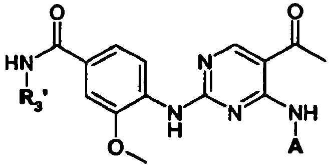

| **#** | **A** | **R₃'** | **UV max [nm]** | **MS (ESI) (M+H)⁺** |
|---|---|---|---|---|
| 1038 | 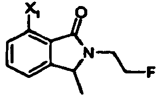 | 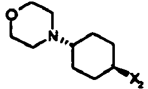 | 326 | 660 |
| 1039 | 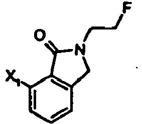 | 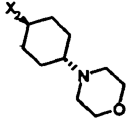 | 326 | 646 |
| 1040 | 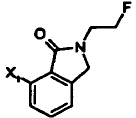 | 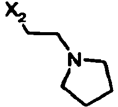 | 328 | 576 |
| 1041 | 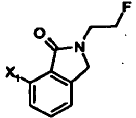 | 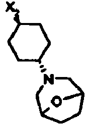 | 318 | 672 |
| 1042 | 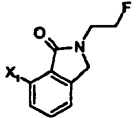 | 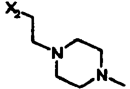 | 326 | 605 |
| 1043 | 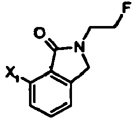 | 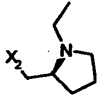 | 330 | 590 |
| 1044 | 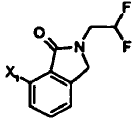 | 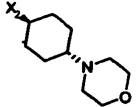 | 318 | 663 |
| 1045 | 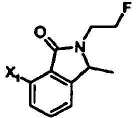 | 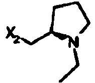 | 330 | 604 |
| 1046 | 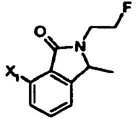 | 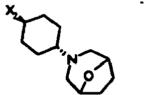 | 326 | 686 |
| 1047 | 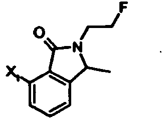 | 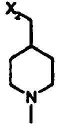 | 326 | 604 |
| 1048 | 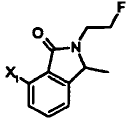 | 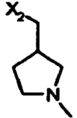 | 330 | 590 |
| 1049 | 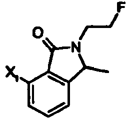 | 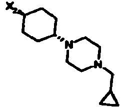 | 326 | 713 |
| 1050 | 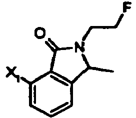 | 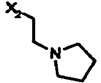 | 330 | 590 |
| 1051 | 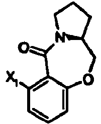 | 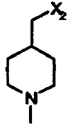 | 250 | 614 |
| 1052 | 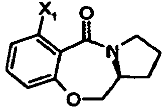 | 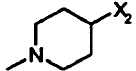 | 334-338 | 600 |
| 1053 | 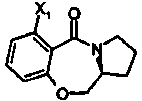 | 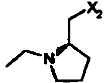 | 334-338 | 614 |
| 1054 | 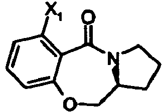 | 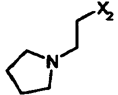 | 338 | 600 |
| 1055 | 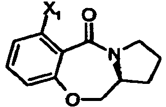 | 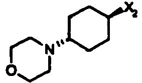 | 338 | 670 |
| 1056 | 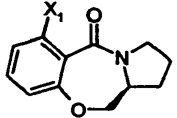 | 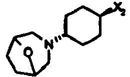 | 334 | 696 |
| 1057 | 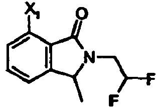 | 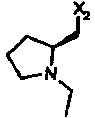 | 330 | 622 |
| 1058 | 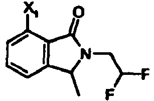 | 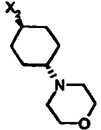 | 327 | 340 |
| 1059 | 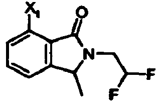 | 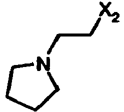 | 330 | 608 |
| 1060 | 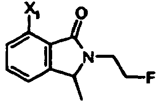 | 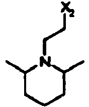 | 330 | 632 |

### Beispiele 1061-1069

Die folgenden Verbindungen sind über ein analoges Verfahren, wie in Beispiel 622 beziehungsweise 623 beschrieben, hergestellt. Das entsprechende Anilin ist in Methode 28 beschrieben.

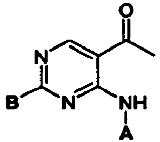

| **#** | **A** | **B** | **UV max [nm]** | **MS (ESI) (M+H)⁺** |
|---|---|---|---|---|
| 1061 | 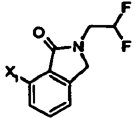 | 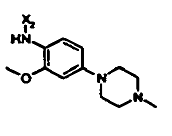 | 254, 316 | 552 |
| 1062 | 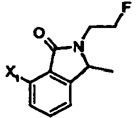 | 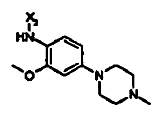 | 254,314 | 548 |
| 1063 | 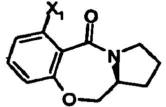 | 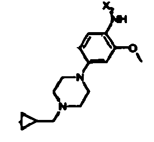 | 250 | 598 |
| 1064 | 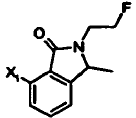 | 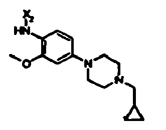 | 254,318 | 588 |
| 1065 | 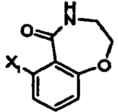 | 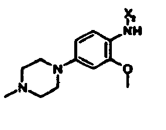 | 250 | 518 |
| 1066 | 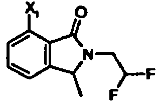 | 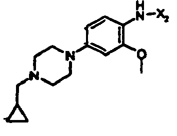 | 232, 318 | 606 |
| 1067 | 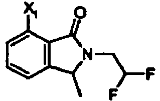 | 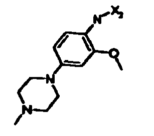 | 250, 310 | 566 |
| 1068 | 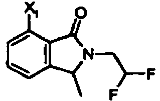 | 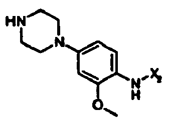 | 254, 318 | 552 |
| 1069 | 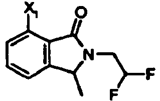 | 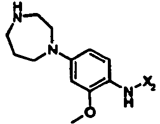 | 262; 314-318 | 566 |

### Beispiele 1070-1071

Die folgenden Verbindungen sind über ein analoges Verfahren, wie in Beispiel 622 beziehungsweise 623 und 53 beschrieben, hergestellt. Das entsprechende Anilin ist in Methode 28 beschrieben. Das für die Darstellung des Amids eingesetzte Amin ist kommerziell erhältlich beziehungsweise in Methode 13 beschrieben.

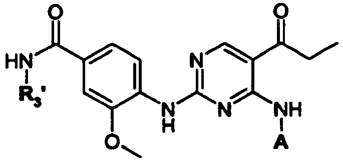

| **#** | **A** | **R₃'** | **UV max [nm]** | **MS (ESI) (M+H)⁺** |
|---|---|---|---|---|
| 1070 | 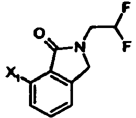 | 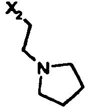 | 330 | 608 |
| 1071 | 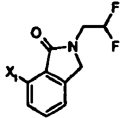 | 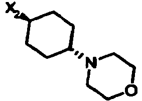 | 330 | 678 |

### Beispiele 1072-1085

Die folgenden Verbindungen sind über ein analoges Verfahren, wie in Beispiel 1037 beschrieben, hergestellt. Das entsprechende Anilin ist in Methode 28 beschrieben. Das für die Darstellung des Amids eingesetzte Amin ist kommerziell erhältlich beziehungsweise in Methode 13 beschrieben.

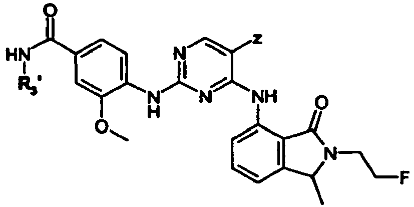

| **#** | **Z** | **R₃'** | **UV max [nm]** | **MS (ESI) (M+H)⁺** |
|---|---|---|---|---|
| 1072 | 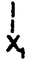 | 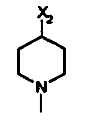 | 285,320 | 674 |
| 1073 | 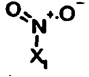 | 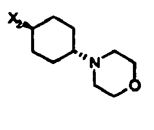 | 326 | 663 |
| 1074 | 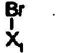 | 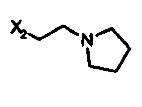 | 306 | 596 |
| 1075 | 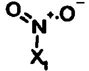 | 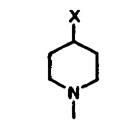 | 326 | 593 |
| 1076 | 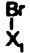 | 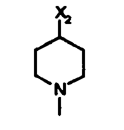 | 262 | 596 |
| 1077 | 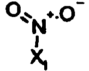 | 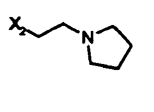 | 326 | 593 |
| 1078 | 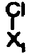 | 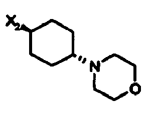 | 318 | 652 |
| 1079 | 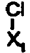 | 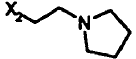 | 325 | 582 |
| 1080 | 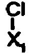 | 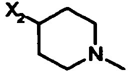 | 319 | 582 |
| 1081 | 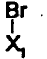 | 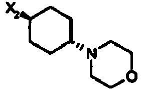 | 302 | 666 |
| 1082 | 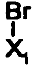 | 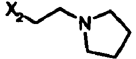 | 322 | 626 |
| 1083 | 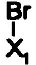 | 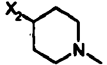 | 318 | 626 |
| 1084 | 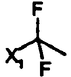 | 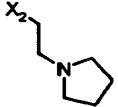 | 286, 318 | 612 |
| 1085 | 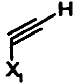 | 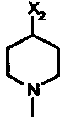 | 280, 325 | 572 |

### Biologische Eigenschaften

Wie durch DNA-Färbung mit darauf folgender FACS Analyse gezeigt werden konnte, ist die, durch die erfindungsgemäßen Verbindungen bewirkte, Pmliferationsinhibition vor allem durch einen Arrest der Zellen in der G2/M Phase des Zellzyklus vermittelt. Die Zellen arretieren abhängig von dem verwendeten Zelltyp für eine bestimmte Zeitspanne in dieser Zellzyklus Phase, bevor der programmierte Zelltod eingeleitet wird. Ein Arrest in der G2/Ni Phase des Zellzyklus kann z.B. durch die Inhibition spezifischer Zellzykluskinasen ausgelöst werden. Auf Grund ihrer biologischen Eigenschaften eignen sich die erfindungsgemäßen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphoma und solide Tumore; Haut-Erkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001). Die neuen Verbindungen können zur Prävention, Kurz- oder Langzeitbehandlung der vorstehend genannten Erkrankungen, auch in Kombination mit anderen Wirkstoffen, die für dieselben Indikationen Verwendung finden, z.B. Zytostatika, Steroide oder Antikörper, verwendet werden.

Die Wirkung der erfindungsgemäßen Verbindungen auf verschiedene Kinasen, beispielsweise auf der Serin-Threonin Kinase PLK-1, wurde in in vitro Kinase Assays mit rekombinant hergestelltem Protein bestimmt. Die Verbindungen zeigen in diesem Assay eine gute bis sehr gute Wirksamkeit auf PLK1, d.h. beispielsweise einen IC50-Wert kleiner 1 µmol/L, in der Regel kleiner 0.1 µmol/L

### Beispiel PLK-1 Kinaseassay

Rekombinantes humanes und an seinem N-terminalen Ende mit GST verbundenes PLK1 Enzym wird aus Bakulovirus infizierten Insektenzellen (Sf21) isoliert. Die Reinigung erfolgt durch Affinitätschromatographie an Glutathion Sepharose Säulen.

4x10⁷ Sf21 Zellen (*Spodoptera frugiperda*) in 200 ml Sf-900 II Serum freien Insektenzellmedium (Life Technologies) werden in eine Spinnerflasche ausgesät. Nach 72 Stunden Inkubation bei 27°C und 70 rpm werden 1x10⁸ Sf21 Zellen in insgesamt 180 ml Medium in eine neue Spinnerflasche ausgesät Nach weiteren 24 Stunden werden 20 ml rekombinanter Baculovirus Stammsuspension zugesetzt und die Zellen 72 Stunden bei 27°C bei 70 rpm kultiviert. 3 Stunden vor dem Ernten wird Okadainsäure zugesetzt (Calbiochem, Endkonzentration 0,1 µM) und die Suspension weiter inkubiert. Die Zellzahl wird bestimmt, die Zellen abzentrifugiert (5 Minuten, 4°C, 800 rpm) und 1x mit PBS (8 g NaCl/1, 0,2 g KCl/1, 1,44 g Na₂HPO₄/l, 0,24 g KH₂PO4/l) gewaschen. Nach nochmaligem Abzentrifugieren wird das Pellet in flüssigem Stickstoff Schock gefroren. Danach wird das Pellet rasch aufgetaut und in eiskaltem Lysispuffer (50 mM HEPES pH 7,5, 10 mM MgCl₂, 1 mM DTT, 5 µg/ml Leupeptin, 5 µg/ml Aprotinin, 100 µM NaF, 100 µM PMSF, 10 mM β-Glycerolphosphat, 0.1 mM Na₃VO₄, 30 mM 4-Nitrophenylphosphate) zu 1x10⁸ Zellen/ 17,5 ml resuspendiert. Die Zellen werden 30 Minuten auf Eis lysiert. Nach dem Entfernen der Zelltrümmer durch Zentrifugation (4000 rpm, 5 Minuten) wird der klare Überstand mit Glutathion Sepharosebeads versetzt (1 ml resuspendierte und gewaschene Beads für 50 ml Überstand) und 30 Minuten bei 4°C auf einem Rotationsbrett inkubiert. Danach werden die Beads mit Lysispuffer gewaschen und das rekombinante Protein mit 1 ml Elutionspuffer/ ml resuspendierte Beads (Elutionspuffer. 100 mM Tris/HCl pH=8,0, 120 mM NaCl, 20 mM reduziertes Glutathion (Sigma G-4251), 10 mM MgCl₂**,** 1 mM DTT) von den Beads eluiert. Die Proteinkonzentration wird mittels Bradford Assay bestimmt.

### Assay

In einem Napf einer 96-Loch Rundbodenplatte (Fa. Greiner bio-one, PS-Microtiterplatte Nr.650101) werden folgende Komponenten zusammengefügt:
- 10 µl zu testende Verbindung in variabler Konzentration(z.B. beginnend bei 300 µM, und Verdünnung in 1:3) in 6% DMSO, 0,5 mg/ml Casein (Sigma C-5890), 60 mM β-Glycerophosphat, 25 mM MOPS pH=7,0, 5 mM EGTA, 15 mM MgCl₂, 1 mM DTT
- 20 µl Substratlösung (25 mM MOPS pH=7,0,15 mM MgCl₂, 1 mM DTT, 2,5 mM EGTA, 30 mM β-Glycerophosphat, 0,25 mg/ml Casein)
- 20 µl Enzymverdünnung (1:100 Verdünnung des Enzymstocks in 25 mM MOPS pH=7,0, 15 mM MgCl₂, 1 mM DTT)
- 10 µl ATP Lösung (45 µM ATP mit 1,11x10⁶ Bq/ml gamma-P33-ATP).

Durch Zusatz der ATP Lösung wird die Reaktion gestartet und 45 Minuten bei 30 °C unter leichtem Schütteln (650 rpm auf IKA Schüttler MTS2) durchgeführt. Die Reaktion wird durch Zusatz von 125 µl eiskalter 5%iger TCA pro Napf gestoppt und mindestens 30 Minuten auf Eis inkubiert. Das Präziptitat wird durch Ernten auf Filterplatten (96-well-Microtiter-Filterplatte: UniFilter-96, GF/B; Fa. Packard; Nr.6005177) übertragen, dann viermal mit 1%iger TCA gewaschen und bei 60°C getrocknet. Nach Zugabe von 35µl Szintillationslösung (Ready-Safe; Beckmann) pro Napf wird die Platte mit Sealing-tape zugeklebt und die präzipitierte Menge P33 mit dem Wallac Betacounter gemessen. Die Messdaten werden mit der Standard Graphpad Software (Levenburg-Marquard Algorhythmus) ausgewertet.

Die anti-proliferative Wirkung der erfindungsgemäßen Verbindungen wird im Cytotoxizitätstest an kultivierten humanen Tumorzellen und/oder in einer FACS-Analyse, beispielsweise an HeLa S3-Zellen, bestimmt. Die Verbindungen zeigen in beiden Testmethoden eine gute bis sehr gute Wirksamkeit, d.h. beispielsweise einen EC50-Wert im HeLa S3-Cytotoxizitätstest kleiner 5 µmol/L, in der Regel kleiner 1 µmol/L.

### Messung der Cytotoxizität an kultivierten humanen Tumorzellen

Zur Messung der Cytotoxizität an kultivierten humanen Tumorzellen werden Zellen der zervikalen Carcinoma Tumorzell-Linie HeLa S3 (erhalten von American Type Culture Collection (ATCC)) in Ham's F12 Medium (Life Technologies) und 10% fötalem Rinderserum (Life Technologies) kultiviert und in der log-Wachstumsphase geerntet. Anschließend werden die HeLa S3 Zellen in 96-well Platten (Costar) mit einer Dichte von 1000 Zellen pro well eingebracht und über Nacht in einem Inkubator (bei 37°C und 5% CO2) inkubiert, wobei auf jeder Platte 6 wells nur mit Medium gefüllt werden (3 wells zur Mediumkontrolle, 3 wells zur Inkubation mit reduzierten AlamarBlue Reagenz). Die Wirksubstanzen werden in verschiedenen Konzentrationen (gelöst in DMSO; DMSO-Endkonzentration: 0.1 %) zu den Zellen zugegeben (jeweils als Dreifachbestimmung). Nach 72 Stunden Inkubation werden zu jedem well 20 µl AlamarBlue Reagenz (AccuMed International) zugesetzt, und die Zellen für weitere 5-7 Stunden inkubiert. Zur Kontrolle wird zu 3 wells je 20 µl reduziertes AlamarBlue Reagenz gegeben (AlamarBlue Reagenz, das für 30 min autoklaviert wird). Nach Inkubation wird der Farbumsatz des AlamarBlue Reagenz in den einzelnen wells in einem Perkin Elmer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wird in Prozent der Kontrolle (HeLa S3 Zellen ohne Inhibitor) berechnet und die Wirkstoffkonzentration, die die Zellaktivität zu 50% hemmt (IC50) abgeleitet. Die Werte werden hierbei aus dem Mittelwert von drei Einzelbestimmungen - unter Korrektur des Leerwertes (Mediumkontrolle)- berechnet.

### FACS- Analyse

Propidium Iodid (PI) bindet stöchiometrisch an doppelsträngige DNA, und ist damit geeignet den Anteil an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N), während Zellen in der G2 oder Mitose einen 4N DNA Gehalt haben.
Für eine PI-Färbung werden beispielsweise 1x10⁶ HeLa S3 Zellen auf eine 75 cm2 Zellkulturflasche ausgesät, nach 24 h wird entweder 0.1 % DMSO als Kontrolle zugesetzt, bzw. die Substanz in verschiedenen Konzentrationen (in 0.1 % DMSO). Die Zellen werden für 24 h mit der Substanz bzw. mit DMSO inkubiert, bevor die Zellen 2 x mit PBS gewaschen und dann mit Trypsin /EDTA abgelöst werden. Die Zellen werden zentrifugiert (1000 Upm, 5 min, 4°C), und das Zellpellet 2 x mit PBS gewaschen, bevor die Zellen in 0.1 ml PBS resuspendiert werden. Anschließend werden die Zellen für 16 Stunden bei 4°C oder alternativ für 2 Stunden bei -20°C mit 80% Ethanol filiert. Die fixierten Zellen werden zentrifugiert (1000 Upm, 5min, 4°C), mit PBS gewaschen und anschließend nochmals zentrifugiert. Das Zellpellet wird in 2 ml 0.25% Triton X-100 in PBS resuspendiert, und 5 min auf Eis inkubiert, bevor 5 ml PBS zugeben werden und erneut zentrifugiert wird. Das Zellpellet wird in 350 µl PI Färbelösung (0.1 mg/ml RNase A (Sigma, No. R-4875), 10 µg/ml Prodium Iodid (Sigma, No. P-4864) in 1 x PBS) resuspendiert. Die Zellen werden für 20 min im Dunkeln mit dem Färbepuffer inkubiert, bevor sie in Probenmessgefäße für das FACS Scan überführt werden. Die DNA Messung erfolgte in einem Becton Dickinson FACS Analyzer, mit einem Argonlaser (500 mW, Emission 488 nm), und dem DNA Cell Quest Programm (BD). Die logarithmische PI Fluoreszenz wird mit einem band-pass Filter (BP 585/42) bestimmt. Die Quantifizierung der Zellpopulationen in den einzelnen Zellzyklusphasen erfolgte mit dem ModFit LT Programm von Becton Dickinson.

Entsprechend werden die erfindungsgemäßen Verbindungen auch auf weiteren Tumorzellen getestet. Beispielsweise sind diese Verbindungen auf Karzinomen verschiedenster Gewebe (z. Bsp. Brust (MCF7); Colon (HCT116), Kopf-Hals (FaDu), Lunge (NCI-H460), Pankreas (BxPC-3), Prostata (DU145)), Sarkome (z. Bsp. SK-UT-1B), Leukämien und Lymphome (z. Bsp. HL-60; Jurkat, THP-1) und anderen Tumoren (z. Bsp. Melanome (BRO), Gliome (U-87MG)) aktiv und könnten in solchen Indikationen eingesetzt werden. Dies belegt die breite Anwendbarkeit der erfindungsgemäßen Verbindungen zur Behandlung verschiedenster Tumortypen.
Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1-90 Gew.-%, bevorzugt 0,5 - 50 Gew.-% der Gesamtzusammensetzung liegen, d.h. in Mengen die ausreichend sind, um den unten angegebenen Dosierungsbereich zu erreichen. Die genannten Dosen können, falls erforderlich, mehrmals täglich gegeben werden.
Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoate, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilfslösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dikalziumphosphat zusammen mit verschiedenen Zusatzstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Dosierung für die intravenöse Anwendung liegt bei 1 - 1000 mg pro Stunde, vorzugsweise zwischen 5 - 500 mg pro Stunde.

Trotzdem kann es gegebenenfalls notwendig sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu wekhen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Formulierungsbeispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der fein gemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feucht granuliert und getrocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der fein gemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel **(1)**, 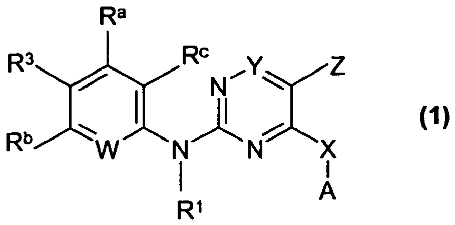 worin
**W** N oder C-R²,
**X** -NR^{1a}, O oder S,
**Y** CH oder N,
**Z** Halogen-C₁₋₃Alkyl-, -COH, -C(=O)-C₁₋₃Alkyl, -C(=O)-C₂₋₃Alkenyl, -C(=O)-C₂₋₃Alkinyl, -C(=O)C₁₋₃Alkyl-Halogen und Pseudohalogen;
**A** ausgewählt ist aus den Formeln (i), (ii) oder (iii) 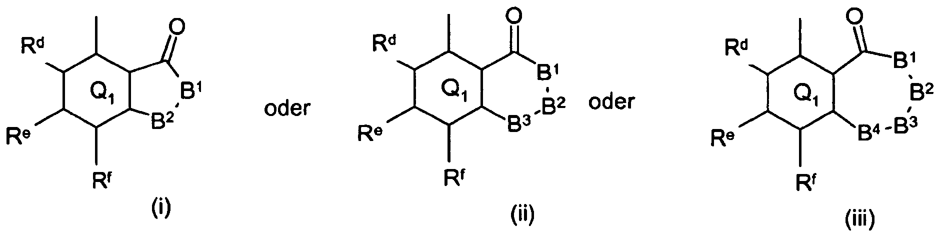
**Q₁** mono- oder bizyklische Arylverbindungen;
**B¹**, **B², B³** und **B⁴** jeweils unabhängig voneinander C-R^{g}R^{h}, N-Rⁱ, O oder S, wobei benachbarte **B¹** - **B⁴** nicht jeweils -O- bedeuten;
**R¹** und **R^{1a}** jeweils unabhängig voneinander Wasserstoff oder Methyl,
**R²** ein Rest ausgewählt aus der Gruppe bestehend aus Wasserstoff, Halogen, -OR⁴, -C(=O)R⁴, -C(=O)NR⁴R⁵, -NR⁴R⁵, -NR⁴C(=O)R⁵, -NR⁴SO₂R⁵, -N=CR⁴R⁵, -C=NRⁱ, -SR⁴, -SOR⁴, -SO₂R⁴, -SO₂NR⁴R⁵ und Pseudohalogen, oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen;
**R^{a}, R^{b}, R^{c}, R^{d}, R^{e}, R^{f}, R^{g}** und **R^{h}** jeweils unabhängig voneinander ein Rest ausgewählt aus der Gruppe bestehend aus Wasserstoff, Halogen, =O, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -C=NRⁱ, -SR⁴, -SOR⁴, -SO₂R⁴, -SO₂NR⁴R⁵, -NR⁴SO₂NR⁵R⁶, -OSO₂NR⁵R⁶ und Pseudohalogen; oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen; und gegebenenfalls können die am gleichen oder an benachbarten C-Atomen befindlichen **R^{g}** und **R^{h}** in jeder Kombination mit einer gemeinsamen gesättigten oder teilweise ungesättigten 3-5-gliedrigen Alkylbrücke, die gegebenfalls ein bis zwei Heteroatome enthalten kann, verbunden sein;
**R¹** ein Rest ausgewählt aus der Gruppe bestehend aus Wasserstoff, =O, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen; oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, R⁸,-NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR4C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen;
und gegebenenfalls können die an benachbarten N-Atomen befindlichen **R¹** miteinander oder **Rⁱ** mit an benachbarten C-Atomen befindlichen **R^{g}** oder **R^{h}** in jeder Kombination mit einer gemeinsamen gesättigten oder teilweise ungesättigten 3-5-gliedrigen Alkylbrücke, die gegebenenfalls ein bis zwei Heteroatome enthalten kann, verbunden sein;
**R³** ausgewählt aus den Formeln (iv) - (x), 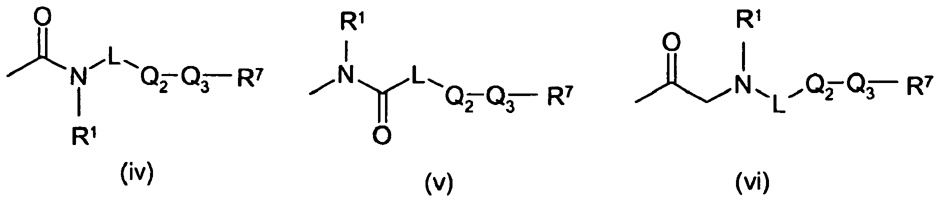 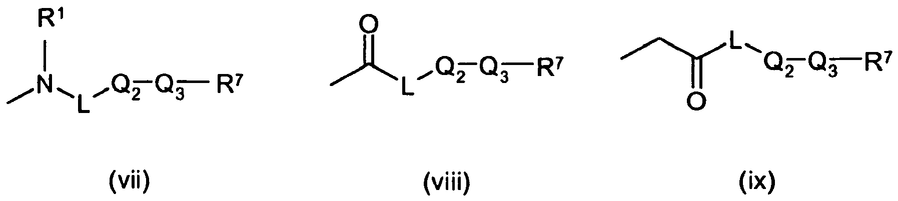
-L-Q₂-Q₃-R⁷ (x)
**R⁴, R⁵** und **R⁶** jeweils unabhängig voneinander Wasserstoff oder ein Rest ausgewählt aus der Gruppe bestehend aus gegebenenfalls einfach oder mehrfach substituiertem C₁₋₅Alkyl, C₂₋₅Alkenyl, C₂₋₅Alkinyl, C₃₋₁₀Cycloalkyl, Aryl, Heterocyclyl und Heteroaryl, wobei der/die Substituenten gleich oder verschieden sein können und ausgewählt werden aus der Gruppe bestehend aus C₃₋₁₀Cycloalkyl, Aryl, Heterocyclyl, Heteroaryl, Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
**L** eine Bindung oder ein Rest ausgewählt aus der Gruppe bestehend aus gegebenenfalls einfach oder mehrfach substituiertem C₁₋₁₆Alkyl, C₂₋₁₆Alkenyl, C₂₋₁₆Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
**Q₂** und **Q₃** jeweils unabhängig voneinander eine Bindung oder ein Rest ausgewählt aus der Gruppe bestehend aus gegebenenfalls einfach oder mehrfach substitutiertem C₁₋₁₆Alkyl, C₂₋₁₆Alkenyl, C₂₋₁₆Alkinyl, C₃₋₁₀Cycloalkyl, Aryl, Heterocyclyl und Heteroaryl wobei der/die Substituent(en) gleich oder verschieden sein können und ausgewählt werden aus der Gruppe bestehend aus Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
**R⁷** Wasserstoff oder ein Rest ausgewählt aus der Gruppe bestehend aus gegebenenfalls einfach oder mehrfach substituiertem C₁₋₁₆Alkyl, C₂₋₁₆Alkenyl, C₂₋₁₆Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen-, NO₂, -OR⁸, -C(=O)R⁸, -C(=O)OR⁸, -C(=O)NR⁸R⁹, -NR⁸R⁹, -NR⁸COR⁹, -NR⁸C(=O)OR⁹, -NR⁸C(=O)NR⁹R¹⁰, -NRC(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
**R⁸**, **R⁹** und **R¹⁰** jeweils unabhängig voneinander Wasserstoff oder ein Rest ausgewählt aus der Gruppe bestehend aus gegebenenfalls substituiertem C₁₋₈Alkyl, C₂₋₈Alkenyl, C₂₋₈Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen, Methyl, Ethyl, Amino, Methylamino, Dimethylamino, -OH und Pseudohalogen;
wobei
**Aryl** monozyklische oder bizyklische Ringe mit 6-12 Kohlenstoffatomen sind;
**Heteroaryl** monozyklische oder bizyklische Ringe sind, welche anstelle eines oder mehrerer Kohlenstoffatome ein oder mehrere, gleiche oder verschiedene Heteroatome enthalten;
**Heterocyclyl** 5-12 Kohlenstoffatome umfassende, gesättigte oder ungesättigte, nicht aromatische mono-, bizyklische, überbrückte oder spirozyklische bizyklische Ringe sind,
welche anstelle eines oder mehrerer Kohlenstoffatome Heteroatome wie Stickstoff, Sauerstoff oder Schwefel tragen;
bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

2. Verbindungen nach Anspruch 1 der allgemeinen Formel **(1)**, worin
**W** C-R² bedeutet und die übrigen Reste wie vorstehend erwähnt definiert sind.

3. Verbindungen nach Anspruch 1 oder 2, wobei
**X** -NR^{1a} oder Sauerstoff,
**R¹** und **R^{1a}** Wasserstoff;
**R³** Formel **(iv)** oder **(x)**, 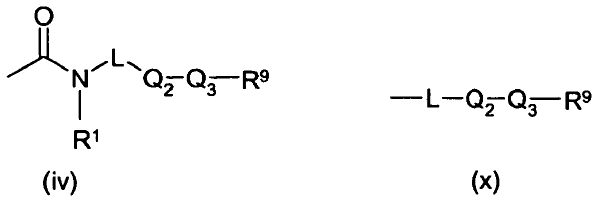
bedeuten und die übrigen Reste wie vorstehend erwähnt definiert sind.

4. Verbindungen nach Anspruch 1-3, wobei
**Y** CH und
**Q₁** monozyklische Arylverbindungen
bedeuten und die übrigen Reste wie vorstehend erwähnt definiert sind.

5. Verbindungen nach Anspruch 1-4, wobei
**R^{c}** ein Rest ausgewählt aus der Gruppe bestehend aus Wasserstoff, -F, -Cl, Methyl und Ethyl
bedeutet und die übrigen Reste wie vorstehend erwähnt definiert sind.

6. Verbindungen nach Anspruch 1-5, wobei
**R^{a}** und **R^{b}** jeweils unabhängig voneinander Wasserstoff oder Fluor;
oder ein gegebenenfalls einfach oder mehrfach substituierter Rest ausgewählt aus der Gruppe bestehend aus C₁₋₂Alkyl, C₂Alkenyl, C₂Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Wasserstoff, Halogen, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁵, -SO₂R⁴, -SO₂NR⁴R⁵, -NR⁴, -SO₂NR⁴R⁵, -OSO₂NR⁴R⁵ und Pseudohalogen
bedeuten und die übrigen Reste wie vorstehend erwähnt definiert sind.

7. Verbindungen nach Anspruch 1-6, wobei
**R^{a}** und **R^{b}** jeweils unabhängig voneinander Wasserstoff oder Fluor bedeuten und die
übrigen Reste wie vorstehend erwähnt definiert sind.

8. Verbindungen - oder deren pharmazeutisch wirksame Salze - nach Ansprüchen 1 - 7 als Arzneimittel.

9. Verbindungen - oder deren pharmazeutisch wirksame Salze - nach Ansprüchen 1-7 zur Herstellung eines Arzneimittels mit antiproliferativer Wirkung.

10. Verbindungen - oder deren pharmazeutisch wirksame Salze - nach Ansprüchen 1-7 zur Herstellung eines Arzneimittels mit antiproliferativer Wirkung mit einem selektiven, kinaseinhibierenden Wirkmechanismus.

11. Verbindungen - oder deren pharmazeutisch wirksame Salze - nach Ansprüchen 1-7 zur Herstellung eines Arzneimittels mit antiproliferativer Wirkung mit einem PLK inhibierenden Wirkmechanismus.

12. Pharmazeutische Zubereitungen, enthaltend als Wirkstoff eine oder mehrere Verbindungen der allgemeinen Formel **(1)** gemäß einem der Ansprüche 1-7 oder deren physiologisch verträgliche Salze gegebenenfalls in Kombination mit üblichen Hilfs- und/oder Trägerstoffen.

13. Verwendung einer Verbindung nach Anspruch 1 - 7 zur Herstellung eines Arzneimittels zur Behandlung und/oder Prävention von Krebs, Infektionen, Entzündungs- und Autoimmunerkrankungen.

14. Pharmazeutische Präparation umfassend eine Verbindung der allgemeinen Formel **(1)** 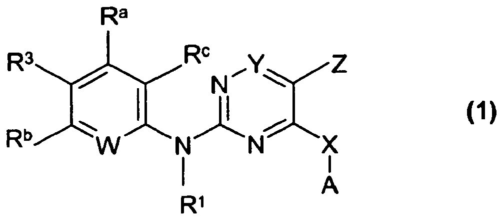 worin
**W** N oder C-R²,
**X** -NR^{1a}, O oder S,
**Y** CH oder N,
**Z** Halogen-C₁₋₃Alkyl-, -COH, -C(=O)-C₁₋₃Alkyl, -C(=O)-C₂₋₃Alkenyl, -C(=O)-C₂₋₃Alkinyl, -C(=O)C₁₋₃Alkyl-Halogen und Pseudohalogen;
**A** ausgewählt ist aus den Formeln **(i)**, **(ii)** oder (**iii**) 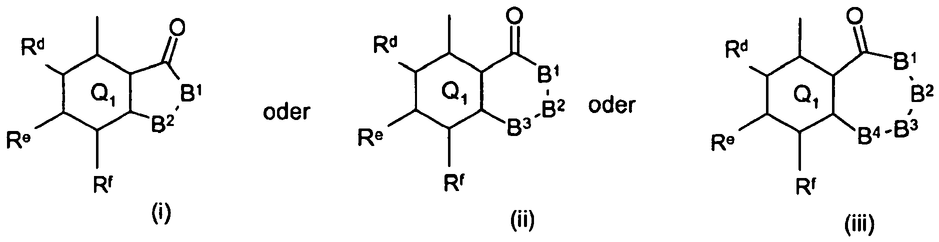
**Q₁** mono- oder bizyklische Arylverbindungen;
**B¹, B², B³** und **B⁴** jeweils unabhängig voneinander C-R^{g}R^{h}, N-Rⁱ, O oder S, wobei benachbarte **B¹ - B⁴ nicht** jeweils -O- bedeuten;
**R¹** und **R^{1a}** jeweils unabhängig voneinander Wasserstoff oder Methyl,
**R²** ein Rest ausgewählt aus der Gruppe bestehend aus Wasserstoff, Halogen, -OR⁴, -C(=O)R⁴, -C(=O)NR⁴R⁵, -NR⁴R⁵, -NR⁴C(=O)R⁵, -NR⁴SO₂R⁵, -N=CR⁴R⁵, -C=NRⁱ, -SR⁴, -SOR⁴, -SO₂R⁴, -SO₂NR⁴R⁵ und Pseudohalogen, oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen;
**R^{a}, R^{b}, R^{c}, R^{d}, R^{e}, R^{f}, R^{g}** und **R^{h}** jeweils unabhängig voneinander ein Rest ausgewählt aus der Gruppe bestehend aus Wasserstoff, Halogen, =O, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -C=NRⁱ, -SR⁴, -SOR⁴, -SO₂R⁴, -SO₂NR⁴R⁵, -NR⁴SO₂NR⁵R⁶, -OSO₂NR⁵R⁶ und Pseudohalogen;
oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, R, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen; und gegebenenfalls können die am gleichen oder an benachbarten C-Atomen befindliche **R^{g}** und **R^{b}** in jeder Kombination mit einer gemeinsamen gesättigten oder teilweise ungesättigten 3-5-gliedrigen Alkylbrücke, die ein bis zwei Heteroatome enthalten kann, verbunden sein;
**R¹** ein Rest ausgewählt aus der Gruppe bestehend aus Wasserstoff, =O, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ oder ein gegebenenfalls einfach oder mehrfach substituierter Rest ausgewählt aus der Gruppe bestehend aus C₁₋₆Alkyl, C₂₋₆Alkenyl, C₂₋₆Alkinyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR4C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ und Pseudohalogen; und gegebenenfalls können die an benachbarten N-Atomen befindlichen **R¹** miteinander oder **R¹** mit an benachbarten C-Atomen befindlichen **R^{g}** oder **R^{h}** in jeder Kombination mit einer gemeinsamen gesättigten oder teilweise ungesättigten 3-5-gliedrigen Alkylbrücke, die ein bis zwei Heteroatome enthalten kann, verbunden sein;
**R³** ausgewählt aus den Formeln **(iv) - (x)**, 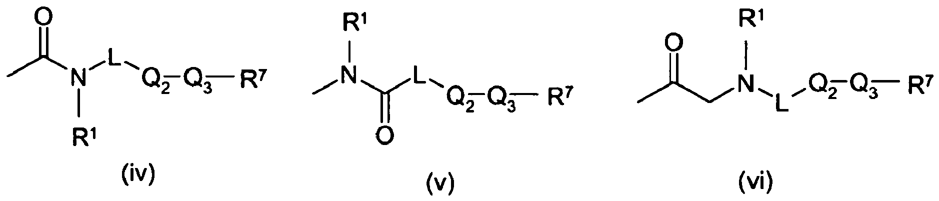 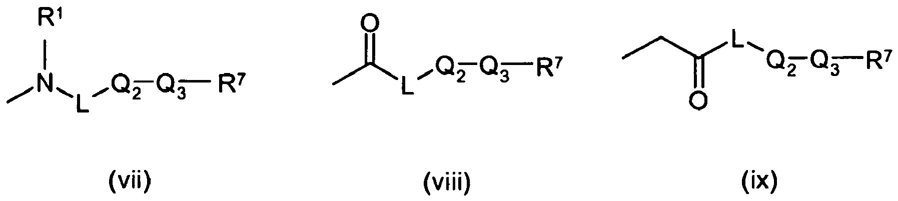
-L-Q₂-Q₃-R₇ (x)
**R⁴, R⁵** und **R⁶** jeweils unabhängig voneinander Wasserstoff oder ein Rest ausgewählt aus der Gruppe bestehend aus gegebenenfalls einfach oder mehrfach substituiertem C₁₋₅Alkyl, C₂₋₅Alkenyl, C₂₋₅Alkinyl, C₃₋₁₀Cycloalkyl, Aryl, Heterocyclyl und Heteroaryl, wobei der/die Substituenten gleich oder verschieden sein können und ausgewählt werden aus der Gruppe bestehend aus C₃₋₁₀Cycloalkyl, Aryl, Heterocyclyl, Heteroaryl, Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
**L** eine Bindung oder ein Rest ausgewählt aus der Gruppe bestehend aus gegebenenfalls einfach oder mehrfach substituiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
**Q₂** und **Q₃** jeweils unabhängig voneinander eine Bindung oder ein Rest ausgewählt aus der Gruppe bestehend aus gegebenenfalls einfach oder mehrfach substituiertem C₁₋₁₆-Alkyl, C₂₋₁₆-Alkenyl, C₂₋₁₆-Alkinyl, C₃₋₁₀-Cycloalkyl, Aryl, Heterocyclyl und Heteroaryl, wobei der/die Substituent(en) gleich oder verschieden sein können und ausgewählt werden aus der Gruppe bestehend aus Halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
**R⁷** Wasserstoff oder ein Rest ausgewählt aus der Gruppe bestehend aus gegebenenfalls einfach oder mehrfach substituiertem C₁₋₁₆Alkyl, C₂₋₁₆Alkenyl, C₂₋₁₆Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen-, NO₂, -OR⁸, -C(=O)R⁸, -C(=O)OR⁸, -C(=O)NR⁸R⁹, -NR⁸R⁹, -NR⁸COR⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ und Pseudohalogen;
**R⁸, R⁹** und **R¹⁰** jeweils unabhängig voneinander Wasserstoff oder ein Rest ausgewählt aus der Gruppe bestehend aus gegebenenfalls substituiertem C₁₋₈Alkyl, C₂₋₈Alkenyl, C₂₋₈Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen, -NH₂, -OH und Pseudohalogen;
wobei
**Aryl** monozyklische oder bizyklische Ringe mit 6-12 Kohlenstoffatomen sind;
**Heteroaryl** monozyklische oder bizyklische Ringe sind, welche anstelle eines oder mehrerer Kohlenstoffatome ein oder mehrere, gleiche oder verschiedene Heteroatome enthalten;
**Heterocyclyl** 5-12 Kohlenstoffatome umfassende, gesättigte oder ungesättigte, nicht aromatische mono-, bizyklische, überbrückte oder spirozyklische bizyklische Ringe sind,
welche anstelle eines oder mehrerer Kohlenstoffatome Heteroatome wie Stickstoff, Sauerstoff oder Schwefel tragen;
bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, und
mindestens eine weitere zytostatische oder zytotoxische Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

## Claims

1. Compounds of general formula (1), 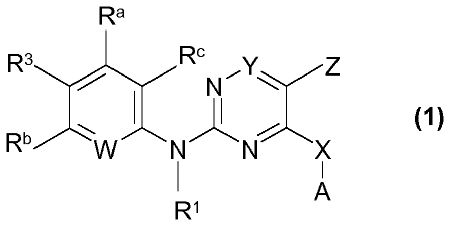 wherein
**W** denotes N or C-R²,
**X** denotes -NR^{1a}, O or S,
**Y** denotes CH or N,
**Z** denotes halogen-C₁₋₃alkyl, -COH, -C(=O)-C₁₋₃alkyl, -C(=O)-C₂₋₃alkenyl, -C(=O)-C₂₋₃alkynyl, -C(=O)C₁₋₃alkyl-halogen and pseudohalogen;
**A** is selected from the formulae (i), (ii) or (iii) 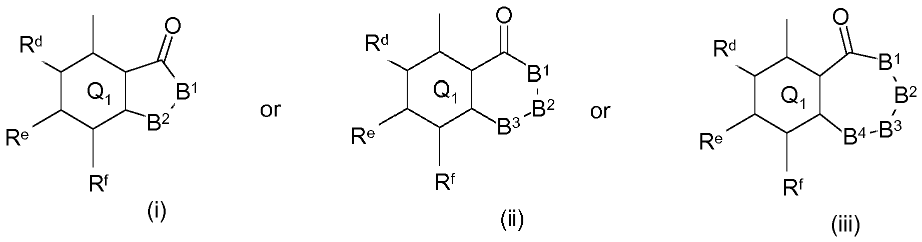
**Q₁** denotes mono- or bicyclic aryl compounds;
**B¹, B², B³** and **B⁴** each independently of one another denote C-R^{g}R^{h}, N-Rⁱ, O or S, while adjacent B¹ - B⁴ do not each denote -O-;
**R¹** and **R^{1a}** each independently of one another denote hydrogen or methyl,
**R²** denotes a group selected from among hydrogen, halogen, -OR⁴, -C(=O)R⁴, -C(=O)NR⁴R⁵, -NR⁴R⁵, -NR⁴C(=O)R⁵, -NR⁴SO₂R⁵, -N=CR⁴R⁵, -C=NRⁱ, -SR⁴, -SOR⁴, -SO₂R⁴, -SO₂NR⁴R⁵ and pseudohalogen, or an optionally mono- or polysubstituted group selected from among C₁₋₆alkyl, C₂₋₆alkenyl, C₂₋₆alkynyl, C₃₋₆-cycloalkyl, aryl, heterocyclyl and heteroaryl, while the substituent(s) may be identical or different and are selected from among halogen, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ and pseudohalogen;
**R^{a}, R^{b}, R^{c}, R^{d}, Re, R^{f}, R^{g}** and **R^{h}** each independently of one another denote a group selected from among hydrogen, halogen, =O, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -C=NRⁱ, -SR⁴, -SOR⁴, -SO₂R⁴, -SO₂NR⁴R⁵, -NR⁴SO₂NR⁵R⁶, -OSO₂NR⁵R⁶ and pseudohalogen; or an optionally mono- or polysubstituted group selected from among C₁₋₆-alkyl, C₂₋₆-alkenyl, C₂₋₆-alkynyl, C₃₋₆-cycloalkyl, aryl, heterocyclyl and heteroaryl, while the substituent(s) may be identical or different and are selected from among halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ and pseudohalogen;
and optionally the **R^{g}** and **R^{h}** located at the same or at adjacent C atoms may be attached in any combination to a common saturated or partially unsaturated 3-5-membered alkyl bridge which may optionally contain one to two heteroatoms;
**Rⁱ** denotes a group selected from among hydrogen, =O, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ and pseudohalogen; or an optionally mono- or polysubstituted group selected from among C₁₋₆alkyl, C₂₋₆alkenyl, C₂₋₆alkynyl, C₃₋₆cycloalkyl, aryl, heterocyclyl and heteroaryl, while the substituent(s) may be identical or different and are selected from among halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ and pseudohalogen; and optionally the **Rⁱ** groups located at adjacent N atoms may be joined together or **Rⁱ** with **R^{g}** or **R^{h}** located at adjacent C atoms may be attached in any combination to a common saturated or partially unsaturated 3-5-membered alkyl bridge which may optionally contain one to two heteroatoms;
**R³** is selected from the formulae (iv) - (x), 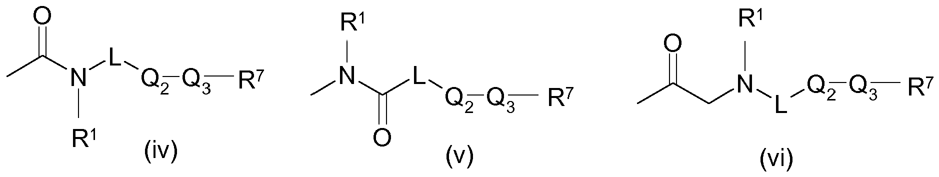 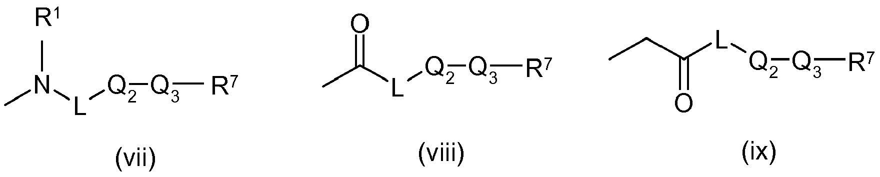
-L-Q₂-Q₃-R⁷ (x)
**R⁴, R⁵** and **R⁶** each independently of one another denote hydrogen or a group selected from among optionally mono- or polysubstituted C₁₋₅-alkyl, C₂₋₅alkenyl, C₂₋₅alkynyl, C₃₋₁₀cycloalkyl, aryl, heterocyclyl and heteroaryl, while the substituent(s) may be identical or different and are selected from among C₃₋₁₀-cycloalkyl, aryl, heterocyclyl, heteroaryl, halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ and pseudohalogen;
**L** denotes a bond or a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, while the substituent(s) may be identical or different and are selected from among halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ and pseudohalogen;
**Q₂** and **Q₃** independently of one another denote a bond or a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, while the substituent(s) may be identical or different and are selected from among halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ and pseudohalogen;
**R⁷** denotes hydrogen or a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, while the substituent(s) may be identical or different and are selected from among halogen, NO₂, -OR⁸, -C(=O)R⁸, -C(=O)OR⁸, -C(=O)NR⁸R⁹, -NR⁸R⁹, -NR⁸COR⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ and pseudohalogen;
**R⁸, R⁹** and **R¹⁰** each independently of one another denote hydrogen or a group selected from among optionally substituted C₁₋₈-alkyl, C₂₋₈-alkenyl, C₂₋₈-alkynyl, C₃₋₁₀-cycloalkyl, aryl, heterocyclyl and heteroaryl, while the substituent(s) may be identical or different and are selected from among halogen, methyl, ethyl, amino, methylamino, dimethylamino, -OH and pseudohalogen;
wherein
**aryl** are monocyclic or bicyclic rings with 6-12 carbon atoms;
**heteroaryl** are monocyclic or bicyclic rings which contain, instead of one or more carbon atoms, one or more identical or different heteroatoms;
**heterocyclyl** are saturated or unsaturated, non-aromatic mono-, bicyclic, bridged or spirocyclic bicyclic C₅₋₁₂ rings which comprise, instead of one or more carbon atoms, heteroatoms such as nitrogen, oxygen or sulphur; optionally in the form of the tautomers, racemates, enantiomers, diastereomers and mixtures thereof, and optionally the pharmacologically acceptable acid addition salts thereof.

2. Compounds according to claim 1 of general formula (1), wherein
**W** denotes C-R² and the other groups are as hereinbefore defined.

3. Compounds according to claim 1 or 2, wherein
**X** denotes -NR^{1a} or oxygen,
**R¹** and **R^{1a}** denote hydrogen;
**R³** denotes formula (iv) or (x), 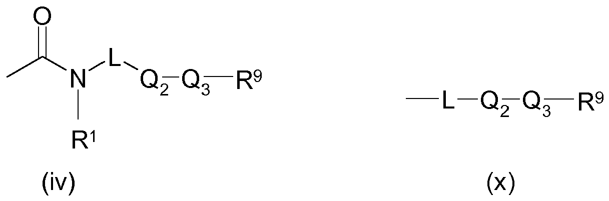
and the other groups are as hereinbefore defined.

4. Compounds according to claim 1 - 3, wherein
**Y** denotes CH and
**Q₁** denotes monocyclic aryl compounds
and the other groups are as hereinbefore defined.

5. Compounds according to claim 1 - 4, wherein
**R^{c}** denotes a group selected from among hydrogen, -F, -Cl, methyl and ethyl
and the other groups are as hereinbefore defined.

6. Compounds according to claim 1 - 5, wherein
**R^{a}** and **R^{b}** each independently of one another denote hydrogen or fluorine;
or an optionally mono- or polysubstituted group selected from among C₁₋₂alkyl, C₂alkenyl, C₂alkynyl, C₃₋₆cycloalkyl, aryl, heterocyclyl and heteroaryl, while the substituent(s) may be identical or different and are selected from among hydrogen, halogen, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁵, -SO₂R⁴, -SO₂NR⁴R⁵, -NR⁴, -SO₂NR⁴R⁵, -OSO₂NR⁴R⁵ and pseudohalogen
and the other groups are as hereinbefore defined.

7. Compounds according to claim 1 - 6, wherein
**R^{a} and R^{b}** each independently of one another denote hydrogen or fluorine
and the other groups are as hereinbefore defined.

8. Compounds - or the pharmaceutically active salts thereof - according to claims 1 - 7 as medicaments.

9. Compounds - or the pharmaceutically active salts thereof - according to claims 1 - 7 for preparing a medicament with an antiproliferative activity.

10. Compounds - or the pharmaceutically active salts thereof - according to claims 1 - 7 for preparing a medicament with an antiproliferative activity having a selective, kinase-inhibiting mechanism of activity.

11. Compounds - or the pharmaceutically active salts thereof - according to claims 1 - 7 for preparing a medicament with an antiproliferative activity having a PLK-inhibiting mechanism of activity.

12. Pharmaceutical preparations, containing as active substance one or more compounds of general formula **(1)** according to one of claims 1 - 7 or the physiologically acceptable salts thereof, optionally in conjunction with conventional excipients and/or carriers.

13. Use of a compound according to claims 1 to 7 for preparing a medicament for the treatment and/or prevention of cancer, infections, inflammatory and autoimmune diseases.

14. Pharmaceutical preparation comprising a compound of general formula **(1)** 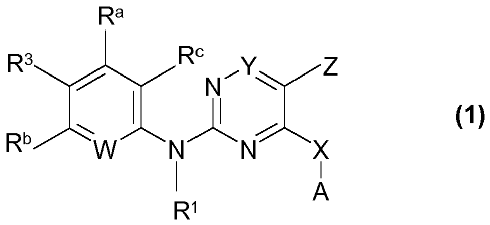 wherein
**W** denotes N or C-R²,
**X** denotes -NR^{1a}, O or S,
**Y** denotes CH or N,
**Z** denotes halogen-C₁₋₃alkyl, -COH, -C(=O)-C₁₋₃alkyl, -C(=O)-C₂₋₃alkenyl, -C(=O)-C₂₋₃alkynyl, -C(=O)C₁₋₃alkyl-halogen and pseudohalogen;
**A** is selected from the formulae (i), (ii) or (iii) 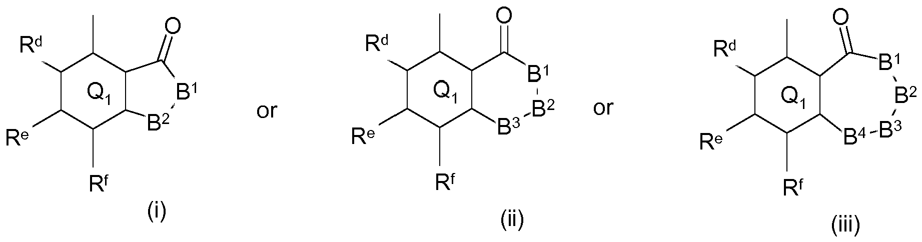
**Q₁** denotes mono- or bicyclic aryl compounds;
**B¹, B², B³** and **B⁴** each independently of one another denote C-R^{g}R^{h}, N-Rⁱ, O or S, while adjacent **B¹ - B⁴** do not each denote -O-;
**R¹** and **R^{1a}** each independently of one another denote hydrogen or methyl,
**R²** denotes a group selected from among hydrogen, halogen, -OR⁴, -C(=O)R⁴, -C(=O)NR⁴R⁵, -NR⁴R⁵, -NR⁴C(=O)R⁵, -NR⁴SO₂R⁵, -N=CR⁴R⁵, -C=NRⁱ, -SR⁴, -SOR⁴, -SO₂R⁴, -SO₂NR⁴R⁵ and pseudohalogen, or an optionally mono- or polysubstituted group selected from among C₁₋₆alkyl, C₂₋₆alkenyl, C₂₋₆alkynyl, C₃₋₆-cycloalkyl, aryl, heterocyclyl and heteroaryl, while the substituent(s) may be identical or different and are selected from among halogen, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ and pseudohalogen;
**R^{a}, R^{b}, R^{c}, R^{d}, Re, R^{f}, R^{g}** and **R^{h}** each independently of one another denote a group selected from among hydrogen, halogen, =O, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -C=NRⁱ, -SR⁴, -SOR⁴, -SO₂R⁴, -SO₂NR⁴R⁵, -NR⁴SO₂NR⁵R⁶, -OSO₂NR⁵R⁶ and pseudohalogen;
or an optionally mono- or polysubstituted group selected from among C₁₋₆-alkyl, C₂₋₆-alkenyl, C₂₋₆-alkynyl , C₃₋₆-cycloalkyl, aryl, heterocyclyl and heteroaryl, while the substituent(s) may be identical or different and are selected from among halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ and pseudohalogen;
and optionally the **R^{g}** and **R^{h}** located at the same or at adjacent C atoms may be attached in any combination to a common saturated or partially unsaturated 3-5-membered alkyl bridge which may contain one to two heteroatoms;
**Rⁱ** denotes a group selected from among hydrogen, =O, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ or an optionally mono- or polysubstituted group selected from among C₁₋₆alkyl, C₂₋₆alkenyl,C₂₋₆alkynyl, C₃₋₆cycloalkyl, aryl, heterocyclyl and heteroaryl, while the substituent(s) may be identical or different and are selected from among halogen, R⁸, -NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR4C(=O)NR⁵R⁶, -NR⁴SO₂R⁵, -N=CR⁵R⁵, -SR⁴, -SOR⁴, -SO₂R⁴, -SO₂NR⁴R⁵, -NR⁴SO₂NR⁵R⁶, -OSO₂NR⁵R⁵ and pseudohalogen; and optionally the **Rⁱ** groups located at adjacent N atoms may be joined together or **Rⁱ** with **R⁹** or **R^{h}** located at adjacent C atoms may be attached in any combination to a common saturated or partially unsaturated 3-5-membered alkyl bridge which may optionally contain one to two heteroatoms;
**R³** is selected from the formulae (iv) - (x), 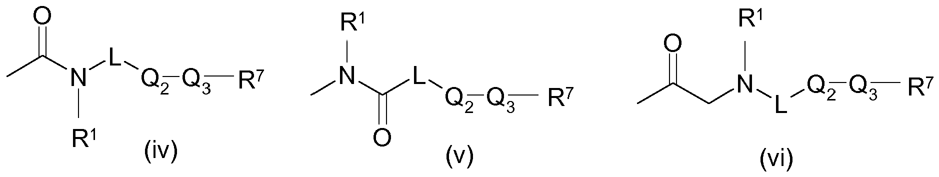 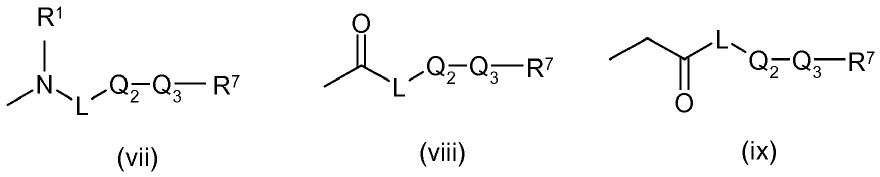
-L-Q₂-Q₃-R⁷ (x)
**R⁴, R⁵** and **R⁶** each independently of one another denote hydrogen or a group selected from among optionally mono- or polysubstituted C₁₋₅-alkyl, C₂₋₅alkenyl, C₂₋₅alkynyl, C₃₋₁₀cycloalkyl, aryl, heterocyclyl and heteroaryl, while the substituent(s) may be identical or different and are selected from among C₃₋₁₀-cycloalkyl, aryl, heterocyclyl, heteroaryl, halogen, -NO₂, -OR⁸, -C(=O)R⁸, -C(=O)OR⁸, -C(=O)NR⁸R⁹, -NR⁸R⁹, NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ and pseudohalogen;
**L** denotes a bond or a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, while the substituent(s) may be identical or different and are selected from among halogen, -NO₂, -OR⁸, -C(=O)R⁸, -C(=O)OR⁸, -C(=O)NR⁸R⁹, -NR⁸R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ and pseudohalogen;
**Q₂** and **Q₃** independently of one another denote a bond or a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, while the substituent(s) may be identical or different and are selected from among halogen, -NO₂, -OR⁸, -C(=O)R⁸, -C(=O)OR⁸, -C(=O)NR⁸R⁹, -NR⁸R⁹, -NR⁸C(=O)R⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹ -NR⁸SO₂NR⁹R¹⁰, -OSO₂NR⁸R⁹ and pseudohalogen;
**R⁷** denotes hydrogen or a group selected from among optionally mono- or polysubstituted C₁₋₁₆-alkyl, C₂₋₁₆-alkenyl, C₂₋₁₆-alkynyl, C₃₋₁₀-cycloalkyl, aryl, heterocyclyl and heteroaryl, while the substituent(s) may be identical or different and are selected from among halogen, NO₂, -OR⁸, -C(=O)R⁸, -C(=O)OR⁸, -C(=O)NR⁸R⁹, -NR⁸R⁹, -NR⁸COR⁹, -NR⁸C(=O)OR⁹, -NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, -OSO₂NR⁸R⁹ and pseudohalogen;
**R⁸, R⁹** and **R¹⁰** each independently of one another denote hydrogen or a group selected from among optionally substituted C₁₋₈-alkyl, C₂₋₈-alkenyl, C₂₋₈-alkynyl, C₃₋₁₀-cycloalkyl, aryl, heterocyclyl and heteroaryl, while the substituent(s) may be identical or different and are selected from among halogen, NH₂, -OH and pseudohalogen;
wherein **aryl** are monocyclic or bicyclic rings with 6-12 carbon atoms;
**heteroaryl** are monocyclic or bicyclic rings which contain, instead of one or more carbon atoms, one or more identical or different heteroatoms;
**heterocyclyl** are saturated or unsaturated, non-aromatic mono-, bicyclic, bridged or spirocyclic bicyclic C₅₋₁₂ rings which comprise, instead of one or more carbon atoms, heteroatoms such as nitrogen, oxygen or sulphur;
optionally in the form of the tautomers, racemates, enantiomers, diastereomers and mixtures thereof, and optionally the pharmacologically acceptable acid addition salts thereof, and
at least one other cytostatic or cytotoxic active substance, optionally in the form of the tautomers, racemates, enantiomers, diastereomers and mixtures thereof, and optionally the pharmacologically acceptable acid addition salts thereof.

## Revendications

1. Composés de formule générale (1), 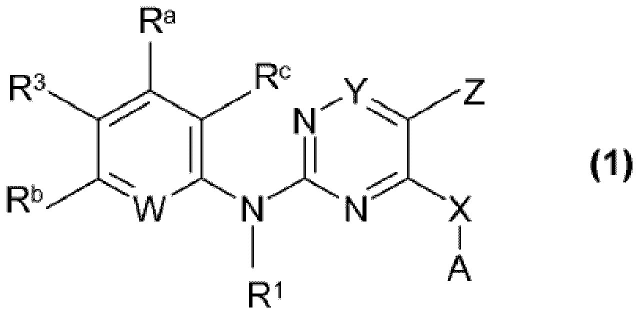 dans laquelle
W représente N ou C-R²,
X représente -NR^{1a}, 0 ou S,
Y représente CH ou N,
Z représente un groupe halogéno-alkyle en C₁ à C₃, - COH, -C(=O)-alkyle en C₁ à C₃, -C(=O)-alcényle en C₂ à C₃, -C(=O)alcinyle en C₂ à C₃, -C(=O)alkyle en C₁ à C₃-halogéno et un pseudohalogène ;
A est choisi parmi les formules (i), (ii) ou (iii) 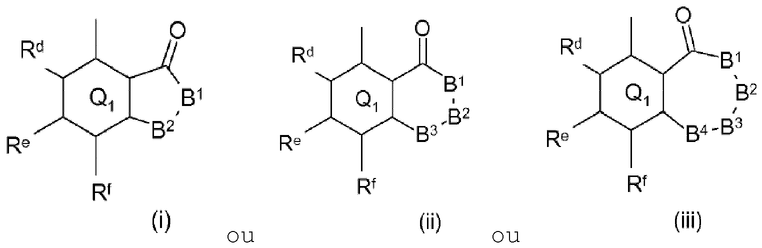
Q₁ représente des composés aryle monocycliques ou bicycliques ;
B¹, B², B³ et B⁴ représentent respectivement, indépendamment les uns des autres, C-R^{g}R^{h}, N-Rⁱ, 0 ou S, où B¹ à B⁴ voisins ne représentent pas respectivement - O- ;
R¹ et R^{1a} représentent respectivement, indépendamment l'un de l'autre, un atome d'hydrogène ou un groupe méthyle,
R² représente un radical choisi dans le groupe comprenant un atome d'hydrogène, d'halogène, -OR⁴, -C(=O)R⁴, -C(=O)NR⁴R⁵, -NR⁴R⁵, -NR⁴C(=O)R⁵, -NR⁴SO₂R⁵, -N=CR⁴R⁵, -C=NRⁱ, -SR⁴, -SOR⁴, -SO₂R⁴, -SO₂NR⁴R⁵ et un pseudohalogène, ou un radical éventuellement monosubstitué ou multisubstitué choisi dans le groupe comprenant un groupe alkyle en C₁ à C₆, alcényle en C₂ à C₆, alcinyle en C₂ à C₆, cycloalkyle en C₃ à C₆, aryle, hétérocyclyle et hétéroaryle, où le/les substituants peuvent être identiques ou différents et sont choisis dans le groupe comprenant un atome d'halogène, un groupe - NO₂, -OR⁴, -C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ et un pseudohalogène ;
R^{a}, R^{b}, R^{c}, R^{d}, R^{e}, R^{f}, R^{g} et R^{h} représentent respectivement, indépendamment les uns des autres, un radical choisi dans le groupe comprenant un atome d'hydrogène, d'halogène, =0, -NO₂, -OR⁴, - C(=O)R⁴, -C(=O)OR⁴, -C(=O)NR⁴R⁵, -NR⁴R⁵, -NR⁴C(=O)R⁵, -NR⁴C (=O)OR⁵, -NR⁴C(=O)NR⁵R⁶, -NR⁴SO₂R⁵, -N=CR⁴R⁵, - C=NRⁱ, -SR⁴, -SOR⁴, -SO₂R⁴, -SO₂NR⁴R⁵, -NR⁴SO₂NR⁵R⁶, - OSO₂NR⁵R⁶ et un pseudohalogène ;
ou un radical éventuellement monosubstitué ou multisubstitué choisi dans le groupe comprenant un groupe alkyle en C₁ à C₆, alcényle en C₂ à C₆, alcinyle en C₂ à C₆, cycloalkyle en C₃ à C₆, aryle, hétérocyclyle et hétéroaryle, où le/les substituants peuvent être identiques ou différents et sont choisis dans le groupe comprenant un atome d'halogène, R⁸, -NO₂, -OR⁴, -C(=O)R⁴, -C(=O)OR⁴, - C(=O)NR⁴R⁵, -NR⁴R⁵, -NR⁴C(=O)R⁵, -NR⁴C(=O)OR⁵, - NR⁴C(=O)NR⁵R⁶, -NR⁴SO₂R⁵, -N=CR⁴R⁵, -SR⁴, -SOR⁴, - SO₂R⁴, -SO₂NR⁴R⁵, -NR⁴SO₂NR⁵R⁶, -OSO₂NR⁵R⁶ et un pseudohalogène ;
et éventuellement les R^{g} et R^{h} qui se trouvent sur les mêmes atomes de C ou des atomes de C voisins peuvent être liés dans chaque combinaison avec un pont alkyle commun saturé ou partiellement insaturé de 3 à 5 chaînons, qui peut éventuellement contenir un à deux hétéroatomes ;
Rⁱ représente un radical choisi dans le groupe comprenant un atome d'hydrogène, =O, -OR⁴, - C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, -NR⁴SO₂R⁵, -N=CR⁴R⁵, - SR⁴, -SOR⁴, -SO₂R⁴, -SO₂NR⁴R⁵, -NR⁴SO₂NR⁵R⁶, - OSO₂NR⁵R⁶ et un pseudohalogène ; ou un radical éventuellement monosubstitué ou multisubstitué choisi dans le groupe comprenant un groupe alkyle en C₁ à C₆, alcényle en C₂ à C₆, alcinyle en C₂ à C₆, cycloalkyle en C₃ à C₆, aryle, hétérocyclyle et hétéroaryle, où le/les substituants peuvent être identiques ou différents et sont choisis dans le groupe comprenant un atome d'halogène, R⁸, -NO₂, - OR⁴, -C(=O)R⁴, -C(=O)OR⁴, -C(=O)NR⁴R⁵, -NR⁴R⁵, - NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ et un pseudohalogène ;
et éventuellement, les R¹ qui se trouvent conjointement sur des atomes de N voisins ou les Rⁱ avec R^{g} ou R^{h} se trouvant sur des atomes de C voisins peuvent être liés dans chaque combinaison à un pont alkyle commun de 3 à 5 chaînons, saturé ou partiellement insaturé, qui peut éventuellement contenir un à deux hétéroatomes ;
R³ est choisi parmi les formules (iv) à (x), 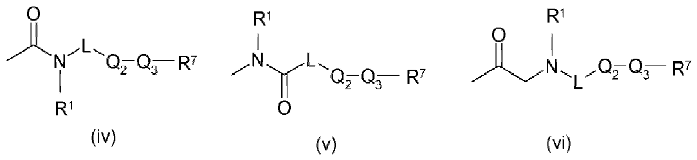 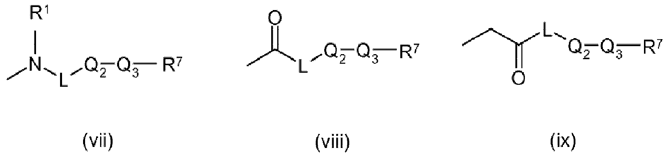
-L-Q₂-Q₃-R⁷ (x)
R⁴, R⁵ et R⁶ représentent respectivement, indépendamment les uns des autres, un atome d'hydrogène ou un radical choisi dans le groupe comprenant les groupes éventuellement monosubstitués ou multisubstitués alkyle en C₁ à C₅, alcényle en C₂ à C₅, alcinyle en C₂ à C₅, cycloalkyle en C₃ à C₁₀, aryle, hétérocyclyle et hétéroaryle, où le/les substituants peuvent être identiques ou différents et sont choisis dans le groupe comprenant un groupe cycloalkyle en C₃ à C₁₀, aryle, hétérocyclyle, hétéroaryle, halogéno, -NO₂, -OR⁸, -C(=O)R⁸, -C(=O)OR⁸, -C(=O)NR⁸R⁹, -NR⁸R⁹, - NR⁸C(=O)R⁹, -NR⁸C (=0) OR⁹, -NR⁸C(=O) NR⁹R¹⁰, - NR⁸C (=0) ONR⁹R¹⁰, -NR⁸SO₂R⁹, -N=CR⁸R⁹, -SR⁸, -SOR⁸, - SO₂R⁸, -SO₂NR⁸R⁹ -NR⁸SO₂NR⁹R¹⁰, -OSO₂NR⁸R⁹ et un pseudohalogène ;
L représente une liaison ou un radical choisi dans le groupe comprenant les groupes éventuellement monosubstitués ou multisubstitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, où le/les substituants peuvent être identiques ou différents et sont choisis dans le groupe comprenant un atome d' halogène, -NO₂, -OR⁸, -C(=O)R⁸, -C(=O)OR⁸, -C(=O)NR⁸R⁹, -NR⁸R⁹, - NR⁸C(=O)R⁹, -NR⁸C(=O)OR⁹, -NR⁸C(=O)NR⁹R¹⁰, - NR⁸C(=O)ONR⁹R¹⁰, -NR⁸SO₂R⁹, -N=CR⁸R⁹, -SR⁸, -SOR⁸, - SO₂R⁸, -SO₂NR⁸R⁹, -NR⁸SO₂NR⁹R¹⁰, -OSO₂NR⁸R⁹ et un pseudohalogène ;
Q₂ et Q₃ représentent respectivement, indépendamment l' un de l' autre, une liaison ou un radical choisi dans le groupe comprenant les groupes éventuellement monosubstitués ou multisubstitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, où le/les substituants peuvent être identiques ou différents et sont choisis dans le groupe comprenant un atome d' halogène, -NO₂, -OR⁸, -C(=O)R⁸, -C(=O)OR⁸, - C(=O)NR⁸R⁹, -NR⁸R⁹, NR⁸C(=O)R⁹, -NR⁸C(=O)OR⁹, - NR⁸C (=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, - OSO₂NR⁸R⁹ et un pseudohalogène ;
R⁷ représente un atome d'hydrogène ou un radical choisi dans le groupe comprenant les groupes éventuellement monosubstitués ou multisubstitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, où le/les substituants peuvent être identiques ou différents et sont choisis dans le groupe comprenant un atome d' halogène, -NO₂, -OR⁸, -C(=O)R⁸, -C(=O)OR⁸, - C(=O)NR⁸R⁹, -NR⁸R⁹, -NR⁸COR⁹, -NR¹⁰C(=O)OR⁹, - NR⁸C(=O)NR⁹R¹⁰, -NR⁸C(=O)ONR⁹R¹⁰, -NR⁸SO₂R⁹, -N=CR⁸R⁹, -SR⁸, -SOR⁸, -SO₂R⁸, -SO₂NR⁸R⁹, -NR⁸SO₂NR⁹R¹⁰, - OSO₂NR⁸R⁹ et un pseudohalogène ;
R⁸, R⁹ et R¹⁰ représentent respectivement, indépendamment les uns des autres, un atome d'hydrogène ou un radical choisi dans le groupe comprenant les groupes éventuellement substitués alkyle en C₁ à C₈, alcényle en C₂ à C₈, alcinyle en C₂ à C₈, cycloalkyle en C₃ à C₁₀, aryle, hétérocyclyle et hétéroaryle, où le/les substituant(s) peuvent être identiques ou différents et sont choisis dans le groupe comprenant un atome d'halogène, un groupe méthyle, éthyle, amino, méthylamino, diméthylamino, -OH et un pseudohalogène ;
dans laquelle
les groupes aryle sont des cycles monocycliques ou bicycliques comportant 6 à 12 atomes de carbone ;
les groupes hétéroaryle sont des cycles monocycliques ou bicycliques qui contiennent, à la place d'un ou
plusieurs atomes de carbone, un ou plusieurs hétéroatomes identiques ou différents ;
les groupes hétérocyclyle sont des cycles monocycliques, bicycliques, pontés ou spirocycliques comprenant 5 à 12 atomes de carbone, saturés ou insaturés, non aromatiques qui portent à la place d'un ou plusieurs atomes de carbone, des hétéroatomes tels que l'azote, l'oxygène ou le soufre ;
éventuellement sous la forme de leurs tautomères, leurs racémates, leurs énantiomères, leurs diastéréomères et leurs mélanges, et éventuellement leurs sels d'addition acides pharmacologiquement inoffensifs.

2. Composés selon la revendication 1 de formule générale (1), dans laquelle
W représente C-R² et les radicaux restants sont tels que définis précédemment.

3. Composés selon la revendication 1 ou 2, dans lesquels
X représente -NR^{1a} ou un atome d'oxygène,
R¹ et R^{1a} représentent un atome d' hydrogène ;
R³ représente la formule (iv) ou (x), 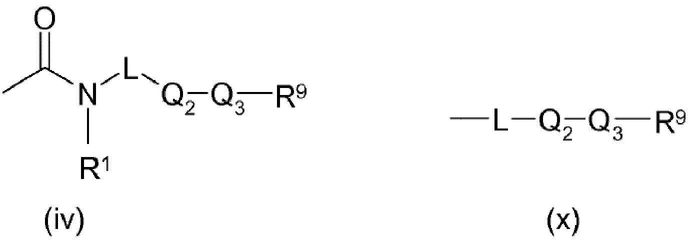
et les radicaux restants sont tels que définis précédemment.

4. Composés selon les revendications 1 à 3, dans lesquels
Y représente CH et
Q₁ représente des composés aryle monocycliques
et les radicaux restants sont tels que définis précédemment.

5. Composés selon les revendications 1 à 4, dans lesquels
R^{C} représente un radical choisi dans le groupe comprenant un atome d'hydrogène, -F, -Cl, un groupe méthyle et éthyle,
et les radicaux restants sont tels que définis précédemment.

6. Composés selon les revendications 1 à 5, dans lesquels
R^{a} et R^{b} représentent respectivement, indépendamment l'un de l'autre, un atome d'hydrogène ou de fluor ;
ou un radical éventuellement monosubstitué ou multisubstitué choisi dans le groupe comprenant un groupe alkyle en C₁ à C₂, alcényle en C₂, alcinyle en C₂, cycloalkyle en C₃ à C₆, aryle, hétérocyclyle et hétéroaryle, où le/les substituants peuvent être identiques ou différents et sont choisis dans le groupe comprenant un atome d'hydrogène, d' halogène, -NO₂, -OR⁴, -C (=O)R⁴, -C(=O)OR⁴, -C(=O)NR⁴R⁵, -NR⁴R⁵, -NR⁴C(=O)R⁵, - NR⁴C(=O)OR⁵, -NR⁴C(=O)NR⁵R⁶, - -NR⁴SO₂R⁵, -N=CR⁴R⁵, -SR⁴, -SOR⁵, -SO₂R⁴, -SO₂NR⁴R⁵, -NR⁴, -SO₂NR⁴R⁵, -OSO₂NR⁴R⁵ et un pseudohalogène,
et les radicaux restants sont tels que définis précédemment.

7. Composés selon les revendications 1 à 6, dans lesquels
R^{a} et R^{b} représentent respectivement, indépendamment l'un de l'autre, un atome d'hydrogène ou de fluor et les radicaux restants sont tels que définis précédemment.

8. Composés - ou leurs sels pharmaceutiquement efficaces - selon les revendications 1 à 7, comme médicaments.

9. Composés - ou leurs sels pharmaceutiquement efficaces - selon les revendications 1 à 7, pour la production d'un médicament ayant un effet antiprolifératif.

10. Composés - ou leurs sels pharmaceutiquement efficaces - selon les revendications 1 à 7, pour la production d'un médicament présentant un effet antiprolifératif ayant un mécanisme d'action sélectif, inhibiteur de kinase.

11. Composés - ou leurs sels pharmaceutiquement efficaces - selon les revendications 1 à 7, pour la production d'un médicament présentant un effet antiprolifératif ayant un mécanisme d'action inhibiteur de la PLK.

12. Compositions pharmaceutiques contenant, comme substance active, un ou plusieurs composés de formule générale (1) selon l'une des revendications 1 à 7 ou leurs sels physiologiquement acceptables, éventuellement en combinaison avec des adjuvants et/ou véhicules habituels.

13. Utilisation d'un composé selon les revendications 1 à 7, pour la production d'un médicament pour le traitement et/ou la prévention du cancer, d'infections, d'inflammations et de maladies auto-immunes.

14. Préparation pharmaceutique comprenant un composé de formule générale (1) 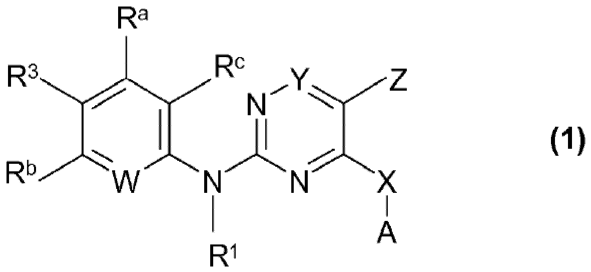 dans laquelle
W représente N ou C-R²,
X représente -NR^{1a}, O ou S,
Y représente CH ou N,
Z représente un groupe halogéno-alkyle en C₁ à C₃, - COH, -C(=O)-alkyle en C₁ à C₃, -C(=O)-alcényle en C₂ à C₃, -C(=O)alcinyle en C₂ à C₃, -C(=O)alkyle en C₁ à C₃-halogéno et un pseudohalogène ;
A est choisi parmi les formules (i), (ii) ou (iii) 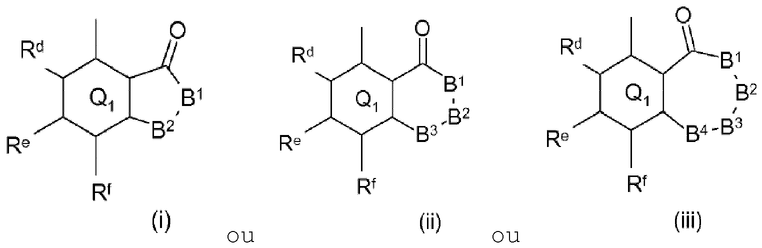
Q₁ représente des composés aryle monocycliques ou bicycliques ;
B¹, B², B³ et B⁴ représentent respectivement, indépendamment les uns des autres, C-R^{g}R^{h}, N-Rⁱ, 0 ou S, où B¹ à B⁴ voisins ne représentent pas respectivement - O- ;
R¹ et R^{1a} représentent respectivement, indépendamment l'un de l'autre, un atome d'hydrogène ou un groupe méthyle,
R² représente un radical choisi dans le groupe comprenant un atome d'hydrogène, d'halogène, -OR⁴, -C(=O)R⁴, -C(=O)NR⁴R⁵, -NR⁴R⁵, -NR⁴C(=O)R⁵, -NR⁴SO₂R⁵, -N=CR⁴R⁵, -C=NR2ⁱ, -SR⁴, -SOR⁴, -SO₂R⁴, -SO₂NR⁴R⁵ et un pseudohalogène, ou un radical éventuellement monosubstitué ou multisubstitué choisi dans le groupe comprenant un groupe alkyle en C₁ à C₆, alcényle en C₂ à C₆, alcinyle en C₂ à C₆, cycloalkyle en C₃ à C₆, aryle, hétérocyclyle et hétéroaryle, où le/les substituants peuvent être identiques ou différents et sont choisis dans le groupe
comprenant un atome d'halogène, un groupe -NO₂, - OR^{4,} -C(=O)R⁴, -C(=O)OR⁴, -C(=O)NR⁴R⁵, -NR⁴R⁵, - NR⁴C(=O)R⁵, -NR⁴C(=O)OR⁵, -NR⁴C (=O) NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ et un pseudohalogène ;
R^{a}, R^{b}, R^{c}, R^{d}, R^{e}, R^{f}, R^{g} et R^{h} représentent respectivement, indépendamment les uns des autres, un radical choisi dans le groupe comprenant un atome d'hydrogène, d'halogène, =O, -NO₂, -OR⁴, - C(=O)R⁴, -C(=O)OR⁴, -C(=O)NR⁴R⁵, -NR⁴R⁵, -NR⁴C(=O)R⁵, -NR⁴C(=O)OR⁵, -NR⁴C(=O)NR⁵R⁶, NR⁴SO₂R⁵, -N=CR⁴R⁵, - C=NR¹, -SR⁴, -SOR⁴, -SO₂R⁴, -SO₂NR⁴R⁵, -NR⁴SO₂NR⁵R⁶, - OSO₂NR⁵R⁶ et un pseudohalogène ; ou un radical éventuellement monosubstitué ou multisubstitué choisi dans le groupe comprenant un groupe alkyle en C₁ à C₆, alcényle en C₂ à C₆, alcinyle en C₂ à C₆, cycloalkyle en C₃ à C₆, aryle, hétérocyclyle et hétéroaryle, où le/les substituants peuvent être identiques ou différents et sont choisis dans le groupe comprenant un atome d'halogène, R, -NO₂, -OR⁴, -C(=O)R⁴, -C(=O)OR⁴, - C(=O)NR⁴R⁵, -NR⁴R⁵, -NR⁴C(=O)R⁵, -NR⁴C(=O)OR⁵, - NR⁴C(=O)NR⁵R⁶, -NR⁴SO₂R⁵, -N=CR⁴R⁵, -SR⁴, -SOR⁴, - SO₂R⁴, -SO₂NR⁴R⁵, -NR⁴SO₂NR⁵R⁶, -OSO₂NR⁵R⁶ et un pseudohalogène ; et éventuellement les R^{g} et R^{h} qui se trouvent sur les mêmes atomes de C ou des atomes de C voisins peuvent être liés dans chaque combinaison avec un pont alkyle commun saturé ou partiellement insaturé de 3 à 5 chaînons, qui peut éventuellement contenir un à deux hétéroatomes ;
R¹ représente un radical choisi dans le groupe comprenant un atome d'hydrogène, =O, -OR⁴, - C(=O)R⁴, -C(=O)OR⁴, -C(=O)NR⁴R⁵, -NR⁴R, -NR⁴C(=O)R⁵, -NR⁴C(=O)OR⁵, -NR⁴C(=O) NR⁵R⁶, -NR⁴SO₂R⁵, -N=CR⁴R⁵, - SR⁴, -SOR⁴, -SO₂R⁴, -SO₂NR⁴R⁵, -NR⁴SO₂NR⁵R⁶, - OSO₂NR⁵R⁶ ou un radical éventuellement monosubstitué ou multisubstitué choisi dans le groupe comprenant un groupe alkyle en C₁ à C₆, alcényle en C₂ à C₆, alcinyle en C₂ à C₆, cycloalkyle en C₃ à C₆, aryle, hétérocyclyle et hétéroaryle, où le/les substituants peuvent être identiques ou différents et sont choisis dans le groupe comprenant un atome d'halogène, R⁸, -NO₂, - OR⁴, -C(=O)R⁴, -C(=O)OR⁴, -C(=O)NR⁴R⁵, -NR⁴R⁵, - NR⁴C(=O)R⁵, -NR⁴C(=O)OR⁵, -NR⁴C(=O)NR⁵R⁶, -NR⁴SO₂R⁵, -N=CR⁴R⁵, -SR⁴, -SOR⁴, -SO₂R⁴, -SO₂NR⁴R⁵, -NR⁴SO₂NR⁵R⁶, -OSO₂NR⁵R⁶ et un pseudohalogène ; et éventuellement, les Rⁱ qui se trouvent conjointement sur des atomes de N voisins ou les Rⁱ avec R^{g} ou R^{h} se trouvant sur des atomes de C voisins peuvent être liés dans chaque combinaison à un pont alkyle commun de 3 à 5 chaînons, saturé ou partiellement insaturé, qui peut éventuellement contenir un à deux hétéroatomes ;
R³ est choisi parmi les formules (iv) à (x), 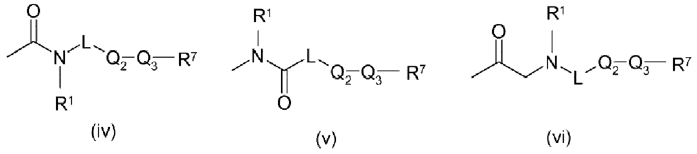 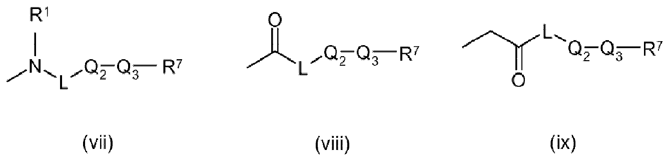
-L-Q₂-Q₃-R⁷ (x)
R⁴, R⁵ et R⁶ représentent respectivement, indépendamment les uns des autres, un atome d'hydrogène ou un radical choisi dans le groupe comprenant les groupes éventuellement monosubstitués ou multisubstitués alkyle en C₁ à C₅, alcényle en C₂ à C₅, alcinyle en C₂ à C₅, cycloalkyle en C₃ à C₁₀, aryle, hétérocyclyle et hétéroaryle, où le/les substituants peuvent être identiques ou différents et sont choisis dans le groupe comprenant un groupe cycloalkyle en C₃ à C₁₀, aryle, hétérocyclyle, hétéroaryle, halogéno, -NO₂, -OR⁸, -C(=O)R⁸, -C(=O)OR⁸, -C(=O)NR⁸R⁹, -NR⁸R⁹, - NR⁸C(=O)R⁹, -NR⁸C(=O)OR⁹, -NR⁸C(=O) NR⁹R¹⁰, - NR⁸C(=O)ONR⁹R¹⁰-NR⁸SO₂R⁹, -N=CR⁸R⁹, -SR⁸, -SOR⁸, - SO₂R⁸, -SO₂NR⁸R⁹, -NR⁸SO₂NR⁹R¹⁰, -OSO₂NR⁸R⁹ et un pseudohalogène ;
L représente une liaison ou un radical choisi dans le groupe comprenant les groupes éventuellement monosubstitués ou multisubstitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, où le/les substituant(s) peuvent être identiques ou différents et sont choisis dans le groupe comprenant un atome d' halogène, -NO₂, -OR⁸, -C(=O)R⁸, -C(=O)OR⁸, -C(=O)NR⁸R⁹, -NR⁸R⁹, - NR⁸C(=O)R⁹, -NR⁸C(=O)OR⁹, -NR⁸C(=O)NR⁹R¹⁰, - NR⁸C(=O)ONR⁹R¹⁰, -NR⁸SO₂R⁹, -N=CR⁸R⁹, -SR⁸, -SOR⁸, - SO₂R⁸, -SO₂NR⁸R⁹ -NR⁸SO₂NR⁹R¹⁰, -OSO₂NR⁸R⁹ et un pseudohalogène ;
Q₂ et Q₃ représentent respectivement, indépendamment l' un de l' autre, une liaison ou un radical choisi dans le groupe comprenant les groupes éventuellement monosubstitués ou multisubstitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, où le/les substituant(s) peuvent être identiques ou différents et sont choisis dans le groupe comprenant un atome d' halogène, -NO₂, -OR⁸, - C(=O)R⁸, -C(=O)OR⁸, -C(=O)NR⁸R⁹, -NR⁸R⁹, NR⁸C(=O)R⁹, -NR⁸C(=O)OR⁹, -NR⁸C(=O)NR⁹R¹⁰, -NR⁸C(=O)ONR⁹R¹⁰, - NR⁸SO₂R⁹, -N=CR⁸R⁹, -SR⁸, -SOR⁸, -SO₂R⁸, -SO₂NR⁸R⁹, - NR⁸SO₂NR⁹R¹⁰, -OSO₂NR⁸R⁹ et un pseudohalogène ;
R⁷ représente un atome d'hydrogène ou un radical choisi dans le groupe comprenant les groupes éventuellement monosubstitués ou multisubstitués alkyle en C₁ à C₁₆, alcényle en C₂ à C₁₆, alcinyle en C₂ à C₁₆, cycloalkyle en C₃ à C₁₀, aryle, hétérocyclyle et hétéroaryle, où le/les substituant(s) peuvent être identiques ou différents et sont choisis dans le groupe comprenant un atome d'halogène, -NO₂, -OR⁸, - C(=O)R⁸, -C(=O)OR⁸, -C(=O)NR⁸R⁹, -NR⁸R⁹, -NR⁸COR⁹, - NR⁸C(=O)OR⁹, -NR⁸C(=O)NR⁹R¹⁰, -NR⁸C(=O)ONR⁹R¹⁰, - NR⁸SO₂R⁹, -N=CR⁸R⁹, -SR⁸, -SOR⁸, -SO₂R⁸, -SO₂NR⁸R⁹, - NR⁸SO₂NR⁹R¹⁰, -OSO₂NR⁸R⁹ et un pseudohalogène ;
R⁸, R⁹ et R¹⁰ représentent respectivement, indépendamment les uns des autres, un atome d'hydrogène ou un radical choisi dans le groupe comprenant les groupes éventuellement substitués alkyle en C₁ à C₈, alcényle en C₂ à C₈, alcinyle en C₂ à C₈, cycloalkyle en C₃ à C₁₀, aryle, hétérocyclyle et hétéroaryle, où le/les substituant(s) peuvent être identiques ou différents et sont choisis dans le groupe comprenant un atome d'halogène, -NH₂, -OH et un pseudohalogène ;
dans laquelle
les groupes aryle sont des cycles monocycliques ou bicycliques comportant 6 à 12 atomes de carbone ;
les groupes hétéroaryle sont des cycles monocycliques ou bicycliques qui contiennent, à la place d'un ou
plusieurs atomes de carbone, un ou plusieurs hétéroatomes identiques ou différents ;
les groupes hétérocyclyle sont des cycles monocycliques, bicycliques, pontés ou spirocycliques comprenant 5 à 12 atomes de carbone, saturés ou insaturés, non aromatiques qui portent, à la place d'un ou plusieurs atomes de carbone, des hétéroatomes tels que l'azote, l'oxygène ou le soufre ;
éventuellement sous la forme de leurs tautomères, leurs racémates, leurs énantiomères, leurs diastéréomères et leurs mélanges, et éventuellement leurs sels d'addition acides pharmacologiquement inoffensifs, et
au moins une autre substance active cytostatique ou cytotoxique, éventuellement sous la forme de leurs tautomères, leurs racémates, leurs énantiomères, leurs diastéréomères et leurs mélanges, et éventuellement leurs sels d'addition acides pharmacologiquement inoffensifs.
